(19) Europäisches Patentamt
European Patent Office
Office européen des brevets

(11) EP 4 122 499 A1

(12) EUROPEAN PATENT APPLICATION

(43) Date of publication:
**25.01.2023 Bulletin 2023/04**

(21) Application number: **21187424.3**

(22) Date of filing: **23.07.2021**

(51) International Patent Classification (IPC):
***A61K 51/04*** *(2006.01)*

(52) Cooperative Patent Classification (CPC):
**A61K 51/0459; A61K 51/0497; C07D 413/14; C07D 417/14**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **3B Pharmaceuticals GmbH**
**12489 Berlin (DE)**

(72) Inventor: **The designation of the inventor has not yet been filed**

(74) Representative: **Bohmann, Armin K.**
**Bohmann**
**Anwaltssozietät**
**Nymphenburger Straße 1**
**80335 München (DE)**

Remarks:
The complete document including Reference Table(s) and the Sequence Listing(s) can be downloaded from the EPO website

# (54) FIBROBLAST ACTIVATION PROTEIN INHIBITORS AND USE THEREOF

(57) The present disclosure relates to fibroblast activation protein (FAP) inhibitors and in particular to a compound of Formula (I)

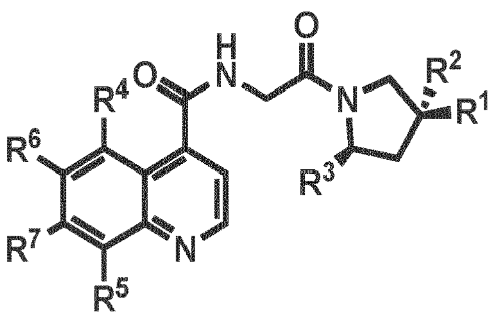

I.

EP 4 122 499 A1

## Description

### FIELD OF INVENTION

The present invention is related to a chemical compound; an inhibitor of fibroblast activation protein (FAP); a composition comprising the compound or inhibitor, respectively; the compound, the inhibitor or the composition, respectively, for use in a method for the diagnosis of a disease; the compound, the inhibitor or the composition, respectively, for use in a method for the treatment of a disease; the compound, the inhibitor or the composition, respectively, for use in a method of diagnosis and treatment of a disease which is also referred to as "thera(g)nosis" or "thera(g)nostics"; the compound, the inhibitor or the composition, respectively, for use in a method for delivering an effector to a FAP-expressing tissue; the compound, the inhibitor or the composition, respectively, for use in a method for the stratification of a group of subjects into subjects which are likely to respond to a treatment of a disease, and into subjects which are not likely to respond to a treatment of a disease; the compound, the inhibitor or the composition, respectively, for use in a method for the identification of a subject, wherein the subject is likely to respond or likely not to respond to a treatment of a disease; the compound, the inhibitor or the composition, respectively, for use in a method for the selection of a subject from a group of subjects, wherein the subject is likely to respond or likely not to respond to a treatment of a disease; a method for the diagnosis of a disease using the compound, the inhibitor or the composition, respectively; a method for the treatment of a disease using the compound, the inhibitor or the composition, respectively; a method for the diagnosis and treatment of a disease which is also referred to as "thera(g)nosis" or "thera(g)nostics, using the compound, the inhibitor or the composition, respectively; a method for the delivery of an effector to a FAP-expressing tissue using the compound, the inhibitor or the composition, respectively; a method for the stratification of a group of subjects into subjects which are likely to respond to a treatment of a disease, and into subjects which are not likely to respond to a treatment of a disease; a method for the identification of a subject, wherein the subject is likely to respond or likely not to respond to a treatment of a disease; and a method for the selection of a subject from a group of subjects, wherein the subject is likely to respond or likely not to respond to a treatment of a disease.

### BACKGROUND

Despite the increasing availability of therapeutic options, cancer is still the second leading cause of death globally. Therapeutic strategies mainly focus on targeting malignant cancer cells itself, ignoring the ever-present surrounding tumor microenvironment (TME) that limit the access of therapeutic cancer cell agents. The TME is part of the tumor mass and consists not only of the heterogeneous population of cancer cells but also of a variety of resident and infiltrating host cells, secreted factors, and extracellular matrix proteins. A dominant cell type found in the TME is the cancer associated fibroblast (CAF). CAFs are often the dominant cell type within a solid tumor mass, and have attracted increasing attention as a player in tumor progression and homeostasis.

Fibroblast activation protein (FAP) has gained notoriety as a marker of CAFs. Due to the omnipresence of CAFs and stroma within tumors, FAP was discovered as a suitable marker for radiopharmaceutical diagnostics and as a suitable target for radiopharmaceutical therapy

Fibroblast activation protein $\alpha$ (FAP) is a type II transmembrane serine protease and a member of the S9 prolyl oligopeptidase family. FAP possesses dual enzyme activity, possessing both post-proline exopeptidase activity (like other DPP enzymes such as DPP4, DPP7, DPP8, DPP9) and endopeptidase activity (similar to prolyl oligopeptidase/endopeptidase (POP/PREP)). Its dipeptidyl peptidase activity allows cleaving two amino acids of the N-terminus after a proline residue. FAP is primarily found to be localized on the cell surface, however a soluble form of the protein has also been described.

FAP expression in the tumor stroma of 90% of epithelial carcinomas was first reported in 1990 under use of a monoclonal antibody, F19. FAP expression on malignant epithelial cells has also been reported. FAP expression in CAFs has been shown for almost all carcinomas and sarcomas. Furthermore, CAFs are present in hematological malignancies. Utilization of FAP as a therapeutic target is therefore not limited to certain tumor entities.

The abundance of FAP-expressing CAFs is described to correlate with poor prognosis. Across a wide range of human tumor indications, FAP expression is described to correlate with higher tumor grade and worse overall survival. FAP as well as FAP-expressing cells present in the tumor microenvironment significantly influence tumor progression. Additionally, due to its relatively selective expression in tumors, FAP is regarded as a suitable target for therapeutic and diagnostic agents.

FAP becomes highly upregulated in stromal cells at sites of active tissue remodeling, including wound healing, fibrosis, arthritis, and atherosclerosis, in addition to cancer. As such, FAP is involved in diseases other than oncology indications, such as those mentioned here. Fibroblastlike synoviocytes in rheumatoid arthritic joints of patients show a significantly increased expression of FAP. Additionally, FAP is recognized as a marker of activated fibroblasts in the injury response, but also as an important player in the healing process of wounds. In fibrotic diseases, upregulated

expression of FAP was also observed e.g. in idiopathic pulmonary fibrosis, Crohn's disease, and liver fibrosis. Additionally, FAP is expressed in arteriosclerotic lesions and upregulated in activated vascular smooth muscle cells.

Soon after its discovery, FAP was utilized as a therapeutic target in cancer. Various FAP targeted strategies have been explored, including e.g. inhibition of FAP enzymatic activity, ablation of FAP-positive cells, or targeted delivery of cytotoxic compounds. However, there remains a significant need for improved diagnostic agents and pharmaceutical agents for the diagnosis and/or treatment of cancer and other diseases and conditions mediated by FAP.

## SUMMARY OF THE INVENTION

One aspect of the present disclosure pertains to a compound of Formula (I)

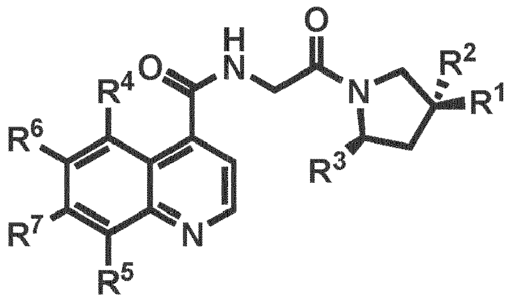

I,

wherein:

$R_1$ is H or F,
$R_2$ is H or F,
$R_3$ is selected from the group consisting of -C(O)-Het1, $-B(OH)_2$, -CN, and $-C(O)-CH_2-OH$,
wherein -C(O)-Het1 is selected from the group consisting of:

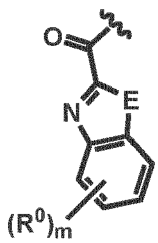 , 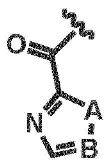, and 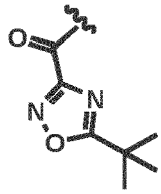 ,

wherein:

A is selected from the group consisting of O, S, and NH;
B is CH or, if A is NH, B is CH or N;
E is O or S;
$R^0$ is independently selected from the group consisting of $(C_1-C_4)$alkyl, $-O-(C_1-C_4)$alkyl, - $COO-(C_1-C_4)$alkyl, F, Cl, Br, I, OH, COOH, and CN; and m is selected from the group consisting of 0, 1, and 2;

$R^4$ is selected from the group consisting of H, Cl, Br, F, and $(C_1-C_2)$alkyl;
one of $R^5$, $R^6$, and $R^7$ is $R^8$-L-, and the other two of $R^5$, $R^6$, and $R^7$ are each independently selected from the group consisting of H, $(C_1-C_4)$alkyl, $-O-(C_1-C_4)$alkyl, $-O-(C_1-C_3)$alkylidene-$(C_6)$aryl, F, Cl, Br, and $O-CF_3$; and
$R^8$-L- is $R^8$-Lin4-Lin3-Lin2-Lin1-,
wherein:

Lin1 is selected from the group consisting of

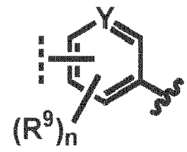, 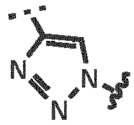,

-O-, N($CH_3$)-, and ($C_1$-$C_4$)alkylidene-C(O)NH, with a proviso that when $R^3$ is CN, then Lin1 is

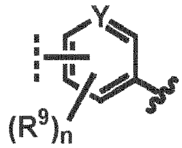 or 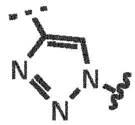,

wherein in Lin1:

Y is CH or N;

indicates attachment to Lin2, and when Lin1 is

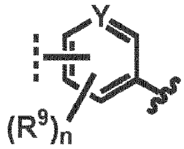

, Lin2 is in *meta*- or *para*-position to and indicates attachment to the quinoline;
$R^9$ is selected from the group consisting of halogen, $CO_2H$, ($C_1$-$C_6$)alkyl, hydroxy substituted ($C_1$-$C_6$)alkyl, and -O-($C_1$-$C_6$)alkyl; and
n is selected from the group consisting of 0, 1, and 2, preferably n is 0 or 1, more preferably n is 0;

Lin2 is selected from the group consisting of -C(O)-NH-, -NH-C(O)-, and -$S(O)_2$-when Lin1 is selected from the group consisting of

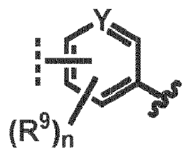, 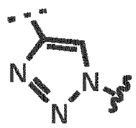,

and ($C_1$-$C_4$)alkylidene-C(O)NH, and Lin2 is absent when Lin1 is -O- or -N($CH_3$)-;
Lin3 is ($C_2$-$C_4$)alkylidene;
Lin4 is selected from the group consisting of -$NR^{10}$,

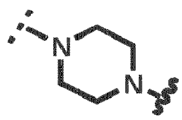,

and

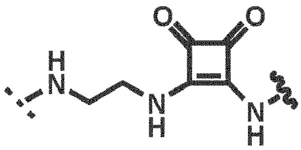

,

wherein in Lin4:

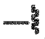 indicates attachment to Lin3,

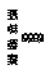 1-: indicates the attachment to $R_8$;

$R^{10}$ is selected from the group consisting of H, $CH_2$-COOH, and ($C_1$-$C_4$)alkyl;; and

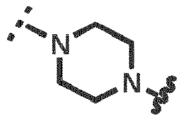

is optionally oxidized to

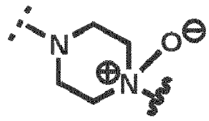

;

$R^8$ is a chelator or a cytotoxic agent; and wherein

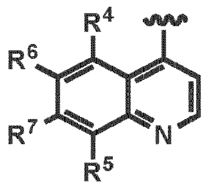

in the formula (I) may optionally be oxidized to its N-oxide

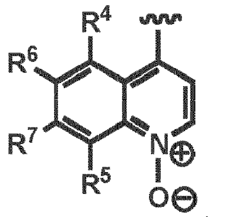

.

Another aspect of the present disclosure pertains to a compound of Formula I, wherein the compound comprises a diagnostically active nuclide or a therapeutically active nuclide.

Another aspect of the present disclosure pertains to a method for the treatment of a disease in a subject, wherein the method comprises administering to the subject a therapeutically effective amount of a compound according to the disclosure. For example, such compounds may be compounds of Formula I.

**BRIEF DESCRIPTION OF THE DRAWINGS**

The following detailed description, given by way of example, but not intended to limit the invention, is further illustrated by reference to the following figures from which further features, embodiments and advantages, may be taken.

Figures 1(a)-1(p) show the percentage of injected dose per gram of tissue (%ID/g) uptake in the kidneys, liver,

bloodpool and HEK-FAP tumor as determined by SPECT-imaging of select compounds post injection into the Swiss nude mice model:

Fig. 1(a): shows the %ID/g uptake of $^{111}$In-3BP-3467, atlh, 3h, 6h, and 24h post injection.

Fig. 1(b): shows the %ID/g uptake of $^{111}$In-3BP-3581, at 1h, 3h, 6h, and 24h post injection.

Fig. 1(c): shows the %ID/g uptake of $^{111}$In-3BP-3621, at 1h, 3h, 6h, and 24h post injection.

Fig. 1(d): shows the %ID/g uptake of $^{111}$In-3BP-3631, at 1h, 3h, 6h, and 24h post injection.

Fig. 1(e) shows the %ID/g uptake of $^{111}$In-3BP-3622, at 1h, 3h, 6h, and 24h post injection.

Fig. 1(f) shows the %ID/g uptake of $^{111}$In-3BP-3772, at 1h, 3h, 6h, and 24h post injection.

Fig. 1(g) shows the %ID/g uptake of $^{111}$In-3BP-3785, at 1h, 3h, 6h, and 24h post injection.

Fig. 1(h) shows the %ID/g uptake of $^{111}$In-3BP-4076, at 1h, 3h, 6h, and 24h post injection.

Fig. 1(i) shows the %ID/g uptake of $^{111}$In-3BP-4808, at 1h, 4h, 24h, and 48h post injection.

Fig. 1(j) shows the %ID/g uptake of $^{111}$In-3BP-4809, at 1h, 4h, 24h, and 48h post injection.

Fig. 1(k) shows the %ID/g uptake of $^{111}$In-3BP-4810, at 1h, 4h, 24h, and 48h post injection.

Fig. 1(l) shows the %ID/g uptake of $^{111}$In-3BP-4811, at 1h, 4h, 24h, and 48h post injection.

Fig. 1(m) shows the %ID/g uptake of $^{111}$In-3BP-4663, at 1h, 4h, 24h, 48h, and 72h post inj ection.

Fig. 1(n) shows the %ID/g uptake of $^{111}$In-3BP-4664, at 1h, 4h, 24h, 48h, and 72h post inj ection.

Fig. 1(o) shows the %ID/g uptake of $^{111}$In-3BP-4665, at 1h, 4h, 24h, 48h, and 72h post inj ection.

Fig. 1(p) shows the %ID/g uptake of reference compound $^{111}$In-3BP-4200 at 1h, 4h, 24h, 48h, and 72h post injection.

Figures 2(a)-2(f) show the percentage of injected dose per gram of tissue (%ID/g) uptake in the kidneys, liver, blood pool, HEK-FAP and/or CHO-FAP tumor as determined by SPECT-imaging of select compounds post injection into the SCID beige mouse model:

Fig. 2(a) shows the %ID/g uptake of $^{111}$In-3BP-4663 at 1h, 4h, 24h, and 48h post injection.

Fig. 2(b) shows the %ID/g uptake of $^{111}$In-3BP-4664 at 1h, 4h, 24h, and 48h post injection.

Fig. 2(c) shows the %ID/g uptake of $^{111}$In-3BP-4665 at 1h, 4h, 24h, and 48h post injection.

Fig. 2(d) shows the %ID/g uptake of $^{111}$In-3BP-4694 at 1h, 4h, 24h, and 48h post injection.

Fig. 2(e) shows the %ID/g uptake of reference compound $^{111}$In-3BP-2929 at 1h, 4h, 24h, and 48h post injection.

Fig. 2(f) shows the %ID/g uptake of $^{111}$In-3BP-4201 at 1h, 4h, 24h, and 48h post injection.

Figures 3(a)-3(d) show SPECT/CT-images of select compounds post injection into a Swiss nude mouse with HEK-FAP tumors:

Fig. 3(a) shows SPECT/CT-images of $^{111}$In-3BP-4809 at 1h, 4h, 24h, and 48h post injection.

Fig. 3(b) shows SPECT/CT-images of $^{111}$In-3BP-4810 at 1h, 4h, 24h, and 48h post injection.

Fig. 3(c) shows SPECT/CT-images of $^{111}$In-3BP-4663 1h, 4h, 24h, 48h, and 72h post injection.

Fig. 3(d) shows SPECT/CT-images of $^{111}$In-3BP-4664 at 1h, 4h, 24h, 48h, and 72h post inj ection.

Figures 4(a)-4(f) show SPECT/CT-images of select compounds post injection into a SCID beige mouse with HEK-FAP (right shoulder) and CHO-FAP (left shoulder) tumors:

Fig. 4(a) shows SPECT/CT-images of $^{111}$In-3BP-4663 at 1h, 4h, 24h, and 48h post injection.

Fig. 4(b) shows SPECT/CT-images of $^{111}$In-3BP-4664 at 1h, 4h, 24h, and 48h post injection.

Fig. 4(c) shows SPECT/CT-images of $^{111}$In-3BP-4665 at 1h, 4h, 24h, and 48h post injection.

Fig. 4(d) shows SPECT/CT-images of $^{111}$In-3BP-4694 at 1h, 4h, 24h and 48h post injection.

Fig. 4(e) shows SPECT/CT-images of reference compound $^{111}$In-3BP-2929 at 1h, 4h, 24h, and 48h post injection.

Fig. 4(f) shows SPECT/CT-images of $^{111}$In-3BP-4201 at 1h, 4h, 24h, and 48h post injection.

## DETAILED DESCRIPTION OF THE INVENTION

The present disclosure relates to novel compounds suitable for use as diagnostic agents and/or pharmaceutical agents, for the diagnosis and/or treatment of cancer and other diseases and conditions mediated by FAP. The present disclosure provides novel compounds, capable of interacting with FAP that can deliver an effector, which can provide for the detection, treatment, and/or management of various diseases associated with one or more FAP-expressing tumors or cells, including cancer.

Specifically, provided herein are compounds that are suitable as a diagnostic agent and/or pharmaceutical agent, particularly if conjugated to a diagnostically and/or therapeutically active effector. Furthermore, compounds provided herein are suitable as a diagnostic agent and/or a pharmaceutical agent, particularly if conjugated to a diagnostically and/or therapeutically active effector, whereby the compound is a potent inhibitor of FAP activity, preferably so that the pIC50 of the compound is equal to or greater than 6.0. Furthermore, compounds provided herein are suitable as diagnostic agents and/or pharmaceutical agents, particularly if conjugated to a diagnostically and/or therapeutically active effector, in the diagnosis and/or therapy of a disease where the diseased cells and/or diseased tissues express FAP. Furthermore, provided herein are compounds suitable for delivering a diagnostically and/or therapeutically effective agent to a diseased cell and/or diseased tissue, respectively, and more particularly a FAP-expressing diseased cell and/or diseased tissue, preferably the diseased tissue comprises or contains cancer associated fibroblasts.

Also, provided herein is a method for the diagnosis of a disease, a method for the treatment and/or prevention of a disease, and a method for the combined diagnosis and treatment of a disease. Preferably such disease is a disease involving FAP-expressing cells and/or tissues, more particularly a FAP-expressing diseased cell and/or diseased tissue, preferably the diseased tissue comprises or contains cancer associated fibroblasts. Furthermore, provided herein is a method for the identification of a subject, wherein the subject is likely to respond or likely not to respond to a treatment of a disease, a method for the selection of a subject from a group of subjects, wherein the subject is likely to respond or likely not to respond to a treatment of a disease.

In addition, provided herein is a pharmaceutical composition containing a compound having the characteristics as outlined above. Furthermore, provided herein is a kit which is suitable for use in any of the above methods.

These and other problems underlying the present invention are solved by, for example, the following embodiments, and/or as set forth in the appended claims.

Embodiment 1. A compound of formula (I):

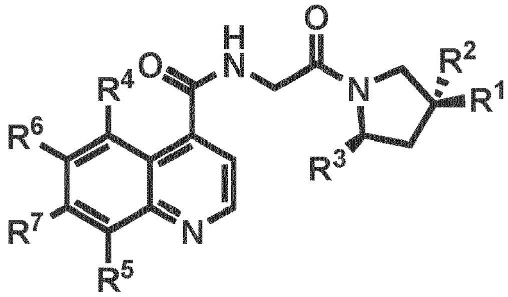

I,

wherein:

$R_1$ is H or F,
$R_2$ is H or F,
$R_3$ is selected from the group consisting of -C(O)-Het1, $-B(OH)_2$, -CN, and $-C(O)-CH_2-OH$,
wherein -C(O)-Het1 is selected from the group consisting of:

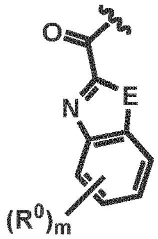
, 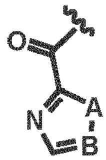
, and 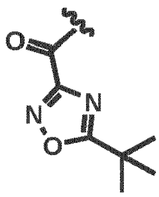
,

wherein:

A is selected from the group consisting of O, S, and NH;
B is CH or, if A is NH, B is CH or N;
E is O or S;
$R^0$ is independently selected from the group consisting of $(C_1-C_4)$alkyl, $-O-(C_1-C_4)$alkyl, - $COO-(C_1-C_4)$alkyl, F, Cl, Br, I, OH, COOH, and CN; and m is selected from the group consisting of 0, 1, and 2;

$R^4$ is selected from the group consisting of H, Cl, Br, F, and $(C_1-C_2)$alkyl;
one of $R^5$, $R^6$, and $R^7$ is $R^8$-L-, and the other two of $R^5$, $R^6$, and $R^7$ are each independently selected from the group consisting of H, $(C_1-C_4)$alkyl, $-O-(C_1-C_4)$alkyl, $-O-(C_1-C_3)$alkylidene-$(C_6)$aryl, F, Cl, Br, and $O-CF_3$; and $R^8$-L- is $R^8$-Lin4-Lin3-Lin2-Lin1-,
wherein:

Lin1 is selected from the group consisting of

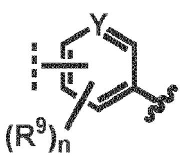
, 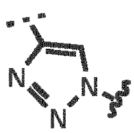
,

-O-, $N(CH_3)$-, and 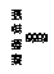 $(C_1-C_4)$alkylidene-C(O)NH- 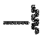, with a proviso that when $R^3$ is CN, then Lin1 is

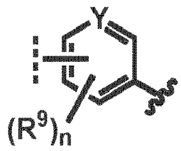 or 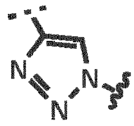,

wherein in Lin1:

Y is CH or N; 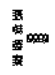 indicates attachment to Lin2, and when Lin1 is

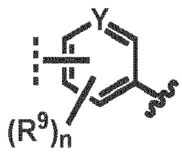,

Lin2 is in *meta*- or *para*-position to 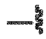 and

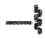 indicates attachment to the quinoline;
$R^9$ is selected from the group consisting of halogen, $CO_2H$, $(C_1-C_6)$alkyl, hydroxy substituted $(C_1-C_6)$alkyl, and -O-$(C_1-C_6)$alkyl; and
n is selected from the group consisting of 0, 1, and 2, preferably n is 0 or 1, more preferably n is 0;

Lin2 is selected from the group consisting of -C(O)-NH-, -NH-C(O)-, and $-S(O)_2$-when Lin1 is selected from the group consisting of

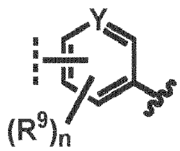, 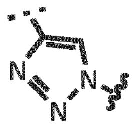,

and 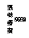 $(C_1-C_4)$alkylidene-C(O)NH-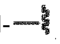, and Lin2 is absent when Lin1 is -O- or $-N(CH_3)-$;
Lin3 is $(C_2-C_4)$alkylidene;
Lin4 is selected from the group consisting of $-NR^{10}$,

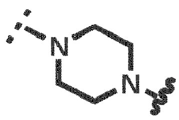,

and

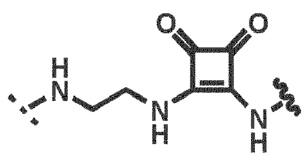,

wherein in Lin4:

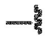 indicates attachment to Lin3,

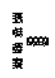 indicates the attachment to $R_8$;
$R^{10}$ is selected from the group consisting of H, $CH_2$-COOH, and $(C_1-C_4)$alkyl; and

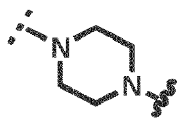

is optionally oxidized to

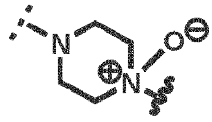
;

$R^8$ is a chelator or a cytotoxic agent; and wherein

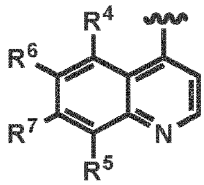

in the formula (I) may optionally be oxidized to its N-oxide

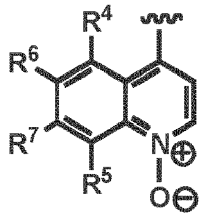
.

The phrase "attachment to the quinoline" as used in this and other embodiments, as well as throughout the present disclosure, refers to the attachment of $R_5$, $R_6$, or $R_7$ to the quinoline ring shown in formula (I), and which quinoline ring may optionally be oxidized to its N-oxide, as mentioned above.

Embodiment 2. The compound of Embodiment 1, wherein $R^3$ is selected from the group consisting of C(O)-Het1, $B(OH)_2$, and $-CO-CH_2-OH$.

Embodiment 3. The compound of Embodiment 1, wherein $R^3$ is -C(O)-Het1, and wherein -C(O)-Het1 is selected from the group consisting of:

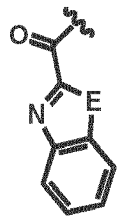 , 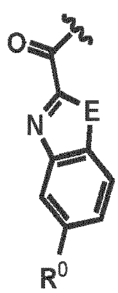 , 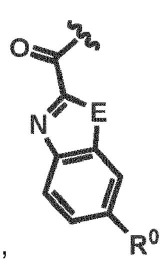 , 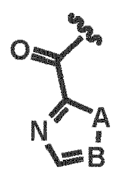 , and 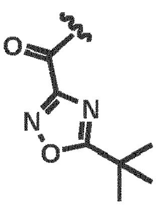 ,

wherein:

A is selected from the group consisting of O, S, and NH;
B is CH or, if A is NH, B is CH or N;

E is O or S; and
$R^0$ is selected from the group consisting of $-CH_3$, $-O-CH_3$, $-COOCH_3$, F, Cl, and Br.

Embodiment 4. The compound of Embodiment 1, wherein $R^3$ is -C(O)-Het1, and wherein -C(O)-Het1 is selected from the group consisting of:

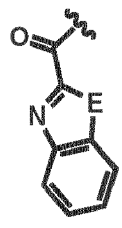 , 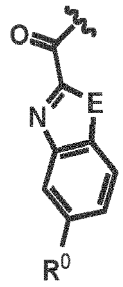 , and 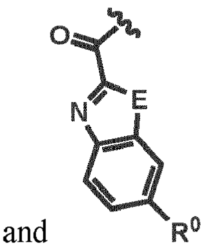 ,

wherein:

E is O or S; and
$R^0$ is selected from the group consisting of $-CH_3$, $-O-CH_3$, $-COOCH_3$, F, Cl, and Br.

Embodiment 5. The compound of Embodiment 1, wherein the compound has a structure of formula (II):

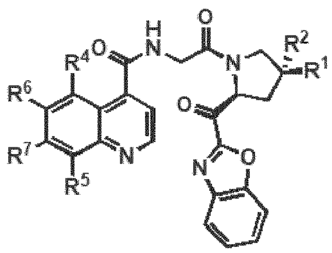

II.

Embodiment 6. The compound of any one of Embodiments 1, 2, 3, 4, and 5, wherein $R^4$ is H or $-CH_3$.

Embodiment 7. The compound of any one of Embodiments 1, 2, 3, 4, 5, and 6, wherein $R^4$ is H.

Embodiment 8. The compound of any one of Embodiments 1, 2, 3, 4, 5, and 6, wherein $R^4$ is $-CH_3$.

Embodiment 9. The compound of any one of Embodiments 1, 2, 3, 4, 5, 6, 7, and 8, preferably of any one of Embodiments 7 and 8, wherein $R^6$ is $R^8$-L.

Embodiment 10. The compound of Embodiment 9, wherein $R^5$ and $R^7$ are each independently selected from the group consisting of H and $-CH_3$.

Embodiment 11. The compound of Embodiment 10, wherein at least one of $R^5$ and $R^7$ is H.

Embodiment 12. The compound of any one of Embodiments 1, 2, 3, 4, 5, 6, 7, and 8, preferably of any one of Embodiments 7 and 8, wherein $R^7$ is $R^8$-L.

Embodiment 13. The compound of Embodiment 12, wherein $R^5$ and $R^6$ are each independently selected from the group consisting of H and $-CH_3$.

Embodiment 14. The compound of Embodiment 13, wherein at least one of $R^5$ and $R^6$ is H.

Embodiment 15. The compound of any one of Embodiments 1, 2, 3, 4, 5, 6, 7, 8, 9, 10, 11, 12, 13, and 14, preferably of any one of Embodiments 2, 3, 4, 5, 6, 7, 8, 9, 10, 11, 12, 13, and 14, more preferably any one of Embodiments

3, 4, 5, 6, 7, 8, 9, 10, 11, 12, 13, and 14, most preferably any one of Embodiments 4, 5, 6, 7, 8, 9, 10, 11, 12, 13, and 14, wherein Lin1 is

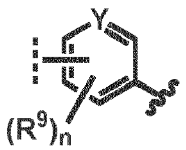 or 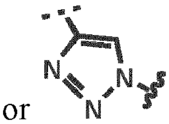 .

Embodiment 16. The compound of any one of Embodiments 1, 2, 3, 4, 5, 6, 7, 8, 9, 10, 11, 12, 13, 14 and 15, preferably of any one of Embodiments 2, 3, 4, 5, 6, 7, 8, 9, 10, 11, 12, 13, 14, and 15, more preferably any one of Embodiments 3, 4, 5, 6, 7, 8, 9, 10, 11, 12, 13, 14, and 15, most preferably any one of Embodiments 4, 5, 6, 7, 8, 9, 10, 11, 12, 13, 14, and 15, wherein Lin1 is

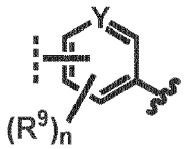 .

Embodiment 17. The compound of any one of Embodiments 15 to 16, wherein Y is CH.

Embodiment 18. The compound of any one of Embodiments 1, 2, 3, 4, 5, 6, 7, 8, 9 10, 11, 12, 13, and 14, preferably of any one of Embodiments 2, 3, 4, 5, 6, 7, 8, 9, 10, 11, 12, 13, and 14, more preferably any one of Embodiments 3, 4, 5, 6, 7, 8, 9, 10, 11, 12, 13, and 14, most preferably any one of Embodiments 4, 5, 6, 7, 8, 9, 10, 11, 12, 13, and 14, wherein Lin1 is

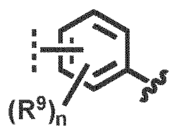 .

Embodiment 19. The compound of any one of Embodiments 1, 2, 3, 4, 5, 6, 7, 8, 9, 10, 11, 12, 13, 14, and 15, preferably any one of Embodiments 2, 3, 4, 5, 6, 7, 8, 9, 10, 11, 12, 13, 14, and 15, more preferably any one of Embodiments 3, 4, 5, 6, 7, 8, 9, 10, 11, 12, 13, 14, and 15, most preferably any one of Embodiments 4, 5, 6, 7, 8, 9, 10, 11, 12, 13, 14, and 15, wherein Lin2-Lin1 is selected from the group consisting of

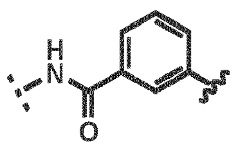

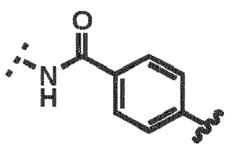 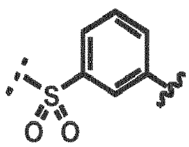 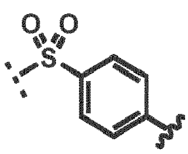 , 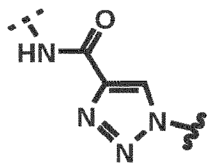 , and 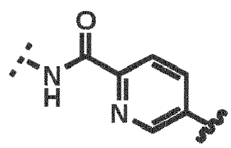 ,

wherein

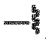 indicates attachment to the quinoline and 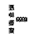 indicates attachment to Lin3.

Embodiment 20. The compound of any one of Embodiments 15, 16, 17, 18, and 19, wherein Lin2-Lin1 is selected from the group consisting of

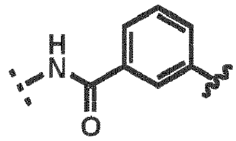,

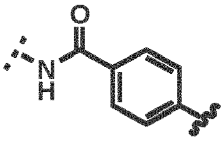, 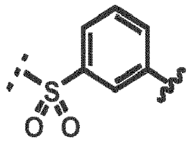, and 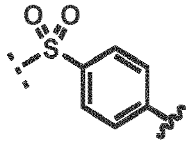,

wherein 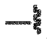 indicates attachment to the quinoline and 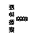 indicates attachment to Lin3.

Embodiment 21. The compound of any one of Embodiments 1, 2, 3, 4, 5, 6, 7, 8, 9, 10, 11, 12, 13, 14, 15, 16, 17, 18 19, and 20, preferably any one of Embodiments 2, 3, 4, 5, 6, 7, 8, 9, 10, 11, 12, 13, 14, 15, 16, 17, 18, 19 and 20, more preferably any one of Embodiments 3, 4, 5, 6, 7, 8, 9, 10, 11, 12, 13, 14, 15, 16, 17, 18, 19 and 20, most preferably any one of Embodiments 4, 5, 6, 7, 8, 9, 10, 11, 12, 13, 14, 15, 16, 17, 18, 19 and 20, wherein Lin3 is -($C_2$-$C_3$)alkylidene.

Embodiment 22. The compound of any one of Embodiments 1, 2, 3, 4, 5, 6, 7, 8, 9, 10, 11, 12, 13, and 14, preferably any one of Embodiments 2, 3, 4, 5, 6, 7, 8, 9, 10, 11, 12, 13 and 14, more preferably any one of Embodiments 3, 4, 5, 6, 7, 8, 9, 10, 11, 12, 13, and 14, most preferably any one of Embodiments 4, 5, 6, 7, 8, 9, 10, 11, 12, 13, and 14, wherein $R^8$-L- is selected from the group consisting of:

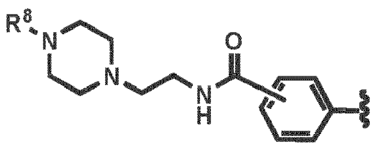, 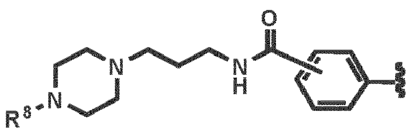, 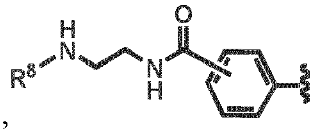,

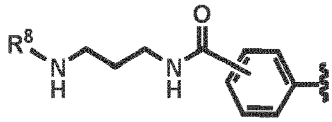, 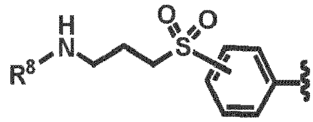,

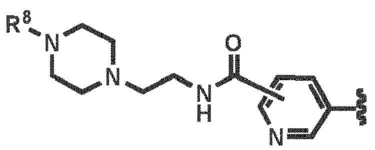, 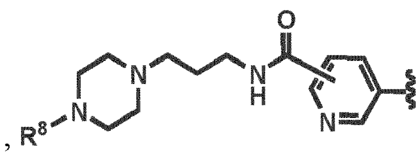, 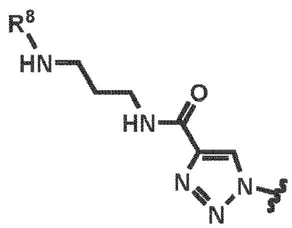,

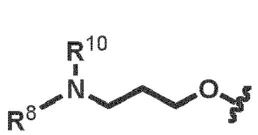, 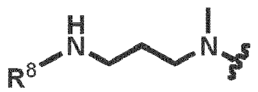, 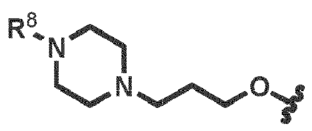, 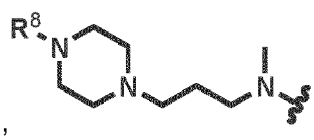,

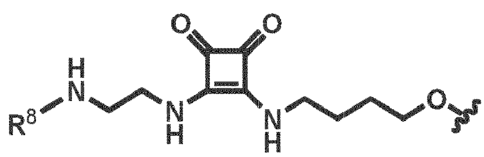, and 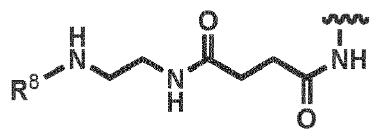.

Embodiment 23. The compound of Embodiment 22, wherein $R^8$-L- is selected from the group consisting of:

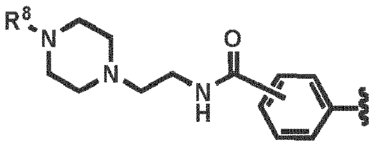, 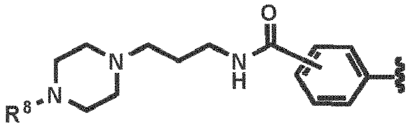, 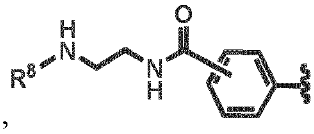, 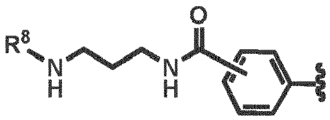, 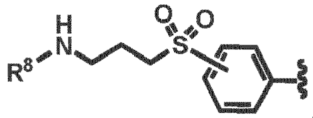, 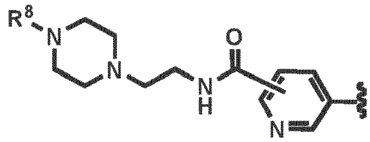, 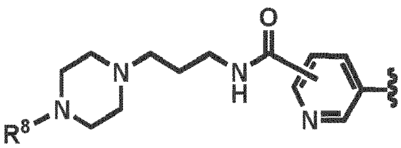, and 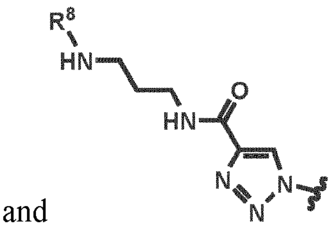.

Embodiment 24. The compound of Embodiment 22, wherein $R^8$-L- is selected from the group consisting of:

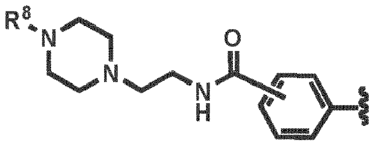, 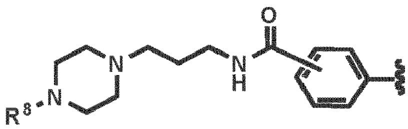, 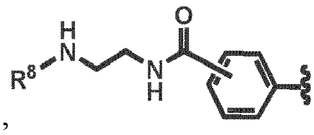, 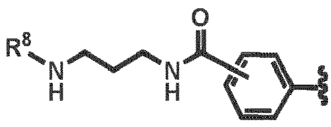, and 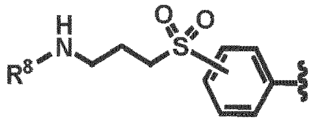.

Embodiment 25. The compound of any one of Embodiments 1, 2, 3, 4, 5, 6, 7, 8, 9, 10, 11, 12, 13, 14, 15, 16, 17, 18, 19, 20, 21, 22, 23 and 24, preferably of any one of Embodiments 2, 3, 4, 5, 6, 7, 8, 9, 10, 11, 12, 13, 14, 15, 16, 17, 18, 19, 20, 21, 22, 23 and 24, more preferably of any one of Embodiments 3, 4, 5, 6, 7, 8, 9, 10, 11, 12, 13, 14, 15, 16, 17, 18, 19, 20, 21, 22, 23 and 24, and most preferably of any one of Embodiments 4, 5, 6, 7, 8, 9, 10, 11, 12, 13, 14, 15, 16, 17, 18, 19, 20, 21, 22, 23 and 24, wherein $R^8$ is a chelator.

Embodiment 26. The compound of Embodiment 25, wherein the chelator is selected from the group consisting of DOTA, DOTAGA, NOPO, PCTA, NOTA, NODAGA, NODA-MPAA, HBED, TETA, CB-TE2A, DTPA, DFO, Macropa, DOTAM, HOPO, TRAP, THP, DATA, NOTP, sarcophagine, FSC, NETA, H4octapa, Pycup, $N_xS_{4-x}$ (N4, N2S2, N3S), Hynic, $^{99m}Tc(CO)_3$-Chelators, more preferably DOTA, DOTAGA, NOPO, PCTA DOTAM, Macropa, NOTA, NODAGA, NODA-MPAA, HBED, CB-TE2A, DFO, THP, N4 and most preferably DOTA, DOTAGA, NOPO, PCTA, DOTAM, Macropa, NOTA, and NODAGA.

Embodiment 27. The compound of Embodiment 25, wherein the chelator is selected from the group consisting of DOTA, NOPO, PCTA and Macropa.

Embodiment 28. The compound of Embodiment 25, wherein the chelator is DOTA.

Embodiment 29. The compound of Embodiment 25, wherein the chelator is NOPO.

Embodiment 30. The compound of Embodiment 25, wherein the chelator is PCTA.

Embodiment 31. The compound of Embodiment 25, wherein the chelator is Macropa.

Embodiment 32. The compound of any one of Embodiments 27, 28, 29, 30, and 31, preferably Embodiment 28, wherein $R^5$ is

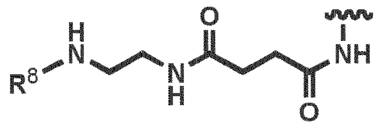
.

Embodiment 33. The compound of any one of Embodiments 1, 2, 3, 4, 5, 6, 7, 8, 9, 10, 11, 12, 13, 14, 15, 16, 17, 18, 19, 20, 21, 22, 23, and 24, preferably of any one of Embodiments 2, 3, 4, 5, 6, 7, 8, 9, 10, 11, 12, 13, 14, 15, 16, 17, 18, 19, 20, 21, 22, 23 and 24, more preferably of any one of Embodiments 3, 4, 5, 6, 7, 8, 9, 10, 11, 12, 13, 14, 15, 16, 17, 18, 19, 20, 21, 22, 23 and 24, and most preferably of any one of Embodiments 4, 5, 6, 7, 8, 9, 10, 11, 12, 13, 14, 15, 16, 17, 18, 19, 20, 21, 22, 23 and 24, , wherein $R^8$ is a cytotoxic agent.

Embodiment 34. The compound of Embodiment 1, wherein $R^3$ is -C(O)-Het1, and wherein -C(O)-Het1 is selected from the group consisting of:

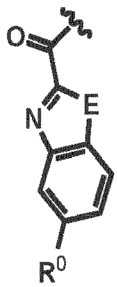 , 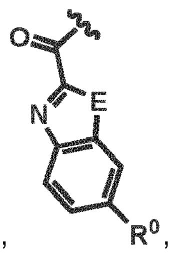, 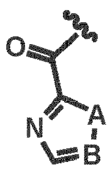, and 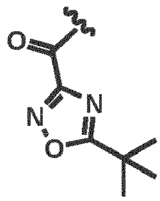,

wherein:

A is selected from the group consisting of O, S, and NH;
B is CH or, if A is NH, B is CH or N;
E is O or S; and
$R^0$ is selected from the group consisting of $-CH_3$, $-O-CH_3$, $-COOCH_3$, F, Cl, and Br.

Embodiment 35. The compound of Embodiment 34, wherein $R^3$ is -C(O)-Het1, and wherein -C(O)-Het1 is selected from the group consisting of:

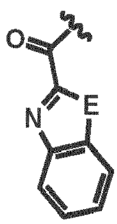, 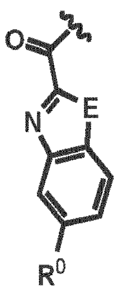, and 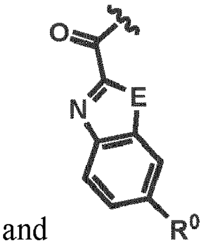,

wherein:

E is O or S; and
$R^0$ is selected from the group consisting of $-CH_3$, $-O-CH_3$, $-COOCH_3$, F, Cl, and Br.

Embodiment 36. The compound of Embodiment 35, wherein the compound has a structure of formula (II):

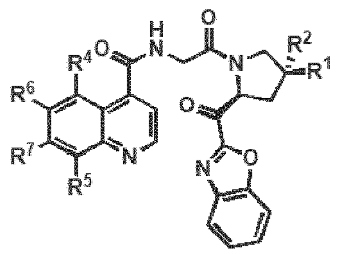

II.

Embodiment 37. The compound of any one of Embodiments 34, 35, and 36, preferably of any one of Embodiments 35, and 36, wherein $R^4$ is H or $-CH_3$.

Embodiment 38. The compound of any one of Embodiments 34, 35, 36 and 37, preferably any one of Embodiments 35, 36, and 37, more preferably any one of Embodiments 36 and 37, wherein $R^4$ is H.

Embodiment 39. The compound of any one of Embodiments 34, 35, 36 and 37, preferably any one of Embodiments 35, 36, and 37, more preferably any one of Embodiments 36 and 37, wherein $R^4$ is $-CH_3$.

Embodiment 40. The compound of any one of Embodiments 34, 35, 36, 37, 38 and 39, preferably of any one of Embodiments 38 and 39, wherein $R^6$ is $R^8$-L.

Embodiment 41. The compound of Embodiment 40, wherein $R^5$ and $R^7$ are each independently selected from the group consisting of H and $-CH_3$.

Embodiment 42. The compound of Embodiment 41, wherein at least one of $R^5$ and $R^7$ is H.

Embodiment 43. The compound of any one of Embodiments 34, 35, 36, 37, 38, and 39, preferably of any one of Embodiments 38 and 39, wherein $R^7$ is $R^8$-L.

Embodiment 44. The compound of Embodiment 43, wherein $R^5$ and $R^6$ are each independently selected from the group consisting of H and $-CH_3$.

Embodiment 45. The compound of Embodiment 44, wherein at least one of $R^5$ and $R^6$ is H.

Embodiment 46. The compound of any one of Embodiments 34, 35, 36, 37, 38, 39, 40, 41, 42, 43, 44, and 45, preferably of any one of Embodiments 35, 36, 37, 38, 39, 40, 41, 42, 43, 44, and 45, more preferably of any one of Embodiments 36, 37, 38, 39, 40, 41, 42, 43, 44, and 45, wherein Lin1 is

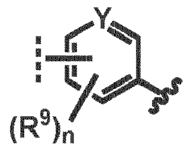

or

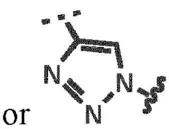

.

Embodiment 47. The compound of any one of Embodiments 34, 35, 36, 37, 38, 39, 40, 41, 42, 43, 44, 45, and 46, preferably of any one of Embodiments 35, 36, 37, 38, 39, 40, 41, 42, 43, 44, 45, and 46, more preferably of any one of Embodiments 36, 37, 38, 39, 40, 41, 42, 43, 44, 45, and 46, wherein Lin1 is

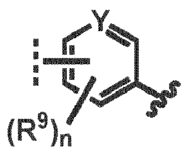

.

Embodiment 48. The compound of any one of Embodiments 46 to 47, wherein Y is CH.

Embodiment 49. The compound of any one of Embodiments 34, 35, 36, 37, 38, 39, 40, 41, 42, 43, 44, and 45,

preferably of any one of Embodiments 35, 36, 37, 38, 39, 40, 41, 42, 43, 44, and 45, more preferably of any one of Embodiments 36, 37, 38, 39, 40, 41, 42, 43, 44, and 45, wherein Lin1 is

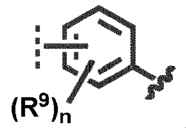
.

Embodiment 50. The compound of any one of Embodiments 34, 35, 36, 37, 38, 39, 40, 41, 42, 43, 44, 45, and 46, preferably of any one of Embodiments 35, 36, 37, 38, 39, 40, 41, 42, 43, 44, 45, and 46, more preferably of any one of Embodiments 36, 37, 38, 39, 40, 41, 42, 43, 44, 45, and 46, wherein Lin2-Lin1 is selected from the group consisting of

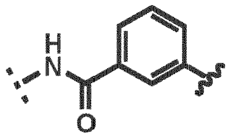 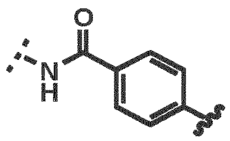 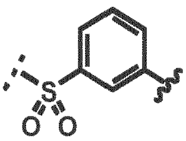 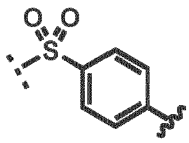, 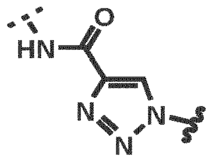,

and

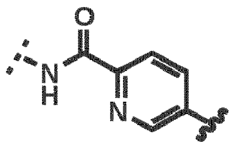,

wherein 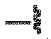 indicates attachment to the quinoline and 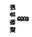 indicates attachment to Lin3.

Embodiment 51. The compound of any one of Embodiments 46, 47, 48, 49, and 50, wherein Lin2-Lin1 is selected from the group consisting of

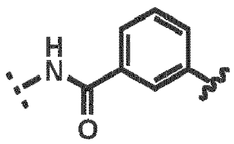,

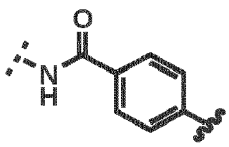, 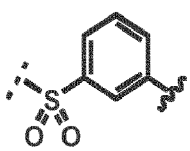, and 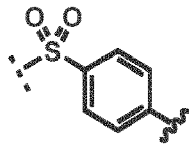,

wherein 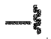 indicates attachment to the quinoline and 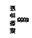 indicates attachment to Lin3.

Embodiment 52. The compound of any one of Embodiments 34, 35, 36, 37, 38, 39, 40, 41, 42, 43, 44, 45, 46, 47, 48, 49, 50, and 51, preferably of any one of Embodiments 35, 36, 37, 38, 39, 40, 41, 42, 43, 44, 45, 46, 47, 48, 49, 50, and 51, more preferably of any one of Embodiments 36, 37, 38, 39, 40, 41, 42, 43, 44, 45, 46, 47, 48, 49, 50, and 51, wherein Lin3 is -($C_2$-$C_3$)alkylidene.

Embodiment 53. The compound of any one of Embodiments 34, 35, 36, 37, 38, 39, 40, 41, 42, 43, 44, and 45, preferably of any one of Embodiments 35, 36, 37, 38, 39, 40, 41, 42, 43, 44, and 45, more preferably of any one of Embodiments 36, 37, 38, 39, 40, 41, 42, 43, 44, and 45, wherein $R^8$-L- is selected from the group consisting of:

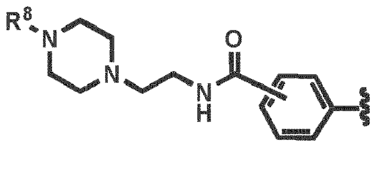, 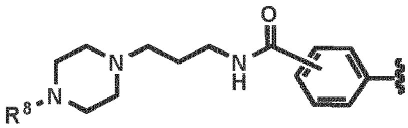, 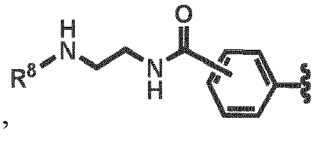,

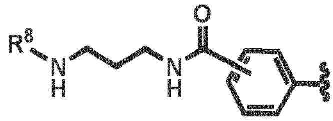, 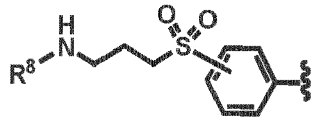,

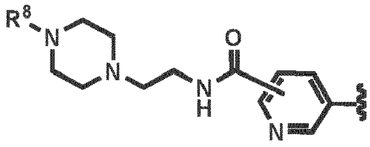, 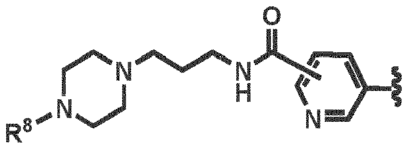, 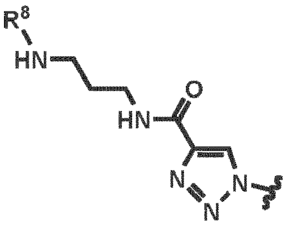,

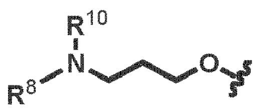, 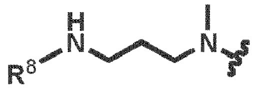, 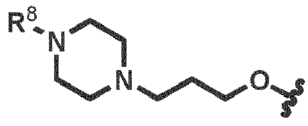, 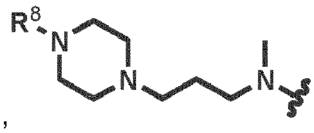,

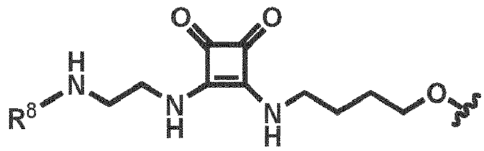, and 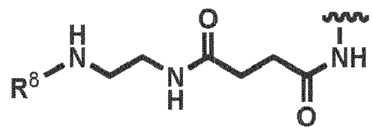.

Embodiment 54. The compound of Embodiment 53, wherein $R^8$-L- is selected from the group consisting of:

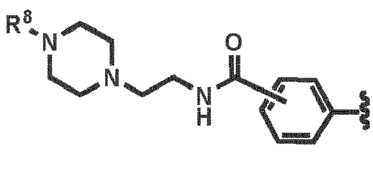, 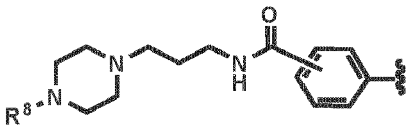, 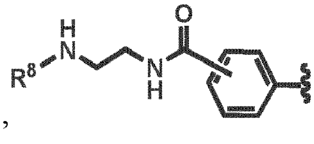,

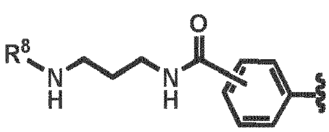, 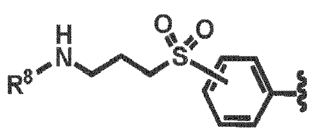, 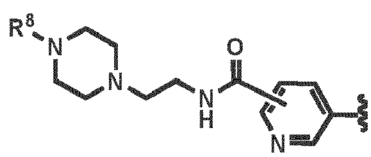,

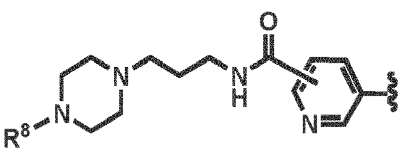, and 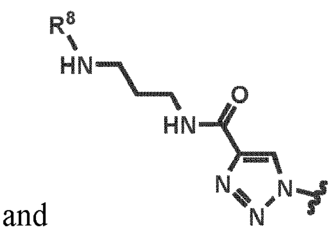.

Embodiment 55. The compound of Embodiment 53, wherein $R^8$-L- is selected from the group consisting of:

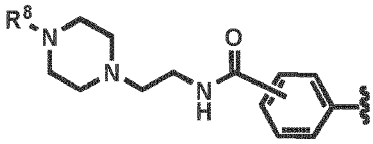, 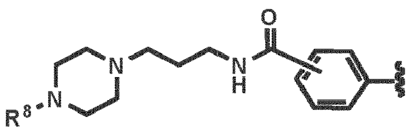, 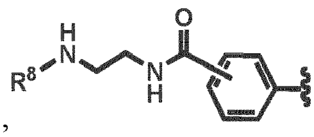,

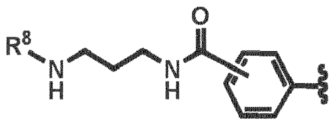, and 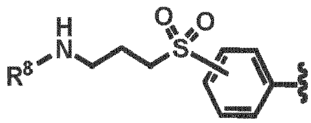.

Embodiment 56. The compound of any one of Embodiments 34, 35, 36, 37, 38, 39, 40, 41, 42, 43, 44, 45, 46, 47, 48, 49, 50, 51, 52, 53, 54 and 55 preferably of any one of Embodiments 35, 36, 37, 38, 39, 40, 41, 42, 43, 44, 45, 46, 47, 48, 49, 50, 51, 52, 53, 54 and 55, more preferably of any one of Embodiments 36, 37, 38, 39, 40, 41, 42, 43, 44, 45, 46, 47, 48, 49, 50, 51, 52, 53, 54 and 55, wherein $R^8$ is a chelator.

Embodiment 57. The compound of Embodiment 56, wherein the chelator is selected from the group consisting of DOTA, DOTAGA, NOPO, PCTA, NOTA, NODAGA, NODA-MPAA, HBED, TETA, CB-TE2A, DTPA, DFO, Macropa, DOTAM, HOPO, TRAP, THP, DATA, NOTP, sarcophagine, FSC, NETA, H4octapa, Pycup, $N_xS_{4-x}$ (N4, N2S2, N3S), Hynic, $^{99m}Tc(CO)_3$-Chelators, more preferably DOTA, DOTAGA, NOPO, PCTA, DOTAM, Macropa, NOTA, NODAGA, NODA-MPAA, HBED, CB-TE2A, DFO, THP, N4 and most preferred DOTA, DOTAGA, NOPO, PCTA, DOTAM, Macropa, NOTA, and NODAGA.

Embodiment 58. The compound of Embodiment 56, wherein the chelator is selected from the group consisting of DOTA, NOPO, PCTA and Macropa.

Embodiment 59. The compound of Embodiment 56, wherein the chelator is DOTA.

Embodiment 60. The compound of Embodiment 56, wherein the chelator is NOPO.

Embodiment 61. The compound of Embodiment 56, wherein the chelator is PCTA.

Embodiment 62. The compound of Embodiment 56, wherein the chelator is Macropa.

Embodiment 63. The compound of any one of Embodiments 59, 60, 61 and 62, preferably Embodiment 59, wherein $R^5$ is

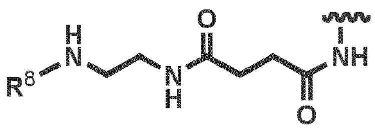.

Embodiment 64. The compound of any one of Embodiments 34, 35, 36, 37, 38, 39, 40, 41, 42, 43, 44, 45, 46, 47, 48, 49, 50, 51, 52, 53, 54, and 55, , and 63, preferably of any one of Embodiments 35, 36, 37, 38, 39, 40, 41, 42, 43, 44, 45, 46, 47, 48, 49, 50, 51, 52, 53, 54, and 55, , more preferably of any one of Embodiments 36, 37, 38, 39, 40, 41, 42, 43, 44, 45, 46, 47, 48, 49, 50, 51, 52, 53, 54, and 55, , wherein $R^8$ is a cytotoxic agent.

Embodiment 65. The compound of Embodiment 1, wherein $R^3$ is -CN, and Lin1 is selected from the group consisting of

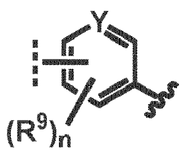 and 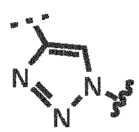.

Embodiment 66. The compound of Embodiment 65, wherein $R^4$ is H or $-CH_3$.

Embodiment 67. The compound of Embodiment 66, wherein $R^4$ is H.

Embodiment 68. The compound of Embodiment 66, wherein $R^4$ is $-CH_3$.

Embodiment 69. The compound of any one of Embodiments 65, 66, 67, 68, and 69, preferably of any one of Embodiments 67 and 68, wherein $R^6$ is $R^8$-L.

Embodiment 70. The compound of Embodiment 69, wherein $R^5$ and $R^7$ are independently selected from the group consisting of H and $-CH_3$.

Embodiment 71. The compound of Embodiment 70, wherein at least one of $R^5$ and $R^7$ is H.

Embodiment 72. The compound of any one of Embodiments 65, 66, 67, and 68, preferably of any one of Embodiments 67 and 68, wherein $R^7$ is $R^8$-L.

Embodiment 73. The compound of Embodiment 72, wherein $R^5$ and $R^6$ are each independently selected from the group consisting of H and $-CH_3$.

Embodiment 74. The compound of Embodiment 73, wherein at least one of $R^5$ and $R^6$ is H.

Embodiment 75. The compound of any one of Embodiments 65, 66, 67, 68, 69, 70, 71, 72, 73, and 74, preferably of any one of Embodiments 66, 67, 68, 69, 70, 71, 72, 73, and 74, wherein Lin1 is

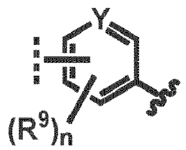.

Embodiment 76. The compound of any one of Embodiments 65 and 75, wherein Y is CH.

Embodiment 77. The compound of any one of Embodiments 65, 66, 67, 68, 69, 70, 71, 72, 73, and 74, preferably of any one of Embodiments 66, 67, 68, 69, 70, 71, 72, 73, and 74, wherein Lin2-Lin1 is selected from the group consisting of

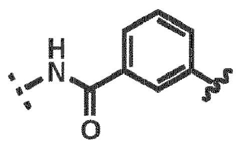

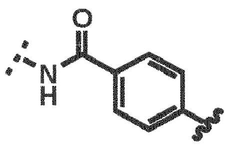 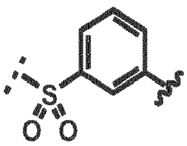 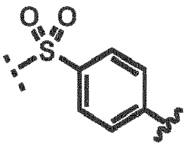, 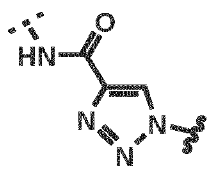, and 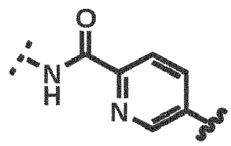,

wherein 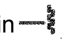 indicates attachment to the quinoline and 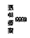 indicates attachment to Lin3.

Embodiment 78. The compound of any one of Embodiments 75, 76, and 77, wherein Lin2-Lin1 is selected from the group consisting of

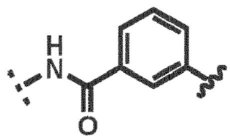, 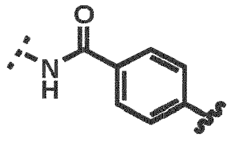,

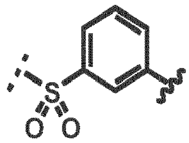, and 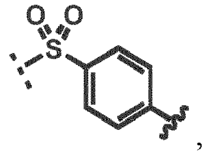,

wherein 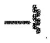 indicates attachment to the quinoline and 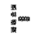 indicates attachment to Lin3.

Embodiment 79. The compound of any one of Embodiments 65, 66, 67, 68, 69, 70, 71, 72, 73, 74, 75, 76, 77, and 78, preferably of any one of Embodiments 66, 67, 68, 69, 70, 71, 72, 73, 74, 75, 76, and 78, wherein Lin3 is -($C_2$-$C_3$)alkylidene.

Embodiment 80. The compound of any one of Embodiments 65, 66, 67, 68, 69, 70, 71, 72, 73, and 74, wherein $R^8$-L- is selected from the group consisting of:

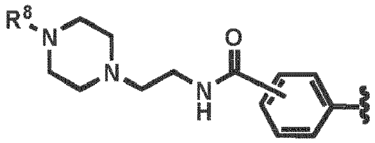, 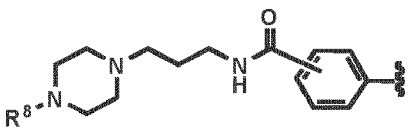, 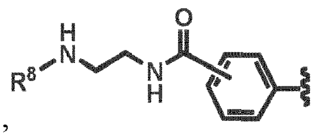,

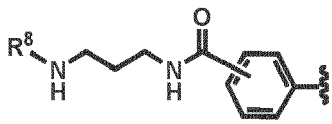, 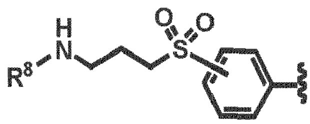,

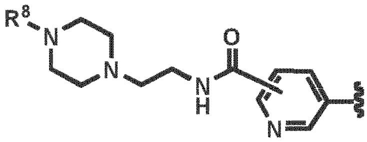, 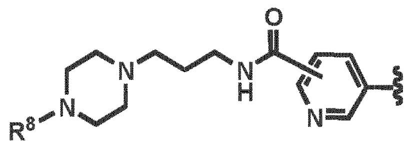,and 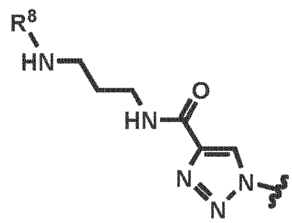

.

Embodiment 81. The compound of any one of Embodiments 65, 66, 67, 68, 69, 70, 71, 72, 73, and 74, wherein $R^8$-L- is selected from the group consisting of:

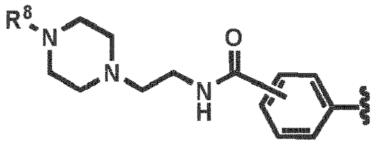, 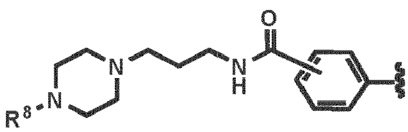, 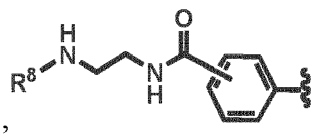,

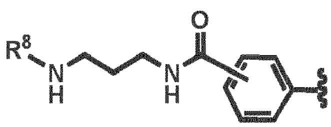, and 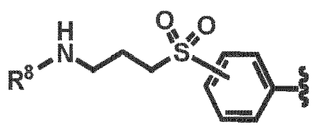.

Embodiment 82. The compound of any one of Embodiments 65, 66, 67, 68, 69, 70, 71, 72, 73, 74, 75, 76, 77, 78, 79, 80, and 81, wherein $R^8$ is a chelator.

Embodiment 83. The compound of Embodiment 82, wherein the chelator is selected from the group consisting of DOTA, DOTAGA, NOPO, PCTA, NOTA, NODAGA, NODA-MPAA, HBED, TETA, CB-TE2A, DTPA, DFO, Macropa, DOTAM, HOPO, TRAP, THP, DATA, NOTP, sarcophagine, FSC, NETA, H4octapa, Pycup, $N_xS_{4-x}$ (N4, N2S2, N3S), Hynic, $^{99m}Tc(CO)_3$-Chelators, more preferably DOTA, DOTAGA, NOPO, PCTA, DOTAM, Macropa, NOTA, NODAGA, NODA-MPAA, HBED, CB-TE2A, DFO, THP, N4 and most preferred DOTA, DOTAGA, NOPO, PCTA, DOTAM, Macropa, NOTA, and NODAGA.

Embodiment 84. The compound of Embodiment 82, wherein the chelator is selected from the group consisting of DOTA, NOPO, PCTA and Macropa.

Embodiment 85. The compound of Embodiment 82, wherein the chelator is DOTA.

Embodiment 86. The compound of Embodiment 82, wherein the chelator is NOPO.

Embodiment 87. The compound of Embodiment 82, wherein the chelator is PCTA.

Embodiment 88. The compound of Embodiment 82, wherein the chelator is Macropa.

Embodiment 89. The compound of any one of Embodiments 65, 66, 67, 68, 69, 70, 71, 72, 73, 74, 75, 76, 77, 78, 79, 80, and 81, wherein $R^8$ is a cytotoxic agent.

Embodiment 90. The compound of Embodiment 1, wherein Lin1 is selected from the group consisting of

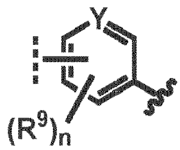, 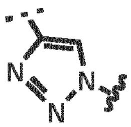,

-O-, $N(CH_3)$-, and 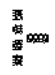 $(C_1-C_4)$alkylidene-C(O)NH 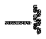, with a proviso that when $R^3$ is CN, then Lin1 is

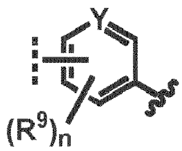 or 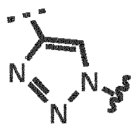,

wherein in Lin1:

Y is CH or N;

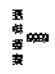 indicates attachment to Lin2, and when Lin1 is

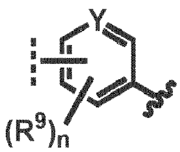,

Lin2 is in *meta*- or *para*-position to 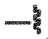 and

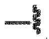 indicates attachment to the quinoline;
$R^9$ is selected from the group consisting of halogen, $CO_2H$, $(C_1-C_6)$alkyl, hydroxy substituted $(C_1-C_6)$alkyl, and -O-$(C_1-C_6)$alkyl; and

n is selected from the group consisting of 0, 1, and 2, preferably n is 0 or 1, more preferably n is 0;

Lin2 is selected from the group consisting of -C(O)-NH-, -NH-C(O)-, and $-S(O)_2-$when Lin1 is selected from the group consisting of

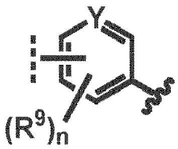,
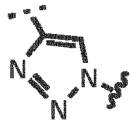,

and ($C_1$- $C_4$)alkylidene-C(O)NH, and Lin2 is absent when Lin1 is -O- or $-N(CH_3)-$.

Embodiment 91. The compound of Embodiment 90, wherein Lin1 is

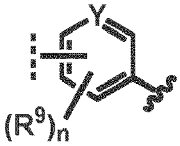 or 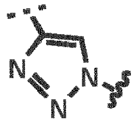.

Embodiment 92. The compound of any one of Embodiments 90 to 91, wherein Lin1 is

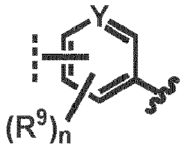.

Embodiment 93. The compound of any one of Embodiments 91 to 92, wherein Y is CH.

Embodiment 94. The compound of any one of Embodiments 90, 91, 92, and 93, wherein Lin1 is

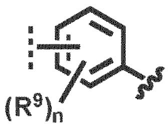.

Embodiment 95. The compound of any one of Embodiments 90, 91, and 92, wherein Lin2-Lin1 is selected from the group consisting of

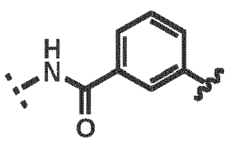
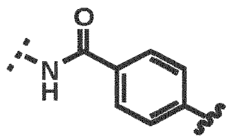

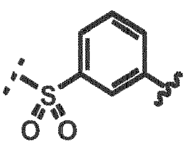
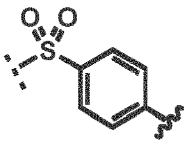,
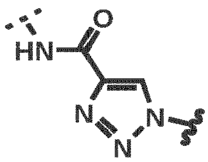, and
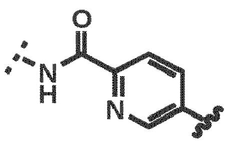,

wherein indicates attachment to the quinoline and indicates attachment to Lin3.

Embodiment 96. The compound of any one of Embodiments 90, 91, 92, 93, 94, and 95, , wherein Lin2-Lin1 is selected from the group consisting of

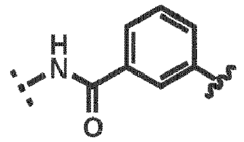

,

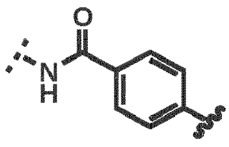

,

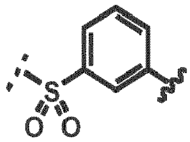

, and

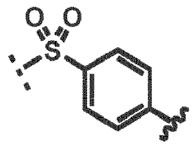

,

wherein indicates attachment to the quinoline and indicates attachment to Lin3.

Embodiment 97. The compound of any one of Embodiments 90, 91, 92, 93, 94, 95, and 96, wherein Lin3 is $-(C_2-C_3)$alkylidene.

Embodiment 98. The compound of Embodiment 90, wherein $R^8$-L- is selected from the group consisting of:

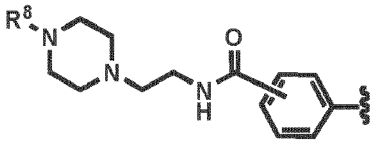

,

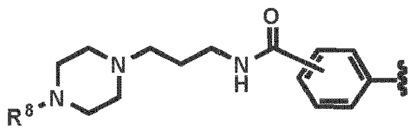

,

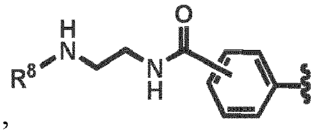

,

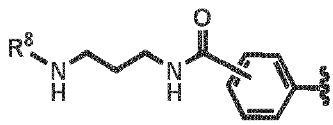

,

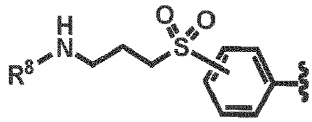

,

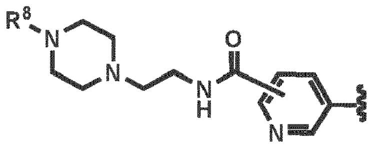

,

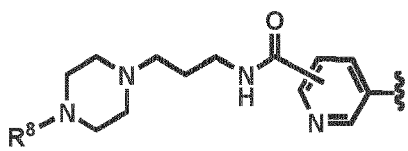

,

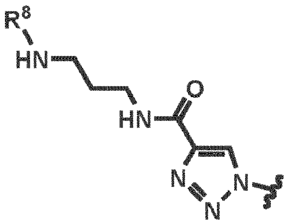

,

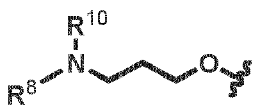

,

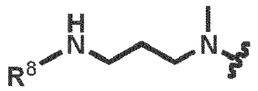

,

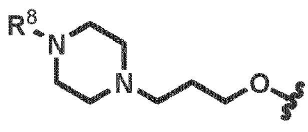

,

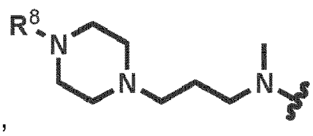

,

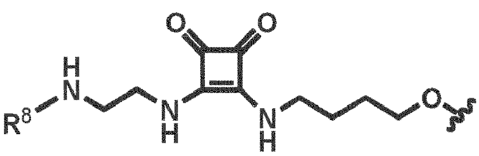

, and

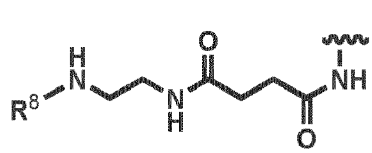

.

Embodiment 99. The compound of Embodiment 98, wherein $R^8$-L- is selected from the group consisting of:

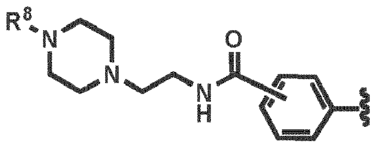, 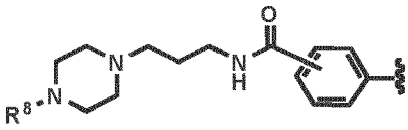, 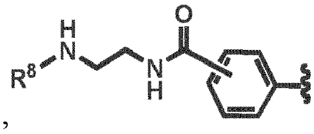,

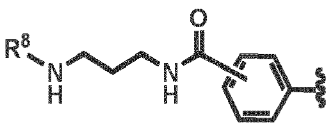, 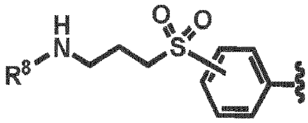, 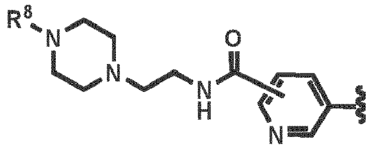,

and

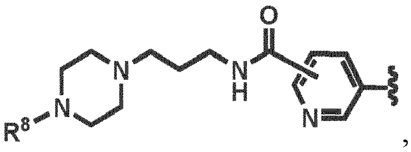, and 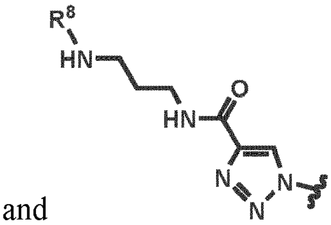.

Embodiment 100. The compound of Embodiment 98, wherein $R^8$-L- is selected from the group consisting of:

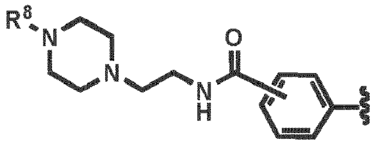, 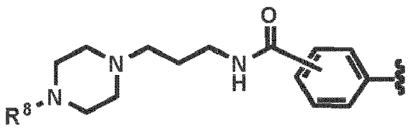, 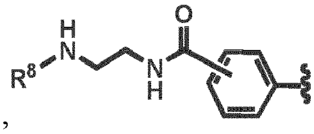,

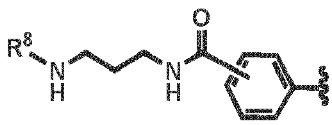, and 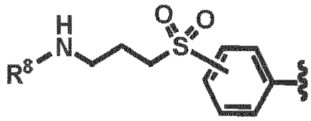.

Embodiment 101. The compound of any one of Embodiments 90,91, 92, 93, 94, 95, 96, 97, 98, 99, and 100, wherein $R^3$ is selected from the group consisting of C(O)-Het1, $B(OH)_2$, and $-CO-CH_2-OH$.

Embodiment 102. The compound of any one of Embodiments 90, 91, 92, 93, 94, 95, 96, 97, 98, 99, and 100, wherein $R^3$ is -C(O)-Het1, and wherein -C(O)-Het1 is selected from the group consisting of:

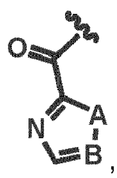, 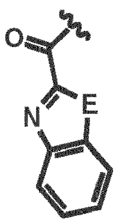, 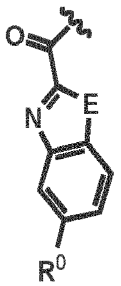, 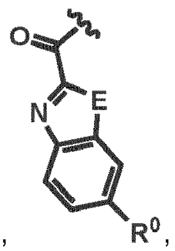, and 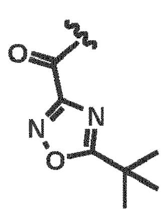,

wherein:

A is selected from the group consisting of O, S, and NH;
B is CH or, if A is NH, B is CH or N;

E is O or S; and

$R^0$ is selected from the group consisting of $-CH_3$, $-O-CH_3$, $-COOCH_3$, F, Cl, and Br.

Embodiment 103. The compound of Embodiments 90, 91, 92, 93, 94, 95, 96, 97, 98, 99, and 100, wherein $R^3$ is -C(O)-Het1, and wherein -C(O)-Het1 is selected from the group consisting of:

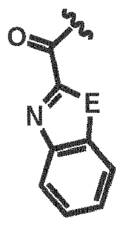
,
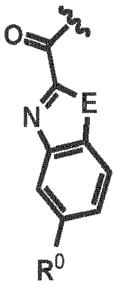
, and
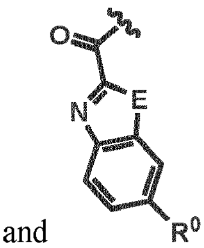
,

wherein:

E is O or S; and

$R^0$ is selected from the group consisting of $-CH_3$, $-O-CH_3$, $-COOCH_3$, F, Cl, and Br.

Embodiment 104. The compound of any one of Embodiments 90, 91, 92, 93, 94, 95, 96, 97, 98, 99, and 100, , wherein the compound has a structure of formula (II):

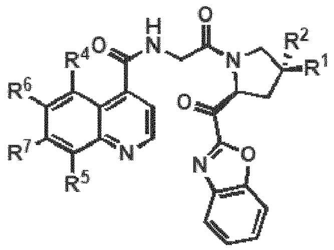
II.

Embodiment 105. The compound of any one of Embodiments 90, 91, 92, 93, 94, 95, 96, 97, 98, 99, 100, 101, 102, 103, and 104, wherein $R^4$ is H or $-CH_3$.

Embodiment 106. The compound of any one of Embodiments 90, 91, 92, 93, 94, 95, 96, 97, 98, 99, 100, 101, 102, 103, 104, and 105, wherein $R^4$ is H.

Embodiment 107. The compound of any one of Embodiments 90, 91, 92, 93, 94, 95, 96, 97, 98, 99, 100, 101, 102, 103, 104, and 105, wherein $R^4$ is $-CH_3$.

Embodiment 108. The compound of any one of Embodiments 90, 91, 92, 93, 94, 95, 96, 97, 98, 99, 100, 101, 102, 103, 104, 105, 106, and 107, preferably of any one of Embodiments 106 and 107, wherein $R^6$ is $R^8$-L.

Embodiment 109. The compound of Embodiment 108, wherein $R^5$ and $R^7$ are each independently selected from the group consisting of H and $-CH_3$.

Embodiment 110. The compound of Embodiment 109, wherein at least one of $R^5$ and $R^7$ is H.

Embodiment 111. The compound of any one of Embodiments 90, 91, 92, 93, 94, 95, 96, 97, 98, 99, 100, 101, 102, 103, 104, 105, 106, and 107, preferably of any one of Embodiments 106 and 107, wherein $R^7$ is $R^8$-L.

Embodiment 112. The compound of Embodiment 111, wherein $R^5$ and $R^6$ are each independently selected from the group consisting of H and $-CH_3$.

Embodiment 113. The compound of Embodiment 112, wherein at least one of $R^5$ and $R^6$ is H.

Embodiment 114. The compound of any one of Embodiments 90, 91, 92, 93, 94, 95, 96, 97, 98, 99, 100, 101, 102, 103, 104, 105, 106, 107, 108, 109, 110, 111, 112, and 113, wherein $R^8$ is a chelator.

Embodiment 115. The compound of Embodiment 114, wherein the chelator is selected from the group consisting of DOTA, DOTAGA, NOPO, PCTA, NOTA, NODAGA, NODA-MPAA, HBED, TETA, CB-TE2A, DTPA, DFO, Macropa, DOTAM, HOPO, TRAP, THP, DATA, NOTP, sarcophagine, FSC, NETA, H4octapa, Pycup, $N_xS_{4-x}$(N4, N2S2, N3S), Hynic, $^{99m}Tc(CO)_3$-Chelators, more preferably DOTA, DOTAGA, NOPO, PCTA, DOTAM, Macropa, NOTA, NODAGA, NODA-MPAA, HBED, CB-TE2A, DFO, THP, N4 and most preferred DOTA, DOTAGA, NOPO, PCTA, DOTAM, Macropa, NOTA, and NODAGA.

Embodiment 116. The compound of Embodiment 114, wherein the chelator is selected from the group consisting of DOTA, NOPO, PCTA and Macropa.

Embodiment 117. The compound of Embodiment 114, wherein the chelator is DOTA.

Embodiment 118. The compound of Embodiment 114, wherein the chelator is NOPO.

Embodiment 119. The compound of Embodiment 114, wherein the chelator is PCTA.

Embodiment 120. The compound of Embodiment 114, wherein the chelator is Macropa.

Embodiment 121. The compound of any one of Embodiments 116, 117, 118, 119, and 120, preferably Claim 117, wherein $R^5$ is

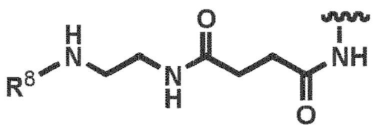

.

Embodiment 122. The compound of any one of Embodiments 90, 91, 92, 93, 94, 95, 96, 97, 98, 99, 100, 101, 102, 103, 104, 105, 106, 107, 108, 109, 110, 111, 112, and 113, wherein $R^8$ is a cytotoxic agent.

Embodiment 123. The compound of Embodiment 1, wherein Lin2-Lin1 is selected from the group consisting of

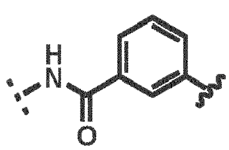 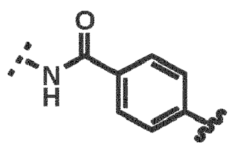 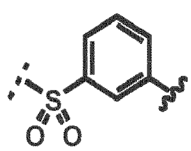 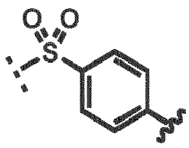,

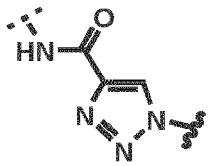, and 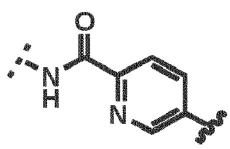,

wherein 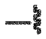 indicates attachment to the quinoline and 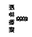 indicates attachment to Lin3.

Embodiment 124. The compound of Embodiment 123, wherein Lin2-Lin1 is selected from the group consisting of

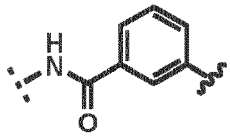, 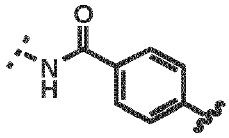, 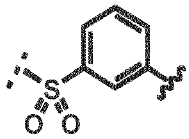,

and

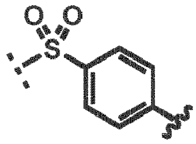,

wherein 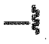 indicates attachment to the quinoline and 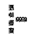 indicates attachment to Lin3.

Embodiment 125. The compound of any one of Embodiments 123 to 124, wherein Lin3 is -($C_2$-$C_3$)alkylidene.

Embodiment 126. The compound of Embodiment 123, wherein $R^8$-L- is selected from the group consisting of:

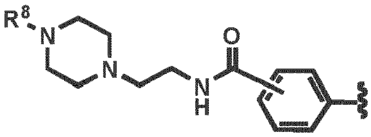, 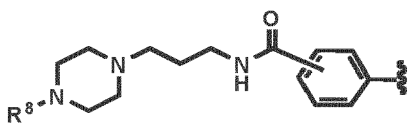, 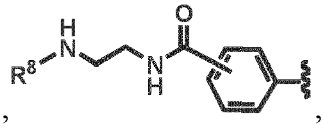,

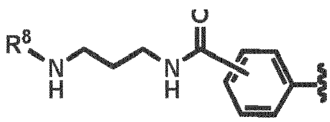, 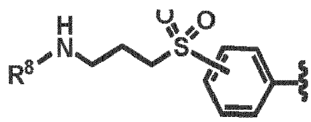,

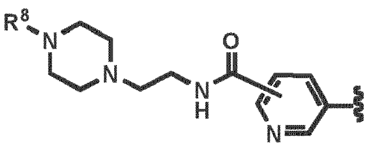, 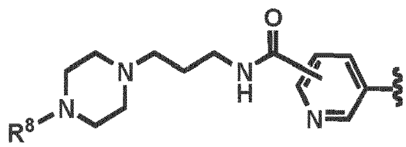, and 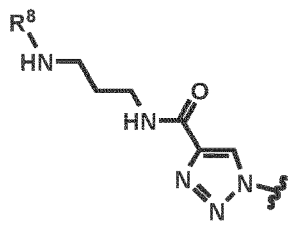

.

Embodiment 127. The compound of Embodiment 126, wherein $R^8$-L- is selected from the group consisting of:

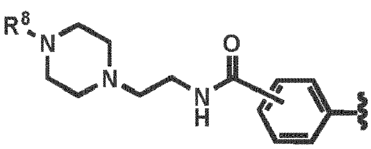, 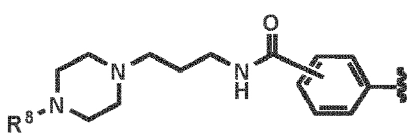, 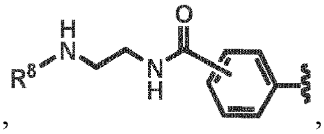,

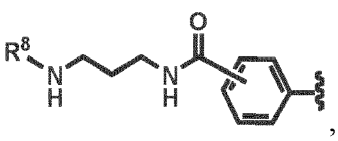, 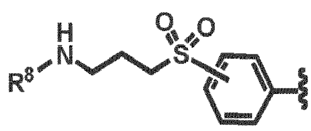, 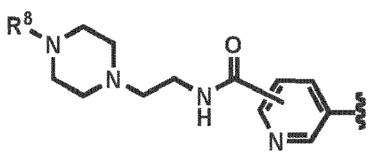,

and

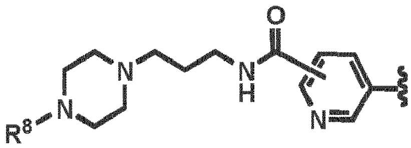.

Embodiment 128. The compound of Embodiment 126, wherein $R^8$-L- is selected from the group consisting of:

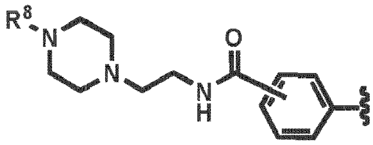, 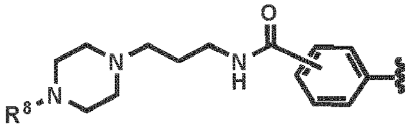, 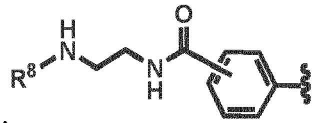,

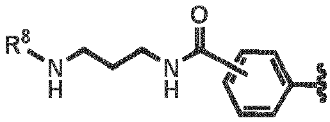, and 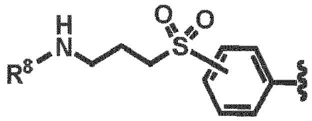.

Embodiment 129. The compound of any one of Embodiments 123, 124, 125, 126, 127, and 128, wherein $R^3$ is selected from the group consisting of C(O)-Het1, $B(OH)_2$, and - CO-$CH_2$-OH.

Embodiment 130. The compound of any one of Embodiments 123,124, 125, 126, 127, 128, and 129, wherein $R^3$ is -C(O)-Het1, and wherein -C(O)-Het1 is selected from the group consisting of:

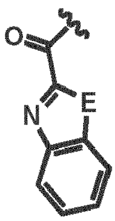, 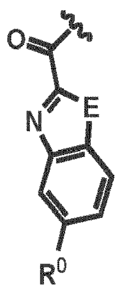, 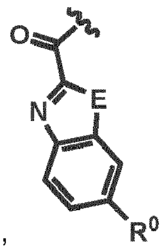, 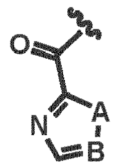 and 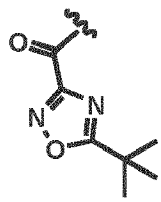,

wherein:

A is selected from the group consisting of O, S, and NH;
B is CH or, if A is NH, B is CH or N;
E is O or S; and
$R^0$ is selected from the group consisting of -$CH_3$, -O-$CH_3$, -$COOCH_3$, F, Cl, and Br.

Embodiment 131. The compound of Embodiments 123, 124, 125, 126, 127, 128, and 129, wherein $R^3$ is -C(O)-Het1, and wherein -C(O)-Het1 is selected from the group consisting of:

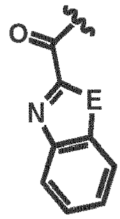, 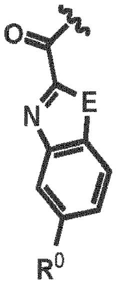, and 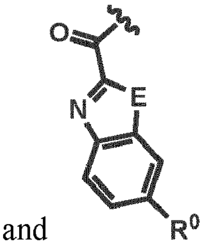,

wherein:

E is O or S; and
$R^0$ is selected from the group consisting of $-CH_3$, $-O-CH_3$, $-COOCH_3$, F, Cl, and Br.

Embodiment 132. The compound of any one of Embodiments 123, 124, 125, 126, 127, 128, and 129, wherein the compound has a structure of formula (II):

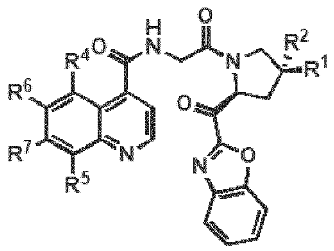

II.

Embodiment 133. The compound of any one of Embodiments 123, 124, 125, 126, 127, 128, 129, 130, 131, and 132, wherein $R^4$ is H or $-CH_3$.

Embodiment 134. The compound of any one of Embodiments 123, 124, 125, 126, 127, 128, 129, 130, 131, 132, and 133, wherein $R^4$ is H.

Embodiment 135. The compound of any one of Embodiments 123, 124, 125, 126, 127, 128, 129, 130, 131, 132, and 133, wherein $R^4$ is $-CH_3$.

Embodiment 136. The compound of any one of Embodiments 123, 124, 125, 126, 127, 128, 129, 130, 131, 132, 133, 134, and 135, wherein $R^6$ is $R^8$-L.

Embodiment 137. The compound of Embodiment 136, wherein $R^5$ and $R^7$ are each independently selected from the group consisting of H and $-CH_3$.

Embodiment 138. The compound of Embodiment 137, wherein at least one of $R^5$ and $R^7$ is H.

Embodiment 139. The compound of any one of Embodiments 123, 124, 125, 126, 127, 128, 129, 130, 131, 132, 133, 134, and 135, preferably of any one of Embodiments 134 and 135, wherein $R^7$ is $R^8$-L.

Embodiment 140. The compound of Embodiment 139, wherein $R^5$ and $R^6$ are each independently selected from the group consisting of H and $-CH_3$.

Embodiment 141. The compound of Embodiment 140, wherein at least one of $R^5$ and $R^6$ is H.

Embodiment 142. The compound of any one of Embodiments 123, 124, 125, 126, 127, 128, 129, 130, 131, 132, 133, 134, 135, 136, 137, 138, 139, 140, and 141, wherein $R^8$ is a chelator.

Embodiment 143. The compound of Embodiment 142, wherein the chelator is selected from the group consisting

of DOTA, DOTAGA, NOPO, PCTA, NOTA, NODAGA, NODA-MPAA, HBED, TETA, CB-TE2A, DTPA, DFO, Macropa, DOTAM, HOPO, TRAP, THP, DATA, NOTP, sarcophagine, FSC, NETA, H4octapa, Pycup, $N_xS_{4-x}$(N4, N2S2, N3S), Hynic, $^{99m}Tc(CO)_3$-Chelators, more preferably DOTA, DOTAGA, NOPO, PCTA, DOTAM, Macropa, NOTA, NODAGA, NODA-MPAA, HBED, CB-TE2A, DFO, THP, N4 and most preferred DOTA, DOTAGA, NOPO, PCTA, DOTAM, Macropa, NOTA, and NODAGA.

Embodiment 144. The compound of Embodiment 142, wherein the chelator is selected from the group consisting of DOTA, NOPO, PCTA and Macropa.

Embodiment 145. The compound of Embodiment 142, wherein the chelator is DOTA.

Embodiment 146. The compound of Embodiment 142, wherein the chelator is NOPO.

Embodiment 147. The compound of Embodiment 142, wherein the chelator is PCTA.

Embodiment 148. The compound of Embodiment 142, wherein the chelator is Macropa.

Embodiment 149. The compound of any one of Embodiments 123, 124, 125, 126, 127, 128, 129, 130, 131, 132, 133, 134, 135, 136, 137, 138, 139, 140, and 141, wherein $R^8$ is a cytotoxic agent.

Embodiment 150. The compound of Embodiment 1, wherein $R^8$-L- is selected from the group consisting of:

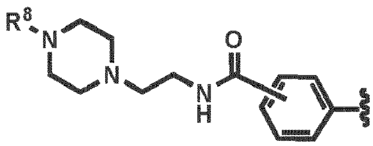, 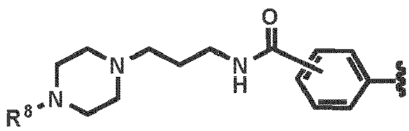, 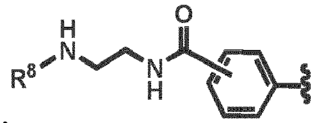,

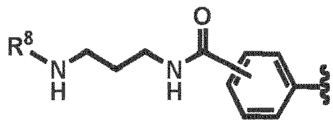, 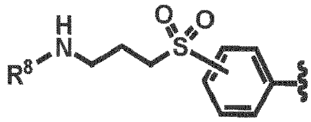,

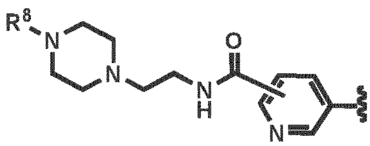, 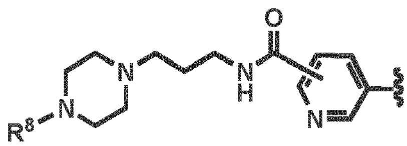, 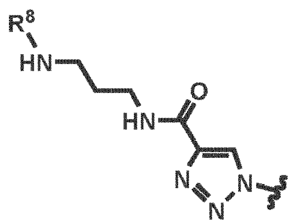,

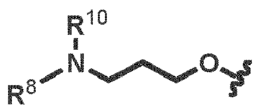, 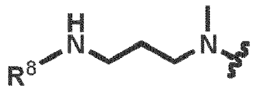, 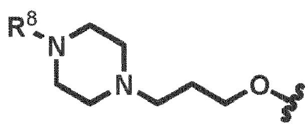, 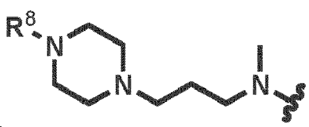,

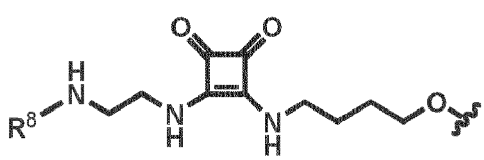, and 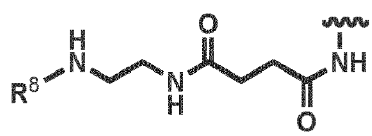.

Embodiment 151. The compound of Embodiment 150, wherein $R^8$-L- is selected from the group consisting of:

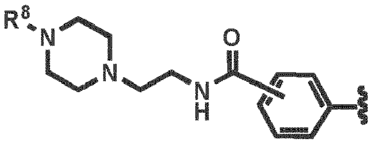, 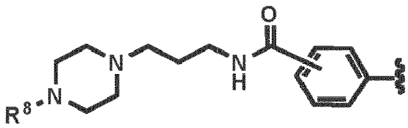, 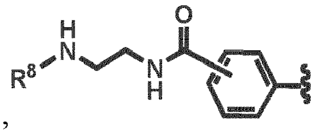,

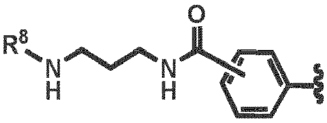, 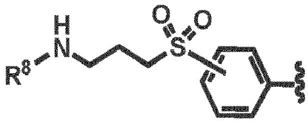, 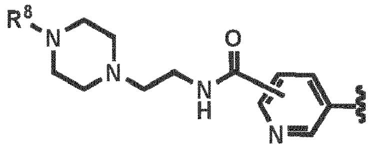,

and

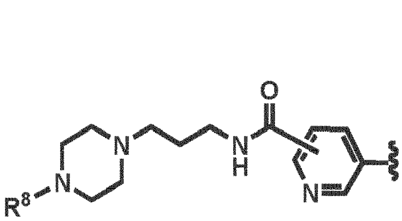, and 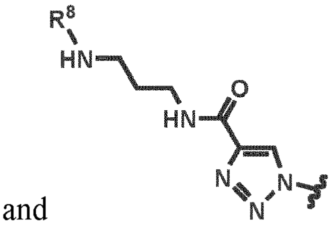.

Embodiment 152. The compound of Embodiment 150, wherein $R^8$-L- is selected from the group consisting of:

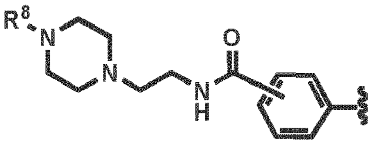, 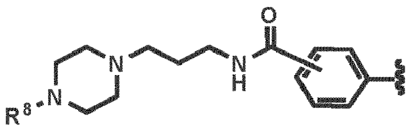, 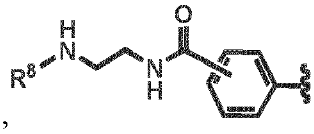,

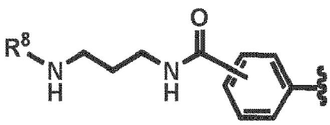, and 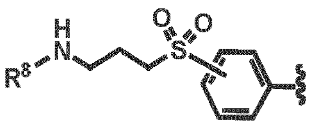.

Embodiment 153. The compound of any one of Embodiments 150, 151, and 152, wherein $R^3$ is selected from the group consisting of C(O)-Het1, $B(OH)_2$, and $-CO-CH_2-OH$.

Embodiment 154. The compound of any one of Embodiments 150, 151,152, and 153, wherein $R^3$ is -C(O)-Het1, and wherein -C(O)-Het1 is selected from the group consisting of:

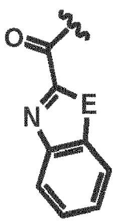, 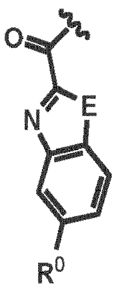, 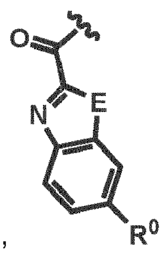, 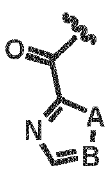, and 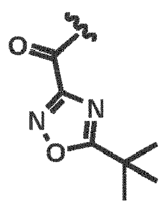,

wherein:

A is selected from the group consisting of O, S, and NH;
B is CH or, if A is NH, B is CH or N;

E is O or S; and
$R^0$ is selected from the group consisting of $-CH_3$, $-O-CH_3$, $-COOCH_3$, F, Cl, and Br.

Embodiment 155. The compound of Embodiments 150, 151, 152, and 153, wherein $R^3$ is -C(O)-Het1, and wherein -C(O)-Het1 is selected from the group consisting of:

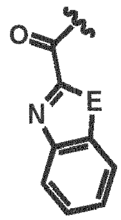, 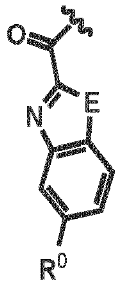, and 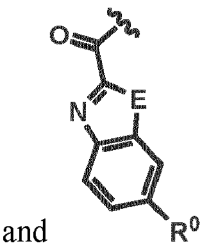,

wherein:

E is O or S; and
$R^0$ is selected from the group consisting of $-CH_3$, $-O-CH_3$, $-COOCH_3$, F, Cl, and Br.

Embodiment 156. The compound of any one of Embodiments 150, 151, 152, and 153, wherein the compound has a structure of formula (II):

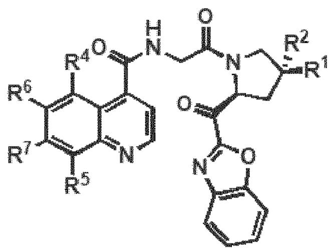

II.

Embodiment 157. The compound of any one of Embodiments 150, 151, 152, 153, 154, 155, and 156, wherein $R^4$ is H or $-CH_3$.

Embodiment 158. The compound of any one of Embodiments 150, 151, 152, 153, 154, 155, 156, and 157, wherein $R^4$ is H.

Embodiment 159. The compound of any one of Embodiments 150, 151,152, 153, 154, 155, 156, and 157, wherein $R^4$ is $-CH_3$.

Embodiment 160. The compound of any one of Embodiments 150, 151, 152, 153, 154, 155, 156, 157, 158, and 159, wherein $R^6$ is $R^8$-L.

Embodiment 160. The compound of Embodiment 160, wherein $R^5$ and $R^7$ are each independently selected from the group consisting of H and $-CH_3$.

Embodiment 162. The compound of Embodiment 161, wherein at least one of $R^5$ and $R^7$ is H.

Embodiment 163. The compound of any one of Embodiments 150, 151, 152, 153, 154, 155, 156, 157, 158, and 159, preferably of any one of Embodiments 158 and 159, wherein $R^7$ is $R^8$-L.

Embodiment 164. The compound of Embodiment 163, wherein $R^5$ and $R^6$ are each independently selected from the group consisting of H and $-CH_3$.

Embodiment 165. The compound of Embodiment 164, wherein at least one of $R^5$ and $R^6$ is H.

Embodiment 166. The compound of any one of Embodiments 150, 151, 152, 153, 154, 155, 156, 157, 158, 159, 160, 161, 162, 163, 164, and 165, wherein $R^8$ is a chelator.

Embodiment 167. The compound of Embodiment 166, wherein the chelator is selected from the group consisting of DOTA, DOTAGA, NOPO, PCTA, NOTA, NODAGA, NODA-MPAA, HBED, TETA, CB-TE2A, DTPA, DFO, Macropa, DOTAM, HOPO, TRAP, THP, DATA, NOTP, sarcophagine, FSC, NETA, H4octapa, Pycup, $N_xS_{4-x}$(N4, N2S2, N3S), Hynic, $^{99m}Tc(CO)_3$-Chelators, more preferably DOTA, DOTAGA, NOPO, PCTA, DOTAM, Macropa, NOTA, NODAGA, NODA-MPAA, HBED, CB-TE2A, DFO, THP, N4 and most preferred DOTA, DOTAGA, NOPO, PCTA, DOTAM, Macropa, NOTA, and NODAGA.

Embodiment 168. The compound of Embodiment 166, wherein the chelator is selected from the group consisting of DOTA, NOPO, PCTA and Macropa.

Embodiment 169. The compound of Embodiment 166, wherein the chelator is DOTA.

Embodiment 170. The compound of Embodiment 166, wherein the chelator is NOPO.

Embodiment 171. The compound of Embodiment 162, wherein the chelator is PCTA.

Embodiment 172. The compound of Embodiment 166, wherein the chelator is Macropa.

Embodiment 173. The compound of any one of Embodiments 168, 169, 170, 171, and 172, preferably Embodiment 169, wherein $R^5$ is

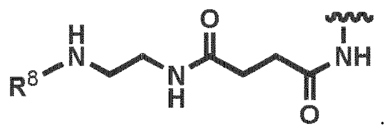

Embodiment 174. The compound of any one of Embodiments 150, 151, 152, 153, 154, 155, 156, 157, 158, 159, 160, 161, 162, 163, 164, and 165, wherein $R^8$ is a cytotoxic agent.

Embodiment 175. The compound of Embodiment 1, wherein the compound is selected from the group consisting of

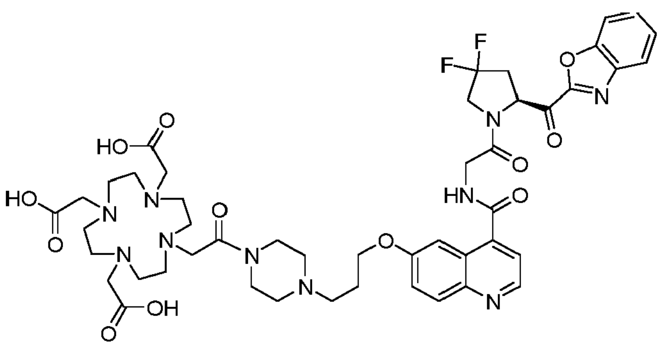

3BP-3412

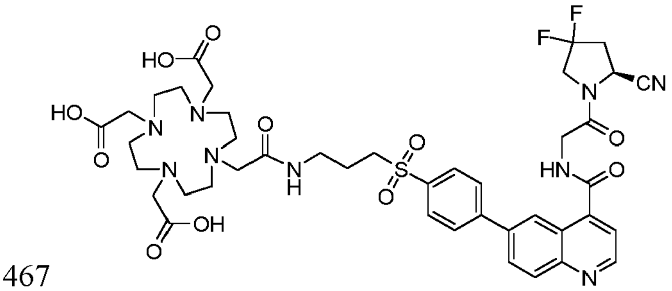

3BP-3467

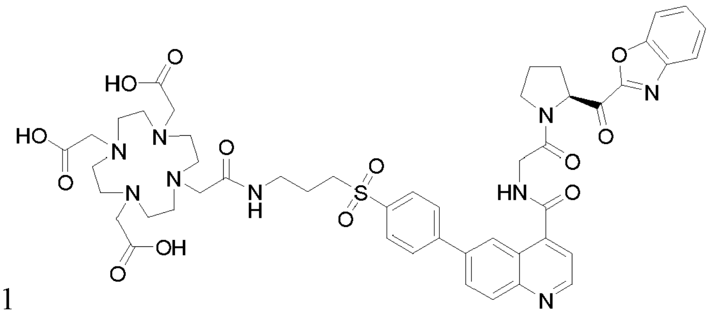

3BP-3581

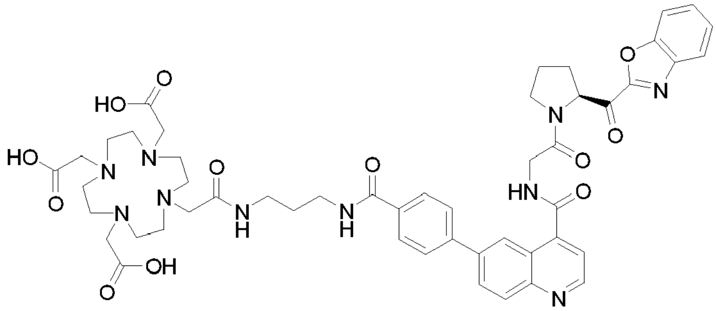

3BP-3582

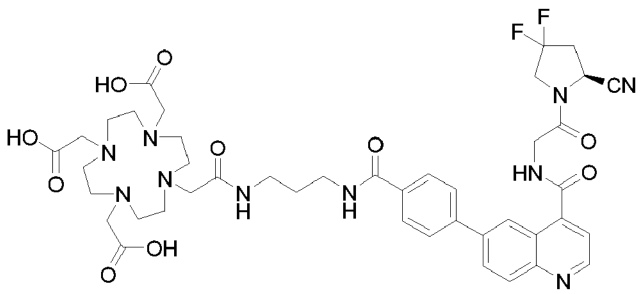

3BP-3621

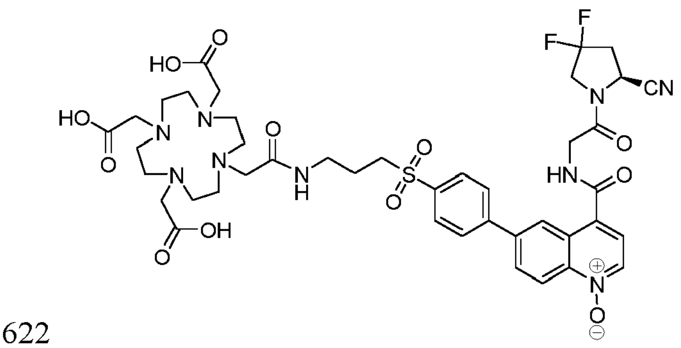

3BP-3622

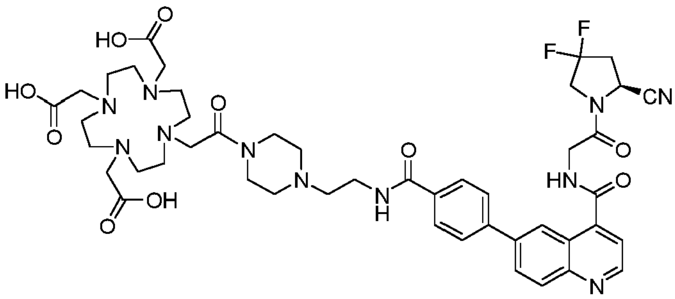

3BP-3631

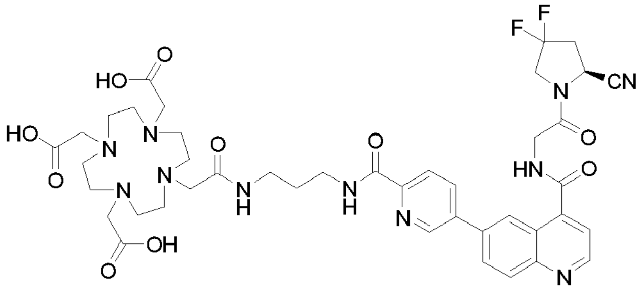

3BP-3772

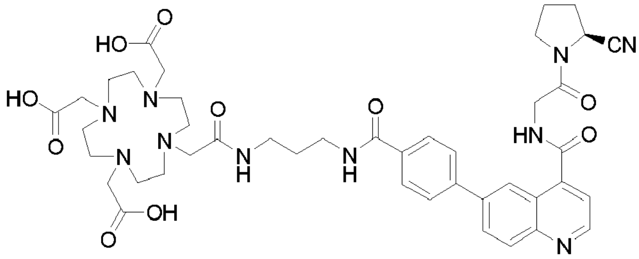

3BP-3785

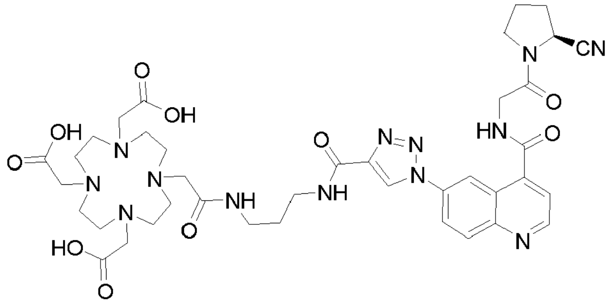

3BP-3951

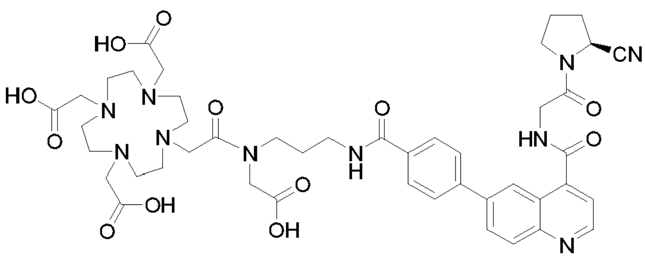

3BP-3954

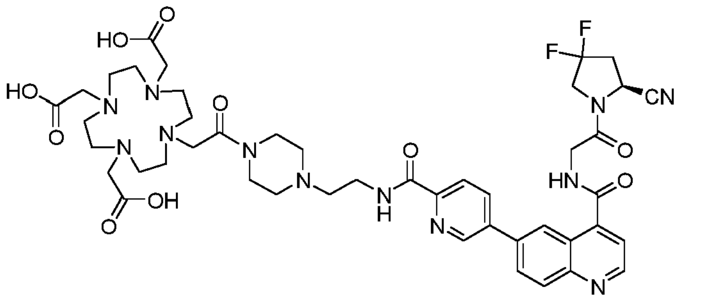

3BP-4076

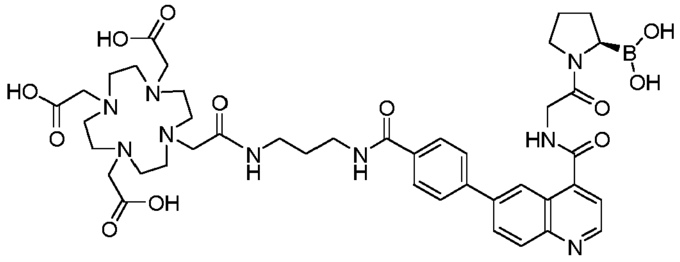

3BP-4201

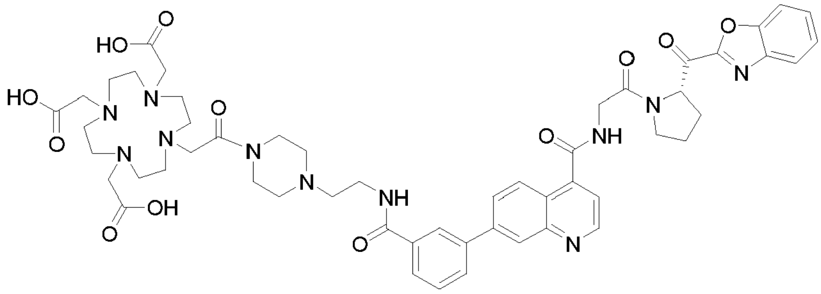

3BP-4663

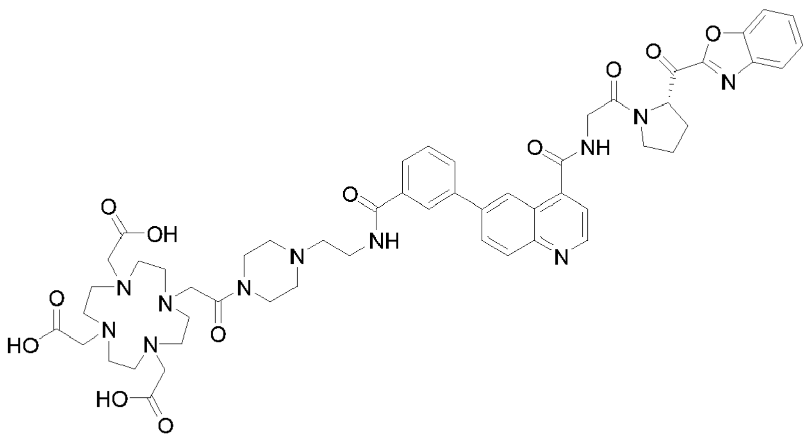

3BP-4664

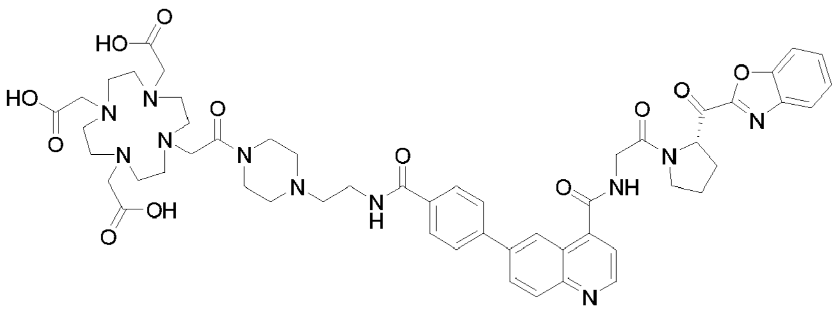

3BP-4665

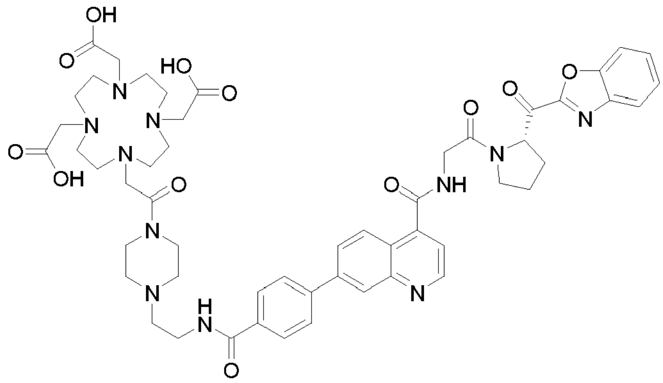

3BP-4694

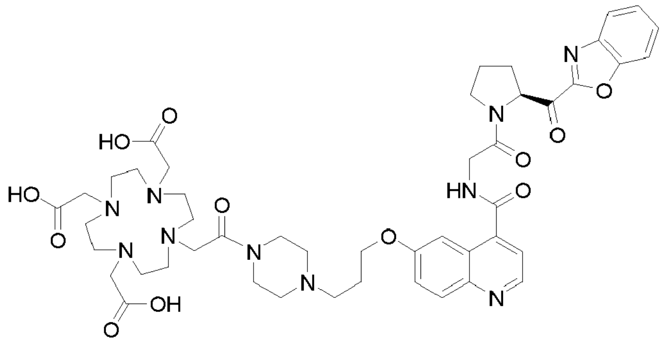

3BP-4808

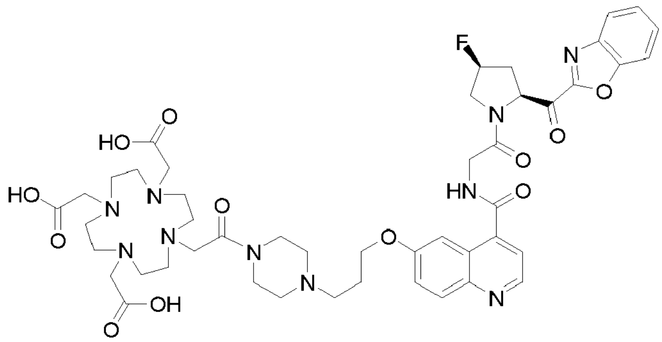

3BP-4809

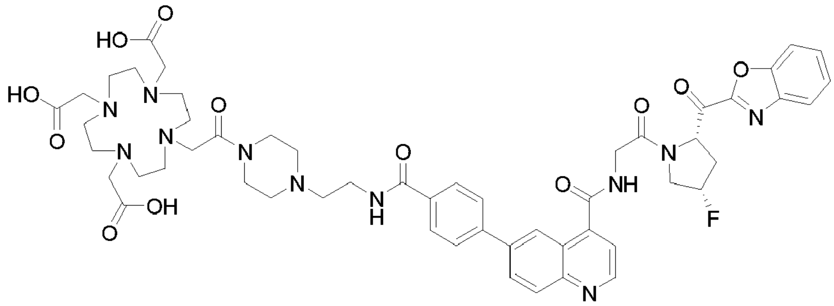

3BP-4810

and

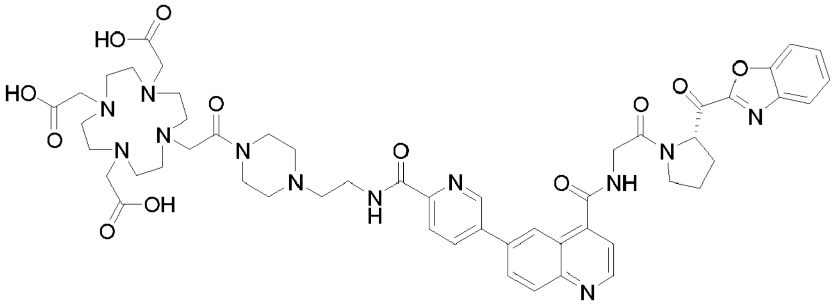

3BP-4811 .

Embodiment 176. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, and 175, wherein the compound comprises a diagnostically active nuclide.

Embodiment 177. The compound of Embodiment 176, wherein the diagnostically active nuclide is a diagnostically active radionuclide.

Embodiment 178. The compound of Embodiment 177, wherein the diagnostically active radionuclide is selected from the group consisting of $^{43}Sc$, $^{44}Sc$, $^{51}Mn$, $^{52}Mn$, $^{64}Cu$, $^{67}Ga$, $^{68}Ga$, $^{86}Y$, $^{89}Zr$, $^{94m}Tc$, $^{99m}Tc$, $^{111}In$, $^{152}Tb$, $^{155}Tb$, $^{177}Lu$ , $^{201}Tl$, $^{203}Pb$, $^{18}F$ $^{76}Br$, $^{77}Br$, $^{123}I$, $^{124}I$, and $^{125}I$.

Embodiment 179. The compound of Embodiment 178, wherein the diagnostically active radionuclide is selected from the group consisting of $^{43}Sc$, $^{44}Sc$, $^{64}Cu$, $^{67}Ga$, $^{68}Ga$, $^{86}Y$,$^{89}Zr$, $^{99m}Tc$, $^{111}In$ $^{152}Tb$, $^{155}Tb$, and $^{203}Pb$.

Embodiment 180. The compound of Embodiment 179 wherein the diagnostically active radionuclide is selected from the group consisting of $^{64}Cu$, $^{68}Ga$, and $^{111}In$.

Embodiment 181. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, and 175, wherein the compound comprises a therapeutically active nuclide.

Embodiment 182. The compound of Embodiment 181, wherein the therapeutically active nuclide is a therapeutically active radionuclide.

Embodiment 183. The compound of Embodiment 182, wherein the therapeutically active radionuclide is selected from the group consisting of $^{47}SC$, $^{67}Cu$, $^{89}Sr$, $^{90}Y$, $^{111}In$, $^{153}Sm$, $^{149}Tb$, $^{161}Tb$, $^{177}Lu$, $^{186}Re$, $^{188}Re$, $^{212}Pb$, $^{213}Bi$, $^{223}Ra$, $^{224}Ra$, $^{225}Ac$, $^{226}Th$, $^{227}Th$, $^{131}I$, and $^{211}At$.

Embodiment 184. The compound of Embodiment 183, wherein the therapeutically active radionuclide is selected from the group consisting of $^{47}Sc$, $^{67}Cu$, $^{90}Y$, $^{177}Lu$, $^{188}Re$, $^{212}Pb$, $^{213}Bi$, $^{225}Ac$, and $^{227}Th$.

Embodiment 185. The compound of Embodiment 184, wherein the therapeutically active radionuclide is selected from the group consisting of $^{90}Y$, $^{177}Lu$, $^{212}Pb$, $^{225}Ac$, and $^{227}Th$.

Embodiment 186. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77,

78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, and 180, for use in a method for diagnosing a disease.

Embodiment 187. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, and 175, 181, 182, 183, 184, and 185 for use in a method for the treatment of a disease.

Embodiment 188. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, and 180, for use in a method for the identification of a subject, wherein the subject is likely to respond or likely not to respond to a treatment of a disease, wherein the method for the identification of a subject comprises carrying out a method of diagnosis using the compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, and 180.

Embodiment 189. The compound for use of Embodiment 188, wherein the method of diagnosis is a method for diagnosing a disease as described in any one of the preceding Embodiments.

Embodiment 190. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, and 180, for use in a method for the selection of a subject from a group of subjects, wherein the subject is likely to respond or likely not to respond to a treatment of a disease, wherein the method for the selection of a subject from a group of subjects comprises carrying out a method of diagnosis using the compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, and 180.

Embodiment 191. The compound for use of Embodiment 190, wherein the method of diagnosis is a method for diagnosing a disease as described in any one of the preceding Embodiments.

Embodiment 192. The compound for use in a method for the stratification of a group of subjects into subjects which

are likely to respond to a treatment of a disease, and into subjects which are not likely to respond to a treatment of a disease, wherein the method for the stratification of a group of subjects comprises carrying out a method of diagnosis using the compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, and 180.

Embodiment 193. The compound for use of Embodiment 192, wherein the method of diagnosis is a method for diagnosing a disease as described in any one of the preceding Embodiments.

Embodiment 194. A composition, preferably a pharmaceutical composition, wherein the composition comprises the compound according to any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, and 175, 181, 182, 183, 184, and 185 and a pharmaceutically acceptable excipient.

Embodiment 195. A kit comprising a compound according to any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, and 185, one or more optional excipient(s), and optionally one or more device(s), wherein the device(s) is/are selected from the group consisting of a labeling device, a purification device, a handling device, a radioprotection device, an analytical device, and an administration device.

It will be acknowledged by a person skilled in the art that a compound of the disclosure is any compound disclosed herein, including but not limited to any compound described in any of the above embodiments and any of the following embodiments.

It will be acknowledged by a person skilled in the art that a composition of the disclosure is any composition disclosed herein, including but not limited to any composition described in any of the above embodiments and any of the following embodiments.

It will be acknowledged by a person skilled in the art that a kit of the disclosure is any kit disclosed herein, including but not limited to any kit described in any of the above embodiments and any of the following embodiments.

The present disclosure provides compounds that can be used for the diagnosis and/or treatment of cancer and other diseases and conditions mediated by fibroblast activation protein (FAP).

The present disclosure provides compounds that can be used for the detection, treatment, and/or management of various diseases associated with FAP-expressing tumors or cells, including cancer and non-oncology diseases.

The compounds of the disclosure provide for highly specific and potent binding to FAP, as well as other desirable properties as described herein.

The compounds of the disclosure have one or more favorable properties, including but not limited to, rapid tumor uptake, prolonged tumor retention, rapid clearance of the compound from non-tumor tissues, improved efficacy, and/or favorable biodistribution properties, with improved toxicity and side effect profiles.

FAP inhibitors of the disclosure exhibit properties for effective clinical utilization, for example, rapid uptake and persistent localization at the target site, with negligible retention in nontargeted tissues.

It will be acknowledged by a person skilled in the art that the expression "aspect of the disclosure" is used synonymously with the term "aspect of the invention" and, respectively, "aspect of the present invention", and that the expression "embodiment of the disclosure" is used synonymously with the term "embodiment of the invention" and,

respectively, "embodiment of the present invention".

Except where otherwise indicated, all numbers expressing quantities of amounts, conditions, and so forth used in the disclosure are to be understood as being modified in all instances by the term "about." Accordingly, unless indicated to the contrary, the numerical parameters set forth in the present disclosure are approximations that may vary depending upon the desired properties sought to be obtained by the present disclosure. At the very least, and not to be considered as an attempt to limit the application of the doctrine of equivalents, each numerical parameter should be construed in light of the number of significant digits and ordinary rounding conventions.

Additionally, the disclosure of numerical ranges within the present disclosure is considered to be a disclosure of all numerical values and ranges within that range. For example, if a range is from 1 to 10, it is deemed to include, for example, 1, 2, 2.2, 3, 4, 5, 6, 7, 7.4, 7.6, 8, 8.7, 9, 9.5, 10, or any other value or range (integer or non-integer) within the range. Moreover, as used herein, the term "at least" includes the stated number, e.g., "at least 50" includes 50.

The expression alkyl as preferably used herein refers each and individually to a saturated, straight-chain or branched hydrocarbon group and is usually accompanied by a qualifier which specifies the number of carbon atoms it may contain. For example the expression $(C_1-C_6)$alkyl means each and individually any of methyl, ethyl, n-propyl, isopropyl, n-butyl, isobutyl, *sec*-butyl, tert-butyl, n-pentyl, 1-methyl-butyl, 1-ethyl-propyl, 3-methyl-butyl, 1,2-dimethylpropyl, 2-methyl-butyl, 1,1-dimethyl-propyl, 2,2-dimethylpropyl, n-hexyl, 1,1-dimethyl-butyl and any other isoform of alkyl groups containing six saturated carbon atoms.

In an embodiment and as preferably used herein, $(C_1-C_2)$alkyl means each and individually any of methyl and ethyl.

In an embodiment and as preferably used herein, $(C_1-C_3)$alkyl means each and individually any of methyl, ethyl, n-propyl and isopropyl.

In an embodiment and as preferably used herein, $(C_1-C_4)$alkyl means each and individually any of methyl, ethyl, n-propyl, isopropyl, n-butyl, isobutyl, sec-butyl and tert-butyl.

In an embodiment and as preferably used herein, $(C_1-C_6)$alkyl means each and individually any of methyl, ethyl, n-propyl, isopropyl, n-butyl, isobutyl, sec-butyl, tert-butyl, n-pentyl, 2-pentyl, 2-methyl-butyl, 3-methyl-butyl, 3-pentyl, 3-methyl-but-2-yl, 2-methyl-but-2-yl, 2,2-dimethylpropyl, n-hexyl, 2-hexyl, 2-methyl-pentyl, 3-methyl-pentyl, 4-methyl-pentyl, 3-hexyl, 2-ethyl-butyl, 2-methyl-pent-2-yl, 2,2-dimethyl-butyl, 3,3-dimethyl-butyl, 3-methyl-pent-2-yl, 4-methyl-pent-2-yl, 2,3-dimethyl-butyl, 3-methyl-pent-3-yl, 2-methyl-pent-3-yl, 2,3-dimethylbut-2-yl and 3,3-dimethyl-but-2-yl.

In an embodiment, and as preferably used herein, "$(C_1-C_8)$alkyl" refers to a saturated or unsaturated, straight-chain or branched hydrocarbon group having from 1 to 8 carbon atoms. Representative $(C_1-C_8)$alkyl groups include, but are not limited to, any of methyl, ethyl, n-propyl, isopropyl, n-butyl, isobutyl, sec-butyl, tert-butyl, n-pentyl, 2-pentyl, 2-methyl-butyl, 3-methyl-butyl, 3-pentyl, 3-methyl-but-2-yl, 2-methyl-but-2-yl, 2,2-dimethylpropyl, n-hexyl, 2-hexyl, 2-methyl-pentyl, 3-methyl-pentyl, 4-methyl-pentyl, 3-hexyl, 2-ethyl-butyl, 2-methylpent-2-yl, 2,2-dimethyl-butyl, 3,3-dimethyl-butyl, 3-methyl-pent-2-yl, 4-methyl-pent-2-yl, 2,3-dimethyl-butyl, 3-methyl-pent-3-yl, 2-methyl-pent-3-yl, 2,3-dimethyl-but-2-yl, 3,3-dimethyl-but-2-yl, n-heptyl, 2-heptyl, 2-methyl-hexyl, 3-methyl-hexyl, 4-methyl-hexyl, 5-methyl-hexyl, 3-heptyl, 2-ethyl-pentyl, 3-ethyl-pentyl, 4-heptyl, 2-methyl-hex-2-yl, 2,2-dimetyhl-pentyl, 3,3-dimetyhl-pentyl, 4,4-dimetyhl-pentyl, 3-methyl-hex-2-yl, 4-methyl-hex-2-yl, 5-methyl-hex-2-yl, 2,3-dimethyl-pentyl, 2,4-dimethyl-pentyl, 3,4-dimethyl-pentyl, 3-methyl-hex-3-yl, 2-ethyl-2-methyl-butyl, 4-methyl-hex-3-yl, 5-methyl-hex-3-yl, 2-ethyl-3-methyl-butyl, 2,3-dimethyl-pent-2-yl, 2,4-dimethyl-pent-2-yl, 3,3-dimethyl-pent-2-yl, 4,4-dimethyl-pent-2-yl, 2,2,3-trimethyl-butyl, 2,3,3-trimethyl-butyl, 2,3,3-trimethyl-but-2-yl, n-octyl, 2-octyl, 2-methyl-heptyl, 3-methyl-heptyl, 4-methyl-heptyl, 5-methyl-heptyl, 6-methyl-heptyl, 3-octyl, 2-ethyl-hexyl, 3-ethyl-hexyl, 4-ethyl-hexyl, 4-octyl, 2-propyl-pentyl, 2-methyl-hept-2-yl, 2,2-dimethyl-hexyl, 3,3-dimethyl-hexyl, 4,4-dimethyl-hexyl, 5,5-dimethyl-hexyl, 3-methyl-hept-2-yl, 4-methyl-hept-2-yl, 5-methyl-hept-2-yl, 6-methyl-hept-2-yl, 2,3-dimethyl-hex-1-yl, 2,4-dimethyl-hex-1-yl, 2,5-dimethyl-hex-1-yl, 3,4-dimethyl-hex-1-yl, 3,5-dimethyl-hex-1-yl, 3,5-dimethyl-hex-1-yl, 3-methyl-hept-3-yl, 2-ethyl-2-methyl-1-yl, 3-ethyl-3-methyl-1-yl, 4-methyl-hept-3-yl, 5-methyl-hept-3-yl, 6-methyl-hept-3-yl, 2-ethyl-3-methyl-pentyl, 2-ethyl-4-methyl-pentyl, 3-ethyl-4-methyl-pentyl, 2,3-dimethyl-hex-2-yl, 2,4-dimethyl-hex-2-yl, 2,5-dimethyl-hex-2-yl, 3,3-dimethyl-hex-2-yl, 3,4-dimethyl-hex-2-yl, 3,5-dimethyl-hex-2-yl, 4,4-dimethyl-hex-2-yl, 4,5-dimethyl-hex-2-yl, 5,5-dimethyl-hex-2-yl, 2,2,3-trimethyl-pentyl, 2,2,4-trimethyl-pentyl, 2,3,3-trimethyl-pentyl, 2,3,4-trimethyl-pentyl, 2,4,4-trimethyl-pentyl, 3,3,4-trimethyl-pentyl, 3,4,4-trimethyl-pentyl, 2,3,3-trimethyl-pent-2-yl, 2,3,4-trimethyl-pent-2-yl, 2,4,4-trimethyl-pent-2-yl, 3,4,4-trimethyl-pent-2-yl, 2,2,3,3-tetramethyl-butyl, 3,4-dimethyl-hex-3-yl, 3,5-dimethyl-hex-3-yl, 4,4-dimethyl-hex-3-yl, 4,5-dimethyl-hex-3-yl, 5,5-dimethyl-hex-3-yl, 3-ethyl-3-methyl-pent-2-yl, 3-ethyl-4-methyl-pent-2-yl, 3-ethyl-hex-3-yl, 2,2-diethyl-butyl, 3-ethyl-3-methyl-pentyl, 4-ethyl-hex-3-yl, 5-methylhept-3-yl, 2-ethyl-3-methyl-pentyl, 4-methyl-hept-4-yl, 3-methyl-hept-4-yl, 2-methyl-hept-4-yl, 3-ethyl-hex-2-yl, 2-ethyl-2-methyl-pentyl, 2-isopropyl-pentyl, 2,2-dimethyl-hex-3-yl, 2,2,4-trimethyl-pent-3-yl and 2-ethyl-3-methyl-pentyl. A $(C_1-C_8)$alkyl group can be unsubstituted or substituted with one or more groups, including, but not limited to, $(C_1-C_8)$alkyl, -O-[$(C_1-C_8)$alkyl], -aryl, -CO-R', -O-CO-R', -CO-OR', $-CO-NH_2$, -CO-NHR', $-CO-NR'_2$, -NH-CO-R', $-SO_2-R'$, -SO-R', -OH, -halogen, $-N_3$, $-NH_2$, -NHR', $-NR'_2$ and -CN; where each R' is independently selected from $-(C_1-C_8)$alkyl and aryl.

In an embodiment, and as preferably used herein, -O-($C_1$-$C_6$)alkyl refers each and individually to an ether oxygen atom to which a -($C_1$-$C_6$)alkyl moiety as defined above is bound, for example, methoxy, ethoxy, n-propoxy, isopropoxy, n-butoxy, isobutoxy, sec-butoxy, tertbutoxy, n-pentoxy, 2-pentoxy, 2-methyl-butoxy, 3-methyl-butoxy, 3-pentoxy, 3-methyl-but-2-oxy, 2-methyl-but-2-oxy, 2,2-dimethylpropoxy, n-hexoxy, 2-methyl-pentoxy, 3-methyl-pentoxy, 4-methyl-pentoxy, 3-hexoxy, 2-ethyl-butoxy, 2-methyl-pent-2-oxy, 2,2-dimethyl-butoxy, 3,3-dimethyl-butoxy, 3-methyl-pent-2-oxy, 4-methyl-pent-2-oxy, 2,3-dimethyl-butoxy, 3-methyl-pent-3-oxy, 2-methyl-pent-3-oxy, 2,3-dimethyl-but-2-oxy, 3,3-dimethylbut-2-oxy.

In an embodiment, and as preferably used herein, -O-($C_1$-$C_4$)alkyl refers each and individually to an ether oxygen atom to which a -($C_1$-$C_4$)alkyl moiety as defined above is bound, for example, methoxy, ethoxy, n-propoxy, isopropoxy, n-butoxy, isobutoxy, sec-butoxy, tertbutoxy.

In an embodiment, and as preferably used herein, -COO-($C_1$-$C_4$)alkyl refers each and individually to an ester group comprising a -($C_1$-$C_4$)alkyl moiety as defined above, for example methyl ester, ethyl ester, propyl ester, or butyl ester.

The expression alkylidene as preferably used herein refers to a saturated straight chain or branched hydrocarbon group wherein two points of substitution are specified. Simple alkyl chains wherein the two points of substitutions are in a maximal distance to each other like methane-1,1-diyl, ethane-1,2-diyl, propane-1,3-diyl, butane-1,4-diyl and pentane-1,5-diyl are also referred to as methylene (which is also referred to as methane-1,1-diyl), ethylene (which is also referred to as ethane-1,2-diyl), propylene (which is also referred to as propane-1,3-diyl), butylene (which is also referred to as butane-1,4-diyl) and pentylene (which is also referred to as pentane-1,5-diyl).

In an embodiment and as preferably used herein, ($C_1$-$C_{10}$)alkylidene means each and individually any of methylene, ethane-1,2-diyl, propane-1,3-diyl, propane-1,2-diyl, butane-1,4-diyl, butane-1,3-diyl, butane-1,2-diyl, 2-methyl-propane-1,2-diyl, 2-methyl-propane-1,3-diyl, pentane-1,5-diyl, pentane-1,4-diyl, pentane-1,3-diyl, pentane-1,2-diyl, pentane-2,3-diyl, pentane-2,4-diyl, any other isomer with 5 carbon atoms, hexane-1,6-diyl, any other isomer with 6 carbon atoms, heptane-1,7-diyl, any other isomer with 7 carbon atoms, octane-1,8-diyl, any other isomer with 8 carbon atoms, nonane-1,9-diyl, any other isomer with 9 carbon atoms, decane-1,10-diyl and any other isomer with 10 carbon atoms, preferably ($C_1$-$C_{10}$) alkylidene means each and individually any of methylene, ethane-1,2-diyl, propane-1,3-diyl, butane-1,4-diyl, pentane-1,5-diyl, hexane-1,6-diyl, heptane-1,7-diyl, octane-1,8-diyl, nonane-1,9-diyl and decane-1,10-diyl. In an embodiment, and as preferably used herein, ($C_2$-$C_4$) alkylidene means each and individually any of methylene, ethane-1,2-diyl, propane-1,3-diyl, propane-1,2-diyl, butane-1,4-diyl, butane-1,3-diyl, butane-1,2-diyl, 2-methyl-propane-1,2-diyl, 2-methylpropane-1,3-diyl. A ($C_1$-$C_{10}$)alkylidene group can be unsubstituted or substituted with one or more groups, including, but not limited to, ($C_1$-$C_8$)alkyl, -O-[($C_1$-$C_8$)alkyl], -aryl, -CO-R', -O-CO-R', -CO-OR', $-CO-NH_2$, -CO-NHR', -CO-NR'$_2$, -NH-CO-R', $-SO_2$-R', -SO-R', -OH, - halogen, $-N_3$, $-NH_2$, -NHR', -NR'$_2$ and -CN; where each R' is independently selected from - ($C_1$-$C_8$)alkyl and aryl.

In an embodiment, and as preferably used herein, -O-($C_1$-$C_3$)alkylidene-($C_6$)aryl, refers to for example, -O-methylene-phenyl, -O-ethylene-phenyl, -O-propylene-phenyl.

In an embodiment, and as preferably used herein, -($C_1$-$C_4$)alkylidene-C(O)NH- refers to for example, methylene-C(O)NH-, -ethylene-C(O)NH-, -propylene-C(O)NH-, and -butylene-C(O)NH-.

In an embodiment, and as preferably used herein, "carbocycle" refers to a saturated, unsaturated or aromatic mono- or bicyclic carbocyclic ring. A carbocycle can be unsubstituted or substituted with one or more groups, including, but not limited to, ($C_1$-$C_8$)alkyl, -O-[($C_1$-$C_8$)alkyl], -aryl, -CO-R', -O-CO-R', -CO-OR', $-CO-NH_2$, -CO-NHR', -CO-NR'$_2$, -NH-CO-R', $-SO_2$-R', -SO-R', -OH, -halogen, $-N_3$, $-NH_2$, -NHR', -NR'$_2$ and -CN; where each R' is independently selected from -($C_1$-$C_8$)alkyl and aryl.

In an embodiment, and as preferably used herein, "heterocycle" refers to a saturated, unsaturated or aromatic mono- or bicyclic heterocyclic ring. A heterocycle group can be unsubstituted or substituted with one or more groups, including, but not limited to, ($C_1$-$C_8$)alkyl, -O-[($C_1$-$C_8$)alkyl], -aryl, -CO-R', -O-CO-R', -CO-OR', $-CO-NH_2$, -CO-NHR', -CO-NR'$_2$, -NH-CO-R', $-SO_2$-R', -SO-R', -OH, -halogen, $-N_3$, $-NH_2$, -NHR', -NR'$_2$ and -CN; where each R' is independently selected from -($C_1$-$C_8$)alkyl and aryl.

In an embodiment, and as preferably used herein, "aryl" refers to a carbocyclic aromatic group. Examples of aryl groups include, but are not limited to, phenyl, naphthyl, and anthracenyl.

In an embodiment and as preferably used herein, ($C_5$-$C_6$)aryl refers to a 5 or 6 carbon carbocyclic aromatic group. In an embodiment, and as preferably used herein, ($C_6$)aryl refers to a 6 carbon carbocyclic aromatic group. A carbocyclic aromatic group can be unsubstituted or substituted with one or more groups including, but not limited to, -($C_1$-$C_8$)alkyl, -O-[($C_1$-$C_8$)alkyl], -aryl, -CO-R', -O-CO-R', -CO-OR', $-CO-NH_2$, -CO-NHR', -CO-NR'$_2$, -NH-CO-R', $-SO_2$-R', -SO-R', -OH, -halogen, $-N_3$, $-NH_2$, -NHR', -NR'$_2$ and -CN; where each R' is independently selected from -($C_1$-$C_8$)alkyl and aryl.

In an embodiment, and as preferably used herein, "heteroaryl" refers to a heterocyclic aromatic group. Examples of heteroaryl groups include, but are not limited to, furane, thiophene, pyridine, pyrimidine, benzothiophene, benzofurane, and quinoline.

In an embodiment, and as preferably used herein, "($C_5$-$C_6$)heteroaryl" refers to a heterocyclic aromatic group consisting of 5 or 6 ring atoms wherein at least one atom is different from carbon, including, for example, nitrogen, sulfur or oxygen. A heterocyclic aromatic group can be unsubstituted or substituted with one or more groups including, but not limited to, -($C_1$-$C_8$)alkyl, -O-[($C_1$-$C_8$)alkyl], -aryl, -CO-R', -O-CO-R', -CO-OR', -CO-$NH_2$, -CO-NHR', -CO-$NR'_2$, -NH-CO-R', -$SO_2$-R', -SO-R', -OH, -halogen, -$N_3$, -$NH_2$, -NHR', -$NR'_2$ and -CN; where each R' is independently selected from -($C_1$-$C_8$)alkyl and aryl.

In an embodiment, and as preferably used herein, atoms with unspecified atomic mass numbers in any structural formula or in any passage of the instant specification are either of unspecified isotopic composition, naturally occurring mixtures of isotopes or individual isotopes. This applies in particular to carbon, oxygen, nitrogen, sulfur, phosphorus, halogens and metal atoms, including but not limited to C, O, N, S, F, P, Cl, Br, At, Sc, Cr, Mn, Co, Fe, Cu, Ga, Sr, Zr, Y, Mo, Tc, Ru, Rh, Pd, Pt, Ag, In, Sb, Sn, Te, I, Pr, Pm, Dy, Sm, Gd, Tb, Ho, Dy, Er, Yb, Tm, Lu, Sn, Re, Rd, Os, Ir, Au, Pb, Bi, Po, Fr, Ra, Ac, Th, and Fm.

In an embodiment, and as preferably used herein, the term "effector domain" refers to a chelator or an effector.

In an embodiment, and as preferably used herein, an "effector" refers to an active agent that inhibits or prevents a cellular function and/or causes cell death or destruction. Effectors include, but are not limited to the following active agents: theragnostically active agents, diagnostically active agents, therapeutically active agents, theragnostically active nuclides, diagnostically active nuclides, therapeutically active nuclides, theragnostically active radionuclides, diagnostically active radionuclide, therapeutically active radionuclide, radioactive isotopes, and cytotoxic agents.

In an embodiment, and as preferably used herein, a "cytotoxic agent" is an agent that damages and/or kills cells, including cancer cells. Cytotoxic agents include, but are not limited to: chemotherapeutic agents or drugs, growth inhibitory agents, enzymes and fragments thereof such as nucleolytic enzymes, antibiotics, toxins such as small molecule toxins or enzymatically active toxins of bacterial, fungal, plant or animal origin, including fragments and/or variants thereof, and the various antitumor or anticancer agents disclosed below. In an embodiment, a radioisotope can be a cytotoxic agent. In an embodiment, and as preferably used herein, an "amine reactive" derivative of a cytotoxic agent is utilized for preparing the respective compounds of the invention. Amine reactive derivatives are preferably comprising at least one functional group which includes, but are not limited to, carboxylic acid, activated carboxylic acid, isocyanate or isothiocyanate.

In an embodiment, and as preferably used herein, a "chelator" is a compound, which is capable of forming a chelate, whereby a chelate is a compound, including, for example, a cyclic compound where a metal or a moiety having an electron gap or a lone pair of electrons participates in the formation of the ring. In certain embodiments, a chelator is this kind of compound where a single ligand occupies more than one coordination site at a central atom. Chelator refers to the un-complexed chelator, or the chelator complexed to any metal complex partner, i.e. any metal which, in principle, can be complexed by the chelator. This metal complex partner is any radioactive or non-radioactive metal complex partner. Examples of the metal complex partner include, but are not limited to, theragnostically active nuclides, diagnostically active nuclides, therapeutically active nuclides, theragnostically active radionuclides, diagnostically active radionuclides, therapeutically active radionuclides, and radioactive isotopes.

In an embodiment, and as preferably used herein, a "diagnostically active compound" is a compound which is suitable for or useful in at least the diagnosis of a disease.

In an embodiment, and as preferably used herein, a "diagnostic agent" or a "diagnostically active agent" is a compound, which is suitable for or useful in at least the diagnosis of a disease.

In an embodiment, and as preferably used herein, a "diagnostically active radionuclide" is a radionuclide, which is suitable for or useful in at least the diagnosis of a disease. It will, however, also be acknowledged by a person skilled in the art that the use of said diagnostically active radionuclide may not be limited to diagnostic purposes, but can encompass their use in therapy and theragnostics.

In an embodiment, and as preferably used herein, a "therapeutically active compound" is a compound, which is suitable for or useful in at least the treatment of a disease.

In an embodiment, and as preferably used herein, a "therapeutic agent" or a "therapeutically active agent" is a compound which is suitable for or useful in at least the treatment of a disease.

In an embodiment, and as preferably used herein, a "therapeutically active radionuclide" is a radionuclide which is suitable for or useful in at least the treatment of a disease. It will, however, also be acknowledged by a person skilled in the art that the use of said therapeutically active radionuclide may not be limited to therapeutical purposes, but can encompass their use in diagnosis and theragnostics.

In an embodiment, and as preferably used herein, a "theragnostically active compound" is a compound, which is suitable for or useful in both the diagnosis and therapy of a disease.

In an embodiment, and as preferably used herein, a "theragnostic agent" or a "theragnostically active agent" is a compound which is suitable for or useful in both the diagnosis and therapy of a disease.

In an embodiment, and as preferably used herein, a "theragnostically active radionuclide" is a radionuclide, which is suitable for or useful in both the diagnosis and therapy of a disease.

In an embodiment, and as preferably used herein, "theragnostics" is a method for the combined diagnosis and therapy of a disease. In certain embodiments, the combined diagnostically and therapeutically active compounds used in theragnostics are radiolabeled.

In an embodiment, and as preferably used herein, "treatment of a disease" is treatment and/or prevention of a disease.

In an embodiment, and as preferably used herein, the terms "treat," "treating," and "treatment" are meant to include alleviating or abrogating a disorder, disease, or condition; or one or more of the symptoms associated with the disorder, disease, or condition; or alleviating or eradicating the cause(s) of the disorder, disease, or condition itself.

In an embodiment, and as preferably used herein, "preventing" or "prevent" describes reducing or eliminating the onset of the symptoms or complications of the disease, condition or disorder.

In an embodiment, and as preferably used herein, the term "subject" or "patient" includes a mammal. The mammal can be, e.g., any mammal, e.g., a human, companion animal, pet, livestock, dog, cat, horse, and cow.

In an embodiment, and as preferably used herein, a "disease involving the FAP protein" is a disease involving cells showing upregulated expression of FAP, which are a or the cause for the disease and/or the symptoms of the disease, or are part of the pathology underlying the disease.

In an embodiment and as preferably used herein, a disease involving FAP is a disease where cells including but not limited to fibroblasts expressing, preferably in an upregulated manner, FAP and tissue either expressing FAP or containing or comprising cells such as fibroblasts, preferably expressing FAP in an upregulated manner respectively, are either a or the cause for the disease and/or the symptoms of the disease, or are part of the pathology underlying the disease. A preferred FAP-expressing cell is a cancer associated fibroblast (CAF). In an embodiment of the disease, preferably when used in connection with the treatment, treating and/or therapy of the disease, affecting the cells, the tissue and pathology, respectively, results in cure, treatment or amelioration of the disease and/or the symptoms of the disease. In an embodiment of the disease, preferably when used in connection with the diagnosis and/or diagnosing of the disease, labeling of the FAP-expressing cells and/or of the FAP-expressing tissue allows discriminating or distinguishing said cells and/or said tissue from healthy or FAP-non-expressing cells and/or healthy or FAP non-expressing tissue. More preferably such discrimination or distinction forms the basis for said diagnosis and diagnosing, respectively. In an embodiment thereof, labeling means the interaction of a detectable label either directly or indirectly with the FAP-expressing cells and/or with the FAP-expressing tissue or tissue containing such FAP-expressing cells; more preferably such interaction involves or is based on the interaction of the label or a compound bearing such label with FAP.

In an embodiment, and as preferably used herein, a "target cell" or "target tissue" is a cell or tissue, which is expressing FAP and is a or the cause for a disease and/or the symptoms of a disease, or is part of the pathology underlying a disease.

In an embodiment, and as preferably used herein, a "non-target cell" or "non-target tissue" is a cell or tissue, which is either not expressing FAP and/or is not a or the cause for a disease and/or the symptoms of a disease, or is part of the pathology underlying a disease.

In an embodiment, and as preferably used herein, a "neoplasm" is an abnormal new growth of cells. The cells in a neoplasm grow more rapidly than normal cells and will continue to grow if not treated. A neoplasm may be benign or malignant.

In an embodiment, and as preferably used herein, a "tumor" is a mass lesion that may be benign or malignant.

In an embodiment, and as preferably used herein, a "cancer" is a malignant neoplasm.

In an embodiment, and as preferably used herein, a "pharmaceutically acceptable excipient" refers to an ingredient other than the active agent(s) and/or compound(s) that is suitable for use in a pharmaceutical composition, including, but not limited to, pharmaceutically acceptable adjuvants, diluents, carriers, buffers, binders, colorants, lubricants, fillers, disintegrants, preservatives, surfactants, and stabilizers.

In an embodiment, and as preferably used herein, a "linkage" is an attachment of two atoms of two independent moieties. A preferred linkage is a chemical bond or a plurality of chemical bonds. Preferably a chemical bond is a covalent bond or a plurality of chemical bonds. Preferably the linkage is a covalent bond or a coordinate bond. As preferably used herein, an embodiment of a coordinate bond is a bond or group of bonds as realized when a metal is bound by a chelator. Depending on the type of atoms linked and their atomic environment different types of linkages are created. These types of linkage are defined by the type of atom arrangements created by the linkage. For instance, the linking of a moiety comprising an amine with a moiety comprising a carboxylic acid leads to a linkage named amide (which is also referred to as amide linkage, -CO-N-, -N-CO-). It will be acknowledged by a person skilled in the art that this and the following examples of creating linkages are only prototypical examples and are by no means limiting the scope of the instant application. It will be acknowledged by a person in the art that the linking of a moiety comprising an isothiocyanate with a moiety comprising an amine leads to thiourea (which is also referred to as a thiourea linkage, -N-CS-N-), and linking of a moiety comprising a C atom with a moiety comprising a thiol-group (-C-SH) leads to thioether (which is also referred to as a thioether linkage, -C-S-C). A non-limiting list of examples of linkages used in connection with the chelator and linker of the disclosure and their characteristic type of atom arrangement is presented Table 1.

Table 1:

| Linkage | Characteristic atom arrangement |
|---|---|
| Amide | 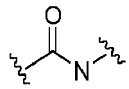 |
| Sulfonamide | 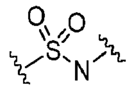 |
| Urea | 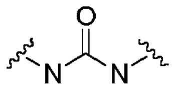 |
| Thioether | 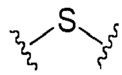 |
| Disulfide | 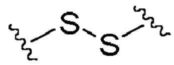 |
| Carbamate | 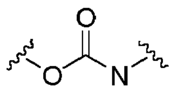 |
| Thiourea | 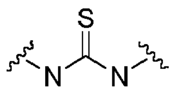 |
| Triazole | 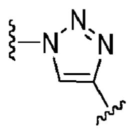 |

Examples of reactive groups which, in some embodiments of the disclosure, are used in the formation of linkages between the chelator and the rest of the molecule. It will, however, be understood by a person skilled in the art that neither the linkages nor the reactive groups forming such linkages for the formation of the compounds of the disclosure are limited to the ones of Table 2.

Table 2:

| first reactive group | second reactive group | (type of) linkage |
|---|---|---|
| amino | carboxylic acid | amide |
| amino | activated carboxylic acid | amide |
| carboxylic acid | amino | amide |
| sulfhydryl | Michael acceptor (e.g., Maleimide) | thioether |
| bromo | sulfhydryl | thioether |
| isothiocyanate | amino | thiourea |
| azide | alkyne | triazole |
| isocyanate | amino | carbamate |

In an embodiment, and as preferably used herein, the term "activated carboxylic acid" refers to a carboxylic acid group with the general formula -CO-X, wherein X is a leaving group. For example, activated forms of a carboxylic acid group may include, but are not limited to, acyl chlorides, symmetrical or unsymmetrical anhydrides, and esters. In

some embodiments, the activated carboxylic acid group is an ester with pentafluorophenol, nitrophenol, benzotriazole, azabenzotriazole, thiophenol or N-hydroxysuccinimide (NHS) as leaving group.

In an embodiment, and as preferably used herein, the term "mediating a linkage" means that a linkage or a type of linkage is established, preferably a linkage between two moieties.

Those skilled in the art will recognize if a stereocenter exists in the compounds disclosed herein. Accordingly, the present disclosure includes possible stereoisomers and includes not only racemic compounds but the individual enantiomers and/or diastereomers as well. When a compound is desired as a single enantiomer or diastereomer, it may be obtained by stereospecific synthesis or by resolution of the final product or any convenient intermediate. Resolution of the final product, an intermediate, or a starting material may be affected by any suitable method known in the art. See, for example, "Stereochemistry of Organic Compounds" by E. L. Eliel, S. H. Wilen, and L. N. Mander (Wiley-Interscience, 1994).

In the present disclosure, the structural formula of the compound represents a certain isomer for convenience in some cases, but the present disclosure includes all isomers, such as geometrical isomers, optical isomers based on an asymmetrical carbon, stereoisomers, tautomers, and the like.

In an embodiment, the compound of the disclosure comprises a chelator. In some embodiments, the chelator forms metal chelates, for example, comprising at least one radioactive metal. The at least one radioactive metal is, for example, useful in or suitable for diagnostic and/or therapeutic and/or theragnostic use and is, for example, useful in or suitable for imaging and/or radiotherapy.

Chelators in principle useful in and/or suitable for the practicing of the instant disclosure, including diagnosis and/or therapy of a disease, are known to the person skilled in the art. A wide variety of respective chelators is available and has been reviewed, e.g., by Banerjee et al. (Banerjee, et al., Dalton Trans, 2005, 24: 3886), and references therein (Price, et al., Chem Soc Rev, 2014, 43: 260; Wadas, et al., Chem Rev, 2010, 110: 2858). Such chelators include, but are not limited to linear, cyclic, macrocyclic, tetrapyridine, N3S, N2S2 and N4 chelators as disclosed in US 5,367,080 A; US 5,364,613 A; US, 5,021,556 A; US 5,075,099 A; and US 5,886,142 A.

In some embodiments, the metal chelator is capable of binding a radioactive nuclide. The binding can be direct, e.g., the metal chelator can make ionic, covalent, dipolar, or ion-dipole interactions with the radioactive atom. The binding can also be indirect, e.g., the metal chelator binds to a molecule that comprises a radioactive atom. In some embodiments, the metal chelator comprises, or is, a macrocycle. In some embodiments, the metal chelator comprises, or is, DOTA or NOTA. In some embodiments, the metal chelator comprises a macrocycle, e.g., a macrocycle comprising an O and/or a N, DOTA, NOTA, one or more amines, one or more ethers, one or more carboxylic acids, EDTA, DTPA, TETA, DO3A, PCTA, or desferrioxamine.

In some embodiments, the metal chelator comprises a plurality of amines. In some embodiments, the metal chelator includes 4 or more N, 4 or more carboxylic acid groups, or a combination thereof. In some embodiments, the metal chelator does not comprise S. In some embodiments, the metal chelator comprises a ring. In some embodiments, the ring comprises an O and/or an N. In some embodiments, the metal chelator is a ring that includes 3 or more N, 3 or more carboxylic acid groups, or a combination thereof. In some embodiments, the metal chelator is polydentate.

Representative chelating agents and their derivatives include, but are not limited to AAZTA, BAT, CDTA, DTA, CyEDTA, EDTMP, DTPMP, DTPA, CyDTPA, Cy2DTPA, DTPA-MA, DTPA-BA, BOPA, NTA, NOC, NOTP, CY-DTA, DTCBP, CTA, cyclam, CB-Cyclam, cyclen, TETA, sarcophagine, CPTA, TEAMA, Cyclen, DO3A, DO2A, TRITA, DATA, DFO, DATA(M), DATA(P), DATA(Ph), DATA(PPh), DEDPA, $H_4$octapa, $H_2$dedpa, $H_5$decapa, $H_2$azapa, H2CHX-DEDPA, DFO-Chx-MAL, DFO-p-SCN, DFO-1AC, DFO-BAC, p-SCN-Bn-DFO, DFO-pPhe-NCS, DFO-HOPO, DFC, diphosphine, DOTA, DOTAGA, DOTA-MFCO, DOTAM, DOTAM-mono-acid, DOTA-MA, DOTA-pNB, DOTA-4AMP, nitro-DOTA, nitro-PA-DOTA, p-NCS-Bz-DOTA, PA-DOTA, DOTA-NCS, DOTA-NHS, CB-DO2A, PCTA, p-$NH_2$-Bn-PCTA, p-SCN-Bn-PCTA, p-SCN-Bn-DOTA, DOTMA, NB-DOTA, H4NB-DOTA, H4TCE-DOTA, HOPO, 3,4,3-(Li-1,2-HOPO), TREN(Me-3,2-HOPO), TCE-DOTA, DOTP, DOTMP, DOTEP, DOTMPE, F-DOTPME, DOTPP, DOTBzP, DOTA-monoamide, DOXP, p-NCS-DOTA, p-NCS-PADOTA, p-NCS-TRITA, TRITA, TETA, 3p-C-DEPA, 3p-C-DEPA-NCS, p-NH2-BN-OXO-DO3A, p-SCN-BN-TCMC, TCMC, 4-aminobutyl-DOTA, azido-mono-amide-DOTA, BCN-DOTA, butyne-DOTA, BCN-DOTA-GA, DOA3P, DO2a2p, DO2A(trans-H2do2a), DO3A, DO3A-thiol, DO3AtBu-N-(2-aminoethyl)ethanamide, DO3TMP-monoamide, DO2AP, CB-DO2A, C3B-DO2A, HP-DO3A, DOTA-NHS-ester, maleimide-DOTA-GA, maleimido-mono-aminde-DOTA, maleimide-DOTA, NH2-DOTA-GA, NH2-PEG4-DOTA-GA, GA, p-$NH_2$-Bn-DOTA, p-NO2-Bn-DOTA, p-SCN-Bn-DOTA, p-SCN-Bz-DOTA, TA-DOTA, TA-DOTA-GA, OTTA, DOXP, TSC, DTC, DTCBP, PTSM, ATSM, H2ATSM, H2PTSM, Dp44mT, DpC, Bp44mT, QT, hybrid thiosemicarbazone-benzothiazole, thiosemicarbazone-styrylpyridine tetradentate ligands $H_2$L2-4, HBED, HBED-CC, dmHBED, dmEHPG, HBED-nn, SHBED, Br-Me2HBED, BPCA, HEHA, BF-HEHA, deferiprone, THP, HYNIC (2-hydrazino nicotinamide), NHS-HYNIC, HYNIC-Kp-DPPB, HYNIC-Ko-DPPB, (HYNIC)(tricine)2, (HYNIC)(EDDA)Cl, p-EDDHA, AIM, AIM A,IAM B, MAMA, MAMA-DGal, MAMA-MGal, MAMA-DA, MAMA-HAD, macropa, macropaquin, macroquin-SO3, $N_xS_{4-x}$, N2S2, N3S, N4, MAG3B, NOTA, NODAGA, SCN-Bz-NOTA-R, NOT-P (NOTMP), NOTAM, p-NCS-NOTA, TACN, TACN-TM, NETA, NETA-monoamine, p-SCN-PhPr-NE3TA, C-NE3TA-NCS, C-NETA-NCS, 3p-C-NETA, NODASA, NOPO, NODA, NODA-MPAA, NO2A, N-benzyl-NODA, C-NOTA, BCNOT-monoamine, maleimido-mono-amide-NOTA, NO2A-azide, NO2A-butyne, NO2AP, NO3AP, N-NOTA, oxo-DO3A, p-NH2-Bn-NOTA, p-$NH_2$-Bn-oxo-DO3A, p-NO2-Bn-cyclen, p-SCN-Bn-NOTA, p-SCN-Bn-oxo-DO3A, TRAP, PEPA, BF-PEPA, pycup, pycup2A, pycup1A1Bn, pycup2Bn, SarAr-R, DiAmSar, AmBaSar-R, siamSar, Sar, Tachpyr, tachpyr-(6-Me), TAM A, TAM B, TAME, TAME-Hex, THP-Ph-NCS, THP-NCS, THP-TATE, NTP, H3THP, THPN, CB-TE2A, PCB-TE1A1P,_TETA-NHS, CPTA, CPTA-NHS, CB-TE1K1P, CB-TE2A, TE2A, H2CB-TE2A, TE2P, CB-TE2P, MM-TE2A, DM-TE2A, 2C-TETA, 6C-TETA, BAT, BAT-6, NHS-BAT ester, SSBAT, SCN-CHX-A-DTPA-P, SCN-TETA, TMT-amine, p-BZ-HTCPP, $H_4$pypa, $H_4$octox, p-NO2-Bn-neunpa, p-SCN-Bn-$H_4$neunpa, TTHA, tBu4pypa-C7-NHS, $H_4$neunpa, H2macropa, BT-DO3A, DO3A-Nprop, DO3AP, DOTPMB, DOTAMAE, DOTAMAP, DO3AMBu, DEPA, p-NO2-Bn-PCTA, symPC2APA, symPCA2PA, asymPC2APA, asymPCA2PA, $^{99m}$Tc$(CO)_3$-Chelators, and MeO-DOTA-NCS.

HYNIC, DTPA, EDTA, DOTA, TETA, bisamino bisthiol (BAT)-based chelators as disclosed in US 5,720,934; desferrioxamine (DFO) as disclosed in Doulias et al. (Doulias, et al., Free Radic BiolMed, 2003, 35: 719), tetrapyridine and $N_3S$, $N_2S_2$ and $N_4$ chelators as disclosed in US 5,367,080 A; US 5,364,613 A; US 5,021,556 A; US 5,075,099 A; and US 5,886,142 A, whereby all of the references are included herein by reference in their entirety. 6-amino-6-methylperhydro-1,4-diazepine-*N,N',N",N"*-tetraacetic acid (AAZTA) is disclosed in Pfister et al. (Pfister, et al., EJNMMI Res, 2015, 5: 74), deferiprone, a 1,2-dimethyl-3,4-hydroxypyridinone and hexadentate tris(3,4-hydroxypyridinone) (THP) are disclosed in Cusnir et al. (Cusnir, et al., Int J Mol Sci, 2017, 18), monoamine-monoamide dithiol (MAMA)-based chelators are disclosed in Demoin et al. (Demoin, et al., Nucl Med Biol, 2016, 43: 802), macropa and analogues are disclosed in Thiele et al. (Thiele, et al., Angew Chem Int Ed Engl, 2017, 56: 14712), 1,4,7,10,13,16-hexaazacyclohexadecane-*N,N',N",N"',N"",N""'*-hexaacetic acid (HEHA) and PEPA analogues are disclosed in Price and Orvig (Price, et al., Chem Soc Rev, 2014, 43: 260), pycup and analogous are disclosed in Boros et al. (Boros, et al., Mol Pharm, 2014, 11: 617), N, N-bis(2-hydroxybenzyl)ethylenediamine-N,N-diacetic acid (HBED), 1,4,7,10-tetrakis (carbamoylmethyl)-1,4,7,10-tetraazacyclododecane (TCM), 2-[(carboxymethyl)]-[5-(4-nitrophenyl-1-[4,7,10-tris-(carboxymethyl)-1,4,7,10-tetraazacyclododecan-1-yl]pentan-2-yl)-amino]acetic acid (3p-C-DEPA), CB-TE2A, TE2A, TE1A1P, DiAmSar, 1-N-(4-aminobenzyl)-3,6,10,13,16,19-hexaazabicyclo[6.6.6]-eicosane-1,8-diamine (SarAr), NETA, , tris(2-mercaptoethyl)-1,4,7-triazacyclononane (TACN-TM), {4-[2-(bis-carboxymethyl-amino)-ethyl]-7-carboxymethyl-[1,4,7]triazonan-1-yl}-acetic acid (NETA), diethylenetriaminepentaacetic acid (DTP), 3-({4,7-bis-[(2-carboxy-ethyl)-hydroxyphosphinoylmethyl]-[1,4,7]triazonan-1-ylmethyl}-hydroxy-phosphinoyl)-propionic acid (TRAP), NOPO, $H_4$octapa, SHBED, BPCA, 3,6,9,15-tetraazabicyclo[9.3.1]-pentadeca-1(15),11,13-triene-3,6,9,-triacetic acid (PCTA), and 1,4,7,10,13-pentaazacyclopentadecane-N,N',N",N"',N""-pentaacetic acid (PEPA) are disclosed in Price and Orvig (Price, et al., Chem Soc Rev, 2014, 43: 260), 1-hydroxy-2-pyridone ligand (HOPO) is disclosed in Allott et al. (Allott, et al., Chem Commun (Camb), 2017, 53: 8529), [4-carboxymethyl-6-(carboxymethyl-methyl-amino)-6-methyl-[1,4]diazepan-1-yl]-acetic acid (DATA) is disclosed in Tornesello et al. (Tornesello, et al., Molecules, 2017, 22: 1282), tetrakis(aminomethyl)methane (TAM) and analogues are disclosed in McAuley 1988 (McAuley, et al., Canadian Journal of Chemistry, 1989, 67: 1657), hexadentate tris(3,4-hydroxypyridinone) (THP) and analogues are disclosed in Ma et al. (Ma, et al., Dalton Trans, 2015, 44: 4884).

The diagnostic and/or therapeutic use of some of the above chelators is described in the prior art. For example, 2-hydrazino nicotinamide (HYNIC) has been widely used in the presence of a coligand for incorporation of $^{99m}$Tc and $^{186,188}$Re (Schwartz, et al., Bioconjug Chem, 1991, 2: 333; Babich, et al., J Nucl Med, 1993, 34: 1964; Babich, et al., Nucl Med Biol, 1995, 22: 25); DTPA is used in Octreoscan® for complexing $^{111}$In and several modifications are described in the literature (Li, et al., Nucl Med Biol, 2001, 28: 145; Brechbiel, et al., Bioconjug Chem, 1991, 2: 187); DOTA-type chelators for radiotherapy applications are described by Tweedle et al. (US Pat 4,885,363); other polyaza macrocycles for chelating trivalent isotopes metals are described by Eisenwiener *et al.* (Eisenwiener, et al., Bioconjug Chem, 2002, 13: 530); and $N_4$-chelators such as a $^{99m}$Tc-$N_4$-chelator have been used for peptide labeling in the case of minigastrin for targeting CCK-2 receptors (Nock, et al., J Nucl Med, 2005, 46: 1727).

In an embodiment, the metal chelator is selected from the group including, but not limited to, DOTA, DOTAGA, DOTAM, DOTP, NOTA, NODAGA, NODA-MPAA, HBED, TETA, CB-TE2A, DTPA, CHX-A"-DTPA, DFO, Macropa, HOPO, TRAP, THP, DATA, NOPO, PCTA, NOTP, sarcophagine, FSC, NETA, NE3TA, $H_4$octapa, pycup, HYNIC, $N_xS_{4-x}$ (N4, N2S2, N3S), $^{99m}$Tc$(CO)_3$-chelators and their analogs, wherein

DOTA stands for 1,4,7,10-tetrazacyclododecane-1,4,7,10-tetraacetic acid,

DOTAGA stand for 1,4,7,10-tetraazacyclodocecane,1-(glutaric acid)-4,7,10-triacetic acid,

DOTAM (also called TCMC) stands for 1,4,7,10-tetrakis[carbamoylmethyl]-1,4,7,10-tetracyclodecane,

DOTP stands for 1,4,7,10-tetraazacyclododecane-1,4,7,10-tetra(methylene phosphonic acid),

NOTA stands for 1,4,7-triazacyclononanetriacetic acid,

NODAGA stands for 1,4,7-triazacyclononane-N-glutaric acid-N',N"-diacetic acid,

NODA-MPAA stands for 1,4,7-triazacyclononane-1,4-diacetate-methyl phenylacetic acid,

HBED stands for bis(2-hydroxybenzyl) ethylenediaminediacetic acid,

TETA stands for 1,4,8,11-tetraazacyclododecane-1,4,8,11-tetraacetic acid,

CB-TE2A stands for 4,11-bis-(carboxymethyl)-1,4,8,11-tetraazabicyclo[6.6.2]-hexadecane,

DTPA stands for diethylenetriaminepentaacetic acid,

CHX-A"-DTPA stands for [(2-{[2-(bis-carboxymethyl-amino)-cyclohexyl]-carboxymethyl-amino}-ethyl)-carboxymethyl-amino]-acetic acid,

DFO stands for the desferal or desferrioxamine type group of chelators, the chemical name of the non-limiting example is N-[5-({3-[5-(acetyl-hydroxy-amino)-pentylcarbamoyl]-propionyl}-hydroxy-amino)-pentyl]-N'-(5-amino-pentyl)-N'-hydroxy-succinamide,

Macropa stands for N,N'-bis[(6-carboxy-2-pyridyl)methyl]-4,13-diaza-18-crown,

HOPO stands for the octadentate hydroxypyridinone-type group of chelators, the structure of a non-limiting example is shown below,

TRAP stands for 3-({4,7-bis-[(2-carboxy-ethyl)-hydroxy-phosphinoylmethyl]-[1,4,7]triazonan-1-ylmethyl} -hydroxy-phosphinoyl)-propionic acid,

THP stands for hexadentate tris(3,4-hydroxypyridinone),

DATA stands for [4-carboxymethyl-6-(carboxymethyl-methyl-amino)-6-methyl-[1,4]diazepan-1-yl]-acetic acid

PCTA stands for 3,6,9,15-tetraazabicyclo[9.3.1]-pentadeca-1(15),1,13-triene-3,6,9,-triacetic acid,

NOPO stands for 1,4,7-triazacyclononane-1,4-bis[methylene(hydroxymethyl)phosphinic acid]-7-[methylene(2-carboxyethyl)phosphinic acid],

NOTP stands for 1,4,7-triazacyclononane-N,N'N"-tris(methylene phosphonic) acid),

Sarcophagine stands for 3,6,10,13,16,19-hexaazabicyclo[6.6.6]icosane,

FSC stands for 3,15,27-triamino-7,19,31-trihydroxy-10,22,34-trimethyl-1,13,25-trioxa-7,19,31-triaza-cyclohexatriaconta-9,21,33-triene-2,8,14,20,26,32-hexaone,

NETA stands for {4-[2-(bis-carboxymethyl-amino)-ethyl]-7-carboxymethyl-[1,4,7]triazonan-1-yl}-acetic acid

NE3TA stands for {4-carboxymethyl-7-[2-(carboxymethyl-amino)-ethyl]-[1,4,7]triazonan-1-yl}-acetic acid,

H4octapa stands for *N,N'*-(6-carboxy-2-pyridylmethyl)-*N,N'*-diacetic acid-1,2-diaminoethane

Pycup stands for 1,8-(2,6-Pyridinedimethylene)-1,4,8,11-tetraazacyclo-tetradecane,

HYNIC stands for 6-hydrazino-nicotinic acid,

$N_xS_{4-x}$ (N4, N2S2, N3S) stands for a group of tetradentate chelators with N-atoms (basic amine or non-basic amide) and thiols as donors stabilizing Tc-complexes, especially Tc(V)-oxo complexes. The structure of one representative non-limiting example N4 is shown below, and

N4 stands for N,N'-bis-(2-amino-ethyl)-propane-1,3-diamine,

$^{99m}Tc(CO)_3$-chelators stands for bi- or tridendate chelators capable of forming stable complexes with technetium tricarbonyl fragments,

and with the chemical structures thereof being as follows:

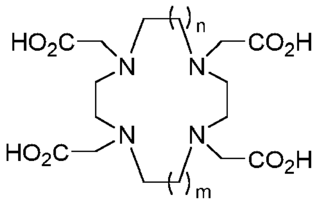

m=n=1 **DOTA**
m = n = 2 **TETA**
m=1, n= 2 **TRITA**

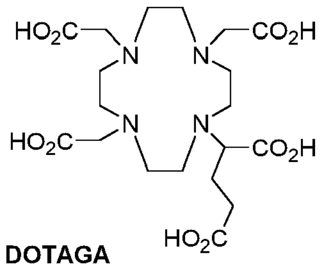

**DOTAGA**

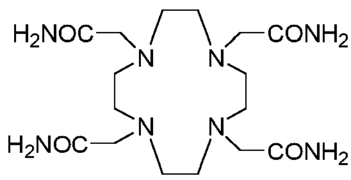

**DOTAM**

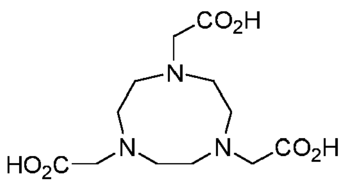

**NOTA**

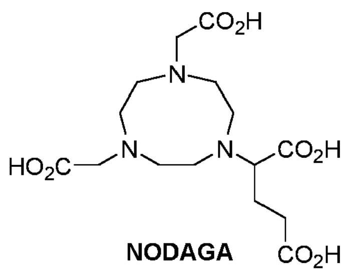

**NODAGA**

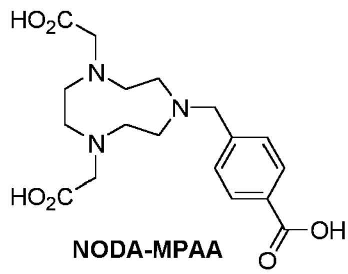

**NODA-MPAA**

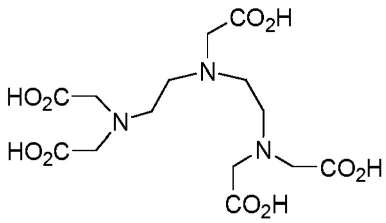

**DTPA**

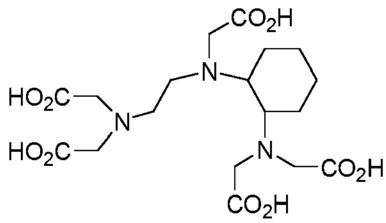

**CHX-A"-DTPA**

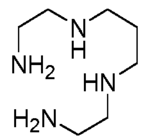

**N4**

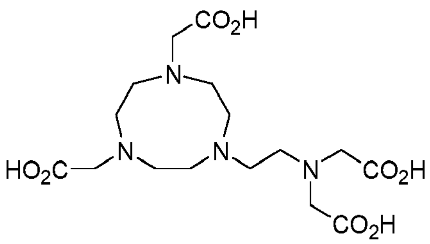

**NETA**

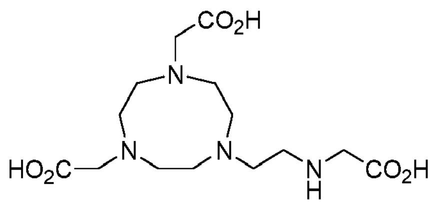

**NE3TA**

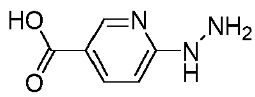

**HYNIC**

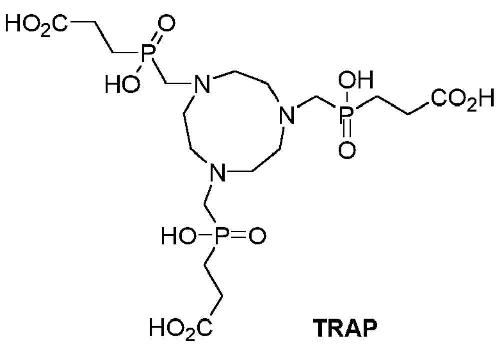

**TRAP**

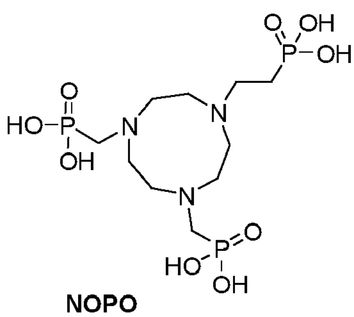

**NOPO**

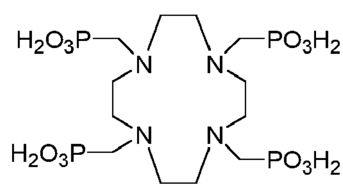

**DOTP**

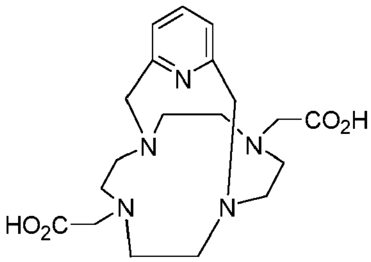

**pycup**

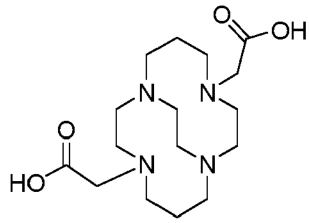

**CB-TE2A**

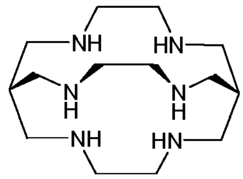

**Sarcophagine**

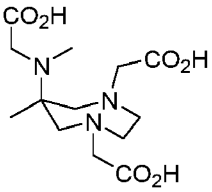

**DATA**

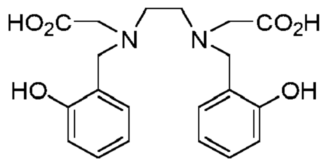

**HBED**

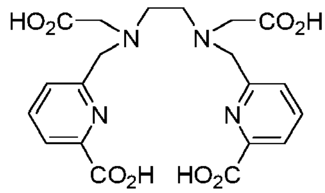

**H4octapa**

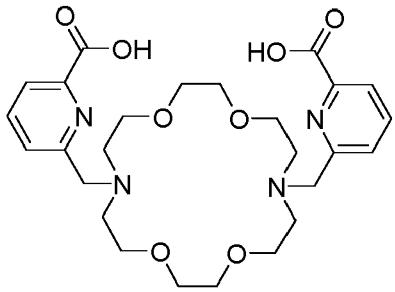

**Macropa**

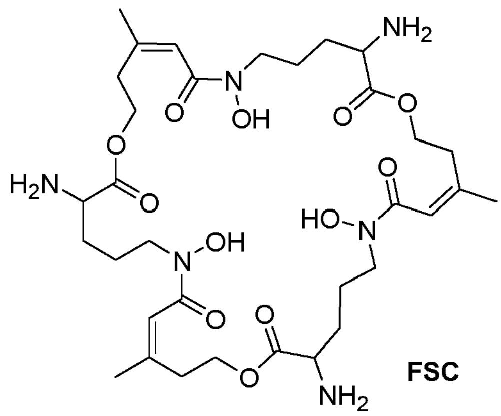

**FSC**

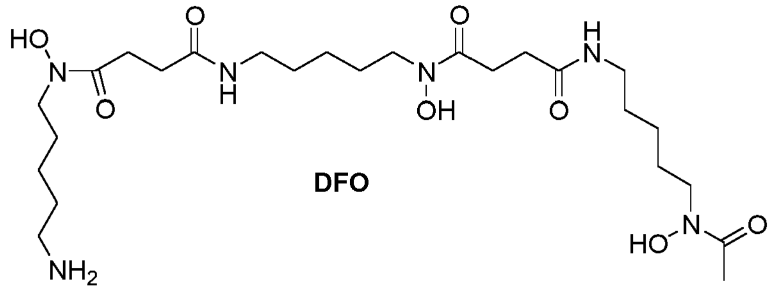

DFO

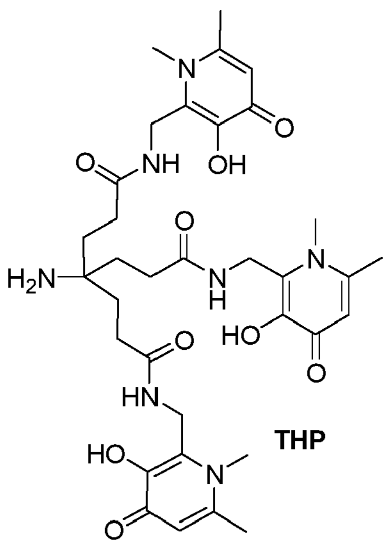

THP

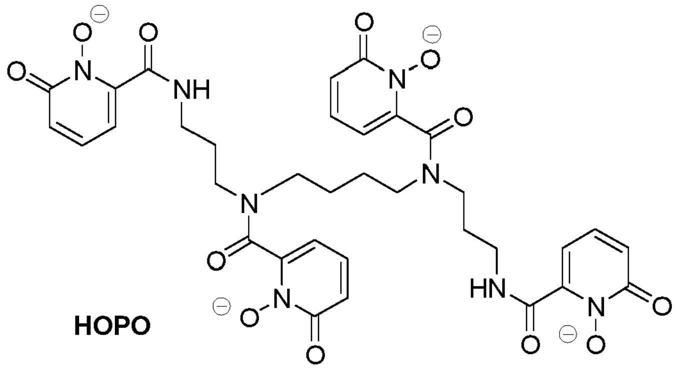

HOPO

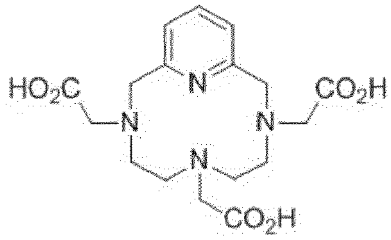

PCTA .

In some embodiments, the metal chelator is selected from the group consisting of DOTA, DOTAGA, NOPO, PCTA, NOTA, NODAGA, NODA-MPAA, HBED, TETA, CB-TE2A, DTPA, DFO, Macropa, DOTAM, HOPO, TRAP, THP, DATA, NOTP, sarcophagine, FSC, NETA, $H_4$octapa, Pycup, $N_xS_{4-x}$ (N4, N2S2, N3S), Hynic, and $^{99m}Tc(CO)_3$-Chelators, and analogs thereof.

In some embodiments, the metal chelator is selected from the group consisting of DOTA, DOTAGA, NOPO, PCTA, DOTAM, Macropa, NOTA, NODAGA, NODA-MPAA, HBED, CB-TE2A, DFO, THP, N4, and analogs thereof.

In some embodiments, the metal chelator is selected from the group consisting of DOTA, DOTAGA, NOPO, PCTA, DOTAM, Macropa, NOTA, and NODAGA, and analogs thereof.

In some embodiments, the metal chelator is selected from the group consisting of DOTA, NOPO, PCTA, and Macropa, and analogs thereof.

In some embodiments, the metal chelator is DOTA and analogs thereof.

It will be acknowledged by a person skilled in the art that the chelator, in principle, may be used regardless of whether the compound of the disclosure is used in or suitable for diagnosis or therapy.

It will be further acknowledged by a person skilled in the art that the presence of a chelator in the compound of the disclosure includes, if not stated otherwise, the possibility that the chelator is complexed to any metal complex partner, i.e., any metal which, in principle, can be complexed by the chelator. An explicitly mentioned chelator of a compound of the disclosure or the general term chelator in connection with the compound of the disclosure refers either to the uncomplexed chelator as such or to the chelator to which any metal complex partner is bound, wherein the metal complex partner is any radioactive or non-radioactive metal complex partner. In some embodiments, the chelator metal complex, i.e. the chelator to which the metal complex partner is bound, is a stable chelator metal complex.

Non-radioactive chelator metal complexes have several applications, e.g., for assessing properties like stability or activity which are otherwise difficult to determine. One aspect is that cold variants of the radioactive versions of the

metal complex partner (e.g., non-radioactive Gallium, Lutetium or Indium complexes) can act as surrogates of the radioactive compounds. Furthermore, they are valuable tools for identifying metabolites *in vitro* or *in vivo,* as well as for assessing toxicity properties of the compounds of disclosure. Additionally, chelator metal complexes can be used in binding assays utilizing the fluorescence properties of some metal complexes with distinct ligands (e.g., Europium salts).

Chelators can be synthesized or are commercially available with a wide variety of (possibly already activated) groups for the conjugation to peptides or amino acids. Direct conjugation of a chelator to an amino-nitrogen of the respective compound of disclosure is possible for chelators selected from the group consisting of DOTA, DOTAGA, NOTA, NODAGA, NODA-MPAA, DTPA, CHX-A"-DTPA, macropa, HBED, CB-TE2A, DFO, THP and N4, for example DOTA, DOTAGA, NODAGA and macropa. For example, the linkage in this respect is an amide linkage.

Direct conjugation of an isothiocyanate-functionalized chelator to an amino-nitrogen of the respective compound of disclosure is possible for chelators selected from the group consisting of DOTA, DOTAGA, NOTA, NODAGA, DTPA, CHX-A"-DTPA, DFO, and THP, for example, DOTA, DOTAGA, NOTA, NODAGA, DTPA, and CHX-A"-DTPA. For example, the linkage in this respect is a thiourea linkage.

Functional groups at a chelator which are ideal precursors for the direct conjugation of a chelator to an amino-nitrogen are known to a person skilled in the art and include, but are not limited to, carboxylic acid, activated carboxylic acid, e.g., active ester like for instance NHS-ester, pentafluorophenol-ester, HOBt-ester and HOAt-ester, isothiocyanate.

It will be acknowledged by a person skilled in the art that the radioactive nuclide which is or which is to be attached to the compound of the disclosure, is selected taking into consideration the disease to be treated and/or the disease to be diagnosed, respectively, and/or the particularities of the patient and patient group, respectively, to be treated and to be diagnosed, respectively.

In the present disclosure, a radioactive nuclide is also referred to as radionuclide. Radioactive decay is the process by which an atomic nucleus of an unstable atom loses energy by emitting ionizing particles (ionizing radiation). There are different types of radioactive decay. A decay, or loss of energy, results when an atom with one type of nucleus, called the parent radionuclide, transforms to an atom with a nucleus in a different state, or to a different nucleus containing different numbers of protons and neutrons. Either of these products is named the daughter nuclide. In some decays the parent and daughter are different chemical elements, and thus the decay process results in nuclear transmutation (creation of an atom of a new element). For example, the radioactive decay can be alpha decay, beta decay, and gamma decay. Alpha decay occurs when the nucleus ejects an alpha particle (helium nucleus). This is the most common process of emitting nucleons, but in rarer types of decays, nuclei can eject protons, or specific nuclei of other elements (in the process called cluster decay). Beta decay occurs when the nucleus emits an electron ($\beta^-$-decay) or positron ($\beta^+$-decay) and a type of neutrino, in a process that changes a proton to a neutron or the other way around. By contrast, there exist radioactive decay processes that do not result in transmutation. The energy of an excited nucleus may be emitted as a gamma ray in gamma decay, or used to eject an orbital electron by interaction with the excited nucleus in a process called internal conversion, or used to absorb an inner atomic electron from the electron shell whereby the change of a nuclear proton to neutron causes the emission of an electron neutrino in a process called electron capture (EC), or may be emitted without changing its number of proton and neutrons in a process called isomeric transition (IT). Another form of radioactive decay, the spontaneous fission (SF), is found only in very heavy chemical elements resulting in a spontaneous breakdown into smaller nuclei and a few isolated nuclear particles.

In an embodiment, described herein are compounds that comprise a radionuclide. Generally, the type of radionuclide used in a therapeutic radiopharmaceutical can be tailored to the specific type of cancer and the type of targeting moiety. Radionuclides that undergo $\alpha$-decay produce particles composed of two neutrons and two protons, and radionuclides that undergo $\beta$-decay emit energetic electrons from their nuclei. Some radionuclides can also emit Auger electrons. In some embodiments, the conjugate comprises an alpha particle-emitting radionuclide. Alpha radiation can cause direct, irreparable double-strand DNA breaks compared with gamma and beta radiation, which can cause single-stranded breaks via indirect DNA damage. The range of these particles in tissue and the half-life of the radionuclide can also be considered in designing the radiopharmaceutical conjugate.

Radionuclides that are $\alpha$-emitters are capable of destroying tumors while causing very limited damage to the surrounding healthy tissue due to the short penetration depth of $\alpha$ particles. Their high linear energy transfer (LET) gives them an increased relative biological effectiveness (RBE) as compared to other radionuclide therapies. Furthermore, when $\alpha$-emitting radionuclides are targeted to specific tumor cells in the body, they can be very effective in destroying metastases, which are difficult to treat by currently employed techniques (de Kruijff et al, 2015 Pharmaceuticals, 8, 321-336).

In an embodiment of the present disclosure, the radionuclide can be used for labeling of the compound of the disclosure.

In an embodiment of the present disclosure, the radionuclide is suitable for complexing with a chelator, leading to a radionuclide chelate complex.

In a further embodiment one or more atoms of the compound of the disclosure are of nonnatural isotopic composition, for example these atoms are radionuclides; for example radionuclides of carbon, oxygen, nitrogen, sulfur,

phosphorus and halogens. These radioactive atoms are typically part of amino acids, in some case halogen containing amino acids, and/or building blocks and in some cases halogenated building blocks each of the compound of the disclosure.

In one embodiment of the present disclosure, the radionuclide has a half-life that allows for diagnostic and/or therapeutic medical use. Specifically, the half-life is between 1 min and 100 days.

In an embodiment of the present disclosure, the radionuclide has a decay energy that allows for diagnostic and/or therapeutic medical use. Specifically, for γ-emitting isotopes, the decay energy is between 0.004 and 10 MeV, for example, between 0.05 and 4 MeV, for diagnostic use. For positron-emitting isotopes, the decay energy is between 0.6 and 13.2 MeV, for example, between 1 and 6 MeV, for diagnostic use. For particle-emitting isotopes, the decay energy is between 0.039 and 10 MeV, for example, between 0.4 and 6.5 MeV, for therapeutic use.

In an embodiment of the present invention, the radionuclide is industrially produced for medical use. Specifically, the radionuclide is available in GMP quality.

In an embodiment of the present disclosure, the daughter nuclide(s) after radioactive decay of the radionuclide are compatible with the diagnostic and/or therapeutic medical use. Furthermore, the daughter nuclides are either stable or further decay in a way that does not interfere with, or may even support, the diagnostic and/or therapeutic medical use. Representative radionuclides, which may be used in connection with the present disclosure are summarized in Table 3. Table 3 includes key properties of relevant radionuclides - half life, decay radiation types, decay energies of transitions with highest probabilities (mean energies for β decays) and absolute intensities (source: https://www-nds.iaea.org/relnsd/vcharthtml/VChartHTML.html; accessed September 4$^{th}$, 2020).

Table 3: Key properties of relevant radionuclides – half life, decay types and decay energies

| Atomic number | Radio-nuclide | Half-life | Decay | Energy in MeV | Intensity (abs) in % |
|---|---|---|---|---|---|
| 6 | **C-11** | 20.4 m | β+ | 0.386 | 99.8 |
| 7 | **N-13** | 10.0 m | β+ | 0.492 | 99.8 |
| 9 | **F-18** | 109.8 m | β+ | 0.250 | 96.7 |
| 11 | **Na-24** | 15.0 h | β- | 0.554 | 99.9 |
| | | | γ | 1.369 | 100.0 |

| Atomic number | Radio-nuclide | Half-life | Decay | Energy in MeV | Intensity (abs) in % |
|---|---|---|---|---|---|
| | | | γ | 2.754 | 99.9 |
| 12 | **Mg-28** | 20.9 h | β- | 0.156 | 94.8 |
| | | | γ | 0.031 | 89.0 |
| | | | γ | 1.342 | 54.0 |
| | | | γ | 0.942 | 36.3 |
| | | | γ | 0.401 | 35.9 |
| 14 | **Si-31** | 157.4 m | β- | 0.596 | 99.9 |
| 15 | **P-32** | 14.3 d | β- | 0.695 | 100.0 |
| 15 | **P-33** | 25.4 d | β- | 0.076 | 100.0 |
| 16 | **S-38** | 170.3 m | β- | 0.373 | 86.6 |
| | | | γ | 1.942 | 86.5 |
| 17 | **Cl-34m1** | 32.0 m | β+ | 1.099 | 28.4 |
| | | | β+ | 0.554 | 25.6 |
| | | | γ | 2.127 | 42.8 |
| | | | γ | 0.146 | 38.3 |
| | | | γ | 1.177 | 14.1 |
| | | | γ | 3.304 | 12.3 |
| 17 | **Cl-38** | 37.2 m | β- | 2.244 | 56.0 |
| | | | β- | 0.420 | 32.9 |
| | | | β- | 1.181 | 11.1 |
| | | | γ | 2.167 | 44.0 |
| | | | γ | 1.643 | 32.9 |
| 17 | **Cl-39** | 56.2 m | β- | 0.792 | 83.0 |
| | | | γ | 1.267 | 53.6 |
| | | | γ | 0.250 | 46.1 |
| | | | γ | 1.517 | 39.3 |
| 18 | **Ar-37** | 35.0 d | EC | 0.814 | 100.0 |
| 18 | **Ar-41** | 109.6 m | β- | 0.459 | 99.2 |
| | | | γ | 1.294 | 99.2 |
| 18 | **Ar-44** | 11.9 m | β- | 0.470 | 93.0 |
| | | | β- | 1.369 | 12.0 |
| | | | β- | 1.264 | 10.0 |
| | | | γ | 0.183 | 66.0 |
| | | | γ | 1.703 | 56.0 |
| | | | γ | 1.886 | 31.0 |
| 9 | **K-42** | 12.4 h | β- | 1.566 | 81.9 |
| | | | β- | 0.824 | 17.6 |
| | | | γ | 1.525 | 18.1 |
| 19 | **K-43** | 22.3 h | β- | 0.305 | 90.9 |
| | | | γ | 0.373 | 86.8 |
| | | | γ | 0.617 | 79.2 |
| | | | γ | 0.397 | 11.9 |
| | | | γ | 0.593 | 11.3 |
| 19 | **K-44** | 22.1 m | β- | 2.601 | 34.0 |
| | | | β- | 0.990 | 28.5 |
| | | | β- | 0.816 | 11.0 |

| Atomic number | Radio-nuclide | Half-life | Decay | Energy in MeV | Intensity (abs) in % |
|---|---|---|---|---|---|
| | | | γ | 1.157 | 58.0 |
| | | | γ | 2.151 | 23.0 |
| 19 | **K-45** | 17.8 m | β- | 0.973 | 51.0 |
| | | | β- | 0.751 | 15.5 |
| | | | β- | 0.733 | 10.0 |
| | | | γ | 0.174 | 74.0 |
| | | | γ | 1.706 | 53.0 |
| | | | γ | 2.354 | 14.1 |
| 20 | **Ca-47** | 4.5 d | β- | 0.243 | 73.0 |
| | | | β- | 0.819 | 27.0 |
| | | | γ | 1.297 | 67.0 |
| 21 | **Sc-43** | 3.9 h | β+ | 0.508 | 70.9 |
| | | | β+ | 0.344 | 17.2 |
| | | | γ | 0.373 | 22.5 |
| 21 | **Sc-44** | 4.0 h | β+ | 0.632 | 94.3 |
| | | | γ | 1.157 | 99.9 |
| 21 | **Sc-44m1** | 58.6 h | γ | 0.271 | 86.7 |
| 21 | **Sc-47** | 3.3 d | β- | 0.143 | 68.4 |
| | | | β- | 0.204 | 31.6 |
| | | | γ | 0.159 | 68.3 |
| 21 | **Sc-48** | 43.7 h | β- | 0.227 | 90.0 |
| | | | β- | 0.159 | 10.0 |
| | | | γ | 0.984 | 100.1 |
| | | | γ | 1.312 | 100.1 |
| | | | γ | 1.038 | 97.6 |
| 21 | **Sc-49** | 57.2 m | β- | 0.824 | 99.9 |
| 22 | **Ti-45** | 184.8 m | β+ | 0.439 | 84.8 |
| | | | EC | 2.066 | 14.9 |
| 23 | **V-47** | 32.6 m | β+ | 0.832 | 96.5 |
| 23 | **V-48** | 16.0 d | β+ | 0.290 | 49.9 |
| | | | EC | 1.717 | 39.2 |
| | | | γ | 0.984 | 100.0 |
| | | | γ | 1.312 | 98.2 |
| 24 | **Cr-48** | 21.6 h | EC | 1.235 | 96.1 |
| | | | γ | 0.308 | 100.0 |
| | | | γ | 0.112 | 96.0 |
| 24 | **Cr-49** | 42.3 m | β+ | 0.625 | 46.4 |
| | | | β+ | 0.653 | 34.7 |
| | | | β+ | 0.694 | 11.7 |
| | | | γ | 0.091 | 53.2 |
| | | | γ | 0.153 | 30.3 |
| | | | γ | 0.062 | 16.4 |
| 24 | **Cr-51** | 27.7 d | EC | 0.752 | 90.1 |
| 25 | **Mn-51** | 46.2 m | β+ | 0.964 | 96.9 |
| 25 | **Mn-52** | 5.6 d | β+ | 0.242 | 29.4 |
| | | | EC | 1.597 | 61.4 |

| Atomic number | Radio-nuclide | Half-life | Decay | Energy in MeV | Intensity (abs) in % |
|---|---|---|---|---|---|
| | | | γ | 1.434 | 100.0 |
| | | | γ | 0.936 | 94.5 |
| | | | γ | 0.744 | 90.0 |
| 25 | **Mn-52m1** | 21.1 m | β+ | 1.174 | 96.4 |
| | | | γ | 1.434 | 98.2 |
| 25 | **Mn-56** | 2.6 h | β- | 1.217 | 56.6 |
| | | | β- | 0.382 | 27.5 |
| | | | β- | 0.255 | 14.5 |
| | | | γ | 0.847 | 98.9 |
| | | | γ | 1.811 | 26.9 |
| | | | γ | 2.113 | 14.2 |
| 26 | **Fe-52** | 8.3 h | β+ | 0.340 | 55.5 |
| | | | EC | 1.825 | 43.6 |
| | | | γ | 0.169 | 99.0 |
| 26 | **Fe-59** | 44.5 d | β- | 0.149 | 53.1 |
| | | | β- | 0.081 | 45.3 |
| | | | γ | 1.099 | 56.5 |
| | | | γ | 1.292 | 43.2 |
| 27 | **Co-55** | 17.5 h | β+ | 0.649 | 46.0 |
| | | | β+ | 0.436 | 25.6 |
| | | | EC | 2.059 | 10.7 |
| | | | γ | 0.931 | 75.0 |
| | | | γ | 0.477 | 20.2 |
| | | | γ | 1.409 | 16.9 |
| 27 | **Co-61** | 1.6 h | β- | 0.475 | 95.6 |
| | | | γ | 0.067 | 84.7 |
| 27 | **Co-62m1** | 13.9 m | β- | 1.286 | 64.0 |
| | | | β- | 0.888 | 19.6 |
| | | | β- | 0.486 | 10.2 |
| | | | γ | 1.173 | 97.7 |
| | | | γ | 1.164 | 70.5 |
| | | | γ | 2.004 | 18.2 |
| 28 | **Ni-56** | 6.1 d | EC | 0.416 | 100.0 |
| | | | γ | 0.158 | 98.8 |
| | | | γ | 0.812 | 86.0 |
| | | | γ | 0.750 | 49.5 |
| | | | γ | 0.270 | 36.5 |
| | | | γ | 0.480 | 36.5 |
| | | | γ | 1.562 | 14.0 |
| 28 | **Ni-57** | 35.6 h | β+ | 0.369 | 35.3 |
| | | | EC | 1.871 | 29.2 |
| | | | EC | 1.345 | 11.9 |
| | | | EC | 1.734 | 10.0 |
| | | | γ | 1.378 | 81.7 |
| | | | γ | 0.127 | 16.7 |
| | | | γ | 1.920 | 12.3 |

| Atomic number | Radio-nuclide | Half-life | Decay | Energy in MeV | Intensity (abs) in % |
|---|---|---|---|---|---|
| 28 | **Ni-65** | 2.5 h | β- | 0.875 | 60.0 |
| | | | β- | 0.221 | 28.4 |
| | | | β- | 0.372 | 10.2 |
| | | | γ | 1.482 | 23.6 |
| | | | γ | 1.116 | 15.4 |
| 28 | **Ni-66** | 54.6 h | β- | 0.073 | 100.0 |
| 29 | **Cu-60** | 23.7 m | β+ | 0.872 | 49.0 |
| | | | β+ | 1.325 | 15.0 |
| | | | β+ | 0.840 | 11.6 |
| | | | γ | 1.333 | 88.0 |
| | | | γ | 1.792 | 45.4 |
| | | | γ | 0.826 | 21.7 |
| 29 | **Cu-61** | 3.3 h | β+ | 0.524 | 51.0 |
| | | | EC | 2.238 | 16.0 |
| | | | EC | 1.582 | 11.1 |
| | | | γ | 0.283 | 12.2 |
| | | | γ | 0.656 | 10.8 |
| 29 | **Cu-64** | 12.7 h | β- | 0.191 | 38.5 |
| | | | β+ | 0.278 | 17.6 |
| | | | EC | 1.673 | 43.2 |
| 29 | **Cu-67** | 61.8 h | β- | 0.121 | 57.0 |
| | | | β- | 0.154 | 22.0 |
| | | | β- | 0.189 | 20.0 |
| | | | γ | 0.185 | 48.7 |
| | | | γ | 0.093 | 16.1 |
| 30 | **Zn-62** | 9.2 h | EC | 1.620 | 32.0 |
| | | | EC | 1.071 | 31.0 |
| | | | EC | 0.982 | 28.6 |
| | | | γ | 0.597 | 26.0 |
| | | | γ | 0.041 | 25.5 |
| | | | γ | 0.548 | 15.3 |
| | | | γ | 0.508 | 14.8 |
| 30 | **Zn-63** | 38.5 m | β+ | 1.042 | 80.3 |
| 30 | **Zn-69** | 56.4 m | β- | 0.322 | 100.0 |
| 30 | **Zn-69m1** | 13.8 h | γ | 0.439 | 94.9 |
| 30 | **Zn-71m1** | 4.0 h | β- | 0.569 | 87.8 |
| | | | γ | 0.386 | 91.4 |
| | | | γ | 0.487 | 61.2 |
| | | | γ | 0.620 | 55.8 |
| | | | γ | 0.512 | 28.7 |
| | | | γ | 0.596 | 27.5 |
| 30 | **Zn-72** | 46.5 h | β- | 0.083 | 85.1 |
| | | | β- | 0.067 | 14.7 |
| | | | γ | 0.145 | 82.8 |
| 31 | **Ga-65** | 15.2 m | β+ | 0.938 | 58.0 |
| | | | β+ | 0.895 | 10.2 |

| Atomic number | Radio-nuclide | Half-life | Decay | Energy in MeV | Intensity (abs) in % |
|---|---|---|---|---|---|
| | | | β+ | 0.966 | 10.0 |
| | | | γ | 0.115 | 54.0 |
| | | | γ | 0.061 | 11.4 |
| 31 | **Ga-66** | 9.5 h | β+ | 1.904 | 51.0 |
| | | | EC | 1.420 | 26.0 |
| | | | γ | 1.039 | 37.0 |
| | | | γ | 2.752 | 22.7 |
| 31 | **Ga-67** | 3.3 d | EC | 0.908 | 52.5 |
| | | | EC | 0.607 | 23.6 |
| | | | EC | 0.816 | 22.7 |
| | | | γ | 0.093 | 38.8 |
| | | | γ | 0.185 | 21.4 |
| | | | γ | 0.300 | 16.6 |
| 31 | **Ga-68** | 67.7 m | β+ | 0.836 | 87.7 |
| 31 | **Ga-70** | 21.1 m | β- | 0.648 | 98.9 |
| 31 | **Ga-72** | 14.1 h | β- | 0.344 | 28.9 |
| | | | β- | 0.226 | 22.4 |
| | | | β- | 0.219 | 15.7 |
| | | | γ | 0.834 | 95.5 |
| | | | γ | 2.202 | 26.9 |
| | | | γ | 0.630 | 26.1 |
| | | | γ | 2.508 | 13.3 |
| | | | γ | 0.894 | 10.1 |
| 31 | **Ga-73** | 4.9 h | β- | 0.461 | 78.6 |
| | | | γ | 0.297 | 79.8 |
| | | | γ | 0.326 | 11.2 |
| | | | γ | 0.053 | 10.5 |
| 32 | **Ge-66** | 2.3 h | β+ | 0.299 | 12.0 |
| | | | EC | 1.718 | 36.3 |
| | | | EC | 1.563 | 19.5 |
| | | | γ | 0.044 | 29.1 |
| | | | γ | 0.382 | 28.3 |
| | | | γ | 0.109 | 10.6 |
| | | | γ | 0.273 | 10.6 |
| 32 | **Ge-67** | 18.9 m | β+ | 1.368 | 74.0 |
| | | | γ | 0.167 | 84.0 |
| 32 | **Ge-69** | 39.1 h | β+ | 0.522 | 21.0 |
| | | | EC | 1.120 | 37.0 |
| | | | EC | 1.355 | 11.5 |
| | | | EC | 1.653 | 10.0 |
| | | | EC | 2.227 | 10.0 |
| | | | γ | 1.107 | 36.0 |
| | | | γ | 0.574 | 13.3 |
| | | | γ | 0.872 | 11.9 |
| 32 | **Ge-71** | 11.4 d | EC | 0.233 | 100.0 |
| 32 | **Ge-75** | 82.8 m | β- | 0.436 | 87.1 |

| Atomic number | Radio-nuclide | Half-life | Decay | Energy in MeV | Intensity (abs) in % |
|---|---|---|---|---|---|
| | | | β- | 0.323 | 11.5 |
| | | | γ | 0.265 | 11.4 |
| 32 | **Ge-77** | 11.2 h | β- | 0.839 | 21.4 |
| | | | β- | 0.584 | 19.2 |
| | | | β- | 0.912 | 16.0 |
| | | | γ | 0.264 | 53.3 |
| | | | γ | 0.211 | 30.0 |
| | | | γ | 0.216 | 27.9 |
| | | | γ | 0.416 | 22.7 |
| | | | γ | 0.558 | 16.8 |
| | | | γ | 0.367 | 14.5 |
| 32 | **Ge-78** | 88.0 m | β- | 0.227 | 96.0 |
| | | | γ | 0.277 | 96.0 |
| 33 | **As-69** | 15.2 m | β+ | 1.349 | 73.3 |
| | | | β+ | 1.238 | 14.0 |
| | | | γ | 0.233 | 10.9 |
| 33 | **As-70** | 52.6 m | β+ | 0.949 | 35.6 |
| | | | β+ | 0.954 | 31.9 |
| | | | γ | 1.039 | 82.0 |
| | | | γ | 0.668 | 22.1 |
| | | | γ | 0.744 | 22.1 |
| | | | γ | 1.114 | 20.6 |
| | | | γ | 0.595 | 18.8 |
| | | | γ | 1.708 | 17.5 |
| | | | γ | 2.019 | 16.6 |
| | | | γ | 0.906 | 11.2 |
| 33 | **As-71** | 65.3 h | β+ | 0.352 | 27.9 |
| | | | EC | 1.834 | 56.0 |
| | | | γ | 0.175 | 82.4 |
| 33 | **As-72** | 26.0 h | β+ | 1.117 | 64.2 |
| | | | β+ | 1.529 | 16.3 |
| | | | γ | 0.834 | 81.0 |
| 33 | **As-74** | 17.8 d | β- | 0.531 | 19.0 |
| | | | β- | 0.243 | 15.4 |
| | | | β+ | 0.408 | 26.1 |
| | | | EC | 1.963 | 33.1 |
| | | | γ | 0.596 | 59.0 |
| | | | γ | 0.635 | 15.4 |
| 33 | **As-76** | 26.2 h | β- | 1.264 | 51.0 |
| | | | β- | 0.993 | 35.2 |
| | | | γ | 0.559 | 45.0 |
| 33 | **As-77** | 38.8 h | β- | 0.229 | 97.0 |
| 33 | **As-78** | 90.7 m | β- | 1.856 | 32.0 |
| | | | β- | 1.560 | 18.5 |
| | | | β- | 0.607 | 15.9 |
| | | | β- | 1.228 | 14.6 |

| Atomic number | Radio-nuclide | Half-life | Decay | Energy in MeV | Intensity (abs) in % |
|---|---|---|---|---|---|
| | | | γ | 0.614 | 54.0 |
| | | | γ | 0.695 | 16.7 |
| | | | γ | 1.309 | 13.0 |
| 34 | **Se-70** | 41.1 m | β+ | 0.569 | 27.2 |
| | | | β+ | 0.402 | 18.2 |
| | | | EC | 1.952 | 26.7 |
| | | | EC | 2.328 | 13.2 |
| | | | γ | 0.050 | 35.8 |
| | | | γ | 0.426 | 29.1 |
| 34 | **Se-72** | 8.4 d | EC | 0.315 | 100.0 |
| | | | γ | 0.046 | 57.2 |
| 34 | **Se-73** | 7.2 h | β+ | 0.555 | 63.9 |
| | | | EC | 2.297 | 33.4 |
| | | | γ | 0.361 | 97.0 |
| | | | γ | 0.067 | 70.0 |
| 34 | **Se-73m1** | 39.8 m | β+ | 0.760 | 12.8 |
| 34 | **Se-81** | 18.5 m | β- | 0.614 | 98.7 |
| 34 | **Se-81m1** | 57.3 m | γ | 0.103 | 12.8 |
| 34 | **Se-83** | 22.3 m | β- | 0.369 | 30.7 |
| | | | β- | 0.349 | 26.3 |
| | | | β- | 0.331 | 16.4 |
| | | | β- | 0.245 | 10.3 |
| | | | γ | 0.357 | 69.4 |
| | | | γ | 0.510 | 42.8 |
| | | | γ | 0.225 | 29.5 |
| | | | γ | 0.718 | 14.7 |
| | | | γ | 0.799 | 14.6 |
| | | | γ | 0.837 | 11.5 |
| 35 | **Br-74** | 25.4 m | β+ | 1.182 | 17.6 |
| | | | β+ | 0.931 | 10.2 |
| | | | γ | 0.635 | 64.1 |
| | | | γ | 0.219 | 18.1 |
| | | | γ | 0.634 | 14.1 |
| 35 | **Br-74m1** | 46.0 m | β+ | 2.445 | 11.0 |
| | | | γ | 0.635 | 91.2 |
| | | | γ | 0.728 | 35.6 |
| | | | γ | 0.634 | 16.4 |
| 35 | **Br-75** | 96.7 m | β+ | 0.773 | 53.0 |
| | | | EC | 2.775 | 11.0 |
| | | | γ | 0.287 | 88.0 |
| 35 | **Br-76** | 16.2 h | β+ | 1.532 | 25.8 |
| | | | EC | 1.893 | 13.5 |
| | | | γ | 0.559 | 74.0 |
| | | | γ | 0.657 | 15.9 |
| | | | γ | 1.854 | 14.7 |
| 35 | **Br-77** | 57.0 h | EC | 1.365 | 43.8 |

| Atomic number | Radio-nuclide | Half-life | Decay | Energy in MeV | Intensity (abs) in % |
|---|---|---|---|---|---|
| | | | EC | 0.844 | 18.6 |
| | | | EC | 1.126 | 15.3 |
| | | | EC | 0.547 | 10.0 |
| | | | γ | 0.239 | 23.1 |
| | | | γ | 0.521 | 22.4 |
| 35 | **Br-80** | 17.7 m | β- | 0.804 | 85.0 |
| 35 | **Br-80m1** | 4.4 h | γ | 0.037 | 39.1 |
| 35 | **Br-82** | 35.3 h | β- | 0.138 | 99.1 |
| | | | γ | 0.777 | 83.6 |
| | | | γ | 0.554 | 71.7 |
| | | | γ | 0.619 | 43.7 |
| | | | γ | 0.698 | 28.4 |
| | | | γ | 1.044 | 27.6 |
| | | | γ | 1.317 | 26.9 |
| | | | γ | 0.828 | 24.2 |
| | | | γ | 1.475 | 16.4 |
| 35 | **Br-83** | 2.4 h | β- | 0.330 | 98.6 |
| 35 | **Br-84** | 31.8 m | β- | 2.051 | 33.0 |
| | | | β- | 1.629 | 13.5 |
| | | | β- | 1.145 | 11.7 |
| | | | β- | 0.235 | 11.5 |
| | | | γ | 0.882 | 42.0 |
| | | | γ | 1.898 | 14.6 |
| 36 | **Kr-74** | 11.5 m | β+ | 0.725 | 32.0 |
| | | | β+ | 0.768 | 25.0 |
| | | | β+ | 0.824 | 10.0 |
| | | | γ | 0.090 | 31.0 |
| | | | γ | 0.203 | 18.0 |
| 36 | **Kr-76** | 14.8 h | EC | 0.995 | 55.0 |
| | | | EC | 0.859 | 26.2 |
| | | | EC | 0.956 | 10.4 |
| | | | γ | 0.316 | 39.0 |
| | | | γ | 0.270 | 21.0 |
| | | | γ | 0.046 | 19.5 |
| | | | γ | 0.407 | 12.1 |
| 36 | **Kr-77** | 74.4 m | β+ | 0.847 | 41.8 |
| | | | β+ | 0.780 | 33.6 |
| | | | γ | 0.130 | 81.0 |
| | | | γ | 0.147 | 37.3 |
| 36 | **Kr-79** | 35.0 h | EC | 1.626 | 55.9 |
| | | | EC | 1.020 | 12.1 |
| | | | EC | 1.365 | 11.6 |
| | | | γ | 0.261 | 12.7 |
| 36 | **Kr-85** | 4.5 h | β- | 0.290 | 78.5 |
| | | | γ | 0.151 | 75.2 |
| | | | γ | 0.305 | 14.0 |

| Atomic number | Radio-nuclide | Half-life | Decay | Energy in MeV | Intensity (abs) in % |
|---|---|---|---|---|---|
| 36 | **Kr-87** | 76.3 m | β- | 1.502 | 41.0 |
| | | | β- | 1.694 | 30.5 |
| | | | γ | 0.403 | 50.0 |
| 36 | **Kr-88** | 2.8 h | β- | 0.167 | 67.0 |
| | | | β- | 1.235 | 14.0 |
| | | | γ | 2.392 | 34.6 |
| | | | γ | 0.196 | 26.0 |
| | | | γ | 2.196 | 13.2 |
| | | | γ | 0.835 | 13.0 |
| | | | γ | 1.530 | 10.9 |
| 37 | **Rb-78** | 17.7 m | β+ | 1.039 | 15.9 |
| | | | β+ | 1.253 | 14.9 |
| | | | γ | 0.455 | 62.7 |
| | | | γ | 0.693 | 12.6 |
| | | | γ | 0.562 | 11.4 |
| | | | γ | 3.438 | 10.8 |
| 37 | **Rb-79** | 22.9 m | β+ | 0.855 | 42.0 |
| | | | β+ | 1.039 | 14.2 |
| | | | β+ | 1.114 | 10.9 |
| | | | β+ | 0.825 | 10.4 |
| | | | γ | 0.688 | 23.4 |
| | | | γ | 0.183 | 19.4 |
| | | | γ | 0.143 | 14.1 |
| | | | γ | 0.130 | 10.9 |
| | | | γ | 0.505 | 10.8 |
| | | | γ | 0.147 | 10.6 |
| 37 | **Rb-81** | 4.6 h | β+ | 0.448 | 25.0 |
| | | | EC | 2.072 | 39.0 |
| | | | EC | 1.597 | 19.9 |
| | | | γ | 0.190 | 64.9 |
| | | | γ | 0.446 | 23.5 |
| 37 | **Rb-82** | 6.5 h | β+ | 0.353 | 19.7 |
| | | | EC | 1.825 | 66.7 |
| | | | γ | 0.777 | 84.4 |
| | | | γ | 0.554 | 62.4 |
| | | | γ | 0.619 | 38.0 |
| | | | γ | 1.044 | 32.1 |
| | | | γ | 0.698 | 26.3 |
| | | | γ | 1.317 | 23.7 |
| | | | γ | 0.828 | 21.0 |
| | | | γ | 1.475 | 15.5 |
| 37 | **Rb-84** | 32.8 d | β+ | 0.759 | 13.1 |
| | | | β+ | 0.341 | 12.6 |
| | | | EC | 0.782 | 56.0 |
| | | | EC | 1.658 | 13.4 |
| | | | γ | 0.882 | 68.9 |

| Atomic number | Radio-nuclide | Half-life | Decay | Energy in MeV | Intensity (abs) in % |
|---|---|---|---|---|---|
| 37 | **Rb-84m1** | 20.3 m | γ | 0.248 | 63.0 |
| | | | γ | 0.464 | 33.1 |
| | | | γ | 0.216 | 31.3 |
| 37 | **Rb-86** | 18.6 d | β- | 0.710 | 91.4 |
| 37 | **Rb-88** | 17.8 m | β- | 2.371 | 76.5 |
| | | | β- | 1.069 | 13.6 |
| | | | γ | 1.836 | 22.8 |
| | | | γ | 0.898 | 14.4 |
| 37 | **Rb-89** | 15.3 m | β- | 0.900 | 37.0 |
| | | | β- | 0.471 | 35.7 |
| | | | β- | 1.985 | 18.8 |
| | | | γ | 1.032 | 63.0 |
| | | | γ | 1.248 | 46.0 |
| | | | γ | 2.196 | 14.5 |
| | | | γ | 0.658 | 10.8 |
| | | | γ | 2.570 | 10.7 |
| | | | γ | 0.948 | 10.0 |
| 38 | **Sr-80** | 106.3 m | EC | 1.276 | 42.0 |
| | | | EC | 1.865 | 31.0 |
| | | | EC | 1.312 | 12.0 |
| | | | γ | 0.589 | 39.0 |
| | | | γ | 0.175 | 10.1 |
| 38 | **Sr-81** | 22.3 m | β+ | 1.169 | 28.0 |
| | | | β+ | 1.102 | 16.0 |
| | | | β+ | 1.310 | 13.0 |
| | | | γ | 0.154 | 34.0 |
| | | | γ | 0.148 | 30.0 |
| | | | γ | 0.443 | 17.5 |
| | | | γ | 0.188 | 15.4 |
| 38 | **Sr-82** | 25.4 d | EC | 0.178 | 100.0 |
| 38 | **Sr-83** | 32.4 h | β+ | 0.548 | 12.4 |
| | | | β+ | 0.529 | 11.0 |
| | | | EC | 1.468 | 25.1 |
| | | | EC | 2.231 | 12.0 |
| | | | EC | 1.849 | 11.5 |
| | | | EC | 2.273 | 11.1 |
| | | | γ | 0.763 | 26.7 |
| | | | γ | 0.382 | 14.0 |
| 38 | **Sr-85m1** | 67.6 m | EC | 1.152 | 13.3 |
| | | | γ | 0.232 | 83.9 |
| | | | γ | 0.151 | 12.8 |
| 38 | **Sr-87** | 2.8 h | γ | 0.389 | 82.2 |
| 38 | **Sr-89** | 50.6 d | β- | 0.587 | 100 |
| 38 | **Sr-91** | 9.7 h | β- | 0.405 | 34.8 |
| | | | β- | 1.128 | 28.6 |
| | | | β- | 0.525 | 25.1 |

| Atomic number | Radio-nuclide | Half-life | Decay | Energy in MeV | Intensity (abs) in % |
|---|---|---|---|---|---|
| | | | γ | 1.024 | 33.5 |
| | | | γ | 0.750 | 23.7 |
| 38 | **Sr-92** | 2.6 h | β- | 0.182 | 97.0 |
| | | | γ | 1.384 | 90.0 |
| 39 | **Y-84** | 39.5 m | β+ | 1.321 | 28.0 |
| | | | β+ | 1.104 | 14.5 |
| | | | γ | 0.793 | 98.3 |
| | | | γ | 0.974 | 78.0 |
| | | | γ | 1.040 | 56.0 |
| | | | γ | 0.661 | 11.3 |
| | | | γ | 0.463 | 10.1 |
| 39 | **Y-85** | 2.7 h | β+ | 0.659 | 38.0 |
| | | | β+ | 0.889 | 24.0 |
| | | | EC | 2.518 | 21.0 |
| | | | γ | 0.232 | 84.0 |
| | | | γ | 0.504 | 60.0 |
| 39 | **Y-85m1** | 4.9 h | β+ | 1.008 | 52.0 |
| | | | EC | 1.157 | 11.7 |
| | | | γ | 0.232 | 22.8 |
| 39 | **Y-86** | 14.7 h | β+ | 0.535 | 11.9 |
| | | | EC | 1.409 | 14.2 |
| | | | EC | 2.243 | 12.9 |
| | | | γ | 1.077 | 82.5 |
| | | | γ | 0.628 | 32.6 |
| | | | γ | 1.153 | 30.5 |
| | | | γ | 0.777 | 22.4 |
| | | | γ | 1.921 | 20.8 |
| | | | γ | 1.854 | 17.2 |
| | | | γ | 0.443 | 16.9 |
| | | | γ | 0.703 | 15.4 |
| 39 | **Y-86m1** | 47.4 m | γ | 0.208 | 93.8 |
| 39 | **Y-87** | 79.8 h | EC | 0.988 | 93.4 |
| | | | γ | 0.485 | 89.8 |
| | | | γ | 0.389 | 82.2 |
| 39 | **Y-87m1** | 13.4 h | γ | 0.381 | 78.1 |
| 39 | **Y-90** | 64.0 h | β- | 0.934 | 100.0 |
| 39 | **Y-90m1** | 3.2 h | γ | 0.203 | 97.3 |
| | | | γ | 0.480 | 90.7 |
| 39 | **Y-91m1** | 49.7 m | γ | 0.556 | 95.0 |
| 39 | **Y-92** | 3.5 h | β- | 1.567 | 85.7 |
| | | | γ | 0.934 | 13.9 |
| 39 | **Y-93** | 10.2 h | β- | 1.217 | 89.5 |
| 39 | **Y-94** | 18.7 m | β- | 2.174 | 41.0 |
| | | | β- | 1.741 | 39.6 |
| | | | γ | 0.919 | 56.0 |
| 39 | **Y-95** | 10.3 m | β- | 1.936 | 64.0 |
| | | | γ | 0.954 | 15.8 |
| 40 | **Zr-86** | 16.5 h | EC | 1.043 | 95.0 |
| | | | γ | 0.243 | 95.8 |
| | | | γ | 0.029 | 21.6 |
| 40 | **Zr-87** | 1.7 h | β+ | 1.014 | 80.5 |
| | | | EC | 3.291 | 13.9 |
| 40 | **Zr-89** | 78.4 h | β+ | 0.396 | 22.7 |
| | | | EC | 1.924 | 76.2 |
| | | | γ | 0.909 | 99.0 |
| 40 | **Zr-97** | 16.7 h | β- | 0.757 | 87.8 |
| | | | γ | 0.743 | 93.1 |
| 41 | **Nb-88** | 14.5 m | β+ | 1.374 | 54.0 |
| | | | β+ | 1.612 | 25.0 |
| | | | γ | 1.083 | 103.0 |
| | | | γ | 1.057 | 100.0 |
| | | | γ | 0.671 | 64.0 |
| | | | γ | 0.503 | 60.0 |
| | | | γ | 0.399 | 31.8 |
| | | | γ | 0.272 | 30.1 |
| | | | γ | 0.077 | 22.4 |
| 41 | **Nb-89** | 2.0 h | β+ | 1.453 | 74.0 |
| 41 | **Nb-89m1** | 66.0 m | β+ | 0.978 | 62.0 |
| | | | β+ | 1.215 | 15.0 |
| | | | EC | 3.212 | 13.0 |
| | | | γ | 0.588 | 95.6 |
| | | | γ | 0.507 | 81.0 |
| 41 | **Nb-90** | 14.6 h | β+ | 0.662 | 51.1 |
| | | | EC | 2.522 | 35.9 |
| | | | γ | 1.129 | 92.7 |
| | | | γ | 2.319 | 82.0 |
| | | | γ | 0.141 | 66.8 |
| | | | γ | 2.186 | 18.0 |
| 41 | **Nb-92** | 10.2 d | EC | 1.207 | 97.3 |
| | | | γ | 0.934 | 99.2 |
| 41 | **Nb-95** | 35.0 d | β- | 0.043 | 100.0 |
| | | | γ | 0.766 | 99.8 |
| 41 | **Nb-95m1** | 3.6 d | γ | 0.236 | 24.8 |
| 41 | **Nb-96** | 23.4 h | β- | 0.251 | 96.7 |
| | | | γ | 0.778 | 96.5 |
| | | | γ | 0.569 | 58.0 |
| | | | γ | 1.091 | 48.5 |
| | | | γ | 0.460 | 26.6 |
| | | | γ | 0.850 | 20.5 |
| | | | γ | 1.200 | 20.0 |
| | | | γ | 0.810 | 11.1 |
| 41 | **Nb-97** | 72.1 m | β- | 0.471 | 98.4 |

| Atomic number | Radio-nuclide | Half-life | Decay | Energy in MeV | Intensity (abs) in % |
|---|---|---|---|---|---|
| | | | γ | 0.658 | 98.2 |
| 41 | **Nb-98m1** | 51.3 m | β- | 0.791 | 27.9 |
| | | | β- | 1.001 | 15.9 |
| | | | β- | 0.550 | 10.4 |
| | | | γ | 0.787 | 93.4 |
| | | | γ | 0.723 | 73.8 |
| | | | γ | 1.169 | 17.8 |
| | | | γ | 0.834 | 10.8 |
| 42 | **Mo-90** | 5.6 h | β+ | 0.477 | 24.9 |
| | | | EC | 2.107 | 56.0 |
| | | | γ | 0.257 | 78.0 |
| | | | γ | 0.122 | 64.0 |
| 42 | **Mo-91** | 15.5 m | β+ | 1.550 | 93.3 |
| 42 | **Mo-93m1** | 6.9 h | γ | 0.685 | 99.9 |
| | | | γ | 1.477 | 99.1 |
| | | | γ | 0.263 | 57.4 |
| 42 | **Mo-99** | 65.9 h | β- | 0.443 | 82.2 |
| | | | β- | 0.133 | 16.4 |
| | | | γ | 0.740 | 12.2 |
| 42 | **Mo-101** | 14.6 m | β- | 0.261 | 20.6 |
| | | | β- | 0.295 | 15.0 |
| | | | β- | 1.079 | 12.8 |
| | | | γ | 0.590 | 19.2 |
| | | | γ | 0.192 | 18.2 |
| | | | γ | 1.012 | 13.0 |
| | | | γ | 0.506 | 11.6 |
| 42 | **Mo-102** | 11.3 m | β- | 0.358 | 94.1 |
| 43 | **Tc-93** | 2.8 h | EC | 1.838 | 56.7 |
| | | | EC | 1.681 | 22.2 |
| | | | γ | 1.363 | 66.2 |
| | | | γ | 1.520 | 24.4 |
| 43 | **Tc-93m1** | 43.5 m | EC | 0.948 | 14.3 |
| | | | γ | 0.392 | 58.3 |
| | | | γ | 2.645 | 14.3 |
| 43 | **Tc-94** | 293.0 m | β+ | 0.358 | 10.5 |
| | | | EC | 1.833 | 70.8 |
| | | | γ | 0.871 | 99.9 |
| | | | γ | 0.703 | 99.6 |
| | | | γ | 0.850 | 95.7 |
| 43 | **Tc-94m1** | 52.0 m | β+ | 1.094 | 67.6 |
| | | | EC | 3.461 | 12.8 |
| | | | γ | 0.871 | 94.2 |
| 43 | **Tc-95** | 20.0 h | EC | 0.925 | 93.8 |
| | | | γ | 0.766 | 93.8 |
| 43 | **Tc-96** | 4.3 d | EC | 0.532 | 79.0 |

| Atomic number | Radio-nuclide | Half-life | Decay | Energy in MeV | Intensity (abs) in % |
|---|---|---|---|---|---|
| | | | EC | 0.218 | 19.5 |
| | | | γ | 0.778 | 99.8 |
| | | | γ | 0.850 | 98.0 |
| | | | γ | 0.813 | 82.0 |
| | | | γ | 1.127 | 15.2 |
| 43 | **Tc-99m1** | 6.0 h | γ | 0.141 | 89.0 |
| 43 | **Tc-101** | 14.2 m | β- | 0.487 | 90.3 |
| | | | γ | 0.307 | 89.0 |
| 43 | **Tc-104** | 18.3 m | β- | 2.326 | 16.0 |
| | | | γ | 0.358 | 89.0 |
| | | | γ | 0.531 | 15.6 |
| | | | γ | 0.535 | 14.7 |
| | | | γ | 0.884 | 10.9 |
| | | | γ | 0.893 | 10.2 |
| 44 | **Ru-94** | 51.8 m | EC | 1.144 | 73.0 |
| | | | EC | 0.619 | 27.0 |
| | | | γ | 0.367 | 75.0 |
| | | | γ | 0.891 | 25.0 |
| 44 | **Ru-95** | 1.6 h | β+ | 0.533 | 12.6 |
| | | | EC | 2.222 | 24.4 |
| | | | EC | 1.132 | 24.0 |
| | | | γ | 0.336 | 69.9 |
| | | | γ | 1.097 | 20.9 |
| | | | γ | 0.627 | 17.8 |
| 44 | **Ru-97** | 2.8 d | EC | 0.892 | 87.7 |
| | | | EC | 0.784 | 11.0 |
| | | | γ | 0.216 | 85.6 |
| | | | γ | 0.324 | 10.8 |
| 44 | **Ru-103** | 39.2 d | β- | 0.064 | 92.0 |
| | | | γ | 0.497 | 91.0 |
| 44 | **Ru-105** | 4.4 h | β- | 0.432 | 47.8 |
| | | | β- | 0.398 | 18.8 |
| | | | β- | 0.406 | 16.9 |
| | | | γ | 0.724 | 47.3 |
| | | | γ | 0.469 | 17.5 |
| | | | γ | 0.676 | 15.7 |
| | | | γ | 0.316 | 11.1 |
| 45 | **Rh-97** | 30.7 m | β+ | 0.923 | 49.1 |
| | | | EC | 3.098 | 19.1 |
| | | | γ | 0.422 | 74.6 |
| | | | γ | 0.840 | 12.0 |
| 45 | **Rh-97m1** | 46.2 m | β+ | 1.155 | 10.4 |
| | | | EC | 1.581 | 20.4 |
| | | | EC | 1.533 | 18.5 |
| | | | γ | 0.189 | 48.5 |
| | | | γ | 2.246 | 13.8 |

| Atomic number | Radio-nuclide | Half-life | Decay | Energy in MeV | Intensity (abs) in % |
|---|---|---|---|---|---|
| | | | γ | 0.422 | 12.7 |
| 45 | **Rh-99** | 16.1 d | EC | 1.485 | 45.0 |
| | | | EC | 1.660 | 31.3 |
| | | | γ | 0.528 | 37.9 |
| | | | γ | 0.353 | 34.5 |
| | | | γ | 0.090 | 33.4 |
| 45 | **Rh-99m1** | 4.7 h | EC | 1.768 | 60.0 |
| | | | EC | 1.491 | 13.9 |
| | | | EC | 0.847 | 12.7 |
| | | | γ | 0.341 | 72.0 |
| | | | γ | 0.618 | 12.3 |
| | | | γ | 1.261 | 11.4 |
| 45 | **Rh-100** | 20.8 h | EC | 0.719 | 68.6 |
| | | | EC | 1.166 | 19.9 |
| | | | γ | 0.540 | 80.6 |
| | | | γ | 2.376 | 32.6 |
| | | | γ | 0.823 | 21.1 |
| | | | γ | 1.553 | 20.7 |
| | | | γ | 1.362 | 15.4 |
| | | | γ | 1.107 | 13.6 |
| | | | γ | 0.446 | 12.0 |
| | | | γ | 1.930 | 11.6 |
| 45 | **Rh-101m1** | 4.3 d | EC | 0.393 | 83.0 |
| | | | γ | 0.307 | 81.0 |
| 45 | **Rh-105** | 35.4 h | β- | 0.179 | 75.0 |
| | | | β- | 0.070 | 19.7 |
| | | | γ | 0.319 | 19.1 |
| 45 | **Rh-106m1** | 131.0 m | β- | 0.318 | 85.0 |
| | | | β- | 0.240 | 14.5 |
| | | | γ | 0.512 | 85.0 |
| | | | γ | 1.047 | 30.4 |
| | | | γ | 0.717 | 28.9 |
| | | | γ | 0.451 | 24.2 |
| | | | γ | 0.616 | 20.2 |
| | | | γ | 0.749 | 19.3 |
| | | | γ | 1.529 | 17.5 |
| | | | γ | 1.128 | 13.7 |
| | | | γ | 0.825 | 13.6 |
| | | | γ | 0.429 | 13.3 |
| | | | γ | 0.805 | 13.0 |
| | | | γ | 0.406 | 11.6 |
| | | | γ | 1.201 | 11.4 |
| 45 | **Rh-107** | 21.7 m | β- | 0.437 | 64.0 |
| | | | β- | 0.569 | 13.0 |
| | | | γ | 0.303 | 66.0 |

| Atomic number | Radio-nuclide | Half-life | Decay | Energy in MeV | Intensity (abs) in % |
|---|---|---|---|---|---|
| 46 | **Pd-98** | 17.7 m | EC | 1.755 | 61.0 |
| | | | EC | 1.029 | 24.0 |
| | | | γ | 0.112 | 58.0 |
| | | | γ | 0.662 | 19.7 |
| | | | γ | 0.107 | 13.9 |
| 46 | **Pd-99** | 21.4 m | β+ | 0.972 | 36.0 |
| | | | EC | 3.198 | 13.0 |
| | | | γ | 0.136 | 73.0 |
| | | | γ | 0.264 | 15.0 |
| 46 | **Pd-100** | 3.6 d | EC | 0.199 | 92.0 |
| | | | γ | 0.084 | 52.0 |
| | | | γ | 0.075 | 48.0 |
| 46 | **Pd-101** | 8.5 h | EC | 1.798 | 52.2 |
| | | | EC | 1.232 | 21.6 |
| | | | EC | 1.510 | 11.4 |
| | | | γ | 0.296 | 19.2 |
| | | | γ | 0.590 | 12.1 |
| 46 | **Pd-103** | 17.0 d | EC | 0.503 | 99.9 |
| 46 | **Pd-109** | 13.6 h | β- | 0.360 | 100.0 |
| 46 | **Pd-111** | 23.4 m | β- | 0.852 | 95.3 |
| 46 | **Pd-111m1** | 5.5 h | γ | 0.172 | 46.0 |
| 46 | **Pd-112** | 21.0 h | β- | 0.077 | 100.0 |
| | | | γ | 0.019 | 27.0 |
| 47 | **Ag-101** | 11.1 m | β+ | 1.319 | 37.0 |
| | | | β+ | 0.896 | 11.2 |
| | | | γ | 0.261 | 52.6 |
| | | | γ | 0.588 | 10.0 |
| 47 | **Ag-103** | 65.7 m | β+ | 0.614 | 14.8 |
| | | | EC | 2.421 | 25.2 |
| | | | EC | 1.414 | 20.5 |
| | | | γ | 0.119 | 31.2 |
| | | | γ | 0.148 | 28.3 |
| | | | γ | 0.267 | 13.3 |
| 47 | **Ag-104** | 69.2 m | EC | 2.014 | 29.0 |
| | | | EC | 2.197 | 12.7 |
| | | | EC | 2.097 | 12.2 |
| | | | γ | 0.556 | 92.6 |
| | | | γ | 0.768 | 65.7 |
| | | | γ | 0.942 | 25.0 |
| | | | γ | 0.926 | 12.5 |
| | | | γ | 0.858 | 10.4 |
| 47 | **Ag-104m1** | 33.5 m | β+ | 1.222 | 58.0 |
| | | | EC | 3.730 | 12.0 |
| | | | γ | 0.556 | 90.0 |
| 47 | **Ag-105** | 41.3 d | EC | 1.000 | 65.0 |
| | | | EC | 0.257 | 17.2 |

| Atomic number | Radio-nuclide | Half-life | Decay | Energy in MeV | Intensity (abs) in % |
|---|---|---|---|---|---|
| | | | γ | 0.345 | 41.4 |
| | | | γ | 0.280 | 30.2 |
| | | | γ | 0.645 | 11.1 |
| | | | γ | 0.064 | 10.5 |
| | | | γ | 0.443 | 10.5 |
| 47 | **Ag-106** | 24.0 m | β+ | 0.868 | 52.6 |
| | | | EC | 2.965 | 30.3 |
| | | | γ | 0.512 | 17.0 |
| 47 | **Ag-106m1** | 8.3 d | EC | 0.298 | 92.0 |
| | | | γ | 0.512 | 88.0 |
| | | | γ | 1.046 | 29.6 |
| | | | γ | 0.717 | 28.9 |
| | | | γ | 0.451 | 28.2 |
| | | | γ | 0.616 | 21.6 |
| | | | γ | 0.748 | 20.6 |
| | | | γ | 1.528 | 16.3 |
| | | | γ | 0.825 | 15.3 |
| | | | γ | 0.406 | 13.4 |
| | | | γ | 0.430 | 13.2 |
| | | | γ | 0.804 | 12.4 |
| | | | γ | 1.128 | 11.8 |
| | | | γ | 1.199 | 11.2 |
| 47 | **Ag-111** | 7.5 d | β- | 0.360 | 92.0 |
| 47 | **Ag-112** | 3.1 h | β- | 1.703 | 54.0 |
| | | | β- | 1.426 | 20.0 |
| | | | γ | 0.618 | 42.0 |
| 47 | **Ag-113** | 5.4 h | β- | 0.794 | 85.0 |
| | | | γ | 0.299 | 10.0 |
| 47 | **Ag-115** | 20.0 m | β- | 1.296 | 60.0 |
| | | | β- | 1.189 | 12.5 |
| | | | γ | 0.229 | 18.0 |
| 48 | **Cd-104** | 57.7 m | EC | 1.046 | 71.8 |
| | | | EC | 0.421 | 28.5 |
| | | | γ | 0.084 | 47.0 |
| | | | γ | 0.709 | 19.5 |
| 48 | **Cd-105** | 55.5 m | β+ | 0.754 | 27.3 |
| | | | EC | 2.713 | 26.7 |
| 48 | **Cd-107** | 6.5 h | EC | 1.324 | 99.7 |
| 48 | **Cd-111** | 48.5 m | γ | 0.245 | 94.0 |
| | | | γ | 0.151 | 29.1 |
| 48 | **Cd-115** | 53.5 h | β- | 0.394 | 62.6 |
| | | | β- | 0.184 | 33.1 |
| | | | γ | 0.528 | 27.4 |
| 48 | **Cd-115m1** | 44.6 d | β- | 0.618 | 97.0 |
| 48 | **Cd-117** | 2.5 h | β- | 0.202 | 32.0 |
| | | | β- | 0.880 | 21.0 |

| Atomic number | Radio-nuclide | Half-life | Decay | Energy in MeV | Intensity (abs) in % |
|---|---|---|---|---|---|
| | | | β- | 0.683 | 13.2 |
| | | | γ | 0.273 | 27.9 |
| | | | γ | 1.303 | 18.4 |
| | | | γ | 0.344 | 17.9 |
| | | | γ | 1.577 | 11.2 |
| 48 | **Cd-117m1** | 3.4 h | β- | 0.216 | 48.0 |
| | | | β- | 0.179 | 20.5 |
| | | | γ | 1.997 | 26.2 |
| | | | γ | 1.066 | 23.1 |
| | | | γ | 0.564 | 14.7 |
| | | | γ | 1.433 | 13.4 |
| | | | γ | 1.029 | 11.7 |
| | | | γ | 1.235 | 11.0 |
| 48 | **Cd-118** | 50.3 m | β- | 0.161 | 100.0 |
| 49 | **In-107** | 32.4 m | β+ | 1.020 | 27.0 |
| | | | EC | 3.220 | 11.5 |
| | | | γ | 0.205 | 47.2 |
| | | | γ | 0.506 | 11.9 |
| | | | γ | 0.321 | 10.2 |
| 49 | **In-108m1** | 39.6 m | β+ | 1.599 | 30.4 |
| | | | γ | 0.633 | 76.4 |
| | | | γ | 1.986 | 12.4 |
| 49 | **In-109** | 4.2 h | EC | 1.813 | 63.4 |
| | | | γ | 0.203 | 74.2 |
| 49 | **In-110** | 4.9 h | EC | 0.756 | 43.8 |
| | | | EC | 0.691 | 29.6 |
| | | | γ | 0.658 | 98.0 |
| | | | γ | 0.885 | 93.0 |
| | | | γ | 0.937 | 68.4 |
| | | | γ | 0.707 | 29.5 |
| | | | γ | 0.642 | 26.0 |
| | | | γ | 0.997 | 10.5 |
| 49 | **In-110m1** | 69.1 m | β+ | 1.015 | 60.7 |
| | | | EC | 3.282 | 26.5 |
| | | | γ | 0.658 | 97.7 |
| 49 | **In-111** | 2.8 d | EC | 0.445 | 100.0 |
| | | | γ | 0.245 | 94.1 |
| | | | γ | 0.171 | 90.6 |
| 49 | **In-112** | 14.9 m | β- | 0.215 | 38.0 |
| | | | β+ | 0.697 | 23.3 |
| | | | EC | 2.582 | 32.0 |
| | | | γ | 0.157 | 13.3 |
| 49 | **In-113** | 99.5 m | γ | 0.392 | 64.9 |
| 49 | **In-114m1** | 49.5 d | γ | 0.190 | 15.6 |
| 49 | **In-115m1** | 4.5 h | β- | 0.279 | 100.0 |
| | | | γ | 0.336 | 45.9 |

| Atomic number | Radio-nuclide | Half-life | Decay | Energy in MeV | Intensity (abs) in % |
|---|---|---|---|---|---|
| 49 | **In-116m1** | 54.3 m | β- | 0.352 | 54.2 |
| | | | β- | 0.296 | 32.5 |
| | | | β- | 0.190 | 10.3 |
| | | | γ | 1.294 | 84.8 |
| | | | γ | 1.097 | 58.5 |
| | | | γ | 0.417 | 27.2 |
| | | | γ | 2.112 | 15.1 |
| | | | γ | 0.819 | 12.1 |
| 49 | **In-117** | 43.2 m | β- | 0.245 | 99.8 |
| | | | γ | 0.553 | 100.0 |
| | | | γ | 0.159 | 87.0 |
| 49 | **In-117m1** | 116.2 m | β- | 0.680 | 18.3 |
| | | | γ | 0.315 | 19.1 |
| | | | γ | 0.159 | 15.9 |
| 49 | **In-119m1** | 18.0 m | β- | 1.094 | 61.2 |
| | | | β- | 1.083 | 33.5 |
| 50 | **Sn-108** | 10.3 m | EC | 1.376 | 87.5 |
| | | | γ | 0.396 | 64.3 |
| | | | γ | 0.273 | 45.5 |
| | | | γ | 0.669 | 22.6 |
| | | | γ | 0.168 | 19.9 |
| | | | γ | 0.104 | 13.8 |
| 50 | **Sn-109** | 18.1 m | γ | 0.650 | 29.6 |
| | | | γ | 1.099 | 29.3 |
| | | | γ | 1.321 | 11.5 |
| 50 | **Sn-110** | 4.2 h | EC | 0.288 | 100.0 |
| | | | γ | 0.280 | 97.1 |
| 50 | **Sn-111** | 35.3 m | β+ | 0.635 | 30.2 |
| | | | EC | 2.451 | 62.7 |
| 50 | **Sn-117** | 14.0 d | γ | 0.159 | 86.4 |
| 50 | **Sn-121** | 27.0 h | β- | 0.116 | 100.0 |
| 50 | **Sn-123m1** | 40.1 m | β- | 0.459 | 99.9 |
| | | | γ | 0.160 | 85.7 |
| 50 | **Sn-125** | 9.6 d | β- | 0.941 | 81.4 |
| | | | γ | 1.067 | 10.0 |
| 50 | **Sn-127** | 2.1 h | β- | 1.325 | 22.0 |
| | | | β- | 0.195 | 11.2 |
| | | | γ | 1.114 | 38.0 |
| | | | γ | 1.096 | 19.0 |
| | | | γ | 0.823 | 11.0 |
| 50 | **Sn-128** | 59.1 m | β- | 0.211 | 84.0 |
| | | | β- | 0.139 | 16.1 |
| | | | γ | 0.482 | 59.0 |
| | | | γ | 0.075 | 28.0 |
| | | | γ | 0.557 | 16.5 |
| | | | γ | 0.681 | 15.9 |
| | | | γ | 0.046 | 13.0 |
| 51 | **Sb-115** | 32.1 m | β+ | 0.675 | 33.3 |
| | | | EC | 2.530 | 63.0 |
| | | | γ | 0.497 | 97.9 |
| 51 | **Sb-116** | 15.8 m | β+ | 1.076 | 40.0 |
| | | | β+ | 0.652 | 13.0 |
| | | | EC | 2.482 | 27.0 |
| | | | EC | 3.413 | 18.0 |
| | | | γ | 1.294 | 85.0 |
| | | | γ | 0.932 | 24.8 |
| | | | γ | 2.225 | 14.6 |
| 51 | **Sb-116m1** | 60.3 m | β+ | 0.619 | 24.0 |
| | | | EC | 2.181 | 58.0 |
| | | | EC | 1.880 | 12.6 |
| | | | γ | 0.100 | 100.0 |
| | | | γ | 0.407 | 100.0 |
| | | | γ | 1.294 | 100.0 |
| | | | γ | 0.973 | 74.0 |
| | | | γ | 0.543 | 48.1 |
| | | | γ | 0.136 | 28.5 |
| | | | γ | 1.072 | 25.5 |
| | | | γ | 0.844 | 11.2 |
| 51 | **Sb-117** | 2.8 h | EC | 1.586 | 97.2 |
| | | | γ | 0.159 | 85.9 |
| 51 | **Sb-118m1** | 5.0 h | EC | 1.332 | 98.3 |
| | | | γ | 1.230 | 100.0 |
| | | | γ | 0.254 | 99.0 |
| | | | γ | 1.051 | 97.0 |
| | | | γ | 0.041 | 30.0 |
| 51 | **Sb-119** | 38.2 h | EC | 0.567 | 100.0 |
| | | | γ | 0.024 | 16.5 |
| 51 | **Sb-120** | 15.9 m | β+ | 0.742 | 41.0 |
| | | | EC | 2.681 | 57.3 |
| 51 | **Sb-120m1** | 5.8 d | EC | 0.199 | 100.0 |
| | | | γ | 1.172 | 100.0 |
| | | | γ | 1.023 | 99.4 |
| | | | γ | 0.197 | 87.0 |
| | | | γ | 0.090 | 79.5 |
| 51 | **Sb-122** | 2.7 d | β- | 0.524 | 66.7 |
| | | | β- | 0.773 | 26.1 |
| | | | γ | 0.564 | 70.7 |
| 51 | **Sb-126** | 12.4 d | β- | 0.146 | 29.0 |
| | | | β- | 0.735 | 20.0 |
| | | | β- | 0.419 | 16.0 |
| | | | γ | 0.667 | 99.6 |
| | | | γ | 0.695 | 99.6 |

| Atomic number | Radio-nuclide | Half-life | Decay | Energy in MeV | Intensity (abs) in % |
|---|---|---|---|---|---|
| | | | γ | 0.415 | 83.3 |
| | | | γ | 0.721 | 53.8 |
| | | | γ | 0.697 | 29.0 |
| | | | γ | 0.857 | 17.6 |
| 51 | **Sb-126m1** | 19.2 m | β- | 0.743 | 83.0 |
| | | | γ | 0.415 | 86.0 |
| | | | γ | 0.666 | 86.0 |
| | | | γ | 0.695 | 82.0 |
| 51 | **Sb-127** | 3.9 d | β- | 0.304 | 35.8 |
| | | | β- | 0.391 | 23.4 |
| | | | β- | 0.265 | 18.0 |
| | | | γ | 0.686 | 36.8 |
| | | | γ | 0.473 | 25.8 |
| | | | γ | 0.784 | 15.1 |
| 51 | **Sb-128** | 9.1 h | β- | 0.792 | 19.0 |
| | | | β- | 0.325 | 15.3 |
| | | | β- | 0.434 | 13.0 |
| | | | γ | 0.743 | 100.0 |
| | | | γ | 0.754 | 100.0 |
| | | | γ | 0.314 | 61.0 |
| | | | γ | 0.527 | 45.0 |
| | | | γ | 0.636 | 36.0 |
| | | | γ | 0.629 | 31.0 |
| | | | γ | 0.654 | 17.0 |
| | | | γ | 0.814 | 13.0 |
| 51 | **Sb-128m1** | 10.4 m | β- | 0.000 | 78.0 |
| | | | γ | 0.743 | 96.0 |
| | | | γ | 0.754 | 96.0 |
| | | | γ | 0.314 | 89.0 |
| 51 | **Sb-129** | 4.4 h | β- | 0.207 | 29.9 |
| | | | β- | 0.165 | 25.5 |
| | | | γ | 0.813 | 48.2 |
| | | | γ | 0.915 | 23.3 |
| | | | γ | 0.545 | 15.4 |
| | | | γ | 1.031 | 15.1 |
| 51 | **Sb-129m1** | 17.7 m | β- | 0.904 | 63.0 |
| | | | β- | 1.043 | 10.0 |
| | | | γ | 0.760 | 85.0 |
| | | | γ | 0.658 | 78.0 |
| | | | γ | 0.434 | 62.0 |
| | | | γ | 1.129 | 15.0 |
| | | | γ | 0.723 | 14.2 |
| 51 | **Sb-130** | 39.5 m | β- | 1.154 | 18.0 |
| | | | γ | 0.793 | 100.0 |
| | | | γ | 0.840 | 100.0 |
| | | | γ | 0.331 | 78.0 |

| Atomic number | Radio-nuclide | Half-life | Decay | Energy in MeV | Intensity (abs) in % |
|---|---|---|---|---|---|
| | | | γ | 0.182 | 65.0 |
| | | | γ | 0.732 | 22.0 |
| | | | γ | 0.935 | 19.0 |
| | | | γ | 0.468 | 18.0 |
| 51 | **Sb-131** | 23.0 m | β- | 0.491 | 29.0 |
| | | | β- | 1.046 | 12.0 |
| | | | γ | 0.943 | 47.1 |
| | | | γ | 0.933 | 26.4 |
| | | | γ | 0.642 | 24.0 |
| 52 | **Te-114** | 15.2 m | β+ | 0.756 | 16.0 |
| | | | EC | 0.686 | 25.0 |
| | | | EC | 2.583 | 24.0 |
| 52 | **Te-116** | 2.5 h | EC | 1.456 | 93.7 |
| | | | γ | 0.094 | 33.1 |
| 52 | **Te-117** | 62.0 m | β+ | 0.809 | 24.1 |
| | | | EC | 2.829 | 28.2 |
| | | | EC | 1.833 | 18.1 |
| | | | EC | 1.249 | 11.4 |
| | | | γ | 0.720 | 64.7 |
| | | | γ | 1.716 | 15.9 |
| | | | γ | 2.300 | 11.2 |
| 52 | **Te-118** | 6.0 d | EC | 0.278 | 100.0 |
| 52 | **Te-119** | 16.1 h | EC | 1.649 | 80.7 |
| | | | γ | 0.644 | 84.1 |
| | | | γ | 0.700 | 10.1 |
| 52 | **Te-119m1** | 4.7 d | EC | 1.188 | 67.0 |
| | | | γ | 1.213 | 66.1 |
| | | | γ | 0.154 | 66.0 |
| | | | γ | 0.271 | 28.0 |
| 52 | **Te-121** | 19.2 d | EC | 0.481 | 82.0 |
| | | | EC | 0.546 | 18.4 |
| | | | γ | 0.573 | 80.4 |
| | | | γ | 0.508 | 17.7 |
| 52 | **Te-127** | 9.4 h | β- | 0.228 | 98.8 |
| 52 | **Te-129** | 69.6 m | β- | 0.547 | 89.0 |
| | | | γ | 0.028 | 16.3 |
| 52 | **Te-129m1** | 33.6 d | β- | 0.609 | 32.0 |
| 52 | **Te-131** | 25.0 m | β- | 0.818 | 59.3 |
| | | | β- | 0.616 | 21.6 |
| | | | β- | 0.382 | 10.0 |
| | | | γ | 0.150 | 68.8 |
| | | | γ | 0.452 | 18.2 |
| 52 | **Te-131m1** | 33.3 h | β- | 0.131 | 26.5 |
| | | | β- | 0.160 | 11.9 |
| | | | γ | 0.774 | 36.8 |
| | | | γ | 0.852 | 19.9 |

| Atomic number | Radio-nuclide | Half-life | Decay | Energy in MeV | Intensity (abs) in % |
|---|---|---|---|---|---|
| | | | γ | 0.794 | 13.4 |
| | | | γ | 1.125 | 11.0 |
| 52 | **Te-132** | 3.2 d | β- | 0.060 | 100.0 |
| | | | γ | 0.228 | 88.0 |
| | | | γ | 0.050 | 15.0 |
| 52 | **Te-133** | 12.5 m | β- | 0.880 | 28.2 |
| | | | β- | 1.067 | 20.3 |
| | | | β- | 0.605 | 13.0 |
| | | | β- | 0.439 | 10.4 |
| | | | γ | 0.312 | 62.4 |
| | | | γ | 0.408 | 27.1 |
| | | | γ | 1.333 | 10.7 |
| 52 | **Te-133m1** | 55.4 m | β- | 0.220 | 13.6 |
| | | | γ | 0.913 | 44.0 |
| | | | γ | 0.648 | 15.5 |
| | | | γ | 0.864 | 12.5 |
| 52 | **Te-134** | 41.8 m | β- | 0.186 | 44.0 |
| | | | β- | 0.214 | 42.0 |
| | | | β- | 0.121 | 14.0 |
| | | | γ | 0.767 | 29.5 |
| | | | γ | 0.210 | 22.7 |
| | | | γ | 0.278 | 21.2 |
| | | | γ | 0.079 | 20.9 |
| | | | γ | 0.435 | 18.9 |
| | | | γ | 0.566 | 18.6 |
| | | | γ | 0.181 | 18.3 |
| | | | γ | 0.743 | 15.3 |
| 53 | **I-118** | 13.7 m | β+ | 2.504 | 32.8 |
| | | | β+ | 2.769 | 16.7 |
| | | | γ | 0.606 | 77.6 |
| | | | γ | 0.545 | 10.0 |
| 53 | **I-119** | 19.1 m | β+ | 1.005 | 50.4 |
| | | | EC | 3.163 | 33.3 |
| | | | γ | 0.258 | 86.3 |
| 53 | **I-120** | 81.6 m | β+ | 1.845 | 29.3 |
| | | | β+ | 2.099 | 19.0 |
| | | | γ | 0.560 | 69.6 |
| | | | γ | 1.523 | 10.9 |
| 53 | **I-120m1** | 53.0 m | β+ | 1.423 | 34.0 |
| | | | EC | 2.813 | 11.0 |
| | | | γ | 0.560 | 100.0 |
| | | | γ | 0.601 | 87.0 |
| | | | γ | 0.615 | 64.0 |
| | | | γ | 1.346 | 19.0 |
| 53 | **I-121** | 2.1 h | β+ | 0.475 | 10.3 |
| | | | EC | 2.080 | 76.2 |

| Atomic number | Radio-nuclide | Half-life | Decay | Energy in MeV | Intensity (abs) in % |
|---|---|---|---|---|---|
| | | | γ | 0.212 | 84.3 |
| 53 | **I-123** | 13.2 h | EC | 1.070 | 97.0 |
| | | | γ | 0.159 | 83.3 |
| 53 | **I-124** | 4.2 d | β+ | 0.687 | 11.7 |
| | | | β+ | 0.975 | 10.7 |
| | | | EC | 2.557 | 25.2 |
| | | | EC | 3.160 | 23.9 |
| | | | EC | 0.866 | 11.6 |
| | | | γ | 0.603 | 62.9 |
| | | | γ | 1.691 | 11.2 |
| | | | γ | 0.723 | 10.4 |
| 53 | **I-126** | 12.9 d | β- | 0.293 | 33.4 |
| | | | β- | 0.462 | 10.3 |
| | | | EC | 1.489 | 28.5 |
| | | | EC | 2.155 | 18.7 |
| | | | γ | 0.389 | 35.6 |
| | | | γ | 0.666 | 32.9 |
| 53 | **I-128** | 25.0 m | β- | 0.833 | 80.0 |
| | | | β- | 0.635 | 11.6 |
| | | | γ | 0.443 | 12.6 |
| 53 | **I-130** | 12.4 h | β- | 0.347 | 48.0 |
| | | | β- | 0.185 | 46.7 |
| | | | γ | 0.536 | 99.0 |
| | | | γ | 0.669 | 96.0 |
| | | | γ | 0.740 | 82.0 |
| | | | γ | 0.418 | 34.2 |
| | | | γ | 1.157 | 11.3 |
| 53 | **I-131** | 8.0 d | β- | 0.192 | 89.6 |
| | | | γ | 0.364 | 81.5 |
| 53 | **I-132** | 2.3 h | β- | 0.422 | 19.0 |
| | | | β- | 0.842 | 19.0 |
| | | | β- | 0.243 | 13.0 |
| | | | β- | 0.608 | 12.3 |
| | | | γ | 0.668 | 98.7 |
| | | | γ | 0.773 | 75.6 |
| | | | γ | 0.955 | 17.6 |
| | | | γ | 0.523 | 16.0 |
| | | | γ | 0.630 | 13.3 |
| 53 | **I-132m1** | 1.4 h | γ | 0.600 | 14.0 |
| | | | γ | 0.773 | 14.0 |
| | | | γ | 0.668 | 13.9 |
| 53 | **I-133** | 20.8 h | β- | 0.439 | 83.4 |
| | | | γ | 0.530 | 87.0 |
| 53 | **I-134** | 52.5 m | β- | 0.474 | 30.4 |
| | | | β- | 0.594 | 16.2 |
| | | | β- | 0.980 | 12.6 |

| Atomic number | Radio-nuclide | Half-life | Decay | Energy in MeV | Intensity (abs) in % |
|---|---|---|---|---|---|
| | | | β- | 0.699 | 11.0 |
| | | | γ | 0.847 | 96.0 |
| | | | γ | 0.884 | 65.1 |
| | | | γ | 1.073 | 14.9 |
| | | | γ | 0.595 | 11.1 |
| | | | γ | 0.622 | 10.6 |
| 53 | **I-135** | 6.6 h | β- | 0.499 | 23.6 |
| | | | β- | 0.324 | 21.8 |
| | | | γ | 1.260 | 28.7 |
| | | | γ | 1.132 | 22.6 |
| 54 | **Xe-120** | 40.0 m | EC | 1.935 | 35.0 |
| | | | EC | 0.994 | 15.9 |
| | | | γ | 0.025 | 29.0 |
| 54 | **Xe-121** | 40.1 m | β+ | 1.245 | 20.0 |
| | | | γ | 0.253 | 12.6 |
| | | | γ | 0.133 | 11.0 |
| 54 | **Xe-122** | 20.1 h | EC | 0.725 | 83.0 |
| 54 | **Xe-123** | 2.1 h | β+ | 0.674 | 17.4 |
| | | | EC | 2.546 | 43.9 |
| | | | EC | 2.517 | 10.6 |
| | | | γ | 0.149 | 48.9 |
| | | | γ | 0.178 | 14.9 |
| 54 | **Xe-125** | 16.9 h | EC | 1.401 | 66.4 |
| | | | EC | 1.456 | 25.3 |
| | | | γ | 0.188 | 53.8 |
| | | | γ | 0.243 | 30.0 |
| 54 | **Xe-127** | 36.3 d | EC | 0.459 | 53.0 |
| | | | EC | 0.287 | 47.6 |
| | | | γ | 0.203 | 68.7 |
| | | | γ | 0.172 | 25.7 |
| | | | γ | 0.375 | 17.3 |
| 54 | **Xe-133** | 5.2 d | β- | 0.101 | 98.5 |
| | | | γ | 0.081 | 36.9 |
| 54 | **Xe-133m1** | 2.2 d | γ | 0.233 | 10.1 |
| 54 | **Xe-135** | 9.1 h | β- | 0.310 | 96.0 |
| | | | γ | 0.250 | 90.0 |
| 54 | **Xe-135m1** | 15.3 m | γ | 0.527 | 80.6 |
| 54 | **Xe-138** | 14.1 m | β- | 0.300 | 35.3 |
| | | | β- | 0.988 | 21.7 |
| | | | β- | 1.005 | 15.0 |
| | | | β- | 1.188 | 11.0 |
| | | | β- | 0.208 | 10.3 |
| | | | γ | 0.258 | 34.1 |
| | | | γ | 0.435 | 22.0 |
| | | | γ | 1.768 | 18.1 |
| | | | γ | 2.016 | 13.3 |
| 55 | **Cs-125** | 46.7 m | β+ | 0.936 | 27.0 |
| | | | EC | 3.104 | 26.0 |
| | | | EC | 2.579 | 21.0 |
| | | | γ | 0.526 | 24.6 |
| 55 | **Cs-127** | 6.3 h | EC | 1.669 | 66.8 |
| | | | EC | 2.081 | 11.1 |
| | | | γ | 0.412 | 62.9 |
| | | | γ | 0.125 | 11.4 |
| 55 | **Cs-129** | 32.1 h | EC | 0.786 | 55.0 |
| | | | EC | 1.197 | 34.0 |
| | | | γ | 0.372 | 30.6 |
| | | | γ | 0.411 | 22.3 |
| 55 | **Cs-130** | 29.2 m | β+ | 0.879 | 43.0 |
| | | | EC | 2.979 | 51.5 |
| | | | γ | 0.080 | 34.6 |
| 55 | **Cs-131** | 9.7 d | EC | 0.355 | 100.0 |
| 55 | **Cs-132** | 6.5 d | EC | 1.457 | 95.5 |
| | | | γ | 0.668 | 97.6 |
| 55 | **Cs-134** | 2.9 h | γ | 0.128 | 12.6 |
| 55 | **Cs-135** | 53.0 m | γ | 0.787 | 99.7 |
| | | | γ | 0.846 | 96.0 |
| 55 | **Cs-136** | 13.0 d | β- | 0.099 | 80.8 |
| | | | β- | 0.121 | 13.6 |
| | | | γ | 0.819 | 99.7 |
| | | | γ | 1.048 | 80.0 |
| | | | γ | 0.341 | 46.8 |
| | | | γ | 1.235 | 20.0 |
| | | | γ | 0.177 | 13.7 |
| | | | γ | 0.274 | 12.7 |
| 55 | **Cs-138** | 32.5 m | β- | 1.200 | 44.0 |
| | | | β- | 1.454 | 13.7 |
| | | | β- | 1.305 | 12.9 |
| | | | γ | 1.436 | 76.3 |
| | | | γ | 0.463 | 30.8 |
| | | | γ | 1.010 | 29.8 |
| | | | γ | 2.218 | 15.2 |
| | | | γ | 0.547 | 10.8 |
| 56 | **Ba-124** | 11.0 m | EC | 2.472 | 14.4 |
| | | | EC | 1.425 | 13.1 |
| | | | EC | 2.642 | 12.0 |
| | | | γ | 0.170 | 22.0 |
| | | | γ | 0.189 | 12.9 |
| 56 | **Ba-126** | 100.0 m | EC | 1.673 | 30.0 |
| | | | EC | 1.439 | 16.8 |
| | | | γ | 0.234 | 19.6 |
| 56 | **Ba-127** | 12.7 m | β+ | 1.083 | 36.1 |

| Atomic number | Radio-nuclide | Half-life | Decay | Energy in MeV | Intensity (abs) in % |
|---|---|---|---|---|---|
| | | | β+ | 1.000 | 15.0 |
| | | | EC | 3.424 | 25.1 |
| | | | EC | 3.243 | 14.0 |
| | | | γ | 0.181 | 12.5 |
| 56 | **Ba-128** | 2.4 d | EC | 0.553 | 84.0 |
| | | | EC | 0.280 | 15.3 |
| | | | γ | 0.273 | 14.5 |
| 56 | **Ba-129** | 2.2 h | β+ | 0.635 | 13.0 |
| | | | EC | 2.436 | 47.0 |
| | | | EC | 2.215 | 20.4 |
| | | | γ | 0.214 | 13.4 |
| 56 | **Ba-129m1** | 2.1 h | EC | 0.796 | 59.5 |
| | | | EC | 0.632 | 15.0 |
| 56 | **Ba-131** | 11.5 d | EC | 0.756 | 52.7 |
| | | | EC | 1.160 | 21.7 |
| | | | EC | 1.003 | 20.2 |
| | | | γ | 0.496 | 48.0 |
| | | | γ | 0.124 | 29.8 |
| | | | γ | 0.216 | 20.4 |
| | | | γ | 0.373 | 14.4 |
| 56 | **Ba-131m1** | 14.6 m | γ | 0.108 | 55.4 |
| 56 | **Ba-133** | 38.9 h | γ | 0.276 | 17.7 |
| 56 | **Ba-135** | 28.7 h | γ | 0.268 | 16.0 |
| 56 | **Ba-139** | 82.9 m | β- | 0.916 | 70.0 |
| | | | β- | 0.841 | 29.7 |
| | | | γ | 0.166 | 23.7 |
| 56 | **Ba-140** | 12.8 d | β- | 0.345 | 40.0 |
| | | | β- | 0.141 | 24.7 |
| | | | β- | 0.362 | 20.0 |
| | | | γ | 0.537 | 24.4 |
| | | | γ | 0.030 | 14.1 |
| 56 | **Ba-141** | 18.3 m | β- | 1.025 | 24.4 |
| | | | β- | 1.108 | 18.1 |
| | | | β- | 0.897 | 12.5 |
| | | | γ | 0.190 | 45.5 |
| | | | γ | 0.304 | 25.2 |
| | | | γ | 0.277 | 23.2 |
| | | | γ | 0.344 | 14.3 |
| 56 | **Ba-142** | 10.6 m | β- | 0.346 | 46.0 |
| | | | β- | 0.398 | 22.0 |
| | | | β- | 0.246 | 15.4 |
| | | | γ | 0.255 | 20.6 |
| | | | γ | 1.204 | 14.3 |
| | | | γ | 0.895 | 13.9 |
| | | | γ | 0.232 | 12.2 |
| | | | γ | 1.079 | 11.5 |
| | | | γ | 0.949 | 10.7 |
| 57 | **La-129** | 11.6 m | β+ | 1.100 | 15.0 |
| | | | β+ | 1.228 | 11.0 |
| | | | EC | 3.460 | 10.9 |
| | | | γ | 0.279 | 24.7 |
| | | | γ | 0.111 | 16.9 |
| 57 | **La-131** | 59.0 m | EC | 2.389 | 24.6 |
| | | | EC | 2.550 | 15.9 |
| | | | γ | 0.108 | 25.0 |
| | | | γ | 0.418 | 17.9 |
| | | | γ | 0.365 | 16.9 |
| | | | γ | 0.285 | 12.4 |
| 57 | **La-132** | 24.3 m | γ | 0.136 | 49.0 |
| | | | γ | 0.465 | 21.6 |
| | | | γ | 0.663 | 11.2 |
| 57 | **La-133** | 3.9 h | EC | 2.047 | 84.0 |
| 57 | **La-135** | 19.5 h | EC | 1.200 | 98.1 |
| 57 | **La-140** | 1.7 d | β- | 0.487 | 43.9 |
| | | | β- | 0.629 | 20.2 |
| | | | β- | 0.441 | 11.1 |
| | | | γ | 1.596 | 95.4 |
| | | | γ | 0.487 | 45.5 |
| | | | γ | 0.816 | 23.3 |
| | | | γ | 0.329 | 20.3 |
| 57 | **La-141** | 3.9 h | β- | 0.999 | 98.1 |
| 57 | **La-142** | 91.1 m | β- | 0.820 | 17.8 |
| | | | β- | 0.755 | 17.3 |
| | | | β- | 1.904 | 16.5 |
| | | | γ | 0.641 | 47.4 |
| | | | γ | 2.398 | 13.3 |
| | | | γ | 2.543 | 10.0 |
| 57 | **La-143** | 14.2 m | β- | 1.409 | 52.0 |
| | | | β- | 1.410 | 32.0 |
| 58 | **Ce-130** | 22.9 m | EC | 2.080 | 39.0 |
| | | | EC | 1.014 | 11.0 |
| | | | γ | 0.131 | 37.0 |
| 58 | **Ce-132** | 3.5 h | EC | 1.088 | 77.1 |
| | | | γ | 0.182 | 77.4 |
| | | | γ | 0.155 | 10.5 |
| 58 | **Ce-133** | 97.0 m | β+ | 0.843 | 19.0 |
| | | | β+ | 0.878 | 18.0 |
| | | | EC | 2.897 | 34.0 |
| | | | EC | 2.974 | 29.0 |
| | | | γ | 0.097 | 45.0 |
| | | | γ | 0.077 | 15.9 |
| | | | γ | 0.558 | 11.4 |

| Atomic number | Radio-nuclide | Half-life | Decay | Energy in MeV | Intensity (abs) in % |
|---|---|---|---|---|---|
| 58 | **Ce-133m1** | 5.1 h | EC | 1.072 | 14.7 |
| | | | γ | 0.477 | 39.3 |
| | | | γ | 0.510 | 20.8 |
| | | | γ | 0.058 | 19.3 |
| | | | γ | 0.131 | 18.0 |
| 58 | **Ce-134** | 3.2 d | EC | 0.380 | 98.9 |
| 58 | **Ce-135** | 17.7 h | EC | 1.154 | 33.7 |
| | | | EC | 1.242 | 30.1 |
| | | | EC | 1.727 | 14.4 |
| | | | γ | 0.266 | 41.8 |
| | | | γ | 0.300 | 23.5 |
| | | | γ | 0.607 | 18.8 |
| | | | γ | 0.518 | 13.6 |
| | | | γ | 0.784 | 10.6 |
| | | | γ | 0.572 | 10.4 |
| 58 | **Ce-137** | 9.0 h | EC | 1.211 | 97.8 |
| 58 | **Ce-137m1** | 34.4 h | γ | 0.254 | 11.1 |
| 58 | **Ce-141** | 32.5 d | β- | 0.130 | 69.7 |
| | | | β- | 0.181 | 30.3 |
| | | | γ | 0.145 | 48.4 |
| 58 | **Ce-143** | 33.0 h | β- | 0.387 | 48.2 |
| | | | β- | 0.511 | 35.0 |
| | | | β- | 0.240 | 13.4 |
| | | | γ | 0.293 | 42.8 |
| | | | γ | 0.057 | 11.7 |
| 58 | **Ce-146** | 13.5 m | β- | 0.224 | 94.3 |
| | | | γ | 0.317 | 55.3 |
| | | | γ | 0.218 | 19.5 |
| 59 | **Pr-134** | 17.0 m | β+ | 2.248 | 32.0 |
| | | | β+ | 1.984 | 20.0 |
| | | | β+ | 1.245 | 11.0 |
| | | | γ | 0.409 | 87.1 |
| | | | γ | 0.556 | 13.1 |
| | | | γ | 0.966 | 13.1 |
| | | | γ | 2.136 | 10.7 |
| 59 | **Pr-134m1** | 11.0 m | β+ | 1.331 | 24.0 |
| | | | β+ | 1.330 | 15.0 |
| | | | EC | 3.961 | 12.0 |
| | | | γ | 0.409 | 89.3 |
| | | | γ | 0.640 | 59.7 |
| | | | γ | 0.215 | 21.4 |
| | | | γ | 0.332 | 19.0 |
| | | | γ | 0.334 | 19.0 |
| | | | γ | 0.974 | 15.3 |
| | | | γ | 0.979 | 12.5 |
| | | | γ | 0.678 | 11.7 |

| Atomic number | Radio-nuclide | Half-life | Decay | Energy in MeV | Intensity (abs) in % |
|---|---|---|---|---|---|
| | | | γ | 0.966 | 11.0 |
| | | | γ | 0.384 | 10.7 |
| | | | γ | 0.556 | 10.7 |
| | | | γ | 1.125 | 10.7 |
| 59 | **Pr-136** | 13.1 m | β+ | 1.384 | 40.6 |
| | | | EC | 4.076 | 17.7 |
| | | | γ | 0.552 | 75.5 |
| | | | γ | 0.540 | 52.1 |
| | | | γ | 1.092 | 18.4 |
| 59 | **Pr-137** | 1.3 h | β+ | 0.755 | 24.8 |
| | | | EC | 2.702 | 68.7 |
| 59 | **Pr-138m1** | 2.0 h | β+ | 0.742 | 23.0 |
| | | | EC | 2.672 | 68.0 |
| | | | γ | 1.038 | 101.0 |
| | | | γ | 0.789 | 100.0 |
| | | | γ | 0.303 | 80.0 |
| 59 | **Pr-139** | 4.4 h | EC | 2.129 | 90.5 |
| 59 | **Pr-142** | 19.1 h | β- | 0.834 | 96.3 |
| 59 | **Pr-143** | 13.6 d | β- | 0.315 | 100.0 |
| 59 | **Pr-144** | 17.3 m | β- | 1.222 | 97.9 |
| 59 | **Pr-145** | 6.0 h | β- | 0.683 | 97.6 |
| 59 | **Pr-146** | 24.1 m | β- | 1.776 | 45.0 |
| | | | β- | 0.889 | 28.7 |
| | | | β- | 1.588 | 13.0 |
| | | | γ | 0.454 | 46.0 |
| | | | γ | 1.525 | 15.0 |
| 59 | **Pr-147** | 13.4 m | β- | 0.727 | 23.4 |
| | | | β- | 0.738 | 21.3 |
| | | | β- | 0.941 | 12.6 |
| | | | β- | 0.502 | 10.2 |
| | | | γ | 0.315 | 17.5 |
| | | | γ | 0.641 | 15.6 |
| | | | γ | 0.578 | 13.7 |
| 60 | **Nd-135** | 12.4 m | β+ | 1.518 | 14.0 |
| | | | γ | 0.204 | 49.6 |
| | | | γ | 0.041 | 22.0 |
| | | | γ | 0.441 | 14.3 |
| 60 | **Nd-136** | 50.7 m | EC | 1.992 | 70.4 |
| | | | EC | 1.566 | 12.2 |
| | | | γ | 0.109 | 32.0 |
| | | | γ | 0.040 | 19.2 |
| | | | γ | 0.575 | 10.6 |
| 60 | **Nd-137** | 38.5 m | β+ | 1.128 | 13.0 |
| | | | EC | 3.016 | 13.0 |
| | | | EC | 3.422 | 11.0 |
| | | | γ | 0.076 | 17.3 |

| Atomic number | Radio-nuclide | Half-life | Decay | Energy in MeV | Intensity (abs) in % |
|---|---|---|---|---|---|
| | | | γ | 0.581 | 13.0 |
| | | | γ | 0.307 | 10.1 |
| 60 | **Nd-138** | 5.0 h | EC | 1.116 | 95.9 |
| 60 | **Nd-139** | 29.7 m | β+ | 0.803 | 24.0 |
| | | | EC | 2.806 | 61.0 |
| 60 | **Nd-139m1** | 5.5 h | EC | 1.203 | 33.8 |
| | | | EC | 1.110 | 12.5 |
| | | | γ | 0.114 | 40.0 |
| | | | γ | 0.738 | 35.1 |
| | | | γ | 0.708 | 26.3 |
| | | | γ | 0.982 | 26.3 |
| | | | γ | 0.828 | 10.3 |
| 60 | **Nd-140** | 3.4 d | EC | 0.429 | 100.0 |
| 60 | **Nd-141** | 2.5 h | EC | 1.824 | 95.6 |
| 60 | **Nd-147** | 11.0 d | β- | 0.264 | 80.2 |
| | | | β- | 0.106 | 15.4 |
| | | | γ | 0.091 | 28.1 |
| | | | γ | 0.531 | 13.4 |
| 60 | **Nd-149** | 1.7 h | β- | 0.541 | 24.7 |
| | | | β- | 0.403 | 21.5 |
| | | | β- | 0.355 | 19.1 |
| | | | β- | 0.515 | 17.5 |
| | | | γ | 0.211 | 25.9 |
| | | | γ | 0.114 | 19.2 |
| | | | γ | 0.270 | 10.7 |
| 60 | **Nd-151** | 12.4 m | β- | 0.400 | 17.9 |
| | | | β- | 0.966 | 14.6 |
| | | | γ | 0.117 | 39.0 |
| | | | γ | 0.256 | 14.8 |
| | | | γ | 1.181 | 13.3 |
| 60 | **Nd-152** | 11.4 m | β- | 0.373 | 52.0 |
| | | | β- | 0.384 | 52.0 |
| | | | β- | 0.266 | 47.0 |
| | | | γ | 0.279 | 26.0 |
| | | | γ | 0.250 | 16.4 |
| 61 | **Pm-141** | 20.9 m | β+ | 1.197 | 49.3 |
| | | | EC | 3.730 | 39.4 |
| 61 | **Pm-148** | 5.4 d | β- | 0.978 | 55.5 |
| | | | β- | 0.343 | 33.4 |
| | | | γ | 1.465 | 22.2 |
| | | | γ | 0.550 | 22.0 |
| | | | γ | 0.915 | 11.5 |
| 61 | **Pm-148m1** | 41.3 d | β- | 0.123 | 54.0 |
| | | | β- | 0.225 | 21.9 |
| | | | β- | 0.156 | 18.7 |
| | | | γ | 0.550 | 94.9 |

| Atomic number | Radio-nuclide | Half-life | Decay | Energy in MeV | Intensity (abs) in % |
|---|---|---|---|---|---|
| | | | γ | 0.630 | 89.0 |
| | | | γ | 0.726 | 32.8 |
| | | | γ | 1.014 | 20.3 |
| | | | γ | 0.414 | 18.7 |
| | | | γ | 0.915 | 17.2 |
| | | | γ | 0.288 | 12.6 |
| | | | γ | 0.600 | 12.5 |
| 61 | **Pm-149** | 53.1 h | β- | 0.369 | 95.9 |
| 61 | **Pm-150** | 2.7 h | β- | 0.895 | 25.9 |
| | | | β- | 0.677 | 19.4 |
| | | | β- | 0.499 | 17.5 |
| | | | β- | 1.427 | 10.0 |
| | | | γ | 0.334 | 68.0 |
| | | | γ | 1.325 | 17.5 |
| | | | γ | 1.166 | 15.8 |
| | | | γ | 0.832 | 11.9 |
| 61 | **Pm-151** | 28.4 h | β- | 0.278 | 42.0 |
| | | | β- | 0.415 | 10.0 |
| | | | β- | 0.417 | 10.0 |
| | | | γ | 0.340 | 22.5 |
| 62 | **Sm-140** | 14.8 m | β+ | 0.779 | 18.0 |
| | | | EC | 2.758 | 57.0 |
| 62 | **Sm-141** | 10.2 m | β+ | 1.434 | 21.2 |
| | | | β+ | 1.419 | 20.1 |
| | | | EC | 4.200 | 10.8 |
| | | | EC | 4.120 | 10.6 |
| | | | γ | 0.404 | 42.5 |
| | | | γ | 0.438 | 37.7 |
| 62 | **Sm-141m1** | 22.6 m | EC | 2.620 | 18.2 |
| | | | EC | 2.673 | 12.8 |
| | | | EC | 2.690 | 11.2 |
| | | | γ | 0.197 | 74.0 |
| | | | γ | 0.432 | 40.4 |
| | | | γ | 0.777 | 20.3 |
| | | | γ | 1.786 | 10.9 |
| 62 | **Sm-142** | 72.5 m | EC | 2.050 | 92.5 |
| 62 | **Sm-153** | 46.3 h | β- | 0.225 | 49.4 |
| | | | β- | 0.200 | 31.3 |
| | | | β- | 0.264 | 18.4 |
| | | | γ | 0.103 | 29.3 |
| 62 | **Sm-155** | 22.3 m | β- | 0.557 | 92.8 |
| | | | γ | 0.104 | 74.6 |
| 62 | **Sm-156** | 9.4 h | β- | 0.223 | 48.0 |
| | | | β- | 0.128 | 44.0 |
| | | | γ | 0.088 | 24.0 |
| | | | γ | 0.204 | 21.0 |

| Atomic number | Radio-nuclide | Half-life | Decay | Energy in MeV | Intensity (abs) in % |
|---|---|---|---|---|---|
| | | | γ | 0.166 | 13.0 |
| 63 | **Eu-145** | 5.9 d | EC | 1.766 | 43.0 |
| | | | EC | 1.112 | 19.3 |
| | | | EC | 1.001 | 16.4 |
| | | | γ | 0.894 | 66.0 |
| | | | γ | 0.654 | 15.0 |
| | | | γ | 1.659 | 14.9 |
| 63 | **Eu-146** | 4.6 d | EC | 2.488 | 14.5 |
| | | | EC | 2.488 | 10.5 |
| | | | γ | 0.747 | 98.0 |
| | | | γ | 0.634 | 44.8 |
| | | | γ | 0.633 | 35.7 |
| | | | γ | 0.665 | 10.3 |
| 63 | **Eu-147** | 24.1 d | EC | 1.524 | 24.9 |
| | | | EC | 1.600 | 20.6 |
| | | | EC | 0.923 | 18.7 |
| | | | EC | 1.722 | 16.9 |
| | | | γ | 0.197 | 24.4 |
| | | | γ | 0.121 | 21.2 |
| 63 | **Eu-150** | 12.8 h | β- | 0.345 | 89.0 |
| 63 | **Eu-152m1** | 9.3 h | β- | 0.704 | 70.0 |
| | | | EC | 0.957 | 26.0 |
| | | | γ | 0.842 | 14.2 |
| | | | γ | 0.963 | 11.6 |
| 63 | **Eu-152m2** | 96.0 m | γ | 0.090 | 69.7 |
| 63 | **Eu-154** | 46.3 m | γ | 0.068 | 37.0 |
| | | | γ | 0.101 | 27.0 |
| | | | γ | 0.036 | 13.0 |
| 63 | **Eu-156** | 15.2 d | β- | 0.965 | 32.0 |
| | | | β- | 0.146 | 29.0 |
| | | | β- | 0.074 | 10.3 |
| 63 | **Eu-157** | 15.2 h | β- | 0.462 | 49.0 |
| | | | β- | 0.296 | 22.0 |
| | | | β- | 0.312 | 15.0 |
| | | | γ | 0.064 | 23.0 |
| | | | γ | 0.411 | 17.8 |
| | | | γ | 0.371 | 11.2 |
| 63 | **Eu-158** | 45.9 m | β- | 0.946 | 25.0 |
| | | | β- | 0.967 | 21.3 |
| | | | γ | 0.944 | 30.0 |
| | | | γ | 0.977 | 16.3 |
| | | | γ | 0.898 | 12.4 |
| | | | γ | 0.079 | 10.5 |
| 63 | **Eu-159** | 18.1 m | β- | 0.927 | 37.0 |
| | | | β- | 0.963 | 19.0 |
| | | | β- | 0.891 | 14.0 |

| Atomic number | Radio-nuclide | Half-life | Decay | Energy in MeV | Intensity (abs) in % |
|---|---|---|---|---|---|
| | | | γ | 0.068 | 19.0 |
| 64 | **Gd-145** | 23.0 m | β+ | 1.024 | 13.0 |
| | | | β+ | 0.969 | 11.8 |
| | | | EC | 3.190 | 23.0 |
| | | | EC | 3.313 | 21.3 |
| | | | γ | 1.758 | 34.2 |
| | | | γ | 1.881 | 32.6 |
| 64 | **Gd-146** | 48.3 d | EC | 0.647 | 72.1 |
| | | | EC | 0.802 | 26.5 |
| | | | γ | 0.155 | 46.9 |
| | | | γ | 0.115 | 44.3 |
| | | | γ | 0.116 | 44.3 |
| 64 | **Gd-147** | 38.1 h | EC | 0.633 | 35.6 |
| | | | EC | 1.192 | 19.0 |
| | | | γ | 0.229 | 58.0 |
| | | | γ | 0.396 | 31.3 |
| | | | γ | 0.929 | 18.4 |
| | | | γ | 0.370 | 15.7 |
| | | | γ | 0.766 | 10.4 |
| 64 | **Gd-149** | 9.3 d | EC | 1.163 | 34.4 |
| | | | EC | 0.518 | 33.9 |
| | | | EC | 0.374 | 14.0 |
| | | | γ | 0.150 | 48.0 |
| | | | γ | 0.299 | 28.6 |
| | | | γ | 0.347 | 23.9 |
| 64 | **Gd-159** | 18.5 h | β- | 0.327 | 58.6 |
| | | | β- | 0.304 | 28.8 |
| | | | β- | 0.189 | 12.2 |
| | | | γ | 0.364 | 11.8 |
| 65 | **Tb-147** | 1.6 h | EC | 2.764 | 34.9 |
| | | | γ | 1.153 | 90.2 |
| | | | γ | 0.695 | 37.3 |
| | | | γ | 0.140 | 30.7 |
| 65 | **Tb-148** | 60.0 m | β+ | 1.791 | 21.8 |
| | | | γ | 0.784 | 84.0 |
| | | | γ | 0.489 | 19.7 |
| | | | γ | 1.079 | 11.4 |
| | | | γ | 0.632 | 10.6 |
| 65 | **Tb-149** | 4.1 h | α | 3.967 | 16.7 |
| | | | EC | 2.431 | 31.5 |
| | | | γ | 0.352 | 29.4 |
| | | | γ | 0.165 | 26.4 |
| | | | γ | 0.389 | 18.4 |
| | | | γ | 0.652 | 16.2 |
| | | | γ | 0.853 | 15.5 |
| | | | γ | 0.817 | 11.6 |

| Atomic number | Radio-nuclide | Half-life | Decay | Energy in MeV | Intensity (abs) in % |
|---|---|---|---|---|---|
| 65 | **Tb-150** | 3.5 h | β+ | 1.358 | 14.0 |
| | | | EC | 4.020 | 11.0 |
| | | | γ | 0.638 | 72.0 |
| | | | γ | 0.511 | 45.0 |
| | | | γ | 0.496 | 14.6 |
| 65 | **Tb-151** | 17.6 h | EC | 1.726 | 48.4 |
| | | | EC | 1.373 | 18.7 |
| | | | γ | 0.287 | 28.3 |
| | | | γ | 0.252 | 26.3 |
| | | | γ | 0.108 | 24.3 |
| | | | γ | 0.587 | 15.6 |
| | | | γ | 0.479 | 15.4 |
| | | | γ | 0.180 | 11.5 |
| | | | γ | 0.395 | 10.8 |
| | | | γ | 0.444 | 10.8 |
| | | | γ | 0.617 | 10.4 |
| 65 | **Tb-152** | 17.5 h | EC | 3.990 | 17.0 |
| | | | γ | 0.344 | 63.5 |
| 65 | **Tb-153** | 2.3 d | EC | 1.358 | 48.0 |
| | | | EC | 1.528 | 15.0 |
| | | | γ | 0.212 | 31.0 |
| 65 | **Tb-154** | 21.5 h | EC | 1.430 | 33.0 |
| | | | EC | 1.363 | 18.7 |
| | | | γ | 0.123 | 26.0 |
| | | | γ | 1.274 | 10.5 |
| 65 | **Tb-154m1** | 9.4 h | γ | 0.123 | 30.0 |
| | | | γ | 0.248 | 22.0 |
| | | | γ | 0.540 | 20.0 |
| | | | γ | 0.649 | 10.9 |
| | | | γ | 1.005 | 10.9 |
| 65 | **Tb-154m2** | 22.7 h | γ | 0.248 | 79.0 |
| | | | γ | 0.347 | 69.0 |
| | | | γ | 1.420 | 46.0 |
| | | | γ | 0.123 | 43.0 |
| | | | γ | 0.226 | 27.0 |
| | | | γ | 0.427 | 17.3 |
| | | | γ | 0.993 | 16.2 |
| 65 | **Tb-155** | 5.3 d | EC | 0.718 | 37.5 |
| | | | EC | 0.556 | 18.0 |
| | | | γ | 0.087 | 32.0 |
| | | | γ | 0.105 | 25.1 |
| 65 | **Tb-156** | 5.4 d | EC | 0.399 | 75.0 |
| | | | EC | 0.510 | 12.0 |
| | | | γ | 0.534 | 67.0 |
| | | | γ | 0.199 | 41.0 |
| | | | γ | 1.222 | 31.0 |

| Atomic number | Radio-nuclide | Half-life | Decay | Energy in MeV | Intensity (abs) in % |
|---|---|---|---|---|---|
| | | | γ | 0.089 | 18.0 |
| | | | γ | 0.356 | 13.6 |
| | | | γ | 1.422 | 12.2 |
| | | | γ | 1.065 | 10.8 |
| | | | γ | 1.154 | 10.4 |
| 65 | **Tb-156m1** | 24.4 h | γ | 0.050 | 74.1 |
| 65 | **Tb-161** | 6.9 d | β- | 0.157 | 65.0 |
| | | | β- | 0.138 | 25.7 |
| | | | γ | 0.026 | 23.2 |
| | | | γ | 0.049 | 17.0 |
| | | | γ | 0.075 | 10.2 |
| 65 | **Tb-163** | 19.5 m | β- | 0.299 | 34.8 |
| | | | β- | 0.279 | 15.4 |
| | | | β- | 0.346 | 11.5 |
| | | | β- | 0.357 | 11.4 |
| | | | γ | 0.351 | 26.0 |
| | | | γ | 0.390 | 24.0 |
| | | | γ | 0.495 | 22.5 |
| | | | γ | 0.422 | 11.5 |
| 66 | **Dy-151** | 17.9 m | γ | 0.386 | 19.4 |
| | | | γ | 0.049 | 18.0 |
| | | | γ | 0.546 | 14.3 |
| | | | γ | 0.176 | 10.6 |
| 66 | **Dy-152** | 2.4 h | EC | 0.343 | 99.9 |
| | | | γ | 0.257 | 97.5 |
| 66 | **Dy-153** | 6.4 h | EC | 1.908 | 26.6 |
| | | | γ | 0.081 | 11.1 |
| | | | γ | 0.214 | 10.9 |
| | | | γ | 0.100 | 10.5 |
| 66 | **Dy-155** | 9.9 h | EC | 1.868 | 64.9 |
| | | | EC | 0.939 | 10.8 |
| | | | γ | 0.227 | 68.4 |
| 66 | **Dy-157** | 8.1 h | EC | 1.013 | 96.0 |
| | | | γ | 0.326 | 93.0 |
| 66 | **Dy-165** | 2.3 h | β- | 0.454 | 83.0 |
| | | | β- | 0.415 | 15.0 |
| 66 | **Dy-166** | 81.6 h | β- | 0.118 | 97.0 |
| | | | γ | 0.082 | 13.8 |
| 67 | **Ho-154** | 11.8 m | β+ | 2.007 | 12.1 |
| | | | γ | 0.335 | 84.4 |
| | | | γ | 0.412 | 15.2 |
| | | | γ | 0.873 | 12.2 |
| | | | γ | 0.569 | 11.1 |
| | | | γ | 0.571 | 11.1 |
| 67 | **Ho-155** | 48.0 m | γ | 0.240 | 12.5 |
| 67 | **Ho-156** | 56.0 m | γ | 0.266 | 66.0 |

| Atomic number | Radio-nuclide | Half-life | Decay | Energy in MeV | Intensity (abs) in % |
|---|---|---|---|---|---|
| | | | γ | 0.138 | 52.0 |
| | | | γ | 0.366 | 14.5 |
| 67 | **Ho-157** | 12.6 m | EC | 2.202 | 53.0 |
| | | | EC | 1.696 | 12.0 |
| | | | γ | 0.280 | 23.1 |
| | | | γ | 0.341 | 17.8 |
| 67 | **Ho-158m2** | 21.3 m | EC | 1.872 | 93.0 |
| 67 | **Ho-159** | 33.1 m | EC | 1.528 | 70.0 |
| | | | EC | 1.442 | 13.8 |
| | | | γ | 0.121 | 36.2 |
| | | | γ | 0.132 | 23.6 |
| | | | γ | 0.310 | 17.2 |
| | | | γ | 0.253 | 13.7 |
| 67 | **Ho-161** | 2.5 h | EC | 0.833 | 76.0 |
| | | | EC | 0.755 | 19.0 |
| | | | γ | 0.026 | 27.0 |
| 67 | **Ho-162** | 15.0 m | EC | 2.090 | 50.0 |
| | | | EC | 2.170 | 40.0 |
| 67 | **Ho-162m1** | 67.0 m | EC | 0.760 | 13.9 |
| | | | γ | 0.185 | 23.9 |
| | | | γ | 1.220 | 23.7 |
| | | | γ | 0.937 | 10.4 |
| | | | γ | 0.283 | 10.3 |
| 67 | **Ho-164** | 28.8 m | β- | 0.322 | 28.0 |
| | | | β- | 0.286 | 12.0 |
| | | | EC | 0.961 | 41.0 |
| | | | EC | 0.888 | 19.0 |
| 67 | **Ho-164m1** | 36.6 m | γ | 0.037 | 11.4 |
| 67 | **Ho-166** | 26.8 h | β- | 0.651 | 49.9 |
| | | | β- | 0.694 | 48.8 |
| 67 | **Ho-167** | 3.1 h | β- | 0.097 | 43.0 |
| | | | β- | 0.208 | 21.0 |
| | | | β- | 0.309 | 15.0 |
| | | | β- | 0.340 | 15.0 |
| | | | γ | 0.347 | 57.0 |
| | | | γ | 0.321 | 24.0 |
| 68 | **Er-156** | 19.5 m | EC | 1.142 | 52.0 |
| | | | EC | 1.208 | 23.0 |
| | | | EC | 1.169 | 18.0 |
| | | | γ | 0.035 | 26.0 |
| 68 | **Er-157** | 18.7 m | EC | 3.266 | 17.0 |
| | | | EC | 3.049 | 11.0 |
| | | | γ | 0.053 | 24.1 |
| | | | γ | 0.391 | 14.2 |
| | | | γ | 0.121 | 10.1 |
| 68 | **Er-158** | 2.3 h | EC | 0.733 | 76.0 |

| Atomic number | Radio-nuclide | Half-life | Decay | Energy in MeV | Intensity (abs) in % |
|---|---|---|---|---|---|
| | | | γ | 0.072 | 10.8 |
| 68 | **Er-159** | 36.0 m | EC | 2.144 | 32.0 |
| | | | EC | 2.119 | 25.0 |
| | | | γ | 0.625 | 33.0 |
| | | | γ | 0.649 | 23.0 |
| 68 | **Er-160** | 28.6 h | EC | 0.263 | 100.0 |
| 68 | **Er-161** | 3.2 h | EC | 1.167 | 66.0 |
| | | | γ | 0.827 | 64.0 |
| | | | γ | 0.211 | 12.2 |
| 68 | **Er-163** | 75.0 m | EC | 1.210 | 99.9 |
| 68 | **Er-165** | 10.4 h | EC | 0.371 | 100.0 |
| 68 | **Er-169** | 9.4 d | β- | 0.101 | 55.0 |
| | | | β- | 0.098 | 45.0 |
| 68 | **Er-171** | 7.5 h | β- | 0.363 | 94.0 |
| | | | γ | 0.308 | 64.0 |
| | | | γ | 0.296 | 28.9 |
| | | | γ | 0.112 | 20.5 |
| 68 | **Er-172** | 49.3 h | β- | 0.079 | 46.4 |
| | | | β- | 0.102 | 46.4 |
| | | | γ | 0.610 | 44.2 |
| | | | γ | 0.407 | 42.1 |
| 69 | **Tm-161** | 30.2 m | γ | 0.046 | 25.0 |
| | | | γ | 1.648 | 19.5 |
| 69 | **Tm-162** | 21.7 m | γ | 0.102 | 17.5 |
| 69 | **Tm-163** | 1.8 h | EC | 0.900 | 16.9 |
| | | | EC | 0.637 | 12.9 |
| | | | γ | 0.104 | 18.6 |
| | | | γ | 0.069 | 11.6 |
| | | | γ | 0.241 | 10.9 |
| 69 | **Tm-165** | 30.1 h | EC | 1.295 | 35.9 |
| | | | EC | 1.236 | 12.2 |
| | | | EC | 0.739 | 10.2 |
| | | | γ | 0.243 | 35.5 |
| | | | γ | 0.047 | 16.9 |
| | | | γ | 0.297 | 12.7 |
| 69 | **Tm-166** | 7.7 h | EC | 0.905 | 59.0 |
| | | | EC | 0.878 | 16.2 |
| | | | γ | 0.779 | 19.1 |
| | | | γ | 2.052 | 17.4 |
| | | | γ | 0.184 | 16.2 |
| | | | γ | 1.274 | 15.0 |
| | | | γ | 0.081 | 11.5 |
| | | | γ | 0.705 | 11.1 |
| | | | γ | 0.786 | 10.0 |
| 69 | **Tm-167** | 9.3 d | EC | 0.541 | 69.0 |
| | | | EC | 0.484 | 29.0 |

| Atomic number | Radio-nuclide | Half-life | Decay | Energy in MeV | Intensity (abs) in % |
|---|---|---|---|---|---|
| | | | γ | 0.208 | 42.0 |
| 69 | **Tm-172** | 63.6 h | β- | 0.668 | 36.0 |
| | | | β- | 0.691 | 29.0 |
| | | | β- | 0.121 | 10.1 |
| | | | β- | 0.076 | 10.0 |
| 69 | **Tm-173** | 8.2 h | β- | 0.296 | 76.0 |
| | | | β- | 0.272 | 22.0 |
| | | | γ | 0.399 | 87.9 |
| 69 | **Tm-175** | 15.2 m | β- | 0.298 | 37.1 |
| | | | β- | 0.000 | 19.0 |
| | | | β- | 0.531 | 12.4 |
| | | | γ | 0.515 | 65.0 |
| | | | γ | 0.941 | 14.6 |
| | | | γ | 0.364 | 12.7 |
| | | | γ | 0.982 | 10.2 |
| 70 | **Yb-162** | 18.9 m | EC | 1.487 | 73.0 |
| | | | EC | 1.650 | 15.0 |
| | | | γ | 0.163 | 40.0 |
| | | | γ | 0.119 | 34.0 |
| 70 | **Yb-163** | 11.1 m | EC | 2.420 | 25.0 |
| | | | EC | 3.417 | 14.0 |
| | | | EC | 3.431 | 14.0 |
| | | | EC | 1.598 | 12.0 |
| | | | γ | 0.860 | 24.0 |
| | | | γ | 0.064 | 15.0 |
| 70 | **Yb-164** | 75.8 m | EC | 1.100 | 96.5 |
| 70 | **Yb-166** | 56.7 h | EC | 0.223 | 100.0 |
| | | | γ | 0.082 | 15.6 |
| 70 | **Yb-167** | 17.5 m | EC | 1.661 | 95.0 |
| | | | γ | 0.113 | 55.0 |
| | | | γ | 0.106 | 22.4 |
| | | | γ | 0.176 | 20.4 |
| 70 | **Yb-169** | 32.0 d | EC | 0.531 | 81.1 |
| | | | EC | 0.437 | 12.3 |
| | | | γ | 0.063 | 43.6 |
| | | | γ | 0.198 | 35.9 |
| | | | γ | 0.177 | 22.3 |
| | | | γ | 0.110 | 17.4 |
| | | | γ | 0.131 | 11.4 |
| | | | γ | 0.308 | 10.1 |
| 70 | **Yb-175** | 4.2 d | β- | 0.140 | 72.9 |
| | | | β- | 0.019 | 20.4 |
| | | | γ | 0.396 | 13.1 |
| 70 | **Yb-177** | 1.9 h | β- | 0.496 | 59.4 |
| | | | β- | 0.434 | 18.5 |
| | | | γ | 0.150 | 18.0 |

| Atomic number | Radio-nuclide | Half-life | Decay | Energy in MeV | Intensity (abs) in % |
|---|---|---|---|---|---|
| 70 | **Yb-178** | 74.0 m | β- | 0.201 | 85.0 |
| | | | β- | 0.071 | 15.0 |
| 71 | **Lu-167** | 51.5 m | γ | 0.030 | 16.3 |
| 71 | **Lu-169** | 34.1 h | EC | 1.332 | 25.2 |
| | | | EC | 0.843 | 15.8 |
| | | | EC | 2.293 | 10.0 |
| | | | γ | 0.961 | 21.2 |
| | | | γ | 0.191 | 18.7 |
| 71 | **Lu-170** | 2.0 d | EC | 1.094 | 14.9 |
| | | | EC | 1.332 | 10.4 |
| 71 | **Lu-171** | 8.2 d | EC | 0.643 | 61.5 |
| | | | EC | 1.383 | 14.0 |
| | | | γ | 0.740 | 48.7 |
| | | | γ | 0.019 | 14.0 |
| | | | γ | 0.667 | 11.2 |
| 71 | **Lu-172** | 6.7 d | EC | 0.446 | 65.0 |
| | | | γ | 1.094 | 62.0 |
| | | | γ | 0.901 | 29.8 |
| | | | γ | 0.182 | 20.6 |
| | | | γ | 0.810 | 16.6 |
| | | | γ | 0.912 | 15.2 |
| | | | γ | 0.079 | 10.6 |
| 71 | **Lu-176m1** | 3.7 h | β- | 0.425 | 61.0 |
| | | | β- | 0.461 | 39.0 |
| 71 | **Lu-177** | 6.6 d | β- | 0.149 | 79.4 |
| | | | β- | 0.047 | 11.7 |
| | | | γ | 0.208 | 10.4 |
| 71 | **Lu-178** | 28.4 m | β- | 0.795 | 63.0 |
| | | | β- | 0.754 | 29.0 |
| 71 | **Lu-178m1** | 23.1 m | β- | 0.365 | 83.7 |
| | | | β- | 0.238 | 13.0 |
| | | | γ | 0.426 | 97.0 |
| | | | γ | 0.326 | 94.1 |
| | | | γ | 0.213 | 81.4 |
| | | | γ | 0.089 | 64.4 |
| | | | γ | 0.093 | 17.2 |
| | | | γ | 0.332 | 11.4 |
| 71 | **Lu-179** | 4.6 h | β- | 0.499 | 87.0 |
| | | | β- | 0.412 | 11.0 |
| | | | γ | 0.214 | 12.0 |
| 72 | **Hf-168** | 26.0 m | EC | 1.258 | 49.0 |
| | | | EC | 0.905 | 11.0 |
| 72 | **Hf-170** | 16.0 h | EC | 0.267 | 43.0 |
| | | | EC | 1.052 | 40.0 |
| | | | EC | 0.854 | 15.0 |
| | | | γ | 0.165 | 26.0 |

| Atomic number | Radio-nuclide | Half-life | Decay | Energy in MeV | Intensity (abs) in % |
|---|---|---|---|---|---|
| | | | γ | 0.621 | 18.0 |
| | | | γ | 0.120 | 15.0 |
| | | | γ | 0.573 | 15.0 |
| 72 | **Hf-173** | 23.6 h | EC | 1.175 | 37.4 |
| | | | EC | 1.337 | 32.0 |
| | | | EC | 1.165 | 21.3 |
| | | | γ | 0.124 | 83.0 |
| | | | γ | 0.297 | 33.9 |
| | | | γ | 0.140 | 12.7 |
| | | | γ | 0.311 | 10.7 |
| 72 | **Hf-177m1** | 51.4 m | γ | 0.277 | 76.1 |
| | | | γ | 0.295 | 70.0 |
| | | | γ | 0.327 | 69.0 |
| | | | γ | 0.312 | 59.0 |
| | | | γ | 0.214 | 41.1 |
| | | | γ | 0.638 | 20.3 |
| | | | γ | 0.607 | 11.6 |
| 72 | **Hf-179m2** | 25.1 d | γ | 0.454 | 68.0 |
| | | | γ | 0.363 | 39.6 |
| | | | γ | 0.123 | 27.7 |
| | | | γ | 0.146 | 27.1 |
| | | | γ | 0.193 | 21.5 |
| | | | γ | 0.410 | 21.5 |
| | | | γ | 0.316 | 20.3 |
| | | | γ | 0.170 | 19.4 |
| | | | γ | 0.236 | 18.8 |
| | | | γ | 0.269 | 11.3 |
| 72 | **Hf-180m1** | 5.5 h | γ | 0.332 | 94.0 |
| | | | γ | 0.443 | 82.0 |
| | | | γ | 0.215 | 81.6 |
| | | | γ | 0.058 | 48.0 |
| | | | γ | 0.093 | 16.5 |
| | | | γ | 0.501 | 14.2 |
| 72 | **Hf-181** | 42.4 d | β- | 0.121 | 93.0 |
| | | | γ | 0.482 | 80.5 |
| | | | γ | 0.133 | 43.3 |
| | | | γ | 0.346 | 15.1 |
| 72 | **Hf-182m1** | 61.5 m | β- | 0.129 | 43.0 |
| | | | β- | 0.296 | 10.0 |
| | | | γ | 0.344 | 46.0 |
| | | | γ | 0.224 | 38.0 |
| | | | γ | 0.507 | 23.0 |
| | | | γ | 0.943 | 22.0 |
| | | | γ | 0.456 | 20.0 |
| | | | γ | 0.051 | 13.2 |
| | | | γ | 0.800 | 10.8 |

| Atomic number | Radio-nuclide | Half-life | Decay | Energy in MeV | Intensity (abs) in % |
|---|---|---|---|---|---|
| 72 | **Hf-183** | 1.0 h | β- | 0.394 | 68.6 |
| | | | β- | 0.558 | 28.7 |
| | | | β- | 0.738 | 14.0 |
| | | | γ | 0.784 | 65.5 |
| | | | γ | 0.073 | 38.0 |
| | | | γ | 0.459 | 29.8 |
| 72 | **Hf-184** | 4.1 h | β- | 0.378 | 47.0 |
| | | | β- | 0.229 | 36.0 |
| | | | β- | 0.290 | 15.2 |
| | | | γ | 0.139 | 46.0 |
| | | | γ | 0.345 | 35.0 |
| | | | γ | 0.181 | 14.1 |
| 73 | **Ta-172** | 36.8 m | EC | 3.280 | 14.0 |
| | | | EC | 3.500 | 12.0 |
| | | | γ | 0.214 | 55.0 |
| | | | γ | 0.095 | 21.3 |
| | | | γ | 1.109 | 14.8 |
| 73 | **Ta-173** | 3.1 h | EC | 2.720 | 28.0 |
| | | | EC | 2.548 | 27.3 |
| | | | γ | 0.172 | 17.5 |
| 73 | **Ta-174** | 1.1 h | β+ | 1.141 | 17.7 |
| | | | EC | 3.547 | 43.0 |
| | | | EC | 3.753 | 11.0 |
| | | | γ | 0.207 | 60.0 |
| | | | γ | 0.091 | 15.9 |
| 73 | **Ta-175** | 10.5 h | EC | 1.732 | 26.3 |
| | | | EC | 1.999 | 12.0 |
| | | | γ | 0.207 | 14.0 |
| | | | γ | 0.349 | 12.0 |
| | | | γ | 0.267 | 10.8 |
| 73 | **Ta-176** | 8.1 h | EC | 1.962 | 11.0 |
| | | | γ | 1.159 | 24.7 |
| | | | γ | 0.088 | 11.9 |
| 73 | **Ta-177** | 56.4 h | EC | 1.166 | 62.0 |
| | | | EC | 1.053 | 35.0 |
| | | | γ | 0.113 | 11.4 |
| 73 | **Ta-178** | 2.4 h | EC | 0.790 | 64.2 |
| | | | EC | 0.458 | 35.8 |
| | | | γ | 0.426 | 97.0 |
| | | | γ | 0.326 | 94.1 |
| | | | γ | 0.213 | 80.8 |
| | | | γ | 0.089 | 66.9 |
| | | | γ | 0.332 | 31.2 |
| | | | γ | 0.093 | 17.3 |
| 73 | **Ta-180** | 8.2 h | β- | 0.221 | 10.4 |
| | | | EC | 0.846 | 61.0 |

| Atomic number | Radio-nuclide | Half-life | Decay | Energy in MeV | Intensity (abs) in % |
|---|---|---|---|---|---|
| | | | EC | 0.752 | 24.9 |
| 73 | **Ta-182m2** | 15.8 m | γ | 0.172 | 48.0 |
| | | | γ | 0.147 | 36.5 |
| | | | γ | 0.185 | 24.0 |
| 73 | **Ta-183** | 5.1 d | β- | 0.191 | 93.0 |
| | | | γ | 0.246 | 27.2 |
| | | | γ | 0.354 | 11.6 |
| | | | γ | 0.108 | 11.2 |
| 73 | **Ta-184** | 8.7 h | β- | 0.399 | 82.0 |
| | | | β- | 0.381 | 14.7 |
| | | | γ | 0.414 | 72.0 |
| | | | γ | 0.253 | 44.0 |
| | | | γ | 0.921 | 32.0 |
| | | | γ | 0.111 | 23.7 |
| | | | γ | 0.318 | 22.8 |
| | | | γ | 0.903 | 15.0 |
| | | | γ | 0.792 | 14.5 |
| | | | γ | 0.537 | 12.7 |
| | | | γ | 0.384 | 12.5 |
| | | | γ | 0.215 | 11.4 |
| | | | γ | 0.461 | 10.7 |
| | | | γ | 0.895 | 10.7 |
| 73 | **Ta-185** | 49.4 m | β- | 0.639 | 68.0 |
| | | | β- | 0.669 | 27.0 |
| | | | γ | 0.178 | 25.7 |
| | | | γ | 0.174 | 22.6 |
| 73 | **Ta-186** | 10.5 m | β- | 0.851 | 58.0 |
| | | | β- | 1.532 | 16.0 |
| | | | β- | 1.121 | 10.0 |
| | | | γ | 0.198 | 50.0 |
| | | | γ | 0.215 | 42.0 |
| | | | γ | 0.511 | 37.0 |
| | | | γ | 0.738 | 29.0 |
| | | | γ | 0.615 | 28.0 |
| | | | γ | 0.122 | 25.0 |
| | | | γ | 0.418 | 12.5 |
| | | | γ | 0.739 | 10.0 |
| 74 | **W-174** | 33.2 m | γ | 0.035 | 15.3 |
| | | | γ | 0.429 | 12.7 |
| 74 | **W-175** | 35.2 m | γ | 0.270 | 12.0 |
| 74 | **W-177** | 132.4 m | EC | 1.796 | 32.6 |
| | | | EC | 0.760 | 15.8 |
| | | | EC | 0.501 | 11.4 |
| | | | γ | 0.116 | 47.3 |
| | | | γ | 0.186 | 15.2 |
| | | | γ | 0.427 | 12.5 |

| Atomic number | Radio-nuclide | Half-life | Decay | Energy in MeV | Intensity (abs) in % |
|---|---|---|---|---|---|
| 74 | **W-178** | 21.6 d | EC | 0.091 | 100.0 |
| 74 | **W-179** | 37.1 m | EC | 1.032 | 99.2 |
| | | | γ | 0.031 | 18.0 |
| 74 | **W-187** | 24.0 h | β- | 0.193 | 66.2 |
| | | | β- | 0.457 | 16.9 |
| | | | γ | 0.686 | 33.2 |
| | | | γ | 0.480 | 26.6 |
| | | | γ | 0.072 | 13.6 |
| | | | γ | 0.134 | 10.4 |
| 74 | **W-190** | 30.0 m | β- | 0.310 | 100.0 |
| | | | γ | 0.158 | 39.0 |
| | | | γ | 0.162 | 11.0 |
| 75 | **Re-177** | 14.0 m | γ | 0.197 | 10.0 |
| 75 | **Re-178** | 13.2 m | β+ | 1.535 | 10.0 |
| | | | β+ | 1.644 | 10.0 |
| | | | EC | 4.417 | 12.0 |
| | | | γ | 0.237 | 44.5 |
| | | | γ | 0.106 | 23.4 |
| 75 | **Re-179** | 19.5 m | EC | 1.997 | 44.0 |
| | | | EC | 1.943 | 19.0 |
| | | | EC | 1.037 | 17.6 |
| | | | γ | 0.430 | 28.1 |
| | | | γ | 0.290 | 27.0 |
| | | | γ | 1.680 | 13.0 |
| | | | γ | 0.415 | 10.6 |
| 75 | **Re-181** | 19.9 h | EC | 0.377 | 22.7 |
| | | | EC | 1.017 | 16.0 |
| | | | EC | 1.377 | 15.0 |
| | | | EC | 1.267 | 10.3 |
| | | | γ | 0.366 | 56.0 |
| | | | γ | 0.361 | 20.0 |
| 75 | **Re-182** | 64.2 h | EC | 0.822 | 24.0 |
| | | | EC | 0.840 | 23.0 |
| | | | EC | 1.043 | 16.4 |
| | | | EC | 0.970 | 14.0 |
| | | | γ | 0.229 | 25.8 |
| | | | γ | 0.068 | 22.2 |
| | | | γ | 1.121 | 22.1 |
| | | | γ | 1.221 | 17.5 |
| | | | γ | 0.100 | 16.5 |
| | | | γ | 1.231 | 14.9 |
| | | | γ | 0.169 | 11.4 |
| | | | γ | 1.076 | 10.6 |
| | | | γ | 0.351 | 10.3 |
| 75 | **Re-182m1** | 14.1 h | EC | 1.511 | 37.0 |
| | | | EC | 1.426 | 29.0 |

| Atomic number | Radio-nuclide | Half-life | Decay | Energy in MeV | Intensity (abs) in % |
|---|---|---|---|---|---|
| | | | EC | 2.700 | 14.0 |
| | | | γ | 0.068 | 38.0 |
| | | | γ | 1.121 | 32.0 |
| | | | γ | 1.222 | 25.0 |
| | | | γ | 1.189 | 15.1 |
| | | | γ | 0.100 | 14.4 |
| 75 | **Re-184** | 35.4 d | EC | 0.578 | 76.5 |
| | | | EC | 0.475 | 17.8 |
| | | | γ | 0.903 | 38.1 |
| | | | γ | 0.792 | 37.7 |
| | | | γ | 0.111 | 17.2 |
| | | | γ | 0.895 | 15.7 |
| 75 | **Re-186** | 3.7 d | β- | 0.359 | 71.0 |
| | | | β- | 0.306 | 21.5 |
| 75 | **Re-188** | 17.0 h | β- | 0.795 | 70.7 |
| | | | β- | 0.729 | 25.8 |
| | | | γ | 0.155 | 15.5 |
| 75 | **Re-188m1** | 18.6 m | γ | 0.064 | 22.3 |
| | | | γ | 0.106 | 11.5 |
| 75 | **Re-189** | 24.3 h | β- | 0.335 | 62.0 |
| | | | β- | 0.253 | 13.0 |
| 75 | **Re-190m1** | 3.2 h | β- | 0.539 | 17.1 |
| | | | γ | 0.187 | 27.8 |
| | | | γ | 0.605 | 14.9 |
| | | | γ | 0.558 | 14.3 |
| | | | γ | 0.569 | 13.7 |
| | | | γ | 0.361 | 12.1 |
| | | | γ | 0.119 | 11.1 |
| | | | γ | 0.371 | 10.3 |
| 76 | **Os-180** | 21.5 m | EC | 1.455 | 99.9 |
| | | | γ | 0.020 | 17.0 |
| 76 | **Os-181** | 105.0 m | EC | 2.528 | 17.0 |
| | | | EC | 2.133 | 13.0 |
| | | | γ | 0.239 | 44.0 |
| | | | γ | 0.827 | 19.9 |
| | | | γ | 0.118 | 12.7 |
| 76 | **Os-182** | 21.8 h | EC | 0.330 | 62.1 |
| | | | EC | 0.577 | 27.0 |
| | | | γ | 0.510 | 52.4 |
| | | | γ | 0.180 | 34.1 |
| 76 | **Os-183** | 13.0 h | EC | 1.654 | 73.8 |
| | | | EC | 1.486 | 17.7 |
| | | | γ | 0.382 | 91.6 |
| | | | γ | 0.114 | 21.1 |
| 76 | **Os-183m1** | 9.9 h | EC | 1.219 | 45.6 |
| | | | EC | 1.213 | 18.6 |

| Atomic number | Radio-nuclide | Half-life | Decay | Energy in MeV | Intensity (abs) in % |
|---|---|---|---|---|---|
| | | | EC | 2.321 | 11.0 |
| | | | γ | 1.102 | 49.2 |
| | | | γ | 1.108 | 22.5 |
| 76 | **Os-191** | 15.4 d | β- | 0.038 | 100.0 |
| | | | γ | 0.129 | 26.5 |
| 76 | **Os-193** | 29.8 h | β- | 0.387 | 59.0 |
| | | | β- | 0.358 | 17.0 |
| | | | β- | 0.333 | 10.6 |
| 76 | **Os-196** | 34.9 m | β- | 0.393 | 85.0 |
| 77 | **Ir-182** | 15.0 m | β+ | 2.001 | 21.0 |
| | | | β+ | 1.874 | 17.0 |
| | | | EC | 5.160 | 14.0 |
| | | | EC | 5.433 | 14.0 |
| | | | γ | 0.274 | 40.0 |
| | | | γ | 0.127 | 35.0 |
| 77 | **Ir-183** | 58.0 m | EC | 3.202 | 14.0 |
| | | | EC | 3.460 | 12.0 |
| 77 | **Ir-184** | 3.1 h | EC | 2.198 | 12.0 |
| | | | γ | 0.264 | 64.4 |
| | | | γ | 0.120 | 30.8 |
| | | | γ | 0.390 | 26.1 |
| | | | γ | 0.961 | 10.2 |
| 77 | **Ir-185** | 14.4 h | γ | 0.254 | 13.3 |
| | | | γ | 1.829 | 10.1 |
| 77 | **Ir-186** | 16.6 h | EC | 1.940 | 20.6 |
| | | | EC | 1.750 | 10.5 |
| | | | γ | 0.297 | 62.3 |
| | | | γ | 0.137 | 41.4 |
| | | | γ | 0.435 | 33.9 |
| 77 | **Ir-186m1** | 1.9 h | EC | 2.077 | 18.6 |
| | | | EC | 3.064 | 14.0 |
| | | | γ | 0.137 | 23.0 |
| | | | γ | 0.767 | 18.4 |
| | | | γ | 0.630 | 15.6 |
| | | | γ | 0.773 | 11.7 |
| 77 | **Ir-187** | 10.5 h | EC | 1.492 | 24.0 |
| | | | EC | 1.427 | 20.0 |
| | | | EC | 0.515 | 13.0 |
| | | | EC | 1.001 | 11.4 |
| | | | EC | 1.428 | 10.0 |
| 77 | **Ir-188** | 41.5 h | EC | 0.577 | 26.6 |
| | | | γ | 0.155 | 29.6 |
| | | | γ | 2.215 | 18.6 |
| | | | γ | 0.633 | 17.9 |
| | | | γ | 0.478 | 14.7 |
| 77 | **Ir-189** | 13.2 d | EC | 0.537 | 40.0 |

| Atomic number | Radio-nuclide | Half-life | Decay | Energy in MeV | Intensity (abs) in % |
|---|---|---|---|---|---|
| | | | EC | 0.467 | 35.0 |
| | | | EC | 0.442 | 11.6 |
| | | | EC | 0.261 | 10.1 |
| 77 | **Ir-190** | 11.8 d | EC | 0.272 | 49.0 |
| | | | EC | 0.791 | 26.0 |
| | | | EC | 0.370 | 10.2 |
| | | | γ | 0.187 | 52.0 |
| | | | γ | 0.605 | 39.9 |
| | | | γ | 0.519 | 34.0 |
| | | | γ | 0.558 | 30.1 |
| | | | γ | 0.569 | 28.5 |
| | | | γ | 0.407 | 23.9 |
| | | | γ | 0.371 | 22.8 |
| | | | γ | 0.361 | 13.0 |
| 77 | **Ir-190m2** | 3.1 h | EC | 0.625 | 91.4 |
| | | | γ | 0.617 | 90.1 |
| | | | γ | 0.503 | 89.4 |
| | | | γ | 0.361 | 86.7 |
| | | | γ | 0.187 | 64.2 |
| 77 | **Ir-194** | 19.3 h | β- | 0.840 | 85.4 |
| | | | γ | 0.328 | 13.1 |
| 77 | **Ir-195** | 2.3 h | β- | 0.320 | 57.0 |
| | | | β- | 0.332 | 25.0 |
| | | | β- | 0.371 | 13.0 |
| | | | γ | 0.099 | 10.0 |
| 77 | **Ir-195m1** | 3.7 h | β- | 0.112 | 36.0 |
| | | | β- | 0.309 | 33.0 |
| | | | γ | 0.099 | 10.7 |
| 77 | **Ir-196m1** | 1.4 h | β- | 0.390 | 80.0 |
| | | | γ | 0.394 | 97.0 |
| | | | γ | 0.521 | 96.0 |
| | | | γ | 0.356 | 94.0 |
| | | | γ | 0.447 | 94.0 |
| | | | γ | 0.647 | 91.0 |
| | | | γ | 0.103 | 18.4 |
| 78 | **Pt-184** | 17.3 m | EC | 1.376 | 29.0 |
| | | | EC | 1.193 | 23.3 |
| | | | EC | 1.215 | 11.5 |
| | | | γ | 0.155 | 31.0 |
| | | | γ | 0.192 | 27.0 |
| | | | γ | 0.071 | 23.0 |
| | | | γ | 0.548 | 23.0 |
| | | | γ | 0.731 | 12.8 |
| 78 | **Pt-186** | 2.1 h | EC | 0.691 | 86.0 |
| | | | γ | 0.689 | 70.0 |
| 78 | **Pt-187** | 2.4 h | EC | 2.890 | 14.1 |

| Atomic number | Radio-nuclide | Half-life | Decay | Energy in MeV | Intensity (abs) in % |
|---|---|---|---|---|---|
| | | | EC | 2.688 | 13.4 |
| | | | EC | 2.181 | 12.9 |
| | | | EC | 2.894 | 10.4 |
| 78 | **Pt-188** | 10.2 d | EC | 0.329 | 44.0 |
| | | | EC | 0.336 | 27.8 |
| | | | EC | 0.524 | 15.0 |
| | | | EC | 0.046 | 12.8 |
| | | | γ | 0.188 | 19.1 |
| | | | γ | 0.195 | 18.4 |
| 78 | **Pt-189** | 10.9 h | EC | 1.259 | 21.0 |
| | | | EC | 1.980 | 21.0 |
| | | | EC | 1.662 | 15.0 |
| | | | EC | 1.803 | 13.0 |
| | | | EC | 1.886 | 12.0 |
| 78 | **Pt-191** | 2.8 d | EC | 0.469 | 33.0 |
| | | | EC | 1.008 | 30.0 |
| | | | EC | 0.926 | 25.0 |
| | | | EC | 0.657 | 12.7 |
| | | | EC | 0.829 | 11.4 |
| | | | γ | 0.539 | 15.9 |
| 78 | **Pt-195** | 4.0 d | γ | 0.099 | 11.7 |
| 78 | **Pt-197** | 19.9 h | β- | 0.198 | 81.2 |
| | | | β- | 0.225 | 10.6 |
| | | | γ | 0.077 | 17.0 |
| 78 | **Pt-197m1** | 95.4 m | γ | 0.347 | 11.1 |
| 78 | **Pt-199** | 30.8 m | β- | 0.613 | 70.7 |
| | | | β- | 0.393 | 12.0 |
| | | | γ | 0.543 | 11.7 |
| 78 | **Pt-200** | 12.6 h | γ | 0.076 | 13.4 |
| 78 | **Pt-202** | 44.0 h | β- | 0.655 | 100.0 |
| 79 | **Au-186** | 10.7 m | β+ | 2.227 | 16.0 |
| | | | γ | 0.192 | 62.0 |
| | | | γ | 0.299 | 25.4 |
| | | | γ | 0.765 | 10.5 |
| 79 | **Au-190** | 42.8 m | EC | 2.059 | 12.0 |
| | | | EC | 3.844 | 11.0 |
| | | | γ | 0.296 | 90.0 |
| | | | γ | 0.302 | 29.7 |
| | | | γ | 0.598 | 12.0 |
| 79 | **Au-191** | 3.2 h | EC | 0.816 | 30.0 |
| | | | EC | 1.880 | 12.0 |
| | | | EC | 1.890 | 12.0 |
| | | | EC | 1.612 | 11.0 |
| | | | γ | 0.586 | 15.0 |
| 79 | **Au-192** | 4.9 h | EC | 3.514 | 14.0 |
| | | | EC | 1.181 | 11.4 |

| Atomic number | Radio-nuclide | Half-life | Decay | Energy in MeV | Intensity (abs) in % |
|---|---|---|---|---|---|
| | | | γ | 0.317 | 59.0 |
| | | | γ | 0.296 | 23.0 |
| 79 | **Au-193** | 17.7 h | EC | 0.887 | 26.0 |
| | | | EC | 1.061 | 20.0 |
| | | | EC | 0.805 | 15.7 |
| | | | EC | 1.075 | 15.0 |
| | | | EC | 0.961 | 12.4 |
| 79 | **Au-194** | 38.0 h | EC | 2.173 | 29.0 |
| | | | EC | 2.509 | 24.0 |
| | | | EC | 0.704 | 11.1 |
| | | | γ | 0.328 | 60.4 |
| | | | γ | 0.294 | 10.6 |
| 79 | **Au-196** | 6.2 d | EC | 1.151 | 67.0 |
| | | | EC | 0.818 | 24.6 |
| | | | γ | 0.356 | 87.0 |
| | | | γ | 0.333 | 22.9 |
| 79 | **Au-196m2** | 9.6 h | γ | 0.148 | 43.5 |
| | | | γ | 0.188 | 30.0 |
| 79 | **Au-198** | 2.7 d | β- | 0.315 | 99.0 |
| | | | γ | 0.412 | 95.6 |
| 79 | **Au-198m1** | 2.3 d | γ | 0.215 | 77.0 |
| | | | γ | 0.097 | 69.0 |
| | | | γ | 0.180 | 49.0 |
| | | | γ | 0.204 | 38.0 |
| | | | γ | 0.334 | 18.0 |
| 79 | **Au-199** | 3.1 d | β- | 0.082 | 72.0 |
| | | | β- | 0.067 | 21.5 |
| | | | γ | 0.158 | 40.0 |
| 79 | **Au-200** | 48.4 m | β- | 0.838 | 79.0 |
| | | | β- | 0.199 | 10.9 |
| | | | γ | 0.368 | 19.0 |
| | | | γ | 1.225 | 10.7 |
| 79 | **Au-200m1** | 18.7 h | β- | 0.169 | 84.0 |
| | | | γ | 0.368 | 86.1 |
| | | | γ | 0.498 | 82.0 |
| | | | γ | 0.579 | 80.0 |
| | | | γ | 0.256 | 79.0 |
| | | | γ | 0.760 | 74.0 |
| | | | γ | 0.181 | 62.0 |
| | | | γ | 0.333 | 14.0 |
| 80 | **Hg-190** | 20.0 m | EC | 1.348 | 85.0 |
| | | | EC | 1.520 | 10.0 |
| | | | γ | 0.143 | 68.0 |
| 80 | **Hg-191** | 50.8 m | EC | 2.674 | 10.2 |
| | | | γ | 0.253 | 57.0 |
| | | | γ | 0.420 | 19.0 |
| | | | γ | 0.579 | 18.0 |
| | | | γ | 0.274 | 13.0 |
| | | | γ | 0.241 | 12.0 |
| 80 | **Hg-192** | 4.9 h | EC | 0.458 | 66.0 |
| | | | EC | 0.607 | 19.7 |
| | | | γ | 0.275 | 52.0 |
| 80 | **Hg-193** | 3.8 h | EC | 2.118 | 34.0 |
| | | | EC | 1.224 | 24.0 |
| | | | EC | 2.305 | 15.0 |
| | | | EC | 1.961 | 13.0 |
| | | | γ | 0.187 | 15.1 |
| | | | γ | 0.382 | 15.0 |
| | | | γ | 0.861 | 12.1 |
| 80 | **Hg-195** | 10.5 h | EC | 1.509 | 71.0 |
| | | | EC | 1.570 | 10.0 |
| 80 | **Hg-195m1** | 41.6 h | EC | 1.427 | 32.0 |
| | | | γ | 0.262 | 31.0 |
| 80 | **Hg-197** | 64.1 h | EC | 0.523 | 96.7 |
| | | | γ | 0.077 | 18.7 |
| 80 | **Hg-197m1** | 23.8 h | γ | 0.134 | 33.5 |
| 80 | **Hg-199** | 42.7 m | γ | 0.158 | 52.3 |
| | | | γ | 0.374 | 13.8 |
| 80 | **Hg-203** | 46.6 d | β- | 0.058 | 100.0 |
| | | | γ | 0.279 | 81.6 |
| 81 | **Tl-194** | 33.0 m | β+ | 1.769 | 21.0 |
| | | | EC | 4.942 | 25.0 |
| | | | EC | 5.370 | 12.0 |
| | | | γ | 0.428 | 75.0 |
| | | | γ | 0.645 | 10.8 |
| 81 | **Tl-194m1** | 32.8 m | EC | 3.710 | 26.0 |
| | | | EC | 3.156 | 15.8 |
| | | | EC | 3.807 | 11.0 |
| | | | EC | 3.355 | 10.0 |
| | | | γ | 0.428 | 99.0 |
| | | | γ | 0.636 | 99.0 |
| | | | γ | 0.749 | 76.0 |
| | | | γ | 0.735 | 22.0 |
| 81 | **Tl-195** | 1.2 h | EC | 1.444 | 15.4 |
| | | | EC | 1.923 | 13.5 |
| | | | EC | 2.244 | 12.0 |
| | | | γ | 0.564 | 11.2 |
| | | | γ | 0.884 | 10.6 |
| 81 | **Tl-196** | 1.8 h | EC | 3.904 | 28.0 |
| | | | γ | 0.426 | 83.0 |
| | | | γ | 0.611 | 11.8 |
| 81 | **Tl-196m1** | 1.4 h | EC | 2.884 | 66.0 |

| Atomic number | Radio-nuclide | Half-life | Decay | Energy in MeV | Intensity (abs) in % |
|---|---|---|---|---|---|
| | | | EC | 2.379 | 15.0 |
| | | | γ | 0.635 | 94.0 |
| | | | γ | 0.426 | 92.0 |
| | | | γ | 0.695 | 90.0 |
| | | | γ | 0.505 | 13.0 |
| 81 | **Tl-197** | 2.8 h | EC | 2.200 | 52.0 |
| | | | EC | 1.622 | 21.0 |
| | | | γ | 0.426 | 13.0 |
| 81 | **Tl-198** | 5.3 h | EC | 1.627 | 11.7 |
| | | | γ | 0.412 | 79.0 |
| | | | γ | 0.676 | 10.6 |
| 81 | **Tl-198m1** | 1.9 h | EC | 2.320 | 24.0 |
| | | | γ | 0.412 | 59.0 |
| | | | γ | 0.637 | 59.0 |
| | | | γ | 0.587 | 54.4 |
| | | | γ | 0.283 | 27.0 |
| 81 | **Tl-199** | 7.4 h | EC | 1.488 | 48.0 |
| | | | EC | 1.033 | 29.0 |
| | | | γ | 0.455 | 12.4 |
| | | | γ | 0.208 | 12.3 |
| 81 | **Tl-200** | 26.1 h | EC | 0.882 | 30.0 |
| | | | EC | 2.088 | 24.9 |
| | | | EC | 0.680 | 13.6 |
| | | | γ | 0.368 | 87.0 |
| | | | γ | 1.206 | 30.0 |
| | | | γ | 0.579 | 13.7 |
| | | | γ | 0.828 | 10.8 |
| 81 | **Tl-201** | 3.0 d | EC | 0.314 | 41.4 |
| | | | EC | 0.479 | 26.3 |
| | | | EC | 0.481 | 20.9 |
| | | | EC | 0.449 | 10.9 |
| | | | γ | 0.167 | 10.0 |
| 81 | **Tl-202** | 12.3 d | EC | 0.923 | 94.3 |
| | | | γ | 0.440 | 91.5 |
| 82 | **Pb-194** | 10.7 m | EC | 1.834 | 23.8 |
| | | | EC | 1.101 | 23.7 |
| | | | γ | 0.582 | 18.9 |
| | | | γ | 1.519 | 16.5 |
| | | | γ | 0.204 | 16.3 |
| 82 | **Pb-195** | 15.0 m | EC | 3.283 | 18.6 |
| | | | EC | 3.767 | 14.0 |
| | | | γ | 0.384 | 91.0 |
| | | | γ | 0.394 | 44.2 |
| | | | γ | 0.878 | 24.3 |
| | | | γ | 0.708 | 14.1 |
| 82 | **Pb-196** | 37.0 m | EC | 1.381 | 33.0 |
| | | | EC | 1.770 | 25.0 |
| | | | EC | 1.883 | 13.0 |
| | | | EC | 1.642 | 11.5 |
| | | | EC | 1.944 | 11.0 |
| | | | γ | 0.253 | 27.0 |
| | | | γ | 0.502 | 27.0 |
| | | | γ | 0.192 | 11.1 |
| | | | γ | 0.367 | 11.1 |
| 82 | **Pb-197m1** | 42.9 m | EC | 3.303 | 22.0 |
| | | | γ | 0.386 | 74.0 |
| | | | γ | 0.223 | 25.0 |
| | | | γ | 0.388 | 25.0 |
| | | | γ | 0.774 | 14.2 |
| 82 | **Pb-198** | 2.4 h | EC | 1.160 | 27.0 |
| | | | EC | 0.794 | 24.0 |
| | | | EC | 1.277 | 22.8 |
| | | | EC | 0.585 | 10.5 |
| | | | γ | 0.290 | 36.0 |
| | | | γ | 0.365 | 19.0 |
| | | | γ | 0.173 | 18.2 |
| 82 | **Pb-199** | 90.0 m | EC | 2.830 | 28.0 |
| | | | EC | 2.463 | 16.0 |
| | | | EC | 1.328 | 12.0 |
| | | | γ | 0.367 | 44.0 |
| 82 | **Pb-199m1** | 12.2 m | γ | 0.424 | 19.6 |
| 82 | **Pb-200** | 21.5 h | EC | 0.657 | 68.0 |
| | | | EC | 0.279 | 14.3 |
| | | | γ | 0.148 | 38.2 |
| 82 | **Pb-201** | 9.3 h | EC | 1.593 | 52.0 |
| | | | EC | 0.647 | 12.3 |
| | | | γ | 0.331 | 77.0 |
| 82 | **Pb-202m1** | 3.5 h | γ | 0.961 | 89.9 |
| | | | γ | 0.422 | 84.0 |
| | | | γ | 0.787 | 49.0 |
| | | | γ | 0.657 | 31.7 |
| 82 | **Pb-203** | 51.9 h | EC | 0.696 | 95.3 |
| | | | γ | 0.279 | 80.9 |
| 82 | **Pb-204** | 66.9 m | γ | 0.899 | 99.2 |
| | | | γ | 0.375 | 94.2 |
| | | | γ | 0.912 | 91.5 |
| 82 | **Pb-209** | 3.2 h | β- | 0.198 | 100.0 |
| 82 | **Pb-211** | 36.1 m | β- | 0.471 | 91.3 |
| 82 | **Pb-212** | 10.6 h | β- | 0.094 | 83.1 |
| | | | β- | 0.172 | 11.9 |
| | | | γ | 0.239 | 43.6 |
| 82 | **Pb-214** | 27.1 m | β- | 0.206 | 45.9 |

| Atomic number | Radio-nuclide | Half-life | Decay | Energy in MeV | Intensity (abs) in % |
|---|---|---|---|---|---|
| | | | β- | 0.226 | 40.2 |
| | | | β- | 0.335 | 11.0 |
| | | | γ | 0.352 | 35.6 |
| | | | γ | 0.295 | 18.4 |
| 83 | **Bi-200** | 36.4 m | EC | 3.964 | 29.0 |
| | | | EC | 3.690 | 25.0 |
| | | | EC | 3.719 | 22.0 |
| | | | γ | 1.026 | 100.0 |
| | | | γ | 0.462 | 98.0 |
| | | | γ | 0.420 | 91.0 |
| | | | γ | 0.245 | 46.0 |
| 83 | **Bi-200m1** | 31.0 m | γ | 1.027 | 96.0 |
| | | | γ | 0.462 | 40.0 |
| | | | γ | 0.420 | 22.8 |
| 83 | **Bi-201** | 103.0 m | EC | 3.211 | 19.0 |
| | | | γ | 0.629 | 26.0 |
| | | | γ | 0.936 | 12.2 |
| | | | γ | 1.014 | 11.6 |
| | | | γ | 0.786 | 10.3 |
| 83 | **Bi-202** | 1.7 h | EC | 3.161 | 11.0 |
| | | | γ | 0.961 | 99.3 |
| | | | γ | 0.422 | 84.0 |
| | | | γ | 0.657 | 60.6 |
| 83 | **Bi-203** | 11.8 h | EC | 0.534 | 21.5 |
| | | | EC | 0.579 | 20.3 |
| | | | γ | 0.820 | 30.0 |
| | | | γ | 0.825 | 14.8 |
| | | | γ | 0.897 | 13.2 |
| | | | γ | 1.847 | 11.6 |
| 83 | **Bi-204** | 11.2 h | EC | 1.270 | 14.7 |
| | | | EC | 1.511 | 11.3 |
| | | | EC | 1.411 | 10.0 |
| | | | EC | 2.182 | 10.0 |
| | | | γ | 0.899 | 99.0 |
| | | | γ | 0.375 | 82.0 |
| | | | γ | 0.984 | 59.0 |
| | | | γ | 0.912 | 13.6 |
| | | | γ | 0.671 | 11.4 |
| | | | γ | 0.912 | 11.2 |
| | | | γ | 0.918 | 10.9 |
| 83 | **Bi-205** | 15.3 d | EC | 0.944 | 32.6 |
| | | | EC | 2.002 | 16.2 |
| | | | EC | 1.094 | 11.2 |
| | | | γ | 1.764 | 32.5 |
| | | | γ | 0.703 | 31.1 |
| | | | γ | 0.988 | 16.1 |

| Atomic number | Radio-nuclide | Half-life | Decay | Energy in MeV | Intensity (abs) in % |
|---|---|---|---|---|---|
| 83 | **Bi-206** | 6.2 d | EC | 0.355 | 49.1 |
| | | | EC | 0.479 | 43.7 |
| | | | γ | 0.803 | 99.0 |
| | | | γ | 0.881 | 66.2 |
| | | | γ | 0.516 | 40.8 |
| | | | γ | 1.719 | 31.9 |
| | | | γ | 0.537 | 30.5 |
| | | | γ | 0.344 | 23.5 |
| | | | γ | 0.184 | 15.8 |
| | | | γ | 0.895 | 15.7 |
| | | | γ | 0.497 | 15.3 |
| | | | γ | 1.098 | 13.5 |
| | | | γ | 0.398 | 10.8 |
| 83 | **Bi-210** | 5.0 d | β- | 0.389 | 100.0 |
| 83 | **Bi-212** | 60.6 m | α | 6.051 | 25.1 |
| | | | β- | 0.834 | 55.4 |
| 83 | **Bi-212m1** | 25.0 m | α | 6.340 | 35.0 |
| | | | α | 6.300 | 26.0 |
| 83 | **Bi-213** | 45.6 m | β- | 0.492 | 65.9 |
| | | | β- | 0.320 | 30.8 |
| | | | γ | 0.440 | 25.9 |
| 83 | **Bi-214** | 19.9 m | β- | 1.269 | 19.1 |
| | | | β- | 0.539 | 17.6 |
| | | | β- | 0.525 | 17.0 |
| | | | γ | 0.609 | 45.5 |
| | | | γ | 1.764 | 15.3 |
| | | | γ | 1.120 | 14.9 |
| 84 | **Po-200** | 11.5 m | α | 5.862 | 11.1 |
| 84 | **Po-201** | 15.6 m | EC | 4.045 | 24.0 |
| | | | γ | 0.890 | 20.5 |
| | | | γ | 0.240 | 14.6 |
| | | | γ | 0.904 | 11.2 |
| 84 | **Po-202** | 44.6 m | γ | 0.689 | 49.0 |
| | | | γ | 0.316 | 13.7 |
| 84 | **Po-203** | 36.7 m | EC | 3.321 | 25.0 |
| | | | EC | 3.139 | 13.1 |
| | | | γ | 0.909 | 55.0 |
| | | | γ | 1.091 | 19.2 |
| | | | γ | 0.894 | 18.7 |
| | | | γ | 0.215 | 14.3 |
| | | | γ | 0.513 | 11.0 |
| 84 | **Po-204** | 3.5 h | EC | 0.961 | 51.3 |
| | | | EC | 0.696 | 23.8 |
| | | | EC | 1.075 | 11.2 |
| | | | γ | 0.884 | 34.3 |
| | | | γ | 0.270 | 31.9 |

| Atomic number | Radio-nuclide | Half-life | Decay | Energy in MeV | Intensity (abs) in % |
|---|---|---|---|---|---|
| | | | γ | 1.016 | 27.6 |
| | | | γ | 0.535 | 15.2 |
| | | | γ | 0.763 | 13.2 |
| | | | γ | 0.063 | 12.3 |
| | | | γ | 0.137 | 11.1 |
| | | | γ | 1.040 | 11.0 |
| 84 | **Po-205** | 1.7 h | EC | 2.552 | 39.0 |
| | | | EC | 1.844 | 16.1 |
| | | | EC | 2.703 | 14.0 |
| | | | γ | 0.872 | 37.0 |
| | | | γ | 1.001 | 28.8 |
| | | | γ | 0.850 | 25.5 |
| | | | γ | 0.837 | 19.2 |
| 84 | **Po-206** | 8.8 d | EC | 0.457 | 74.1 |
| | | | EC | 0.914 | 19.3 |
| | | | γ | 1.032 | 31.7 |
| | | | γ | 0.511 | 23.2 |
| | | | γ | 0.286 | 22.9 |
| | | | γ | 0.807 | 21.8 |
| | | | γ | 0.338 | 18.5 |
| | | | γ | 0.522 | 15.1 |
| 84 | **Po-207** | 5.8 h | EC | 1.915 | 63.7 |
| | | | EC | 0.849 | 21.2 |
| | | | EC | 2.162 | 10.9 |
| | | | γ | 0.992 | 59.2 |
| | | | γ | 0.743 | 28.4 |
| | | | γ | 0.912 | 17.0 |
| 85 | **At-205** | 26.9 m | γ | 0.719 | 44.0 |
| | | | γ | 0.669 | 12.4 |
| 85 | **At-206** | 30.6 m | EC | 4.189 | 29.0 |
| | | | EC | 4.584 | 12.0 |
| | | | γ | 0.701 | 98.0 |
| | | | γ | 0.477 | 86.0 |
| | | | γ | 0.396 | 48.0 |
| | | | γ | 0.734 | 10.2 |
| 85 | **At-207** | 1.8 h | EC | 1.600 | 17.5 |
| | | | EC | 1.673 | 11.3 |
| | | | γ | 0.814 | 45.0 |
| | | | γ | 0.588 | 19.5 |
| | | | γ | 0.301 | 12.9 |
| 85 | **At-208** | 1.6 h | EC | 1.413 | 19.5 |
| | | | EC | 2.422 | 16.2 |
| | | | EC | 3.454 | 11.0 |
| | | | γ | 0.687 | 97.6 |
| | | | γ | 0.660 | 89.0 |
| | | | γ | 0.178 | 48.6 |
| | | | γ | 0.845 | 19.7 |
| | | | γ | 1.028 | 16.8 |
| | | | γ | 0.990 | 10.7 |
| 85 | **At-209** | 5.4 h | EC | 1.171 | 70.9 |
| | | | γ | 0.545 | 90.9 |
| | | | γ | 0.782 | 83.3 |
| | | | γ | 0.790 | 63.5 |
| | | | γ | 0.195 | 23.5 |
| | | | γ | 0.239 | 12.5 |
| 85 | **At-210** | 8.1 h | EC | 1.071 | 70.0 |
| | | | EC | 0.955 | 19.0 |
| | | | γ | 1.181 | 99.0 |
| | | | γ | 0.245 | 79.0 |
| | | | γ | 1.483 | 46.5 |
| | | | γ | 1.437 | 29.0 |
| | | | γ | 1.600 | 13.4 |
| 85 | **At-211** | 7.2 h | α | 5.870 | 41.8 |
| | | | EC | 0.785 | 57.9 |
| 86 | **Rn-208** | 24.4 m | α | 6.140 | 62.0 |
| 86 | **Rn-209** | 28.8 m | α | 6.039 | 16.9 |
| | | | EC | 3.208 | 40.0 |
| | | | EC | 3.546 | 20.0 |
| | | | γ | 0.408 | 50.4 |
| | | | γ | 0.746 | 22.8 |
| | | | γ | 0.337 | 14.5 |
| 86 | **Rn-210** | 2.4 h | α | 6.041 | 96.0 |
| 86 | **Rn-211** | 14.6 h | α | 5.784 | 17.3 |
| | | | EC | 0.413 | 68.0 |
| | | | γ | 0.674 | 45.4 |
| | | | γ | 1.363 | 32.7 |
| | | | γ | 0.678 | 29.0 |
| | | | γ | 0.442 | 23.1 |
| | | | γ | 1.127 | 22.2 |
| | | | γ | 0.947 | 16.3 |
| 86 | **Rn-212** | 23.9 m | α | 6.264 | 100.0 |
| 86 | **Rn-221** | 25.0 m | α | 6.037 | 16.2 |
| | | | β- | 0.271 | 27.2 |
| | | | γ | 0.186 | 21.6 |
| 86 | **Rn-222** | 3.8 d | α | 5.489 | 99.9 |
| 86 | **Rn-223** | 24.3 m | β- | 0.676 | 24.0 |
| | | | β- | 0.648 | 21.0 |
| | | | β- | 0.438 | 14.0 |
| | | | β- | 0.387 | 11.0 |
| 87 | **Fr-212** | 20.0 m | α | 6.262 | 16.3 |
| | | | α | 6.383 | 10.3 |
| | | | EC | 2.434 | 20.5 |

| Atomic number | Radio-nuclide | Half-life | Decay | Energy in MeV | Intensity (abs) in % |
|---|---|---|---|---|---|
| | | | EC | 3.481 | 15.4 |
| | | | EC | 3.620 | 13.1 |
| | | | γ | 1.275 | 46.0 |
| | | | γ | 0.228 | 42.6 |
| | | | γ | 1.186 | 14.1 |
| 87 | **Fr-222** | 14.2 m | β- | 0.604 | 54.0 |
| | | | β- | 0.688 | 38.0 |
| | | | γ | 0.206 | 50.0 |
| | | | γ | 0.111 | 13.1 |
| 87 | **Fr-223** | 22.0 m | β- | 0.362 | 70.0 |
| | | | β- | 0.350 | 15.0 |
| | | | β- | 0.293 | 10.1 |
| | | | γ | 0.050 | 34.0 |
| 88 | **Ra-223** | 11.4 d | α | 5.716 | 51.6 |
| | | | α | 5.607 | 25.2 |
| | | | γ | 0.269 | 13.9 |
| 88 | **Ra-224** | 3.6 d | α | 5.685 | 94.9 |
| 88 | **Ra-225** | 14.9 d | β- | 0.093 | 69.5 |
| | | | β- | 0.105 | 30.5 |
| | | | γ | 0.040 | 30.0 |
| 88 | **Ra-227** | 42.2 m | β- | 0.438 | 37.2 |
| | | | β- | 0.431 | 25.2 |
| | | | β- | 0.323 | 19.0 |
| | | | γ | 0.027 | 14.1 |
| 88 | **Ra-230** | 93.0 m | β- | 0.218 | 40.0 |
| | | | β- | 0.193 | 16.0 |
| | | | β- | 0.183 | 11.0 |
| | | | γ | 0.072 | 10.0 |
| 89 | **Ac-224** | 2.8 h | EC | 1.192 | 90.0 |
| | | | γ | 0.216 | 52.3 |
| | | | γ | 0.131 | 26.9 |
| 89 | **Ac-225** | 9.9 d | α | 5.830 | 50.7 |
| | | | α | 5.793 | 18.1 |
| 89 | **Ac-226** | 29.4 h | β- | 0.281 | 49.0 |
| | | | β- | 0.367 | 24.0 |
| | | | β- | 0.340 | 10.0 |
| | | | EC | 0.386 | 11.3 |
| | | | γ | 0.230 | 26.9 |
| | | | γ | 0.158 | 17.5 |
| 89 | **Ac-228** | 6.2 h | β- | 0.385 | 29.9 |
| | | | β- | 0.610 | 11.7 |
| | | | γ | 0.911 | 25.8 |
| | | | γ | 0.969 | 15.8 |
| | | | γ | 0.338 | 11.3 |
| 89 | **Ac-229** | 62.7 m | β- | 0.387 | 79.0 |
| 90 | **Th-226** | 30.6 m | α | 6.337 | 75.5 |

| Atomic number | Radio-nuclide | Half-life | Decay | Energy in MeV | Intensity (abs) in % |
|---|---|---|---|---|---|
| | | | α | 6.234 | 22.8 |
| 90 | **Th-227** | 18.7 d | α | 6.038 | 24.2 |
| | | | α | 5.978 | 23.5 |
| | | | α | 5.757 | 20.4 |
| | | | γ | 0.236 | 12.9 |
| 90 | **Th-231** | 25.5 h | β- | 0.080 | 40.0 |
| | | | β- | 0.085 | 32.0 |
| | | | β- | 0.056 | 12.1 |
| | | | β- | 0.080 | 12.0 |
| | | | γ | 0.026 | 14.1 |
| 90 | **Th-233** | 21.8 m | β- | 0.411 | 50.0 |
| | | | β- | 0.414 | 30.0 |
| | | | β- | 0.378 | 16.0 |
| 90 | **Th-234** | 24.1 d | β- | 0.054 | 78.0 |
| | | | β- | 0.028 | 14.0 |
| 90 | **Th-236** | 37.3 m | β- | 0.359 | 80.0 |
| 91 | **Pa-227** | 38.3 m | α | 6.466 | 42.7 |
| | | | α | 6.416 | 12.8 |
| 91 | **Pa-228** | 22.0 h | EC | 0.720 | 31.8 |
| 91 | **Pa-229** | 1.5 d | EC | 0.311 | 93.0 |
| 91 | **Pa-230** | 17.4 d | EC | 0.359 | 39.6 |
| | | | EC | 1.257 | 21.0 |
| | | | γ | 0.952 | 30.0 |
| 91 | **Pa-232** | 1.3 d | β- | 0.089 | 73.0 |
| | | | β- | 0.079 | 24.5 |
| | | | γ | 0.969 | 42.3 |
| | | | γ | 0.894 | 19.6 |
| | | | γ | 0.150 | 10.4 |
| 91 | **Pa-233** | 27.0 d | β- | 0.041 | 26.7 |
| | | | β- | 0.062 | 26.0 |
| | | | β- | 0.071 | 24.0 |
| | | | β- | 0.046 | 16.2 |
| | | | γ | 0.312 | 38.5 |
| 91 | **Pa-234** | 6.7 h | β- | 0.137 | 34.0 |
| | | | β- | 0.194 | 20.4 |
| | | | β- | 0.137 | 12.9 |
| | | | γ | 0.131 | 18.9 |
| | | | γ | 0.946 | 14.0 |
| | | | γ | 0.883 | 10.0 |
| 91 | **Pa-235** | 24.4 m | β- | 0.476 | 100.0 |
| 92 | **U-229** | 58.0 m | α | 6.360 | 12.8 |
| 92 | **U-230** | 20.2 d | α | 5.888 | 67.4 |
| | | | α | 5.818 | 32.0 |
| 92 | **U-231** | 4.2 d | EC | 0.297 | 50.0 |
| | | | EC | 0.279 | 50.0 |
| | | | γ | 0.026 | 14.6 |

| Atomic number | Radio-nuclide | Half-life | Decay | Energy in MeV | Intensity (abs) in % |
|---|---|---|---|---|---|
| 92 | **U-237** | 6.8 d | β- | 0.065 | 51.0 |
| | | | β- | 0.069 | 42.0 |
| | | | γ | 0.060 | 34.5 |
| | | | γ | 0.208 | 21.2 |
| 92 | **U-239** | 23.5 m | β- | 0.390 | 69.0 |
| | | | β- | 0.419 | 18.7 |
| | | | γ | 0.075 | 53.2 |
| 92 | **U-240** | 14.1 h | β- | 0.108 | 75.0 |
| | | | β- | 0.094 | 25.0 |
| 92 | **U-242** | 16.8 m | β- | 0.395 | 79.0 |
| | | | β- | 0.370 | 13.0 |
| | | | γ | 0.068 | 10.0 |
| 93 | **Np-231** | 48.8 m | EC | 1.700 | 88.2 |
| 93 | **Np-232** | 14.7 m | EC | 1.556 | 94.0 |
| | | | γ | 0.327 | 53.0 |
| | | | γ | 0.820 | 33.9 |
| | | | γ | 0.867 | 24.9 |
| | | | γ | 0.864 | 20.7 |
| | | | γ | 0.282 | 20.1 |
| 93 | **Np-233** | 36.2 m | EC | 1.030 | 97.0 |
| 93 | **Np-234** | 4.4 d | EC | 0.208 | 29.0 |
| | | | EC | 1.810 | 26.0 |
| | | | EC | 0.239 | 17.0 |
| | | | EC | 0.573 | 10.2 |
| | | | γ | 1.558 | 18.5 |
| | | | γ | 1.527 | 11.1 |
| 93 | **Np-236m1** | 22.5 h | β- | 0.178 | 20.0 |
| | | | EC | 0.930 | 18.0 |
| 93 | **Np-238** | 2.1 d | β- | 0.072 | 44.8 |
| | | | β- | 0.412 | 41.1 |
| | | | β- | 0.060 | 11.5 |
| | | | γ | 0.984 | 25.1 |
| | | | γ | 1.029 | 18.2 |
| 93 | **Np-239** | 2.4 d | β- | 0.126 | 45.0 |
| | | | β- | 0.093 | 44.0 |
| | | | γ | 0.106 | 25.3 |
| | | | γ | 0.278 | 14.5 |
| | | | γ | 0.228 | 10.7 |
| 93 | **Np-240** | 61.9 m | β- | 0.276 | 79.0 |
| | | | β- | 0.347 | 17.0 |
| | | | β- | 0.376 | 10.0 |
| | | | γ | 0.566 | 27.6 |
| | | | γ | 0.974 | 25.9 |
| | | | γ | 0.601 | 20.0 |
| | | | γ | 0.896 | 14.8 |
| | | | γ | 0.448 | 13.4 |

| Atomic number | Radio-nuclide | Half-life | Decay | Energy in MeV | Intensity (abs) in % |
|---|---|---|---|---|---|
| 93 | **Np-241** | 13.9 m | β- | 0.432 | 70.0 |
| | | | β- | 0.366 | 30.8 |
| 94 | **Pu-232** | 33.8 m | α | 6.600 | 15.0 |
| | | | EC | 0.960 | 80.0 |
| 94 | **Pu-235** | 25.3 m | EC | 1.139 | 57.0 |
| | | | EC | 1.105 | 37.0 |
| 94 | **Pu-237** | 45.6 d | EC | 0.220 | 79.0 |
| | | | EC | 0.187 | 11.8 |
| 94 | **Pu-243** | 5.0 h | β- | 0.172 | 60.0 |
| | | | β- | 0.144 | 21.0 |
| | | | γ | 0.084 | 23.0 |
| 94 | **Pu-245** | 10.5 h | β- | 0.301 | 53.0 |
| | | | β- | 0.111 | 15.0 |
| | | | β- | 0.405 | 10.0 |
| | | | γ | 0.327 | 27.0 |
| 94 | **Pu-246** | 10.8 d | β- | 0.047 | 85.0 |
| | | | γ | 0.044 | 25.0 |
| | | | γ | 0.224 | 23.5 |
| 95 | **Am-235** | 10.3 m | EC | 2.442 | 31.9 |
| 95 | **Am-237** | 73.6 m | EC | 1.200 | 49.7 |
| | | | EC | 1.325 | 10.0 |
| | | | γ | 0.280 | 47.3 |
| 95 | **Am-238** | 98.0 m | EC | 1.297 | 54.0 |
| | | | EC | 1.655 | 14.8 |
| | | | EC | 2.260 | 11.0 |
| | | | γ | 0.963 | 28.0 |
| | | | γ | 0.919 | 23.0 |
| | | | γ | 0.561 | 10.9 |
| 95 | **Am-239** | 11.9 h | EC | 0.517 | 71.0 |
| | | | EC | 0.290 | 18.1 |
| | | | γ | 0.278 | 15.0 |
| | | | γ | 0.228 | 11.3 |
| 95 | **Am-240** | 50.8 h | EC | 0.354 | 98.6 |
| | | | γ | 0.988 | 72.2 |
| | | | γ | 0.889 | 24.7 |
| 95 | **Am-242** | 16.0 h | β- | 0.186 | 45.0 |
| | | | β- | 0.200 | 37.0 |
| | | | EC | 0.707 | 10.6 |
| 95 | **Am-244** | 10.1 h | β- | 0.110 | 100.0 |
| | | | γ | 0.744 | 66.0 |
| | | | γ | 0.898 | 28.0 |
| | | | γ | 0.154 | 16.0 |
| 95 | **Am-244m1** | 26.0 m | β- | 0.512 | 72.0 |
| | | | β- | 0.496 | 25.0 |
| 95 | **Am-245** | 2.1 h | β- | 0.281 | 78.0 |
| | | | β- | 0.193 | 15.0 |

| Atomic number | Radio-nuclide | Half-life | Decay | Energy in MeV | Intensity (abs) in % |
|---|---|---|---|---|---|
| 95 | **Am-246** | 39.0 m | β- | 0.391 | 100.0 |
| | | | γ | 0.679 | 64.0 |
| | | | γ | 0.205 | 44.0 |
| | | | γ | 0.154 | 31.0 |
| | | | γ | 0.756 | 16.1 |
| 95 | **Am-246m1** | 25.0 m | β- | 0.429 | 37.5 |
| | | | β- | 0.519 | 16.1 |
| | | | β- | 0.419 | 14.2 |
| | | | γ | 1.079 | 27.8 |
| | | | γ | 0.799 | 24.8 |
| | | | γ | 1.062 | 17.2 |
| | | | γ | 1.036 | 12.7 |
| 95 | **Am-247** | 23.0 m | β- | 0.443 | 63.0 |
| | | | β- | 0.465 | 31.0 |
| | | | γ | 0.285 | 25.0 |
| 96 | **Cm-239** | 2.7 h | γ | 0.188 | 36.0 |
| | | | γ | 0.146 | 11.9 |
| 96 | **Cm-240** | 27.0 d | α | 6.291 | 70.9 |
| | | | α | 6.248 | 28.8 |
| 96 | **Cm-241** | 32.8 d | EC | 0.131 | 42.2 |
| | | | EC | 0.115 | 26.7 |
| | | | EC | 0.296 | 26.0 |
| | | | γ | 0.472 | 71.0 |
| 96 | **Cm-251** | 16.8 m | β- | 0.474 | 73.0 |
| | | | β- | 0.274 | 14.0 |
| | | | γ | 0.543 | 11.0 |
| 97 | **Bk-245** | 5.0 d | EC | 0.558 | 90.0 |
| | | | γ | 0.253 | 31.0 |
| 97 | **Bk-246** | 1.8 d | EC | 0.508 | 49.0 |
| | | | EC | 0.474 | 21.0 |
| | | | EC | 0.226 | 10.8 |
| | | | γ | 0.799 | 62.0 |
| 97 | **Bk-248** | 23.7 h | β- | 0.267 | 45.0 |
| | | | β- | 0.252 | 20.0 |
| 97 | **Bk-250** | 3.2 h | β- | 0.228 | 83.4 |
| | | | γ | 0.989 | 45.0 |
| | | | γ | 1.032 | 35.6 |
| 97 | **Bk-251** | 55.6 m | β- | 0.286 | 90.0 |
| 97 | **Bk-251-m1** | 23.7 h | EC | 0.706 | 23.0 |
| 98 | **Cf-244** | 19.4 m | α | 7.209 | 52.0 |
| | | | α | 7.174 | 17.0 |
| 98 | **Cf-245** | 45.0 m | α | 7.138 | 32.4 |
| 98 | **Cf-246** | 35.7 h | α | 6.750 | 79.3 |
| | | | α | 6.708 | 20.6 |
| 98 | **Cf-247** | 3.1 h | EC | 0.572 | 74.0 |
| | | | EC | 0.530 | 21.0 |
| 98 | **Cf-253** | 17.8 d | β- | 0.065 | 50.0 |
| | | | β- | 0.079 | 50.0 |
| 98 | **Cf-255** | 85.0 m | β- | 0.218 | 100.0 |
| 99 | **Es-249** | 102.2 m | EC | 1.070 | 39.0 |
| | | | EC | 1.450 | 25.0 |
| | | | EC | 0.637 | 10.0 |
| | | | γ | 0.380 | 41.0 |
| 99 | **Es-250** | 8.6 h | EC | 0.622 | 53.2 |
| | | | EC | 0.600 | 41.0 |
| | | | EC | 0.642 | 10.0 |
| | | | γ | 0.829 | 72.0 |
| | | | γ | 0.303 | 22.0 |
| | | | γ | 0.349 | 20.1 |
| | | | γ | 0.384 | 13.8 |
| 99 | **Es-250m1** | 2.2 h | EC | 2.100 | 50.0 |
| | | | EC | 1.068 | 23.2 |
| | | | γ | 0.989 | 13.3 |
| | | | γ | 1.032 | 10.6 |
| 99 | **Es-251** | 33.0 h | EC | 0.377 | 80.0 |
| | | | EC | 0.199 | 22.0 |
| 99 | **Es-253** | 20.5 d | α | 6.633 | 89.9 |
| 99 | **Es-254m1** | 39.3 h | β- | 0.138 | 56.0 |
| | | | β- | 0.361 | 25.0 |
| | | | β- | 0.125 | 16.0 |
| | | | γ | 0.649 | 29.0 |
| | | | γ | 0.694 | 24.8 |
| | | | γ | 0.689 | 12.5 |
| 99 | **Es-255** | 39.8 d | β- | 0.080 | 92.0 |
| 99 | **Es-256m1** | 7.6 h | β- | 0.075 | 86.0 |
| | | | β- | 0.037 | 14.0 |
| | | | γ | 0.862 | 50.3 |
| | | | γ | 0.231 | 30.7 |
| | | | γ | 0.173 | 24.8 |
| | | | γ | 1.093 | 23.7 |
| | | | γ | 0.218 | 14.6 |
| | | | γ | 0.834 | 14.0 |
| | | | γ | 0.106 | 13.1 |
| | | | γ | 0.135 | 13.1 |
| 100 | **Fm-250** | 30.0 m | α | 7.435 | 75.0 |
| | | | α | 7.396 | 15.0 |
| 100 | **Fm-251** | 5.3 h | EC | 1.432 | 82.0 |
| | | | EC | 1.384 | 11.0 |
| 100 | **Fm-252** | 25.4 h | α | 7.039 | 84.0 |
| | | | α | 6.998 | 15.0 |
| 100 | **Fm-254** | 3.2 h | α | 7.192 | 84.9 |
| | | | α | 7.150 | 14.2 |

| Atomic number | Radio-nuclide | Half-life | Decay | Energy in MeV | Intensity (abs) in % |
|---|---|---|---|---|---|
| 100 | **Fm-255** | 20.1 h | α | 7.022 | 93.4 |
| 101 | **Md-255** | 27.0 m | EC | 0.981 | 60.0 |
| | | | EC | 0.812 | 27.0 |
| 101 | **Md-256** | 77.7 m | EC | 1.970 | 58.0 |
| | | | EC | 0.644 | 13.0 |
| | | | EC | 0.596 | 10.0 |
| 101 | **Md-257** | 5.5 h | α | 7.074 | 14.0 |
| | | | EC | 0.407 | 100.0 |
| | | | γ | 0.371 | 12.0 |
| 102 | **No-259** | 58.0 m | α | 7.505 | 75.0 |
| | | | EC | 0.486 | 25.0 |

In an embodiment of the present disclosure, the radionuclide is used for diagnosis. In some embodiments, the radioactive isotope is selected from the group including, but not limited to, $^{43}Sc$, $^{44}Sc$, $^{51}Mn$, $^{52}Mn$, $^{64}Cu$, $^{67}Ga$, $^{68}Ga$, $^{86}Y$, $^{89}Zr$, $^{94m}Tc$, $^{99m}Tc$, $^{111}In$, $^{152}Tb$, $^{155}Tb$, $^{177}Lu$, $^{201}Tl$, $^{203}Pb$, $^{18}F$, $^{76}Br$, $^{77}Br$, $^{123}I$, $^{124}I$, and $^{125}I$. In some embodiments, the radionuclide is selected from $^{43}Sc$, $^{44}Sc$, $^{64}Cu$, $^{67}Ga$, $^{68}Ga$, $^{86}Y$, $^{89}Zr$, $^{99m}Tc$, $^{111}In$, $^{152}Tb$, $^{155}Tb$, and $^{203}Pb$. In some embodiments, the radionuclide is selected from $^{64}Cu$, $^{68}Ga$, and $^{111}In$. It will, however, also be acknowledged by a person skilled in the art that the use of said radionuclide is not limited to diagnostic purposes, but encompasses their use in therapy and theragnostics when conjugated to the compound of the disclosure.

In an embodiment of the present disclosure, the radionuclide is used for therapy. In some embodiments, the radioactive isotope is selected from $^{47}Sc$, $^{67}Cu$, $^{89}Sr$, $^{90}Y$, $^{111}In$, $^{153}Sm$, $^{149}Tb$, $^{161}Tb$, $^{177}Lu$, $^{186}Re$, $^{188}Re$, $^{212}Pb$, $^{213}Bi$, $^{223}Ra$, $^{224}Ra$ $^{225}Ac$, $^{226}Th$, $^{227}Th$, $^{131}I$, and $^{211}At$. In some embodiments, the radioactive isotope is selected from $^{47}Sc$, $^{67}Cu$, $^{90}Y$, $^{177}Lu$, $^{188}Re$, $^{212}Pb$, $^{213}Bi$, $^{225}Ac$, and $^{227}Th$. In some embodiments, the radionuclide is selected from $^{90}Y$, $^{177}Lu$, $^{212}Pb$, $^{225}Ac$, and $^{227}Th$. It will, however, also be acknowledged by a person skilled in the art that the use of said radionuclide is not limited to therapeutic purposes, but encompasses their use in diagnostic and theragnostics when conjugated to the compound of the disclosure.

In an embodiment, the compound of the disclosure is present as a pharmaceutically acceptable salt.

In certain embodiments, a "pharmaceutically acceptable salt" of a compound of the present disclosure is an acid salt or a base salt that is generally considered in the art to be suitable for use in contact with the tissues of human beings or animals without excessive toxicity or carcinogenicity, and, for example, without irritation, allergic response, or other problem or complication. Such salts include mineral and organic acid salts of basic residues, such as amines, as well as alkali or organic salts of acidic residues such as carboxylic acids. Compounds of the disclosure are capable of forming internal salts, which are also pharmaceutically acceptable salts.

Suitable pharmaceutically acceptable salts include, but are not limited to, salts of acids, such as hydrochloric, phosphoric, hydrobromic, malic, glycolic, fumaric, sulfuric, sulfamic, sulfanilic, formic, toluenesulfonic, methanesulfonic, benzene sulfonic, ethane disulfonic, 2-hydroxyethylsulfonic, nitric, benzoic, 2-acetoxybenzoic, citric, tartaric, lactic, stearic, salicylic, glutamic, ascorbic, pamoic, succinic, fumaric, maleic, propionic, hydroxymaleic, hydroiodic, phenylacetic, alkanoic such as acetic, HOOC-$(CH_2)_n$-COOH where n is any integer from 0 to 4, *i.e.,* 0, 1, 2, 3, or 4, and the like. Similarly, pharmaceutically acceptable cations include, but are not limited to sodium, potassium, calcium, aluminum, lithium and ammonium. Those of ordinary skill in the art will recognize further pharmaceutically acceptable salts for the compounds provided herein. In general, a pharmaceutically acceptable acid or base salt can be synthesized from a parent compound that contains a basic or acidic moiety by any conventional chemical method. Briefly, such salts can be prepared by reacting the free acid or base forms of these compounds with a stoichiometric amount of the appropriate base or acid in water or in an organic solvent, or in a mixture of the two. Generally, the use of non-aqueous media, such as ether, ethyl acetate, ethanol, isopropanol or acetonitrile, is preferred.

In certain embodiments, a "pharmaceutically acceptable solvate" of a compound of the disclosure is a solvate of the compound of the disclosure formed by association of one or more solvent molecules to one or more molecules of a compound of the disclosure. In some embodiments, the solvent is one which is generally considered in the art to be suitable for use in contact with the tissues of human beings or animals without excessive toxicity or carcinogenicity, and for example, without irritation, allergic response, or other problem or complication. Such solvent includes an organic solvent, such as alcohols, ethers, esters and amines.

In certain embodiments, a "hydrate" of a compound of the disclosure is formed by association of one or more water molecules to one or more molecules of a compound of the disclosure. Such hydrates include, but are not limited to, a hemi-hydrate, mono-hydrate, dihydrate, trihydrate and tetrahydrate. Independent of the hydrate composition, all hydrates are generally considered as pharmaceutically acceptable.

The compound of the disclosure has a high binding affinity to FAP and a high inhibitory activity on FAP. Because of this high binding affinity, the compound of the disclosure is effective as, useful as, and/or suitable as a targeting agent and, if conjugated to another moiety, as a targeting moiety, where the target is FAP and/or a cell and/or tissue expressing FAP. In terms of cells and tissues thus targeted by the compound of the disclosure any cell and tissue, respectively, expressing FAP is or may be targeted.

In an embodiment, the compound interacts with a fibroblast activation protein (FAP), preferably with human FAP having an amino acid sequence of SEQ ID NO: 1 or a homolog thereof, wherein the amino acid sequence of the homolog has an identity of FAP that is at least 85% to the amino acid sequence of SEQ ID NO: 1. In preferred embodiments, the identity is 90%, preferably 95 %, 96 %, 97 %, 98 % or 99%.

The identity between two nucleic acid molecules can be determined as known to the person skilled in the art. More specifically, a sequence comparison algorithm may be used for calculating the percent sequence homology for the test sequence(s) relative to the reference sequence, based on the designated program parameters. The test sequence is preferably the sequence or protein or polypeptide which is said to be identical or to be tested whether it is identical, and if so, to what extent, to a different protein or polypeptide, whereby such different protein or polypepetide is also referred to as the reference sequence and is preferably the protein or polypeptide of wild type, more preferably the human FAP of SEQ ID NO: 1.

Optimal alignment of sequences for comparison can be conducted, e.g., by the local homology algorithm of Smith & Waterman (Smith, et al., Advances in Applied Mathematics, 1981, 2: 482), by the homology alignment algorithm of Needleman & Wunsch (Needleman, et al., JMol Biol, 1970, 48: 443), by the search for similarity method of Pearson & Lipman (Pearson, et al., Proc Natl Acad Sci USA, 1988, 85: 2444), by computerized implementations of these algorithms (GAP, BESTFIT, FASTA, and TFASTA in the Wisconsin Genetics Software Package, Genetics Computer Group, 575 Science Dr., Madison, Wis.), or by visual inspection.

One example of an algorithm that is suitable for determining percent sequence identity is the algorithm used in the basic local alignment search tool (hereinafter "BLAST "), see, e.g. Altschul et al., 1990 (Altschul, et al., J Mol Biol, 1990, 215: 403) and Altschul et al., 1997 (Altschul, et al., Nucleic Acids Res, 1997, 25: 3389). Software for performing BLAST analyses is publicly available through the National Center for Biotechnology Information (hereinafter "NCBI"). The default parameters used in determining sequence identity using the software available from NCBI, e.g., BLASTN (for nucleotide sequences) and BLASTP (for amino acid sequences) are described in McGinnis et al. (McGinnis, et al., Nucleic Acids Res, 2004, 32: W20).

It is within the present disclosure that the compound of the disclosure is used or is for use in a method for the treatment of a disease as disclosed herein. In certain embodiments, such a method for the treatment of a disease as disclosed herein comprises the step of administering to a subject in need thereof a therapeutically effective amount of the compound of the disclosure. Such a method includes, but is not limited to, curative or adjuvant cancer treatment. It is used as palliative treatment where cure is not possible, and the aim is for local disease control or symptomatic relief or as therapeutic treatment where the therapy has survival benefit and it can be curative.

The method for the treatment of a disease as disclosed herein includes the treatment of the diseases disclosed herein, including tumors and cancer, and may be used either as the primary therapy or as second, third, fourth, or last line therapy. It is also within the present disclosure to combine the compound of the disclosure with further therapeutic approaches. It is well known to the person skilled in the art that the precise treatment intent including curative, adjuvant, neoadjuvant, therapeutic, or palliative treatment intent will depend on the tumor type, location, and stage, as well as the general health of the patient.

FAP expression in CAFs was shown for almost all carcinomas and sarcomas (Pure, et al., Oncogene, 2018, 37: 4343; Busek, et al., Front Biosci (Landmark Ed), 2018, 23: 1933). In an embodiment of the present disclosure, the disease is selected from the group comprising neoplasm nos, neoplasm benign, neoplasm uncertain whether benign or malignant, neoplasm malignant, neoplasm metastatic, neoplasm malignant uncertain whether primary or metastatic, tumor cells benign, tumor cells uncertain whether benign or malignant, tumor cells malignant, malignant tumor small cell type, malignant tumor giant cell type, malignant tumor fusiform cell type, epithelial neoplasms nos, epithelial tumor benign, carcinoma in situ nos, carcinoma nos, carcinoma metastatic nos, carcinomatosis, epithelioma benign, epithelioma malignant, large cell carcinoma nos, carcinoma undifferentiated type nos, carcinoma anaplastic type nos, pleomorphic carcinoma, giant cell and spindle cell carcinoma, giant cell carcinoma, spindle cell carcinoma, pseudosarcomatous carcinoma, polygonal cell carcinoma, spheroidal cell carcinoma, tumorlet, small cell carcinoma nos, oat cell carcinoma, small cell carcinoma, fusiform cell type, papillary and squamous cell neoplasms, papilloma nos, papillary carcinoma in situ, papillary carcinoma nos, verrucous papilloma, verrucous carcinoma nos, squamous cell papilloma, papillary squamous cell carcinoma, inverted papilloma, papillomatosis nos, squamous cell carcinoma in situ nos, squamous cell

carcinoma nos, squamous cell carcinoma metastatic nos, squamous cell carcinoma, keratinizing type nos, squamous cell carcinoma large cell nonkeratinizing type, squamous cell carcinoma small cell nonkeratinizing type, squamous cell carcinoma spindle cell type, adenoid squamous cell carcinoma, squamous cell carcinoma in situ with questionable stromal invasion, squamous cell carcinoma microinvasive, queyrat's erythroplasia, bowen's disease, lymphoepithelial carcinoma, basal cell neoplasms, basal cell tumor, basal cell carcinoma nos, multicentric basal cell carcinoma, basal cell carcinoma, morphea type, basal cell carcinoma fibroepithelial type, basosquamous carcinoma, metatypical carcinoma, intraepidermal epithelioma of jadassohn, trichoepithelioma, trichofolliculoma, tricholemmoma, pilomatrixoma, transitional cell papillomas and carcinomas, transitional cell papilloma nos, urothelial papilloma, transitional cell carcinoma in situ, transitional cell carcinoma nos, schneiderian papilloma, transitional cell papilloma, inverted type, schneiderian carcinoma, transitional cell carcinoma spindle cell type, basaloid carcinoma, cloacogenic carcinoma, papillary transitional cell carcinoma, adenomas and adenocarcinomas, adenoma nos, bronchial adenoma nos, adenocarcinoma in situ, adenocarcinoma nos, adenocarcinoma metastatic nos, scirrhous adenocarcinoma, linitis plastica, superficial spreading adenocarcinoma, adenocarcinoma intestinal type, carcinoma diffuse type, monomorphic adenoma, basal cell adenoma, islet cell adenoma, islet cell carcinoma, insulinoma nos, insulinoma malignant, glucagonoma nos, glucagonoma malignant, gastrinoma nos, gastrinoma malignant, mixed islet cell and exocrine adenocarcinoma, bile duct adenoma, cholangiocarcinoma, bile duct cystadenoma, bile duct cystadenocarcinoma, liver cell adenoma, hepatocellular carcinoma nos, hepatocholangioma benign, combined hepatocellular carcinoma and cholangiocarcinoma, trabecular adenoma, trabecular adenocarcinoma, embryonal adenoma, eccrine dermal cylindroma, adenoid cystic carcinoma, cribriform carcinoma, adenomatous polyp nos, adenocarcinoma in adenomatous polyp, tubular adenoma nos, tubular adenocarcinoma, adenomatous polyposis coli, adenocarcinoma in adenomatous polyposis coli, multiple adenomatous polyps, solid carcinoma nos, carcinoma simplex, carcinoid tumor nos, carcinoid tumor malignant, carcinoid tumor argentaffin nos, carcinoid tumor argentaffin malignant, carcinoid tumor nonargentaffin nos, carcinoid tumor nonargentaffin malignant, mucocarcinoid tumor malignant, composite carcinoid, pulmonary adenomatosis, bronchiolo-alveolar adenocarcinoma, alveolar adenoma, alveolar adenocarcinoma, papillary adenoma nos, papillary adenocarcinoma nos, villous adenoma nos, adenocarcinoma in villous adenoma, villous adenocarcinoma, tubulovillous adenoma, chromophobe adenoma, chromophobe carcinoma, acidophil adenoma, acidophil carcinoma, mixed acidophil-basophil adenoma, mixed acidophil-basophil carcinoma, oxyphilic adenoma, oxyphilic adenocarcinoma, basophil adenoma, basophil carcinoma, clear cell adenoma, clear cell adenocarcinoma nos, hypernephroid tumor, renal cell carcinoma, clear cell adenofibroma, granular cell carcinoma, chief cell adenoma, water-clear cell adenoma, water-clear cell adenocarcinoma, mixed cell adenoma, mixed cell adenocarcinoma, lipoadenoma, follicular adenoma, follicular adenocarcinoma nos, follicular adenocarcinoma well differentiated type, follicular adenocarcinoma trabecular type, microfollicular adenoma, macrofollicular adenoma, papillary and follicular adenocarcinoma, nonencapsulated sclerosing carcinoma, multiple endocrine adenomas, juxtaglomerular tumor, adrenal cortical adenoma nos, adrenal cortical carcinoma, adrenal cortical adenoma compact cell type, adrenal cortical adenoma heavily pigmented variant, adrenal cortical adenoma clear cell type, adrenal cortical adenoma glomerulosa cell type, adrenal cortical adenoma mixed cell type, endometrioid adenoma nos, endometrioid adenoma, borderline malignancy, endometrioid carcinoma, endometrioid adenofibroma nos, endometrioid adenofibroma borderline malignancy, endometrioid adenofibroma malignant, adnexal and skin appendage neoplasms, skin appendage adenoma, skin appendage carcinoma, sweat gland adenoma, sweat gland tumor nos, sweat gland adenocarcinoma, apocrine adenoma, apocrine adenocarcinoma, eccrine acrospiroma, eccrine spiradenoma, hidrocystoma, papillary hydradenoma, papillary syringadenoma, syringoma nos, sebaceous adenoma, sebaceous adenocarcinoma, ceruminous adenoma, ceruminous adenocarcinoma, mucoepidermoid neoplasms, mucoepidermoid tumor, mucoepidermoid carcinoma cystic, mucinous, and serous neoplasms, cystadenoma nos, cystadenocarcinoma nos, serous cystadenoma nos, serous cystadenoma borderline malignancy, serous cystadenocarcinoma nos, papillary cystadenoma nos, papillary cystadenoma borderline malignancy, papillary cystadenocarcinoma nos, papillary serous cystadenoma nos, papillary serous cystadenoma borderline malignancy, papillary serous cystadenocarcinoma, serous surface papilloma nos, serous surface papilloma borderline malignancy, serous surface papillary carcinoma, mucinous cystadenoma nos, mucinous cystadenoma borderline malignancy, mucinous cystadenocarcinoma nos, papillary mucinous cystadenoma nos, papillary mucinous cystadenoma borderline malignancy, papillary mucinous cystadenocarcinoma, mucinous adenoma, mucinous adenocarcinoma, pseudomyxoma peritonei, mucin-producing adenocarcinoma, signet ring cell carcinoma, metastatic signet ring cell carcinoma, ductal, lobular, and medullary neoplasms, intraductal carcinoma noninfiltrating nos, infiltrating duct carcinoma, comedocarcinoma, noninfiltrating, comedocarcinoma nos, juvenile carcinoma of the breast, intraductal papilloma, noninfiltrating intraductal papillary adenocarcinoma, intracystic papillary adenoma, noninfiltrating intracystic carcinoma, intraductal papillomatosis nos, subareolar duct papillomatosis, medullary carcinoma nos, medullary carcinoma with amyloid stroma, medullary carcinoma with lymphoid stroma, lobular carcinoma in situ, lobular carcinoma nos, infiltrating ductular carcinoma, inflammatory carcinoma, paget's disease mammary, paget's disease and infiltrating duct carcinoma of breast, paget's disease extramammary, acinar cell neoplasms, acinar cell adenoma, acinar cell tumor, acinar cell carcinoma, complex epithelial neoplasms, adenosquamous carcinoma, adenolymphoma, adenocarcinoma with squamous metaplasia, adenocarcinoma with cartilaginous and osseous metaplasia, adenocarcinoma with spindle

cell metaplasia, adenocarcinoma with apocrine metaplasia, thymoma benign, thymoma malignant, specialized gonadal neoplasms, sex cord-stromal tumor, thecoma nos, theca cell carcinoma, luteoma nos, granulosa cell tumor nos, granulosa cell tumor malignant, granulosa cell-theca cell tumor, androblastoma benign, androblastoma nos, androblastoma malignant, sertoli-leydig cell tumor, gynandroblastoma, tubular androblastoma nos, sertoli cell carcinoma, tubular androblastoma with lipid storage, leydig cell tumor benign, leydig cell tumor nos, leydig cell tumor malignant, hilar cell tumor, lipid cell tumor of ovary, adrenal rest tumor, paragangliomas and glomus tumors, paraganglioma nos, paraganglioma malignant, sympathetic paraganglioma, parasympathetic paraganglioma, glomus jugulare tumor, aortic body tumor, carotid body tumor, extra-adrenal paraganglioma nos, extra-adrenal paraganglioma, malignant, pheochromocytoma nos, pheochromocytoma malignant, glomangiosarcoma, glomus tumor, glomangioma, nevi and melanomas, pigmented nevus nos, malignant melanoma nos, nodular melanoma, balloon cell nevus, balloon cell melanoma, halo nevus, fibrous papule of the nose, neuronevus, magnocellular nevus, nonpigmented nevus, amelanotic melanoma, junctional nevus, malignant melanoma in junctional nevus, precancerous melanosis nos, malignant melanoma in precancerous melanosis, hutchinson's melanotic freckle, malignant melanoma in hutchinson's melanotic freckle, superficial spreading melanoma, intradermal nevus, compound nevus, giant pigmented nevus, malignant melanoma in giant pigmented nevus, epithelioid and spindle cell nevus, epithelioid cell melanoma, spindle cell melanoma nos, spindle cell melanoma type a, spindle cell melanoma type b, mixed epithelioid and spindle cell melanoma, blue nevus nos, blue nevus malignant, cellular blue nevus, soft tissue tumors and sarcomas nos, soft tissue tumor, benign, sarcoma nos, sarcomatosis nos, spindle cell sarcoma, giant cell sarcoma, small cell sarcoma, epithelioid cell sarcoma, fibromatous neoplasms, fibroma nos, fibrosarcoma nos, fibromyxoma, fibromyxosarcoma, periosteal fibroma, periosteal fibrosarcoma, fascial fibroma, fascial fibrosarcoma, infantile fibrosarcoma, elastofibroma, aggressive fibromatosis, abdominal fibromatosis, desmoplastic fibroma, fibrous histiocytoma nos, atypical fibrous histiocytoma, fibrous histiocytoma malignant, fibroxanthoma nos, atypical fibroxanthoma, fibroxanthoma malignant, dermatofibroma nos, dermatofibroma protuberans, dermatofibrosarcoma nos, myxomatous neoplasms, myxoma nos, myxosarcoma, lipomatous neoplasms, lipoma nos, liposarcoma nos, fibrolipoma, liposarcoma well differentiated type, fibromyxolipoma, myxoid liposarcoma, round cell liposarcoma, pleomorphic liposarcoma, mixed type liposarcoma, intramuscular lipoma, spindle cell lipoma, angiomyolipoma, angiomyoliposarcoma, angiolipoma nos, angiolipoma infiltrating, myelolipoma, hibernoma, lipoblastomatosis, myomatous neoplasms, leiomyoma nos, intravascular leiomyomatosis, leiomyosarcoma nos, epithelioid leiomyoma, epithelioid leiomyosarcoma, cellular leiomyoma, bizarre leiomyoma, angiomyoma, angiomyosarcoma, myoma, myosarcoma, rhabdomyoma nos, rhabdomyosarcoma nos, pleomorphic rhabdomyosarcoma, mixed type rhabdomyosarcoma, fetal rhabdomyoma, adult rhabdomyoma, embryonal rhabdomyosarcoma, alveolar rhabdomyosarcoma, complex mixed and stromal neoplasms, endometrial stromal sarcoma, endolymphatic stromal myosis, adenomyoma, pleomorphic adenoma, mixed tumor, malignant nos, mullerian mixed tumor, mesodermal mixed tumor, mesoblastic nephroma, nephroblastoma nos, epithelial nephroblastoma, mesenchymal nephroblastoma, hepatoblastoma, carcinosarcoma nos, carcinosarcoma embryonal type, myoepithelioma, mesenchymoma benign, mesenchymoma nos, mesenchymoma malignant, embryonal sarcoma, fibroepithelial neoplasms, brenner tumor nos, brenner tumor, borderline malignancy, brenner tumor malignant, fibroadenoma nos, intracanalicular fibroadenoma nos, pericanalicular fibroadenoma, adenofibroma nos, serous adenofibroma, mucinous adenofibroma, cellular intracanalicular fibroadenoma, cystosarcoma phyllodes nos, cystosarcoma phyllodes malignant, juvenile fibroadenoma, synovial neoplasms, synovioma benign, synovial sarcoma nos, synovial sarcoma spindle cell type, synovial sarcoma, epithelioid cell type, synovial sarcoma, biphasic type, clear cell sarcoma of tendons and aponeuroses, mesothelial neoplasms, mesothelioma benign, mesothelioma malignant, fibrous mesothelioma benign, fibrous mesothelioma malignant, epithelioid mesothelioma benign, epithelioid mesothelioma malignant, mesothelioma biphasic type benign, mesothelioma biphasic type malignant, adenomatoid tumor nos, germ cell neoplasms, dysgerminoma, seminoma nos, seminoma anaplastic type, spermatocytic seminoma, germinoma, embryonal carcinoma nos, endodermal sinus tumor, polyembryoma, gonadoblastoma, teratoma benign, teratoma nos, teratoma malignant nos, teratocarcinoma, malignant teratoma, undifferentiated type, malignant teratoma, intermediate type, dermoid cyst, dermoid cyst with malignant transformation, struma ovarii nos, struma ovarii malignant, strumal carcinoid, trophoblastic neoplasms, hydatidiform mole nos, invasive hydatidiform mole, choriocarcinoma, choriocarcinoma combined with teratoma, malignant teratoma trophoblastic, mesonephromas, mesonephroma benign, mesonephric tumor, mesonephroma malignant, endosalpingioma, blood vessel tumors, hemangioma nos, hemangiosarcoma, cavernous hemangioma, venous hemangioma, racemose hemangioma, kupffer cell sarcoma, hemangioendothelioma benign, hemangioendothelioma nos, hemangioendothelioma malignant, capillary hemangioma, intramuscular hemangioma, kaposi's sarcoma, angiokeratoma, verrucous keratotic hemangioma, hemangiopericytoma benign, hemangiopericytoma nos, hemangiopericytoma malignant, angiofibroma nos, hemangioblastoma, lymphatic vessel tumors, lymphangioma nos, lymphangiosarcoma, capillary lymphangioma, cavernous lymphangioma, cystic lymphangioma, lymphangiomyoma, lymphangiomyomatosis, hemolymphangioma, osteomas and osteosarcomas, osteoma nos, osteosarcoma nos, chondroblastic osteosarcoma, fibroblastic osteosarcoma, telangiectatic osteosarcoma, osteosarcoma in paget's disease of bone, juxtacortical osteosarcoma, osteoid osteoma nos, osteoblastoma, chondromatous neoplasms, osteochondroma, osteochondromatosis nos, chondroma nos, chondromatosis nos, chondrosarcoma nos, juxtacortical chondroma, juxtacortical

chondrosarcoma, chondroblastoma nos, chondroblastoma malignant, mesenchymal chondrosarcoma, chondromyxoid fibroma, giant cell tumors, giant cell tumor of bone nos, giant cell tumor of bone malignant, giant cell tumor of soft parts nos, malignant giant cell tumor of soft parts, miscellaneous bone tumors, ewing's sarcoma, adamantinoma of long bones, ossifying fibroma, odontogenic tumors, odontogenic tumor benign, odontogenic tumor nos, odontogenic tumor malignant, dentinoma, cementoma nos, cementoblastoma benign, cementifying fibroma, gigantiform cementoma, odontoma nos, compound odontoma, complex odontoma, ameloblastic fibro-odontoma, ameloblastic odontosarcoma, adenomatoid odontogenic tumor, calcifying odontogenic cyst, ameloblastoma nos, ameloblastoma malignant, odontoameloblastoma, squamous odontogenic tumor, odontogenic myxoma, odontogenic fibroma nos, ameloblastic fibroma, ameloblastic fibrosarcoma, calcifying epithelial odontogenic tumor, miscellaneous tumors, craniopharyngioma, pinealoma, pineocytoma, pineoblastoma, melanotic neuroectodermal tumor, chordoma, gliomas, glioma malignant, gliomatosis cerebri, mixed glioma, subependymal glioma, subependymal giant cell astrocytoma, choroid plexus papilloma nos, choroid plexus papilloma malignant, ependymoma nos, ependymoma anaplastic type, papillary ependymoma, myxopapillary ependymoma, astrocytoma nos, astrocytoma, anaplastic type, protoplasmic astrocytoma, gemistocytic astrocytoma, fibrillary astrocytoma, pilocytic astrocytoma, spongioblastoma nos, spongioblastoma polare, astroblastoma, glioblastoma nos, giant cell glioblastoma, glioblastoma with sarcomatous component, primitive polar spongioblastoma, oligodendroglioma nos, oligodendroglioma, anaplastic type, oligodendroblastoma, medulloblastoma nos, desmoplastic medulloblastoma, medullomyoblastoma, cerebellar sarcoma nos, monstrocellular sarcoma, neuroepitheliomatous neoplasms, ganglioneuroma, ganglioneuroblastoma, ganglioneuromatosis, neuroblastoma nos, medulloepithelioma nos, teratoid medulloepithelioma, neuroepithelioma nos, spongioneuroblastoma, ganglioglioma, neurocytoma, pacinian tumor, retinoblastoma nos, retinoblastoma differentiated type, retinoblastoma undifferentiated type, olfactory neurogenic tumor, esthesioneurocytoma, esthesioneuroblastoma, esthesioneuroepithelioma, meningiomas, meningioma nos, meningiomatosis nos, meningioma malignant, meningotheliomatous meningioma, fibrous meningioma, psammomatous meningioma, angiomatous meningioma, hemangioblastic meningioma, hemangiopericytic meningioma, transitional meningioma, papillary meningioma, meningeal sarcomatosis, nerve sheath tumor, neurofibroma nos, neurofibromatosis nos, neurofibrosarcoma, melanotic neurofibroma, plexiform neurofibroma, neurilemmoma nos, neurinomatosis, neurilemmoma malignant, neuroma nos, granular cell tumors and alveolar soft part sarcoma, granular cell tumor nos, granular cell tumor, malignant, alveolar soft part sarcoma, lymphomas nos or diffuse, lymphomatous tumor benign, malignant lymphoma nos, malignant lymphoma non hodgkin's type, malignant lymphoma, undifferentiated cell type nos, malignant lymphoma stem cell type, malignant lymphoma convoluted cell type nos, lymphosarcoma nos, malignant lymphoma lymphoplasmacytoid type, malignant lymphoma immunoblastic type, malignant lymphoma mixed lymphocytic-histiocytic nos, malignant lymphoma centroblastic-centrocytic diffuse, malignant lymphoma follicular center cell nos, malignant lymphoma lymphocytic well differentiated nos, malignant lymphoma lymphocytic intermediate differentiation nos, malignant lymphoma centrocytic malignant lymphoma follicular center cell, cleaved nos, malignant lymphoma lymphocytic poorly differentiated nos, prolymphocytic lymphosarcoma, malignant lymphoma centroblastic type nos, malignant lymphoma follicular center cell noncleaved nos, reticulosarcomas, reticulosarcoma nos, reticulosarcoma pleomorphic cell type, reticulosarcoma nodular, hodgkin's disease, hodgkin's disease nos, hodgkin's disease lymphocytic predominance, hodgkin's disease mixed cellularity, hodgkin's disease lymphocytic depletion nos, hodgkin's disease lymphocytic depletion diffuse fibrosis, hodgkin's disease lymphocytic depletion reticular type, hodgkin's disease nodular sclerosis nos, hodgkin's disease nodular sclerosis cellular phase, hodgkin's paragranuloma, hodgkin's granuloma, hodgkin's sarcoma, lymphomas nodular or follicular, malignant lymphoma nodular nos, malignant lymphoma mixed lymphocytic-histiocytic nodular, malignant lymphoma centroblastic-centrocytic follicular, malignant lymphoma lymphocytic well differentiated nodular, malignant lymphoma lymphocytic intermediate differentiation nodular, malignant lymphoma follicular center cell cleaved follicular, malignant lymphoma lymphocytic poorly differentiated nodular, malignant lymphoma centroblastic type follicular malignant lymphoma follicular center cell noncleaved follicular, mycosis fungoides, mycosis fungoides, sezary's disease, miscellaneous reticuloendothelial neoplasms, microglioma, malignant histiocytosis, histiocytic medullary reticulosis, letterer-siwe's disease, plasma cell tumors, plasma cell myeloma, plasma cell tumor, benign, plasmacytoma nos, plasma cell tumor malignant, mast cell tumors, mastocytoma nos, mast cell sarcoma, malignant mastocytosis, burkitt's tumor, burkitt's tumor, leukemias, leukemias nos, leukemia nos, acute leukemia nos, subacute leukemia nos, chronic leukemia nos, aleukemic leukemia nos, compound leukemias, compound leukemia, lymphoid leukemias, lymphoid leukemia nos, acute lymphoid leukemia, subacute lymphoid leukemia, chronic lymphoid leukemia, aleukemic lymphoid leukemia, prolymphocytic leukemia, plasma cell leukemias, plasma cell leukemia, erythroleukemias, erythroleukemia, acute erythremia, chronic erythremia, lymphosarcoma cell leukemias, lymphosarcoma cell leukemia, myeloid leukemias, myeloid leukemia nos, acute myeloid leukemia, subacute myeloid leukemia, chronic myeloid leukemia, aleukemic myeloid leukemia, neutrophilic leukemia, acute promyelocytic leukemia, basophilic leukemias, basophilic leukemia, eosinophilic leukemias, eosinophilic leukemia, monocytic leukemias, monocytic leukemia nos, acute monocytic leukemia, subacute monocytic leukemia, chronic monocytic leukemia, aleukemic monocytic leukemia, miscellaneous leukemias, mast cell leukemia, megakaryocytic leukemia, megakaryocytic myelosis, myeloid sarcoma, hairy cell leukemia, miscellaneous myeloproliferative and lymphoproliferative disorders, polycythemia vera, acute panmyelosis, chronic myeloproliferative disease,

myelosclerosis with myeloid metaplasia, idiopathic thrombocythemia, chronic lymphoproliferative disease.

In an embodiment of the present invention, the disease is selected from the group comprising tumors of pancreas, pancreatic adenocarcinoma, tumors of head of pancreas, of body of pancreas, of tail of pancreas, of pancreatic duct, of islets of langerhans, neck of pancreas, tumor of prostate, prostate adenocarcinoma, prostate gland, neuroendocrine tumors, breast cancer, tumor of central portion of breast, upper inner quadrant of breast, lower inner quadrant of breast, upper outer quadrant of breast, lower outer quadrant of breast, axillary tail of breast, overlapping lesion of breast, juvenile carcinoma of the breast, tumors of parathyroid gland, myeloma, lung cancer, small cell lung cancer, non-small cell lung cancer, tumor of main bronchus, of upper lobe lung, of middle lobe lung, of lower lobe lung, colorectal carcinoma, tumor of ascending colon, of hepatic flexure of colon, of transverse colon, of splenic flexure of colon, of descending colon, of sigmoid colon, of overlapping lesion of colon, of small intestine, tumors of liver, liver cell adenoma, hepatocellular carcinoma, hepatocholangioma, ombined hepatocellular carcinoma and cholangiocarcinoma, hepatoblastoma, ovarian carcinoma,sarcoma, osteosarcoma, fibrosarcoma, gastrointestinal stroma tumors, gastrointestinal tract, gastric carcinoma, thyroid carcinoma, medullary thyroid carcinoma, thyroid gland, renal cell carcinoma, renal pelvis, tumors of bladder, bladder carcinoma, tumors of trigone bladder, of dome bladder, of lateral wall bladder, of posterior wall bladder, of ureteric orifice, of urachus, overlapping lesion of bladder, basal cell carcinoma, basal cell neoplasms, basal cell tumor, basal cell carcinoma, multicentric basal cell carcinoma, basaloid carcinoma, basal cell adenoma, squamous cell carcinoma, oral squamous cell carcinoma, *squamous cell carcinoma* of the *larynx,* cervical carcinoma, tumors of exocervix, of overlapping lesion of cervix uteri, of cervix uteri, of isthmus uteri, tumors of uterus, tumors of ovary, tumors of cervical esophagus, of thoracic esophagus, of abdominal esophagus, of upper third of esophagus, of esophagus middle third, of esophagus lower third, of overlapping lesion of esophagus, endometrial carcinoma, head and neck cancer, lymphoma, malignant mesothelioma, mesothelial neoplasms, mesothelioma, fibrous mesothelioma, fibrous mesothelioma, epithelioid mesothelioma, epithelioid mesothelioma, duodenal carcinoma, neuroendocrine tumors, neuroendocrine tumors of the lung, neuroendocrine tumors of the pancreas, neuroendocrine tumors of the foregut, neuroendocrine tumors of the midgut, neuroendocrine tumors of the hindgut, gastroenteropancreatic neuroendocrine tumors, neuroendocrine carcinomas, neuroendocrine tumors of the breast, neuroendocrine tumors o the ovaries, testicular cancer, thymic carcinoma, tumors of stomach, fundus stomach, body stomach, gastric antrum, pylorus, lesser curvature of stomach, greater curvature of stomach, overlapping lesion of stomach, paragangliomas, ganglioma, melanomas, malignant melanoma, nodular melanoma, amelanotic melanoma, superficial spreading melanoma, epithelioid cell melanoma, spindle cell melanoma, mixed epithelioid and spindle cell melanoma.

In a still further embodiment, the aforementioned indications may occur in organs and tissues selected from the group comprising external upper lip, external lower lip, external lip nos, upper lip mucosa, lower lip mucosa, mucosa lip nos, commissure lip, overlapping lesion of lip, base of tongue nos, dorsal surface tongue nos, border of tongue, ventral surface of tongue nos, anterior 2/3 of tongue nos, lingual tonsil, overlapping lesion of tongue, tongue nos, upper gum, lower gum, gum nos, anterior floor of mouth, lateral floor of mouth, overlapping lesion of floor of mouth, floor of mouth nos, hard palate, soft palate nos, uvula, overlapping lesion of palate, palate nos, cheek mucosa, vestibule of mouth, retromolar area, overlapping lesion of other and unspecified parts of mouth, mouth nos, parotid gland, submaxillary gland, sublingual gland, overlapping lesion of major salivary glands, major salivary gland nos, tonsillar fossa, tonsillar pillar, overlapping lesion of tonsil, tonsil nos, vallecula, anterior surface of epiglottis, lateral wall oropharynx, posterior wall oropharynx, branchial cleft, overlapping lesion of oropharynx, oropharynx nos, superior wall of nasopharynx, posterior wall nasopharynx, lateral wall nasopharynx, anterior wall nasopharynx, overlapping lesion of nasopharynx, nasopharynx nos, pyriform sinus, postcricoid region, hypopharyngeal aspect of aryepiglottic fold, posterior wall hypopharynx, overlapping lesion of hypopharynx, hypopharynx nos, pharynx nos, laryngopharynx, waldeyer’s ring, overlapping lesion of lip oral cavity and pharynx, cervical esophagus, thoracic esophagus, abdominal esophagus, upper third of esophagus, middle third of esophagus, esophagus lower third, overlapping lesion of esophagus, esophagus nos, cardia nos, fundus stomach, body stomach, gastric antrum, pylorus, lesser curvature of stomach nos, greater curvature of stomach nos, overlapping lesion of stomach, stomach nos, duodenum, jejunum, ileum, meckel’s diverticulum, overlapping lesion of small intestine, small intestine nos, cecum, appendix, ascending colon, hepatic flexure of colon, transverse colon, splenic flexure of colon, descending colon, sigmoid colon, overlapping lesion of colon, colon nos, rectosigmoid junction, rectum nos, anus nos, anal canal, cloacogenic zone, overlapping lesion of rectum anus and anal canal, liver, intrahepatic bile duct, gallbladder, extrahepatic bile duct, ampulla of vater, overlapping lesion of biliary tract, biliary tract nos, head of pancreas, body pancreas, tail pancreas, pancreatic duct, islets of langerhans, neck of pancreas, overlapping lesion of pancreas, pancreas nos, intestinal tract nos, overlapping lesion of digestive system, gastrointestinal tract nos, nasal cavity, middle ear, maxillary sinus, ethmoid sinus, frontal sinus, sphenoid sinus, overlapping lesion of accessory sinuses, accessory sinus nos, glottis, supraglottis, subglottis, laryngeal cartilage, overlapping lesion of larynx, larynx nos, trachea, main bronchus, upper lobe lung, middle lobe lung, lower lobe lung, overlapping lesion of lung, lung nos, thymus, heart, anterior mediastinum, posterior mediastinum, mediastinum nos, pleura nos, overlapping lesion of heart mediastinum and pleura, upper respiratory tract nos, overlapping lesion of respiratory system and intrathoracic organs, respiratory tract nos, upper limb long bones joints, upper limb short bones joints, lower limb long bones joints, lower limb short

bones joints, overlapping lesion of bones joints and articular cartilage of limbs, bone limb nos, skull and facial bone, mandible, vertebral column, rib sternum clavicle, pelvic bone, overlapping lesion of bones joints and articular cartilage, bone nos, blood, bone marrow, spleen, reticuloendothelial system nos, hematopoietic system nos, skin lip nos, eyelid nos, external ear, skin face, skin scalp neck, skin trunk, skin limb upper, skin limb lower, peripheral nerve head neck, peripheral nerve shoulder arm, peripheral nerve leg, peripheral nerve thorax, peripheral nerve abdomen, peripheral nerve pelvis, peripheral nerve trunk, overlapping lesion of peripheral nerves and autonomic nervous system, autonomic nervous system nos, retroperitoneum, peritoneum, peritoneum nos, overlapping lesion of retroperitoneum and peritoneum, connective tissue head, connective tissue arm, connective tissue leg, connective tissue thorax, connective tissue abdomen, connective tissue pelvis, connective tissue trunk nos, overlapping lesion of connective subcutaneous and other soft tissues, connective tissue nos, nipple, central portion of breast, upper inner quadrant of breast, lower inner quadrant of breast, upper outer quadrant of breast, lower outer quadrant of breast, axillary tail of breast, overlapping lesion of breast, breast nos, labium majus, labium minus, clitoris, overlapping lesion of vulva, vulva nos, vagina nos, endocervix, exocervix, overlapping lesion of cervix uteri, cervix uteri, isthmus uteri, endometrium, myometrium, fundus uteri, overlapping lesion of corpus uteri, corpus uteri, uterus nos, ovary, fallopian tube, broad ligament, round ligament, parametrium, uterine adnexa, wolffian body, overlapping lesion of female genital organs, female genital tract nos, prepuce, glans penis, body penis, overlapping lesion of penis, penis nos, prostate gland, undescended testis, descended testis, testis nos, epididymis, spermatic cord, scrotum nos, tunica vaginalis, overlapping lesion of male genital organs, male genital organs nos, kidney nos, renal pelvis, ureter, trigone bladder, dome bladder, lateral wall bladder, posterior wall bladder, ureteric orifice, urachus, overlapping lesion of bladder, bladder nos, urethra, paraurethral gland, overlapping lesion of urinary organs, urinary system nos, conjunctiva, cornea nos, retina, choroid, ciliary body, lacrimal gland, orbit nos, overlapping lesion of eye and adnexa, eye nos, cerebral meninges, spinal meninges, meninges nos, cerebrum, frontal lobe, temporal lobe, parietal lobe, occipital lobe, ventricle nos, cerebellum nos, brain stem, overlapping lesion of brain, brain nos, spinal cord, cauda equina, olfactory nerve, optic nerve, acoustic nerve, cranial nerve nos, overlapping lesion of brain and central nervous system, nervous system nos, thyroid gland, adrenal gland cortex, adrenal gland medulla, adrenal gland nos, parathyroid gland, pituitary gland, craniopharyngeal duct, pineal gland, carotid body, aortic body, overlapping lesion of endocrine glands and related structures, endocrine gland nos, head face or neck nos, thorax nos, abdomen nos, pelvis nos, upper limb nos, lower limb nos, other illdefined sites, overlapping lesion of illdefined sites, lymph node face head neck, intrathoracic lymph node, intra-abdominal lymph nodes, lymph node axilla arm, lymph node inguinal region leg, lymph node pelvic, lymph nodes of multiple regions, lymph node nos, unknown primary site.

In some embodiments, the disease is a neoplasm, preferably a cancer or tumor. In some embodiments, the neoplasm, cancer and tumor are selected from the group comprising a solid tumor, an epithelial tumor, bladder cancer, breast cancer, cervical cancer, colorectal cancer, cholangiocarcinoma, endometrial cancer, esophageal cancer, gastric cancer, gastrointestinal stromal tumors, head and neck cancer, liver cancer, lung cancer, melanoma, mesothelioma, neuroendocrine tumors and carcinomas, ovarian cancer, pancreatic cancer, prostate cancer, renal cell carcinoma, salivary carcinoma, sarcoma, squamous cell carcinoma, and thyroid cancer, and combinations thereof.

In some embodiments, the neoplasm, cancer and tumor are individually selected from the group comprising breast cancer, colorectal cancer, cholangiocarcinoma, head and neck cancer, lung cancer, mesothelioma, neuroendocrine tumors and carcinomas, ovarian cancer, pancreatic cancer, prostate cancer, sarcoma, and squamous cell carcinoma, and combinations thereof.

In some embodiments, the disease is a non-oncology disease. In some embodiments, the disease is selected from the group comprising: inflammatory disease, cardiovascular disease, autoimmune disease, and fibrotic disease. In some embodiments, the disease is selected from the group consisting of atherosclerosis, arthritis, rheumatoid arthritis, cardiovascular disease involving atherosclerotic plaques, atherosclerotic pathology caused by rupture of plaques, acute coronary syndrome, myocardial infarction, thrombosis, or vessel occlusion, idiopathic pulmonary fibrosis, Crohn's disease, and liver fibrosis.

In some embodiments, the subjects treated with the presently disclosed compounds may be treated in combination with other non-surgical anti-proliferative (e.g., anti-cancer) drug therapy. In some embodiments, the compounds may be administered in combination with an anti-cancer compound such as a cytostatic compound. A cytostatic compound is a compound (e.g., a small molecule, a nucleic acid, or a protein) that suppresses cell growth and/or proliferation. In some embodiments, the cytostatic compound is directed towards the malignant cells of a tumor. In some embodiments, the cytostatic compound is one which inhibits the growth and/or proliferation of vascular smooth muscle cells or fibroblasts.

In some embodiments, the herein-described compounds are used or are for use in combination with a chemotherapeutic agent, e.g., a DNA damaging chemotherapeutic agent. Non-limiting examples of DNA damaging chemotherapeutic agents include topoisomerase I inhibitors, topoisomerase II inhibitors; alkylating agents; DNA intercalators; DNA intercalators and free radical generators such as bleomycin; and nucleoside mimetics.

In some embodiments, a compound described herein can be administered alone or in combination with one or more additional therapeutic agents. For example, the combination therapy can include a composition comprising a conjugate described herein co-formulated with, and/or coadministered with, one or more additional therapeutic agents,

e.g., one or more anti-cancer agents, e.g., cytotoxic or cytostatic agents, immune checkpoint inhibitors, hormone treatment, vaccines, and/or immunotherapies. In some embodiments, the conjugate is administered in combination with other therapeutic treatment modalities, including surgery, cryosurgery, and/or chemotherapy. Such combination therapies may advantageously utilize lower dosages of the administered therapeutic agents, thus avoiding possible toxicities or complications associated with the various monotherapies.

Suitable anti-proliferative drugs or cytostatic compounds to be used in combination with the presently disclosed compounds include anti-cancer drugs. Numerous anti-cancer drugs which may be used are well known and include, but are not limited to: Acivicin; Aclarubicin; Acodazole Hydrochloride; Acronine; Adozelesin; Aldesleukin; Altretamine; Ambomycin; Ametantrone Acetate; Aminoglutethimide; Amsacrine; Anastrozole; Anthramycin; Asparaginase; Asperlin; Azacitidine; Azaribine; Azetepa; Azotomycin; Batimastat; Benzodepa; Bicalutamide; Bisantrene Hydrochloride; Bisnafide Dimesylate; Bizelesin; Bleomycin; Bleomycin Sulfate; Brequinar Sodium; Bropirimine; Bryostatin-1; Busulfan; Cactinomycin; Calusterone; Caracemide; Carbetimer; Carboplatin; Carmustine; Carubicin Hydrochloride; Carzelesin; Cedefingol; Celebrex; Chlorambucil; Cirolemycin; Cisplatin; Cladribine; Crisnatol Mesylate; Cyclophosphamide; Cytarabine; Dacarbazine; Dactinomycin; Daunorubicin; Daunorubicin Hydrochloride; Decitabine; Dexormaplatin; Dezaguanine; Dezaguanine Mesylate; Diaziquone; Docetaxel; Doxorubicin; Doxorubicin Hydrochloride; Doxorubicin Glucuronide; Cyanomorpholino Doxorubicin; 2-Pyrrolinodoxorubicin (2P-DOX), Droloxifene; Droloxifene Citrate; Dromostanolone Propionate; Duazomycin; Edatrexate; Eflornithine Hydrochloride; Elsamitrucin; Enloplatin; Enpromate; Epipropidine; Epirubicin Hydrochloride; Epirubicin Glucuronide; Erbulozole; Esorubicin Hydrochloride; Estramustine; Estramustine Phosphate Sodium; Etanidazole; Etoposide; Etoposide Phosphate; Etoposide Glucuronide; Etoprine; Fadrozole Hydrochloride; Fazarabine; Fenretinide; Floxuridine (FUdR); 3',5'-O-dioleoyl-FudR (FUdR-dO); Fludarabine; Fludarabine Phosphate; Fluorouracil; Fluorocitabine; Flutamide; Fluoxymesterone; Fosquidone; Fostriecin Sodium; Gemcitabine; Gemcitabine Hydrochloride; Hydroxyurea; Hydroxyprogesterone caproate; Idarubicin; Idarubicin Hydrochloride; Ifosfamide; Ilmofosine; Interferon Alfa-2a; Interferon Alfa-2b; Interferon Alfa-nl; Interferon Alfa-n3; Interferon Beta-I a; Interferon Gamma-I b; Iproplatin; Irinotecan Hydrochloride; L-asparaginase; Lanreotide Acetate; Letrozole; Leucovorin; Leuprolide Acetate; Liarozole Hydrochloride; Lometrexol Sodium; Lomustine; Losoxantrone Hydrochloride; Masoprocol; Maytansine; Mechlorethamine; Mechlorethamine Hydrochloride; Medroprogesterone acetate; Megestrol Acetate; Melengestrol Acetate; Melphalan; Menogaril; Mercaptopurine; 6- Mercaptopurine; Methotrexate; Methotrexate Sodium; Metoprine; Meturedepa; Mithramycin; Mitindomide; Mitocarcin; Mitocromin; Mitogillin; Mitomalcin; Mitomycin; Mitosper; Mitotane; Mitoxantrone; Mitoxantrone Hydrochloride; Mycophenolic Acid; Niraparib; Nocodazole; Nogalamycin; Olaparib; Ormaplatin; Oxisuran; Paclitaxel; Pegaspargase; Peliomycin; Pentamustine; Peplomycin Sulfate; Perfosfamide; Phenyl Butyrate, Pipobroman; Piposulfan; Piroxantrone Hydrochloride; Plicamycin; Plomestane; Porfimer Sodium; Porfiromycin; Prednimustine; Prednisone; Procarbazine; Procarbazine Hydrochloride; PSI-341, Puromycin; Puromycin Hydrochloride; Pyrazofurin; Riboprine; Rogletimide; Rucaparib; Safingol; Safingol Hydrochloride; Semustine; Semustine Streptozocin; Simtrazene; Sparfosate Sodium; Sparsomycin; Spirogermanium Hydrochloride; Spiromustine; Spiroplatin; Streptonigrin; Streptozocin; Sulofenur; Talazoparib; Talisomycin; Taxol; Taxotere; Tecogalan Sodium; Tegafur; Teloxantrone Hydrochloride; Temoporfin; Teniposide; Teroxirone; Testolactone; Thiamiprine; Thioguanine; Thiotepa; Tiazofurin; Tirapazamine; Topotecan; Topotecan Hydrochloride; Toremifene Citrate; Trestolone Acetate; Triciribine Phosphate; Trimetrexate; Trimetrexate Glucuronate; Tubulozole Hydrochloride; Uracil Mustard; Uredepa; Vapreotide; Velaparib; Velcade; Verteporfin; Vinblastine; Vinblastine Sulfate; Vincristine; Vincristine Sulfate; Vindesine; Vindesine Sulfate; Vinepidine Sulfate; Vinglycinate Sulfate; Vinleurosine Sulfate; Vinorelbine; Vinorelbine Tartrate; Vinrosidine Sulfate; Vinzolidine Sulfate; Vorozole; Zeniplatin; Zinostatin; and Zorubicin Hydrochloride.

Other anti-cancer drugs include, but are not limited to: 20-epi-1,25 dihydroxyvitamin D3; 5-ethynyluracil; abiraterone; acylfulvene; adecypenol; adozelesin; ALL-TK antagonists; ambamustine; amidox; amifostine; aminolevulinic acid; amrubicin; anagrelide; andrographolide; angiogenesis inhibitors; antagonist D; antagonist G; antarelix; anti-dorsalizing morphogenetic protein-1; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bisaziridinylspermine; bisnafide; bistratene A; bortezomib; breflate; budotitane; buthionine sulfoximine; calicheamicin; calcipotriol; calphostin C; camptothecin derivatives; canarypox IL-2; capecitabine; carboxamide-amino-triazole; carboxyamidotriazole; CaRest M3; CARN 700; cartilage derived inhibitor; casein kinase inhibitors (ICOS); castanospermine; cecropin B; cetrorelix; chlorins; chloroquinoxaline sulfonamide; cicaprost; cis-porphyrin; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; daunomycin glucuronide; daunorubicin; dehydrodidemnin B; diethylstilbestrol; deslorelin; dexamethasone; dexifosfamide; dexrazoxane; dexverapamil; didemnin B; didox; diethylnorspermine; azacytidine; dihydro-5-azacytidine; dihydrotaxol, 9-; dioxamycin; diphenyl spiromustine; docosanol; dolasetron; doxifluridine; dronabinol; duocarmycin SA; ebselen; ecomustine; edelfosine; edrecolomab; eflomithine; elemene; emitefur; epirubicin; epristeride; estramustine analogue; estrogen agonists; estrogen antagonists; etanidazole; ethinyl estradiol; etoposide phosphate; exemestane; filgrastim; finasteride; flavopiridol; flezelastine; fluasterone; fludarabine; fluorodaunorunicin hydrochloride; forfenimex; formestane; fotemustine; gadolinium texaphyrin; gallium nitrate; galocitabine; ganirelix; gelatinase inhibitors; glutathione inhibitors; hepsulfam; heregulin; hexamethylene bisacetamide; hypericin; ibandronic acid; idoxifene; idramantone; ilmofosine; ilomastat; imidazoacridones; imiquimod; immunostimulant peptides; insulin-like growth factor-I receptor inhibitor; interferon agonists; interferons; interleukins; iobenguane; iododoxorubicin; ipomeanol, 4-; irinotecan; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anti-cancer compound; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; 06-benzylguanine; octreotide; okicenone; oligonucleotides; onapristone; ondansetron; ondansetron; oracin; oral cytokine inducer; osaterone; oxaliplatin; oxaunomycin; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinumtriamine complex; porfimer sodium; porfiromycin; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rohitukine; romurtide; roquinimex; rubiginone B1; ruboxyl; saintopin; SarCNU; sarcophytol A; sargramostim; Sdi 1 mimetics; senescence derived inhibitor 1; sense oligonucleotides; signal transduction inhibitors; signal transduction modulators; single chain antigen binding protein; sizofuran; SN-38; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen; tamoxifen methiodide; tauromustine; taxanes; tazarotene; tecogalan sodium; tegafur; tellurapyrylium; telomerase inhibitors; temozolomide; testosterone proprionate, tetrachlorodecaoxide; tetrazomine; thaliblastine; thalidomide; thiocoraline; thrombopoietin; thrombopoietin mimetic; thymalfasin; thymopoietin receptor agonist; thymotrinan; thyroid stimulating hormone; tin ethyl etiopurpurin; titanocene dichloride; topsentin; toremifene; totipotent stem cell factor; translation inhibitors; tretinoin; triacetyluridine; triciribine; tropisetron; turosteride; tyrosine kinase inhibitors; tyrphostins; UBC inhibitors; ubenimex; urogenital sinus-derived growth inhibitory factor; urokinase receptor antagonists; variolin B; vector system, erythrocyte gene therapy; velaresol; veramine; verdins; vinorelbine; vinxaltine; vitaxin; zanoterone; zilascorb; zinostatin stimalamer; abrin; ricine; ribonuclease; onconase; rapLR1; DNase I; Staphylococcal enterotoxin-A; pokeweed antiviral protein; gelonin; diphtheria toxin; Pseudomonas exotoxin; and Pseudomona endotoxin; or combinations of these.

In some embodiments, the drug to be used in combination with the disclosed compounds is selected from duocarmycin and its analogues, dolastatins, combretastatin, calicheamicin, N-acetyl-γ-calicheamycin (CMC), a calicheamycin derivative, maytansine and analogues thereof, DM-I, auristatin E, auristatin EB (AEB), auristatin EFP (AEFP), monomethyl auristatin E (MMAE), monomethyl auristatin F (MMAF), tubulysin, disorazole, the epothilones, Paclitaxel, docetaxel, Topotecan, echinomycin, estramustine, cemadotine, eleutherobin, methopterin, actinomycin, daunorubicin, the daunorubicin conjugates, mitomycin C, mitomycin A, vincristine, retinoic acid, camptothecin, a camptothecin derivative, SN38, maytansine, a derivative of the maytansinoid type, DM1, DM4, TK1, amanitin, a pyrrolobenzodiazepine, a pyrrolobenzodiazepine dimer, methotrexate, ilomedine, aspirin, an IMIDs, lenalidomide, pomalidomide.

The presently disclosed compounds can also be used in combination with any of the following treatments:

Therapy in combination with compounds targeting the androgen receptor, including androgen depletion approaches and antiandrogens. Such inhibitors include but are not limited to enzalutamide, apalutamide, darolutamide, etc.

Therapy in combination with inhibitors of Poly(ADP-ribose) polymerases (PARP), a class of chemotherapeutic agents directed at targeting cancers with defective DNA-damage repair (Yuan, et al., Expert Opin Ther Pat, 2017, 27: 363). Such PARP inhibitors include but are not limited to olaparib, rucaparib, velaparib, niraparib, talazoparib, pamiparib, iniparib, E7449, and A-966492.

Therapy in combination with inhibitors of signaling pathways and mechanisms leading to repair of DNA single and double strand breaks as, e.g., nuclear factor-kappaB signaling (Pilie, et al., Nat Rev Clin Oncol, 2019, 16: 81; Zhang, et al, Chin J Cancer, 2012, 31: 359). Such inhibitors include but are not limited to inhibitors of ATM and ATR kinases, checkpoint kinase 1 and 2, DNA-dependent protein kinase, and WEE1 kinase (Pilie, et al., Nat Rev Clin Oncol, 2019, 16: 81).

Therapy in combination with an immunomodulator (Khalil, et al., Nat Rev Clin Oncol, 2016, 13: 394), a cancer vaccine (Hollingsworth, et al., NPJ Vaccines, 2019, 4: 7), an immune checkpoint inhibitor (e.g., PD-1, PD-L1, CTLA-4-inhibitor) (Wei, et al., Cancer Discov, 2018, 8: 1069), a Cyclin-D-Kinase 4/6 inhibitor (Goel, et al., Trends Cell Biol, 2018, 28: 911), an antibody being capable of binding to a tumor cell and/or metastases and being capable of inducing antibodydependent cellular cytotoxicity (ADCC) (Kellner, et al., Transfus Med Hemother, 2017, 44: 327), a T cell- or NK cell engager (e.g., bispecific antibodies) (Yu, et al., J Cancer Res Clin Oncol, 2019, 145: 941), a cellular therapy using expanded autologous or allogeneic immune cells (e.g., chimeric antigen receptor T (CAR-T) cells) (Khalil, et al., Nat Rev Clin Oncol, 2016, 13: 394). Immune checkpoint inhibitors include, but are not limited to nivolumab, ipilimumab, pembrolizumab, atezolizumab, avelumab, durvalumab, and cemiplimab.

According to the present disclosure, the compounds may be administered prior to, concurrent with, or following other anti-cancer compounds. The administration schedule may involve administering the different agents in an alternating fashion. In other embodiments, the compounds may be delivered before and during, or during and after, or before and after, or before and during and after treatment with other therapies. In some embodiments, the compound is administered more than 24 hours before the administration of the other anti-proliferative treatment. In some embodiments, more than one anti-proliferative therapy may be administered to a subject. For example, the subject may receive the present compounds, in combination with both surgery and at least one other anti-proliferative compound. In some embodiments, the compound may be administered in combination with more than one anti-cancer drug.

In some embodiments, the compounds of the present disclosure are used to detect cells and tissues overexpressing FAP, whereby such detection is achieved by conjugating a detectable label to the compounds of the disclosure, for example a detectable radionuclide. In some embodiments, the cells and tissues detected are diseased cells and tissues and/or are either a or the cause for the disease and/or the symptoms of the disease, or are part of the pathology underlying the disease. In some embodiments, the diseased cells and tissues are causing and/or are part of an oncology indication (e.g., neoplasms, tumors, and cancers) or a non-oncology indication (e.g. inflammatory disease, cardiovascular disease, autoimmune disease, and fibrotic disease).

In some embodiments, the compounds of the present disclosure are used to treat cells and tissues overexpressing FAP. In some embodiments, the cells and tissues treated are diseased cells and tissues and/or are either a or the cause for the disease and/or the symptoms of the disease, or are part of the pathology underlying the disease. In some embodiments, the diseased cells and tissues are causing and/or are part of an oncology indication (e.g., neoplasms, tumors, and cancers) and the therapeutic activity is achieved by conjugating a therapeutically active effector to the compounds of the present disclosure, for example, a therapeutically active radionuclide. In some embodiments, the diseased cells and tissues are causing and/or are part of a non-oncology indication (e.g. inflammatory disease, cardiovascular disease, autoimmune disease, and fibrotic disease) and the therapeutic activity is achieved by inhibition of the enzymatic activity of FAP.

In a further embodiment, particularly if the disease is a non-oncology disease or a non-oncology indication (e.g. inflammatory disease, cardiovascular disease, autoimmune disease, and fibrotic disease), the compounds of the present invention are administered in therapeutically effective amounts; preferably the compound of the present invention does not comprise a therapeutically active nuclide. An effective amount is a dosage of the compound sufficient to provide a therapeutically or medically desirable result or effect in the subject to which the compound is administered. The effective amount will vary with the particular condition being treated, the age and physical condition of the subject being treated, the severity of the condition, the duration of the treatment, the nature of the concurrent or combination therapy (if any), the specific route of administration and like factors within the knowledge and expertise of the health practitioner. For example, in connection with methods directed towards treating subjects having a condition characterized by abnormal cell proliferation, an effective amount to inhibit proliferation would be an amount sufficient to reduce or halt altogether the abnormal cell proliferation so as to slow or halt the development of or the progression of a cell mass such as, for example, a tumor. As used in the embodiments, "inhibit" embraces all of the foregoing.

In some embodiments, a therapeutically effective amount will be an amount necessary to extend the dormancy

of micrometastases or to stabilize any residual primary tumor cells following surgical or drug therapy.

Generally, when using an unconjugated compound without a therapeutically active radionuclide, a therapeutically effective amount may vary based on factors, such as the subject's age, condition, and sex, as well as the nature and extent of the disease in the subject, all of which can be determined by one of ordinary skill in the art. The dosage may be adjusted by the individual physician or veterinarian, particularly in the event of any complication. In some embodiments, a therapeutically effective amount includes, but not is limited to, an amount in a range from 0.1 μg/kg to about 2000 mg/kg, or from 1.0 μg/kg to about 1000 mg/kg, or from about 0.1 mg/kg to about 500 mg/kg, or from about 1.0 mg/kg to about 100 mg/kg, in one or more dose administrations daily, for one or more days. If desired, the effective daily dose of the active compound may be administered as two, three, four, five, six, or more sub-doses, for example administered separately at appropriate intervals throughout the day, optionally, in unit dosage forms. In some embodiments, the compounds are administered for more than 7 days, more than 10 days, more than 14 days, or more than 20 days. In some embodiments, the compound is administered over a period of weeks or months or years. In some embodiments, the compound is delivered on alternate days. For example, the agent is delivered every two days, or every three days, or every four days, or every five days, or every six days, or every week, or every month.

In some embodiments, the compounds of the present disclosure are for use in the treatment and/or prevention of a disease, whereby such treatment is radionuclide therapy.

For example, radionuclide therapy makes use of or is based on different forms of radiation emitted by a radionuclide. Such radiation can, for example, be any one of radiation of photons, radiation of electrons including but not limited to $\beta^-$-particles and Auger-electrons, radiation of protons, radiation of neutrons, radiation of positrons, radiation of α-particles or an ion beam. Depending on the kind of particle or radiation emitted by said radionuclide, radionuclide therapy can, for example, be distinguished as photon radionuclide therapy, electron radionuclide therapy, proton radionuclide therapy, neutron radionuclide therapy, positron radionuclide therapy, α-particle radionuclide therapy or ion beam radionuclide therapy. All of these forms of radionuclide therapy are encompassed by the present disclosure, and all of these forms of radionuclide therapy can be realized by the compound of the disclosure, wherein a radionuclide attached to the compound of the disclosure, for example as an effector, is providing for this kind of radiation.

Radionuclide therapy preferably works by damaging the DNA of cells. The damage is caused by a photon, electron, proton, neutron, positron, α-particle or ion beam directly or indirectly ionizing the atoms which make up the DNA chain. Indirect ionization happens as a result of the ionization of water, forming free radicals, notably hydroxyl radicals, which then damage the DNA.

In the most common forms of radionuclide therapy, most of the radiation effect is through free radicals. Because cells have mechanisms for repairing DNA damage, breaking the DNA on both strands proves to be the most significant technique in modifying cell characteristics. Because cancer cells generally are undifferentiated and stem cell-like, they reproduce more, and have a diminished ability to repair sub-lethal damage compared to most healthy differentiated cells. The DNA damage is inherited through cell division, accumulating damage to the cancer cells, causing them to die or reproduce more slowly.

Oxygen is a potent radiosensitizer, increasing the effectiveness of a given dose of radiation by forming DNA-damaging free radicals. Therefore, use of high-pressure oxygen tanks, blood substitutes that carry increased oxygen, hypoxic cell radiosensitizers such as misonidazole and metronidazole, and hypoxic cytotoxins, such as tirapazamine may be applied.

Other factors that are considered when selecting a radioactive dose include whether the patient is receiving chemotherapy, whether radiation therapy is being administered before or after surgery, and the degree of success of surgery.

The total radioactive dose may be fractionated, i.e., spread out over time in one or more treatments for one or more of several important reasons. For example, fractionation allows normal cells time to recover, while tumor cells are generally less efficient in repair between fractions. For example, fractionation also allows tumor cells that were in a relatively radio-resistant phase of the cell cycle during one treatment to cycle into a sensitive phase of the cycle before the next fraction is given. Similarly, tumor cells that were chronically or acutely hypoxic and, therefore, more radioresistant, may reoxygenate between fractions, improving the tumor cell kill.

It is generally known that different cancers respond differently to radiation therapy. The response of a cancer to radiation is described by its radiosensitivity. Highly radiosensitive cancer cells are rapidly killed by modest doses of radiation. These include leukemias, most lymphomas, and germ cell tumors.

It is important to distinguish radiosensitivity of a particular tumor, which to some extent is a laboratory measure, from "curability" of a cancer by an internally delivered radioactive dose in actual clinical practice. For example, leukemias are not generally curable with radiotherapy, because they are disseminated through the body. Lymphoma may be radically curable if it is localized to one area of the body. Similarly, many of the common, moderately radioresponsive tumors can be treated with curative doses of radioactivity if they are at an early stage. This applies, for example, to non-melanoma skin cancer, head and neck cancer, non-small cell lung cancer, cervical cancer, anal cancer, and prostate cancer.

The response of a tumor to radiotherapy is also related to its size. For complex reasons, very large tumors do not respond as well to radiation as smaller tumors or microscopic disease. Various strategies are used to overcome this effect. The most common technique is surgical resection prior to radiotherapy. This is most commonly seen in the treatment of breast cancer with wide local excision or mastectomy followed by adjuvant radiotherapy. Another method is to shrink the tumor with neoadjuvant chemotherapy prior to radical radionuclide therapy. A third technique is to enhance the radiosensitivity of the cancer by giving certain drugs during a course of radiotherapy. Examples of radiosensiting drugs include, but are not limited to Cisplatin, Nimorazole, and Cetuximab.

Introperative radiotherapy is a special type of radiotherapy that is delivered immediately after surgical removal of the cancer. This method has been employed in breast cancer (TARGeted Introperative radioTherapy), brain tumors and rectal cancers.

Radionuclide therapy is in itself painless. Many low-dose palliative treatments cause minimal or no side effects. Treatment with higher doses may cause varying side effects during treatment (acute side effects), in the months or years following treatment (long-term side effects), or after retreatment (cumulative side effects). The nature, severity, and longevity of side effects depends on the organs that receive the radiation, the treatment itself (type of radionuclide, dose, fractionation, concurrent chemotherapy), and the patient.

It is within the present disclosure that the method for the treatment of a disease of the invention may realize each and any of the above strategies which are as such known in the art, and which insofar constitute further embodiments of the disclosure.

It is also within the present disclosure that the compound of the disclosure is used in a method for the diagnosis of a disease as disclosed herein. In some embodiments, such a method comprises the step of administering to a subject in need thereof a diagnostically effective amount of the compound of the disclosure.

In accordance with the present disclosure, an imaging method is selected from the group consisting of scintigraphy, Single Photon Emission Computed Tomography (SPECT), Positron Emission Tomography (PET), computed tomography (CT), and combinations thereof.

Scintigraphy is a form of diagnostic test or method used in nuclear medicine, wherein radiopharmaceuticals are internalized by cells, tissues and/or organs, for example, internalized *in vivo,* and radiation emitted by said internalized radiopharmaceuticals is captured by external detectors (gamma cameras) to form and display two-dimensional images. In contrast thereto, SPECT and PET forms and displays three-dimensional images. Because of this, SPECT and PET are classified as separate techniques to scintigraphy, although they also use gamma cameras to detect internal radiation. Scintigraphy is unlike a diagnostic X-ray where external radiation is passed through the body to form an image.

Single Photon Emission Tomography (SPECT) scans are a type of nuclear imaging technique using gamma rays. They are very similar to conventional nuclear medicine planar imaging using a gamma camera. Before the SPECT scan, the patient is injected with a radiolabeled chemical emitting gamma rays that can be detected by the scanner. A computer collects the information from the gamma camera and translates this into two-dimensional cross-sections. These cross-sections can be added back together to form a three-dimensional image of an organ or a tissue. SPECT involves detection of gamma rays emitted singly, and sequentially, by the radionuclide provided by the radiolabeled chemical. To acquire SPECT images, the gamma camera is rotated around the patient. Projections are acquired at defined points during the rotation, typically every 3 - 6 degrees. In most cases, a full 360 degree rotation is used to obtain an optimal reconstruction. The time taken to obtain each projection is also variable, but 15 - 20 seconds is typical. This gives a total scan time of 15 - 20 minutes. Multi-headed gamma cameras are faster. Since SPECT acquisition is very similar to planar gamma camera imaging, the same radiopharmaceuticals may be used.

Positron Emitting Tomography (PET) is a non-invasive, diagnostic imaging technique for measuring the biochemical status or metabolic activity of cells within the human body. PET is unique since it produces images of the body's basic biochemistry or functions. Traditional diagnostic techniques, such as X-rays, CT scans, or MRI, produce images of the body's anatomy or structure. The premise with these techniques is that any changes in structure or anatomy associated with a disease can be seen. Biochemical processes are also altered by a disease, and may occur before any gross changes in anatomy. PET is an imaging technique that can visualize some of these early biochemical changes. PET scanners rely on radiation emitted from the patient to create the images. Each patient is given a minute amount of a radioactive pharmaceutical that either closely resembles a natural substance used by the body or binds specifically to a receptor or molecular structure. As the radioisotope undergoes positron emission decay (also known as positive beta decay), it emits a positron, the antiparticle counterpart of an electron. After traveling up to a few millimeters, the positron encounters an electron and annihilates, producing a pair of annihilation (gamma) photons moving in opposite directions. These are detected when they reach a scintillation material in the scanning device, creating a burst of light, which is detected by photomultiplier tubes or silicon avalanche photodiodes. The technique depends on simultaneous or coincident detection of the pair of photons. Photons that do not arrive in pairs, i.e., within a few nanoseconds, are ignored. All coincidences are forwarded to the image processing unit where the final image data is produced using image reconstruction procedures.

SPECT/CT and PET/CT is the combination of SPECT and PET with computed tomography (CT). The key

benefits of combining these modalities are improving the reader's confidence and accuracy. With traditional PET and SPECT, the limited number of photons emitted from the area of abnormality produces a very low-level background that makes it difficult to anatomically localize the area. Adding CT helps determine the location of the abnormal area from an anatomic perspective and categorize the likelihood that this represents a disease.

It is within the present disclosure that the method for the diagnosis of a disease of the disclosure may realize each and any of the above strategies which are as such known in the art, and which insofar constitute further embodiments of the disclosure.

In some embodiments, compounds of the disclosure may advantageously be used in a method for the identification of a subject or a method for the selection of a subject from a group of subjects or the method for the stratification of a group of subjects, wherein the subject is likely to respond or likely not to respond to a treatment of a disease, wherein the method comprises carrying out a method of diagnosis using compounds according to the disclosure. In certain embodiments, such methods may advantageously optimize drug treatment, including minimizing risks and maximizing efficacy, for example by helping healthcare professionals identify subjects who might benefit the most from a given therapy and avoid unnecessary treatments.

In some embodiments, compounds of the present disclosure can be useful to stratify patients, i.e., to create subsets within a patient population that provide more detailed information about how the patient will respond to a given drug. Stratification can be a critical component to transforming a clinical trial from a negative or neutral outcome to one with a positive outcome by identifying the subset of the population most likely to respond to a novel therapy.

Stratification includes the identification of a group of patients with shared "biological" characteristics to select the optimal management for the patients and achieve the best possible outcome in terms of risk assessment, risk prevention and achievement of the optimal treatment outcome.

In some embodiments, a compound of the present disclosure may be used to assess or detect, a specific disease as early as possible (which is a diagnostic use), the risk of developing a disease (which is a susceptibility/risk use), the evolution of a disease including indolent vs. aggressive (which is a prognostic use) and it may be used to predict the response and the toxicity to a given treatment (which is a predictive use).

It is also within the present disclosure that the compounds of the disclosure may be used in a theragnostic method. The concept of theragnostics is to combine a therapeutic agent with a corresponding diagnostic test that can increase the clinical use of the therapeutic drug. The concept of theragnostics is becoming increasingly attractive and is widely considered the key to improving the efficiency of drug treatment by helping doctors identify patients who might profit from a given therapy and hence avoid unnecessary treatments.

The concept of theragnostics is to combine a therapeutic agent with a diagnostic test that allows doctors to identify those patients who will benefit most from a given therapy. In an embodiment, a compound of the present disclosure is used for the diagnosis of a patient, i.e., identification and localization of the primary tumor mass as well as potential local and distant metastases. Furthermore, the tumor volume can be determined, especially utilizing three-dimensional diagnostic modalities such as SPECT or PET. Only those patients having FAP-positive tumor masses and who, therefore, might profit from a given therapy are selected for a particular therapy and hence unnecessary treatments are avoided. For example, such therapy is a FAP-targeted therapy using a compound of the present disclosure. In some embodiments, chemically identical tumor-targeted diagnostics, including, for example, imaging diagnostics for scintigraphy, PET or SPECT and radiotherapeutics are applied. Such compounds only differ in the radionuclide and therefore usually have a very similar if not identical pharmacokinetic profile. This can be realized using a chelator and a diagnostic or therapeutic radiometal. Alternatively, this can be realized using a precursor for radio labeling and radiolabeling with either a diagnostic or a therapeutic radionuclide. In one embodiment diagnostic imaging is used by means of quantification of the radiation of the diagnostic radionuclide and subsequent dosimetry which is known to those skilled in the art and the prediction of drug concentrations in the tumor compared to vulnerable side effect organs. Thus, a truly individualized drug dosing therapy for the patient is achieved.

In some embodiments, the theragnostic method is realized with only one theragnostically active compound such as a compound of the present disclosure labeled with a radionuclide emitting diagnostically detectable radiation (e.g., positrons or gamma rays) as well as therapeutically effective radiation (e.g., electrons or alpha particles).

The disclosure also contemplates a method of intraoperatively identifying/disclosing diseased tissues expressing FAP in a subject. Such method uses a compound of the disclosure, whereby in some embodiments such compound of the disclosure comprises as the effector a diagnostically active agent such as a diagnostically active radionuclide.

According to a further embodiment of the disclosure, the compound of the disclosure, particularly if complexed with a radionuclide, may be employed as adjunct or adjuvant to any other tumor treatment including, surgery as the primary method of treatment of most isolated solid cancers, radiation therapy involving the use of ionizing radiation in an attempt to either cure or improve the symptoms of cancer using either sealed internal sources in the form of brachytherapy or external sources, chemotherapy such as alkylating agents, antimetabolites, anthracyclines, plant alkaloids, topoisomerase inhibitors, and other antitumor agents, hormone treatments that modulate tumor cell behavior without directly attacking those cells, targeted agents which directly target a molecular abnormality in certain types of cancer

including monoclonal antibodies and tyrosine kinase inhibitors, angiogenesis inhibitors, immunotherapy, cancer vaccination, palliative care including actions to reduce the physical, emotional, spiritual, and psycho-social distress to improve the patient's quality of life and alternative treatments including a diverse group of health care systems, practices, and products that are not part of conventional medicine.

In an embodiment of the methods of the disclosure, the subject is a patient. In an embodiment, a patient is a subject which has been diagnosed as suffering from or which is suspected of suffering from or which is at risk of suffering from or developing a disease, whereby the disease is a disease as described herein, a disease involving FAP.

Dosages employed in practicing the methods for treatment and diagnosis, respectively, where a radionuclide is used and more specifically attached to or part of the compound of the disclosure will vary depending, e.g., on the particular condition to be treated, for example the known radiosensitivity of the tumor type, the volume of the tumor and the therapy desired. In general, the dose is calculated on the basis of radioactivity distribution to each organ and on observed target uptake. A $\gamma$-emitting complex may be administered once or at several times for diagnostic imaging. In animals, an indicated dose range may be, for example, from 0.1 ng/kg to 5 mg/kg of the compound of the disclosure complexed, e.g., with 1 kBq to 200 MBq of a $\gamma$-emitting radionuclide, including, but not limited to, $^{111}$In or $^{89}$Zr. An $\alpha$- or $\beta$-emitting complex of the compound of the disclosure may be administered at several time points, e.g., over a period of 1 to 3 weeks or longer. In animals, an indicated dosage range may be, for example, from 0.1 ng/kg to 5 mg/kg of the compound of the disclosure complexed, e.g., with 1 kBq to 200 MBq of an $\alpha$- or $\beta$-emitting radionuclide, including, but not limited to, $^{225}$Ac or $^{177}$Lu. In larger mammals, including, for example, humans, an indicated dosage range may be, for example, from 0.1 ng/kg to 5 mg/kg or for example 0.1 ng/kg to 100 $\mu$g/kg of the compound of the disclosure complexed with, e.g., 10 to 1000 MBq of a $\gamma$-emitting radionuclide, including, but not limited to, $^{111}$In or $^{89}$Zr. In larger mammals, including, for example, humans, an indicated dosage range may be, for example, from 0.1 ng/kg to 5 mg/kg or for example, from 0.1 ng/kg to 100 $\mu$g/kg of the compound of the disclosure complexed with, e.g., 1 to 100000 MBq of an $\alpha$- or $\beta$-emitting radionuclide, including, but not limited to, $^{225}$Ac or $^{177}$Lu.

In certain embodiments, uptake can be measured in terms of absorbed dose (mGy/MBq), SUVmax, SUVmean. In animals, uptake across tissues is reported in injected dose/gram ID/g. Sensitivity to radiation is tumor and non-tumor tissue dependent. The favorable tumor to non-tumor tissue uptake of the present compounds allows delivery of a radioactive nuclide at a dose that could reduce tumor growth, or partially or completely destroys the tumor. At such dose, no permanent or critical damage to non-tumor tissue is expected.

In a further aspect, the instant disclosure is related to a composition and a pharmaceutical composition in particular, comprising the compound of the disclosure.

The pharmaceutical composition of the present disclosure comprises at least one compound of the disclosure and, optionally, one or more carrier substances, excipients and/or adjuvants. The pharmaceutical composition may additionally comprise, for example, one or more of water, buffers such as, e.g., neutral buffered saline or phosphate buffered saline, ethanol, mineral oil, vegetable oil, dimethylsulfoxide, carbohydrates such as *e.g.,* glucose, mannose, sucrose or dextrans, mannitol, proteins, adjuvants, polypeptides or amino acids such as glycine, antioxidants, chelating agents such as EDTA or glutathione and/or preservatives. Furthermore, one or more other active ingredients may, but need not, be included in the pharmaceutical composition of the disclosure.

The pharmaceutical composition of the disclosure may be formulated for any appropriate route of administration, including, for example, topical such as, e.g., transdermal or ocular, oral, buccal, nasal, vaginal, rectal or parenteral administration. In an embodiment, and as preferably used herein, the term parenteral includes subcutaneous, intradermal, intravascular such as, e.g., intravenous, intramuscular, intrathecal and intraperitoneal injection, as well as any similar injection or infusion technique. In some embodiments, the route of administration is intravenous administration.

In an embodiment of the disclosure the compound of the disclosure comprising a radionuclide is administered by any conventional route, in particular intravenously, e.g., in the form of injectable solutions or suspensions. The compound of the disclosure may also be administered advantageously by infusion, e.g., by an infusion of 30 to 60 min.

In some embodiments, depending on the site of the tumor, the compound of the disclosure may be administered as close as possible to the tumor site, e.g., by means of a catheter. Such administration may be carried out directly into the tumor tissue or into the surrounding tissue or into the afferent blood vessels. The compound of the disclosure may also be administered repeatedly in doses, including, in some embodiments, in divided doses.

According to an embodiment of the disclosure, a pharmaceutical composition of the disclosure comprises a stabilizer, e.g., a free radical scavenger, which inhibits autoradiolysis of the compound of the disclosure. Suitable stabilizers include, e.g., serum albumin, ascorbic acid, retinol, gentisic acid or a derivative thereof, or an amino acid infusion solution such, e.g., used for parenteral protein feeding, for example, free from electrolyte and glucose, for example a commercially available amino acid infusion such as Proteinsteril® KE Nephro. In some embodiments, ascorbic acid and gentisic acid are used.

A pharmaceutical composition of the disclosure may comprise further additives, e.g., an agent to adjust the pH between 7.2 and 7.4, e.g., sodium or ammonium acetate or $Na_2HPO_4$. In some embodiments, the stabilizer is added to the non-radioactive compound of the disclosure and introduction of the radionuclide, for instance the complexation with

the radionuclide, is performed in the presence of the stabilizer, either at room temperature or, for example, at a temperature of from 40 to 120° C. The complexation may conveniently be performed under air free conditions, e.g., under $N_2$ or Ar. In some embodiments, further stabilizer may be added to the composition after complexation.

Excretion of the compound of the disclosure, particularly if the effector is a radionuclide, essentially takes place through the kidneys. In some embodiments, further protection of the kidneys from radioactivity accumulation may be achieved by administration of lysine or arginine or an amino acid solution having a high content of lysine and/or arginine, e.g., a commercially available amino acid solution such as Synthamin®-14 or -10, prior to the injection of or together with the compound of the disclosure, particularly if the effector is a radionuclide. In some embodiments, protection of the kidneys may also be achieved by administration of plasma expanders, such as, e.g., gelofusine, either instead of or in addition to amino acid infusion. In some embodiments, protection of the kidneys may also be achieved by administration of diuretics providing a means of forced diuresis which elevates the rate of urination. Such diuretics include high ceiling loop diuretics, thiazides, carbonic anhydrase inhibitors, potassium-sparing diuretics, calcium-sparing diuretics, osmotic diuretics and low ceiling diuretics. In some embodiments, a pharmaceutical composition of the disclosure may contain, apart from a compound of the disclosure, at least one of these further compounds intended for or suitable for kidney protection, including, for example, kidney protection of the subject to which the compound of the disclosure is administered.

It will be understood by a person skilled in the art that the compounds of the disclosure are disclosed herein for use in various methods. It will be further understood by a person skilled in the art that the composition of the disclosure and the pharmaceutical composition of the disclosure can be equally used in said various methods. It will also be understood by a person skilled in the art that the composition of the disclosure and the pharmaceutical composition are disclosed herein for use in various methods. It will be equally understood by a person skilled in the art that the compounds of the disclosure can be equally used in said various methods.

It will be acknowledged by a person skilled in the art that the composition and/or the pharmaceutical composition as disclosed herein may contain one or more further compounds in addition to the compound of the disclosure. To the extent that such one or more further compounds are disclosed herein as being part of the composition of the disclosure and/or of the pharmaceutical composition of the disclosure, it will be understood that such one or more further compounds can be administered separately from the compound of the disclosure to the subject which is exposed to or the subject of a method of the disclosure. Such administration of the one or more further compounds can be performed prior to, concurrently with or after the administration of the compound of the invention. It will also be acknowledged by a person skilled in the art that in a method of the invention, apart from a compound of the invention, one or more further compounds may be administered to a subject. Such administration of the one or more further compounds can be performed prior to, concurrently with or after the administration of the compound of the disclosure. To the extent that such one or more further compounds are disclosed herein as being administered as part of a method of the disclosure, it will be understood that such one or more further compounds are part of a composition of the disclosure and/or of a pharmaceutical composition of the disclosure. It is within the present disclosure that the compound of the disclosure and the one or more further compounds may be contained in the same or a different formulation. It is also within the present disclosure that the compound of the disclosure and the one or more further compounds are not contained in the same formulation, but are contained in the same package containing a first formulation comprising a compound of the disclosure, and a second formulation comprising the one or more further compounds, whereby the type of formulation may be the same or may be different.

It is within the present disclosure that more than one type of a compound of the disclosure may be contained in the composition of the disclosure and/or the pharmaceutical composition of the disclosure. It is also within the present disclosure that more than one type of a compound of the disclosure may be used, preferably administered, in a method of the disclosure.

It will be acknowledged that a composition of the disclosure and a pharmaceutical composition of the disclosure may be manufactured in conventional manner.

Radiopharmaceuticals have decreasing content of radioactivity with time, as a consequence of the radioactive decay. The physical half-life of the radionuclide is often short for radiopharmaceutical diagnostics. In these cases, the final preparation has to be done shortly before administration to the patient. This is in particular the case for positron emitting radiopharmaceuticals for tomography (PET radiopharmaceuticals). It often leads to the use of semi-manufactured products such as radionuclide generators, radioactive precursors and kits.

In some embodiments, a kit of the disclosure comprises apart from one or more than one compounds of the disclosure typically at least one of the followings: instructions for use, final preparation and/or quality control, one or more optional excipient(s), one or more optional reagents for the labeling procedure, optionally one or more radionuclide(s) with or without shielded containers, and optionally one or more device(s), whereby the device(s) is/are selected from the group comprising a labeling device, a purification device, an analytical device, a handling device, a radioprotection device or an administration device.

Shielded containers known as "pigs" for general handling and transport of radiopharmaceutical containers come

in various configurations for holding radiopharmaceutical containers such as bottles, vials, syringes, etc. One form includes a removable cover that allows access to the held radiopharmaceutical container. When the pig cover is in place, the radiation exposure is acceptable.

In some embodiments, a labeling device is selected from the group of open reactors, closed reactors, microfluidic systems, nanoreactors, cartridges, pressure vessels, vials, temperature controllable reactors, mixing or shaking reactors and combinations thereof.

In some embodiments, a purification device is selected from the group of ion exchange chromatography columns or devices, size-exclusion chromatography columns or devices, affinity chromatography columns or devices, gas or liquid chromatography columns or devices, solid phase extraction columns or devices, filtering devices, centrifugations vials columns or devices and combinations thereof.

In some embodiments, an analytical device is selected from the group of tests or test devices to determine the identity, radiochemical purity, radionuclidic purity, content of radioactivity and specific radioactivity of the radiolabelled compound and combinations thereof.

In some embodiments, a handling device is selected from the group consisting of devices for mixing, diluting, dispensing, labeling, injecting and administering radiopharmaceuticals to a subject and combinations thereof.

In some embodiments, a radioprotection device is used in order to protect doctors and other personnel from radiation when using therapeutic or diagnostic radionuclides. In some embodiments, the radioprotection device is selected from the group consisting of devices with protective barriers of radiation-absorbing material selected from the group consisting of aluminum, plastics, wood, lead, iron, lead glass, water, rubber, plastic, cloth, devices ensuring adequate distances from the radiation sources, devices reducing exposure time to the radionuclide, devices restricting inhalation, ingestion, or other modes of entry of radioactive material into the body and devices providing combinations of these measures.

In some embodiments, an administration device is selected from the group of syringes, shielded syringes, needles, pumps, and infusion devices and combinations thereof Syringe shields are commonly hollow cylindrical structures that accommodate the cylindrical body of the syringe and are constructed of lead or tungsten with a lead glass window that allows the handler to view the syringe plunger and liquid volume within the syringe.

It will be acknowledged by a person skilled in the art that in the instant description the terms disclosure and invention are used interchangeably.

It will be further acknowledged by a person skilled in the art that in the instant description the terms pharmaceutical agent and therapeutic agent are used interchangeably.

## EXAMPLES

The following Examples have been included to provide guidance to one of ordinary skill in the art for practicing representative embodiments of the presently disclosed subject matter. In light of the present disclosure and the general level of skill in the art, those of skill will appreciate that the following Examples are intended to be exemplary only and that numerous changes, modifications, and alterations can be employed without departing from the scope of the presently disclosed subject matter. The synthetic descriptions and specific examples that follow are intended for the purposes of illustration only, and are not to be construed as limiting in any manner the preparation of compounds of the present disclosure by other methods.

Abbreviations used in the instant application and the following examples in particular are as follows:

| |
|---|
| 4PL means four parameter logistic curve fitting |
| ACN means acetonitrile |
| AcOH means acetic acid |
| AIBN means azobisisobutyronitrile |
| Alloc means allyloxycarbonyl |
| AMC means 7-amino-4-methylcoumarin |
| Boc means *tert*-butyloxycarbonyl |
| $Boc_2O$ means di-*tert*-butyl dicarbonate |
| BSA means bovine serum albumin |
| BuLi means n-butyllithium |
| conc. means concentrated |

(continued)

| |
|---|
| CT means computed tomography |
| Cy5 means Cyanine-5 |
| DAD means Diode Array Detector |
| DCM means dichloromethane |
| DIPEA means diisopropylethylamine |
| DICOM means Digital Imaging and Communications in Medicine |
| DME means dimethoxyethane |
| DMF means *N*,*N*-dimethylformamide |
| DMP means Dess-Martin-Periodinane |
| DMSO means dimethyl sulfoxide |
| DOTA means 1,4,7,10-tetraazacyclododecane-1,4,7,10-tetraacetic acid |
| DOTA-NHS ester is 1,4,7,10-Tetraazacyclododecane-1,4,7,10-tetraacetic acid mono-N-hydroxysuccinimide |
| DOTA(tBu)$_3$-OH means tri-*tert*-butyl-1,4,7,1 0-tetraazacyclo-dodecane-1,4,7,10-tetraacetate |
| DTPA means diethylenetriaminepentaacetic acid |
| EA means ethyl acetate |
| EDT means 1,2-ethanedithiol |
| EOS means end of synthesis |
| eq. means equivalents |
| ESI means electrospray ionization |
| Et2O means diethylether |
| FACS means fluorescence-activated cell sorting |
| FAP means fibroblast activation protein |
| FBS means fetal bovine serum |
| h means hour(s) |
| HATU means *O*-(7-azabenzotriazol-1-yl)-*N,N,N',N'*-tetramethyluronium hexafluorophosphate |
| HL means half-life |
| HPLC means high-performance liquid chromatography |
| IC50 means half-maximal inhibitory concentration |
| $K_D$ means dissociation constant |
| $K_i$ means inhibitory constant |
| $k_{on}$ means association rate |
| $k_{off}$ means dissociation rate |
| KO$^t$Bu is potassium 2-methylpropan-2-olate |
| LC means liquid chromatography |
| M means molar, i.e. mol per Liter |
| MBq means megabecquerel |
| *m*CPBA means 3-chlorobenzene-1-carboperoxoic acid |
| MeOH means methanol |
| min means minute(s) |

(continued)

| |
|---|
| MS means mass spectrometry |
| MTBE means methyl-*tert*-butylether |
| MW means molecular weight |
| NBS means *N*-bromosuccinimide |
| m/z means mass divided by charge |
| *n*-BuOH means *n*-butanol |
| n.d. means not determined |
| PBS means phosphate buffered saline |
| pIC50 means the negative log of the IC50 value when converted to molar |
| rh means recombinant human |
| rm means recombinant mouse |
| RP means reversed phase |
| RT means room temperature |
| $R_t$ means retention time |
| RU means response unit |
| SCK means single cycle kinetic |
| SPECT means single photon emission computed tomography |
| SPR means surface plasmon resonance |
| SUVmax means maximum standardized uptake values |
| tBu means *tert.* butyl |
| $t_{1/2}$ means half-life |
| TFA means trifluoroacetic acid or trifluoroacetate |
| TIPS means triisopropylsilane |
| TOF means time-of-flight detection |
| Tris means tris(hydroxymethyl)aminomethane |
| w/o means without |

**Example 1: Material and Methods**

The materials and methods as well as general methods are further illustrated by the following examples.

Solvents:
Solvents were used in the specified quality without further purification. Acetonitrile (Super Gradient, HPLC, VWR - for analytical purposes; PrepSolv, Merck - for preparative purposes); cyclohexane; dichloromethane (synthesis grade, Roth); *N*,*N*-dimethylformamide (peptide synthesis grade, Biosolve); methanol (synthesis grade, Roth); methyl-tert-butylether (synthesis grade, Roth); tetrahydrofuran (puriss. grade, Sigma-Aldrich).

Water: Milli-Q Plus, Millipore, demineralized.

Chemicals:
Chemicals were synthesized according to or in analogy to literature procedures or purchased from Sigma-Aldrich-Merck (Deisenhofen, Germany), Bachem (Bubendorf, Switzerland),-VWR (Darmstadt, Germany), Novabiochem (Merck Group, Darmstadt, Germany), Iris Biotech (Marktredwitz, Germany), Roth (Karlsruhe, Deutschland), or other companies and used in the assigned quality without further purification.

HPLC/MS analyses

As one typical non-limiting example HPLC/MS analyses were performed by injection of 5 μl of a solution of the sample, using a 2-step gradient for all chromatograms (5-65% B in 12 min, followed by 65-90% in 0.5 min, A: 0.1% TFA in water and B: 0.1% TFA in ACN). A typical set-up and instrument configuration is the following: RP columns were from Agilent (Type Poroshell 120, 2.7μm, EC-C18, 50 x 3.00 mm, flow 0.8 ml, HPLC at room temperature); Mass spectrometer: Agilent 6230 LC/TOF-MS, ESI ionization. MassHunter Qualitative Analysis B.07.00 SP2 was used as software. UV detection was done at λ = 230 nm. Retention times ($R_t$) are indicated in the decimal system (e.g., 1.9 min = 1 min 54 s) and are referring to detection in the UV spectrometer. For the evaluation of observed compound masses, the 'Find Compounds by Formula'-feature was used. In particular, the individual 'neutral mass of a compound (in units of Daltons)'-values and the corresponding isotope distribution pattern were used to confirm compound identity.

Preparation of compounds:

Specific embodiments for the preparation of compounds of the disclosure are provided in the following examples. Unless otherwise specified, all starting materials and reagents are of standard commercial grade, and are used without further purification, or are readily prepared from such materials by routine methods. Those skilled in the art of organic synthesis will recognize in light of the instant disclosure that starting materials and reaction conditions may be varied including additional steps employed to produce compounds encompassed by the present disclosure.

**Example 2: Synthesis**

An appropriate synthesis strategy for compounds of the invention is the late stage introduction of the chelator which typically includes a compound equipped with a protecting group at corresponding nitrogen as precursor. Example 2a illustrates some synthesis routes to N-Boc-protected precursors (**1-8**) which finally are converted to the compounds of invention by applying a general two-step method.

**Example 2a: Synthesis of N-Boc-protected precursors, shown below as Intermediates (1-8)**

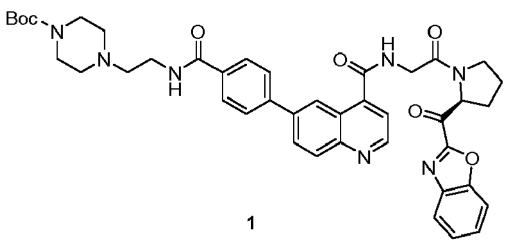

1

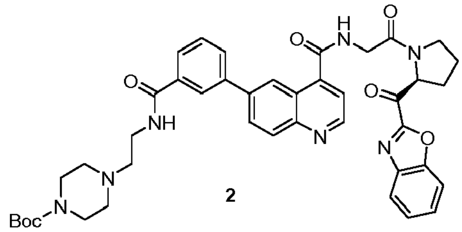

2

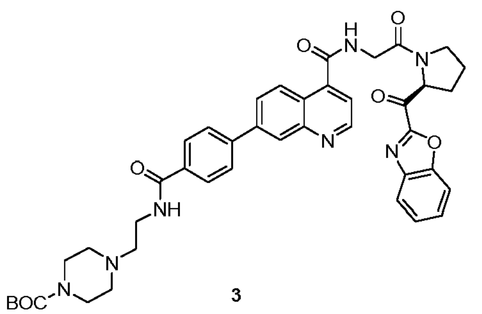

3

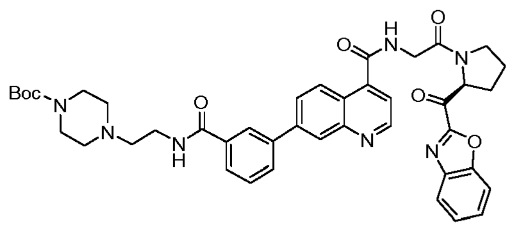

4

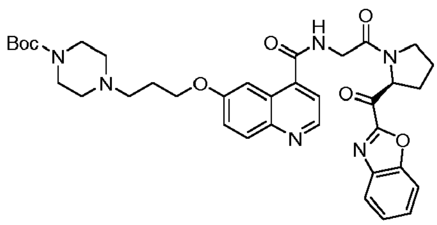

**5**

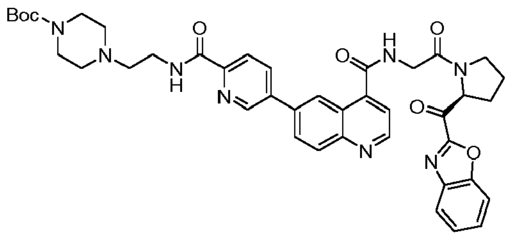

**6**

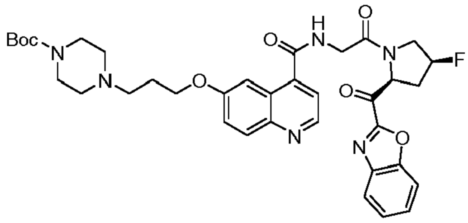

**7**

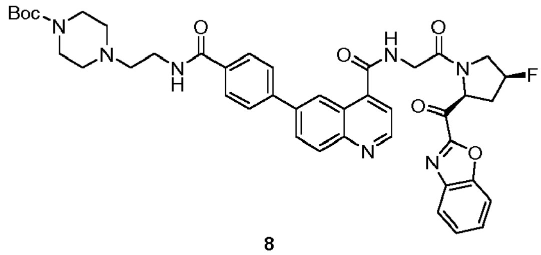

**8**

Preparation of Boc-protected precursor. Intermediate(**4**), for the synthesis of DOTA-compound 3BP-4694

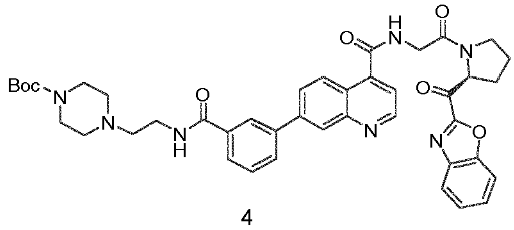

4

The synthesis of the title compound is depicted in the following reaction scheme.

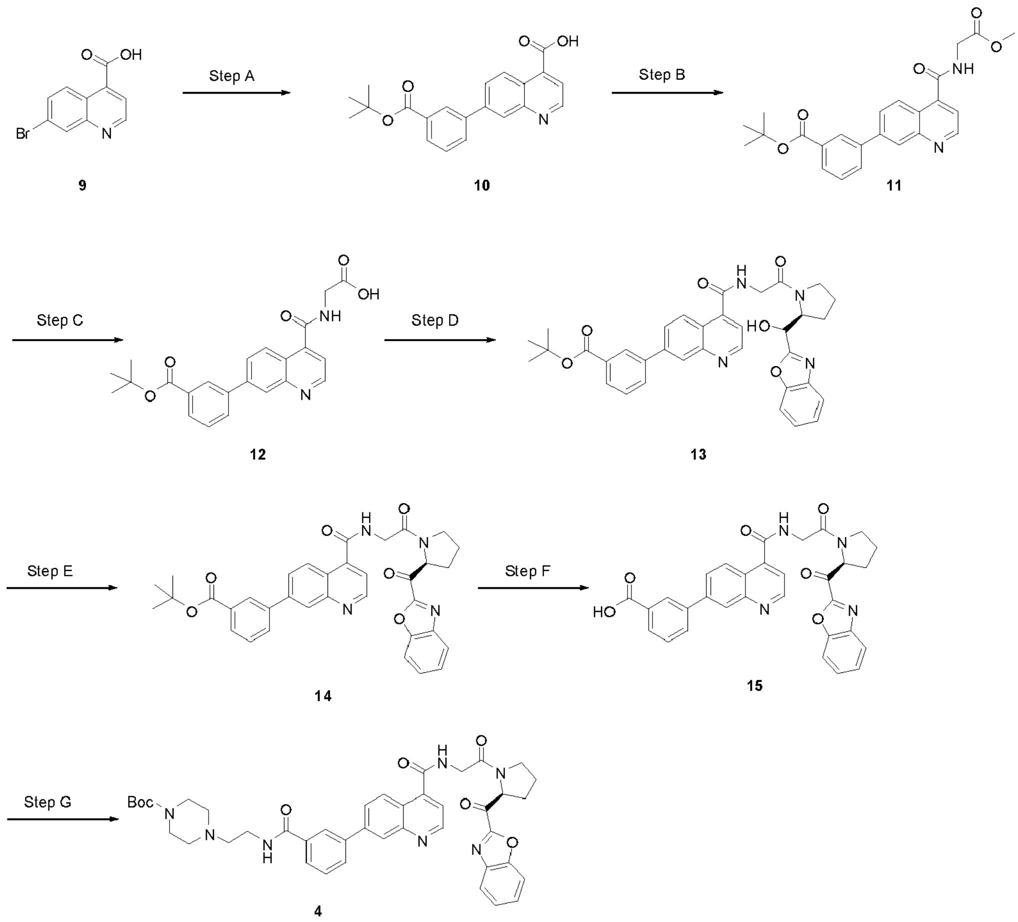


9 10 11 12 13 14 15 4

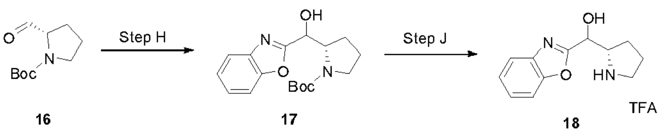


16 17 18

Step A: Synthesis of 7-(3-(*tert*-butoxycarbonyl)phenyl)quinoline-4-carboxylic acid (**10**). 7-Bromoquinoline-4-carboxylic acid (**9**, 100 mg, 0.397 mmol) and 3-(*tert*-butoxycarbonyl)phenylboronic acid (105.7 mg, 0.476 mmol) were dissolved in a mixture of DME (4 mL) and 2M aqueous $NaHCO_3$ solution (1.190 mL, 2.380 mmol). The flask was evaporated and ventilated with nitrogen and the procedure was repeated. After addition of bis(triphenylphosphine)palladium(II) dichloride (75.4 mg, 0.198 mmol), the mixture was stirred at 80 °C. After complete conversion, the mixture was cooled to RT, filtrated and evaporated to dryness to yield the title compound. The crude was used without further purification. MS (m/z): 350.3 [M+H$^+$]

Step B: Synthesis of *tert-butyl* 3-(4-(2-methoxy-2-oxoethylcarbamoyl)quinolin-7-yl)benzoate (**11**). A solution of 7-(3-(*tert*-butoxycarbonyl)phenyl)quinoline-4-carboxylic acid (**10**, 138.6 mg, 0.397 mmol) and glycine methyl ester hydrochloride (69.7 mg, 0.555 mmol) were dissolved in dry DMF (10 mL). The solution was cooled to 0 °C and HATU (226.3 mg, 0.595 mmol) and DIPEA (269.9 µL, 1.587 mmol) were added at this temperature. The reaction mixture was stirred and allowed to warm to RT. After complete conversion (LCMS), the mixture was evaporated and purified by flash chromatography on silica gel (eluent: DCM/ MeOH) to afford the title compound. MS (m/z): 421.0 [M+H$^+$]

Step C: Synthesis of 2-(7-(3-(tert-butoxycarbonyl)phenyl)quinoline-4-carboxamido)acetic acid (**12**). Lithiumhydroxid monohydrate (46.6 mg, 1.111 mmol) was added to a solution of *tert*-butyl 3-(4-(2-methoxy-2-oxoethylcarbamoyl)quinolin-7-yl)benzoate (**11**, 233.5 mg, 0.555 mmol) in a mixture of THF (5.0 mL) and $H_2O$ (0.5 mL). The mixture was stirred for 3 h. After complete conversion, the mixture was neutralized using 1M aqueous HCl and subsequently evaporated to dryness to yield the title compound. The product was used without further purification. MS (m/z):

407.1 [M+H+]

Step D: Synthesis of *tert*-butyl 3-(4-(2-((*S*)-2-((*R*)-benzo[*d*]oxazol-2-yl(hydroxy)methyl)pyrrolidin-1-yl)-2-oxoethylcarbamoyl)quinolin-7-yl)benzoate (**13**). A solution of 2-(7-(3-(*tert*-butoxycarbonyl)phenyl)quinoline-4-carboxamido)acetic acid (**12**, 129.0 mg, 0.317 mmol) and (2*S*)-2-(benzo[*d*]oxazol-2-yl(hydroxy)methyl)pyrrolidinium 2,2,2-trifluoroacetate (**18**, 90.1 mg, 0.413 mg) in dry DMF (3.2 mL) was cooled to 0 °C. Subsequently, HATU (181.0 mg, 0.476 mmol) and DIPEA (108 μL, 0.635 mmol) were added. The mixture was stirred and allowed to reach RT. After stirring for 15 min, the volatiles were removed *in vacuo* and the crude product was purified by chromatography on silica (elution with DCM/ MeOH) to yield the title product. MS (m/z): 607.5 [M+H+]

Step E: Synthesis of (*S*)-*tert*-butyl 3-(4-(2-(2-(benzo[*d*]oxazole-2-carbonyl)pyrrolidin-1-yl)-2-oxoethylcarbamoyl)quinolin-7-yl)benzoate (**14**). DMP (107.7 mg, 0.254 mmol) was added to a solution of *tert*-butyl 3 -(4-(2-((*S*)-2-((*R*)-benzo[*d*]oxazol-2-yl(hydroxy)methyl)pyrrolidin-1-yl)-2-oxoethylcarbamoyl)quinolin-7-yl)benzoate (**13**, 77.0 mg, 0.127 mmol) in DCM (1.3 mL). After stirring for 3 h at RT, the mixture was concentrated to dryness and the crude product was subject to flash column chromatography on silica gel (elution with DCM/ MeOH). MS (m/z): 605.2 [M+H+]

Step F: Synthesis of (*S*)-3-(4-(2-(2-(benzo[*d*]oxazole-2-carbonyl)pyrrolidin-1-yl)-2-oxoethylcarbamoyl)quinolin-7-yl)benzoic acid (**15**). (*S*)-*tert*-Butyl 3-(4-(2-(2-(benzo[d]oxazole-2-carbonyl)pyrrolidin-1-yl)-2-oxoethylcarbamoyl)quinolin-7-yl)benzoate (**14**, 60.0 mg, 0.10 mmol) was dissolved in a mixture of TFA (152.9 μL, 1.99 mmol) and DCM (408 μL) and stirred at RT for 3 h. After complete conversion, the mixture was concentrated to dryness and used without further purification. MS (m/z): 549.1 [M+H+]

Step G: Synthesis of (*S*)-*tert*-butyl 4-(2-(3-(4-(2-(2-(benzoMoxazole-2-carbonyl)pyrrolidin-1-yl)-2-oxoethylcarbamoyl)quinolin-7-yl)benzamido)ethyl)piperazine-1-carboxylate (**4**). A solution of (*S*)-3-(4-(2-(2-(benzo[*d*]oxazole-2-carbonyl)pyrrolidin-1-yl)-2-oxoethylcarbamoyl)quinolin-7-yl)benzoic acid (**15**, 57.9 mg, 0.106 mmol) and *tert*-butyl 4-(2-aminoethyl)piperazine-1-carboxylate (33.9 mg, 0.148 mmol) in dry DMF was cooled to 0 °C. Subsequently, HATU (60.2 mg, 0.158 mmol) and DIPEA (35.9 μL, 0.211 mmol) were added at this temperature. After stirring for 30 min at RT the mixture was concentrated to dryness and subject to reverse phase HPLC to yield the title compound. MS (m/z): 760.6 [M+H+]

Step H: Synthesis of (2*S*)-*tert*-butyl 2-(benzo[*d*]oxazol-2-yl(hydroxy)methyl)pyrrolidine-1-carboxylate (**17**). Benzo[*d*]oxazole (1.793 g, 15.056 mmol) was dissolved under an atmosphere of Argon in dry THF (2 mL/ mmol) and cooled to -20 °C. At this temperature, isopropylmagnesium chloride (2 M in diethyl ether, 9.034 mL, 18.068 mmol) was added dropwise. Stiring at -20 °C was continued 1 h before adding a solution of (*S*)-*tert*-butyl 2-formylpyrrolidine-l-carboxylate (**16**, 2.359 mL, 12.547 mmol) in dry THF (12.5 mL). The mixture was stirred for 2 h and allowed to reach room temperature. After 2 h the reaction was quenched by addition of sat. aqueous $NH_4Cl$ solution (12 mL) and stirring of this mixture for 1 h. The reaction mixture was extracted with EA (3 x 10 mL). The combined organic layers were washed with sat. aqueous NaCl solution, dried ($Na_2SO_4$), and evaporated to dryness. The crude mixture was purified by flash column chromatography on silica (elution with heptane/ EA) to afford the title compound. MS (m/z): 319.3 [M+H+]

Step J: Synthesis of (2*S*)-2-(benzo[*d*]oxazol-2-yl(hydroxy)methyl)pyrrolidinium 2,2,2-trifluoroacetate (**18**). (2*S*)-*tert*-Butyl 2-(benzo[*d*]oxazol-2-yl(hydroxy)methyl)pyrrolidine-1-carboxylate (**17**, 1.550 g, 4.869 mmol) was dissolved in dry DCM (36 mL) and TFA (7.501 mL, 97.371 mmol) was added dropwise. The mixture was stirred at RT for 1 h. After complete conversion (1 h), the volatiles were removed *in vacuo* to yield the title compound. The product was used without further purification. MS (m/z): 218.9 [M+H+]

The corresponding N-Boc-protected precursors, Intermediates **1, 2,** and **3** (for the synthesis of compounds 3BP-4663, 3BP-4664, and 3BP-4665 respectively), were prepared following an analogous procedure to that described for Intermediate **4.** Someone skilled in the art will recognize that a simple variation in the substitution pattern of the starting materials for step A lead to the desired regioisomeric target molecules.

In addition, it is simple to introduce a variety of other Boc-protected diamines into the structures by varying the building-block introduced in step C. For instance, the synthesis of the intermediate for 3BP-3582 was performed in analogy to precursor **4** described above, but in step G, mono-Boc-protected diamino-propane was coupled instead of the Boc-piperazine building block. Deprotection and DOTA coupling followed to give 3BP-3582.

Preparation of Boc-protected precursor. Intermediate (**6**), for the synthesis of 3BP-4809

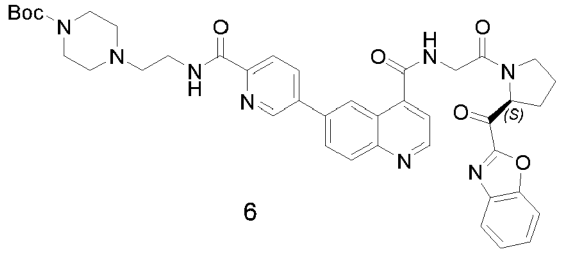

6

The synthesis of the title compound is depicted in the following reaction scheme.

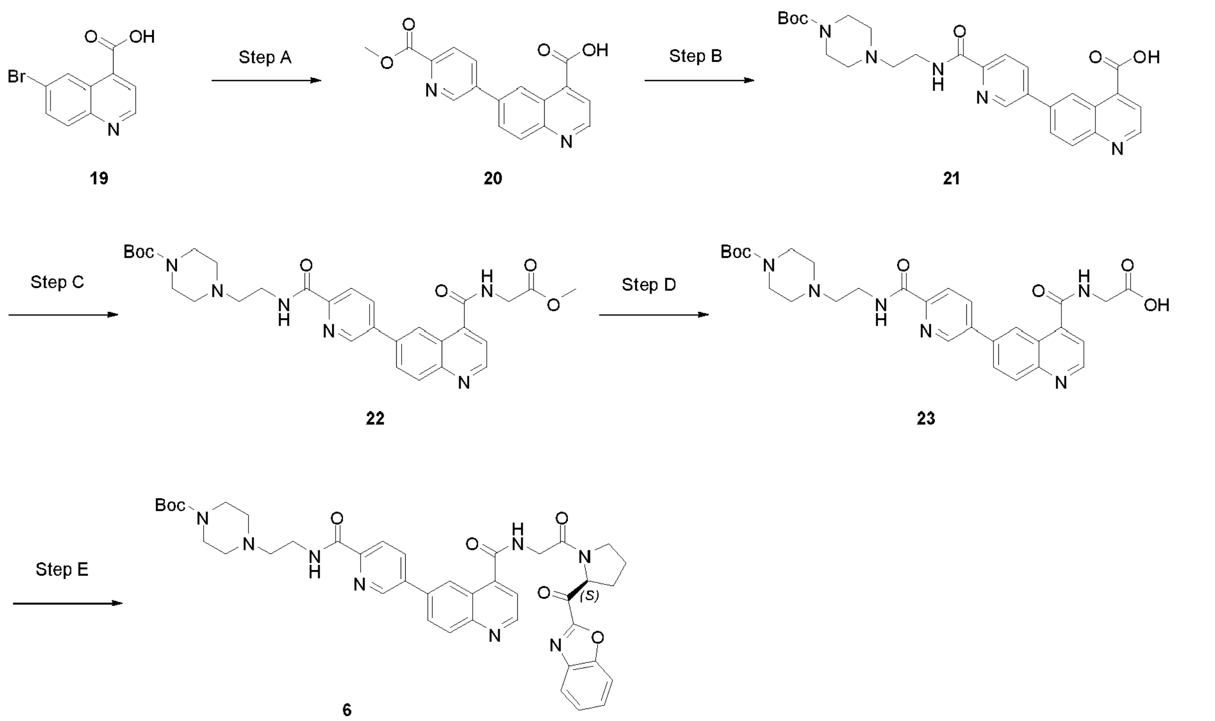


19 20 21 22 23 6

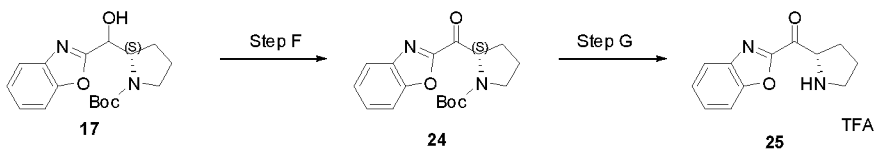


17 24 25 TFA

Step A: Synthesis of 6-(6-(methoxycarbonyl)pyridin-3-yl)quinoline-4-carboxylic acid (**20**). 6-Bromoquinoline-4-carboxylic acid (**19**, 100.0 mg, 0.397 mmol), 2-(methylcarboxy)pyridine-5-boronic acid pinacol ester (125.3 mg, 0.476 mmol), bis(triphenylphosphine)palladium(II) dichloride (75.4 mg, 0.198 mmol), and 2M aqueous $NaHCO_3$ solution (1.190 mL, 2.380 mmol) were reacted according to the synthesis of 7-(3-(*tert*-butoxycarbonyl)phenyl)quinoline-4-carboxylic acid (**10**) to give the title compound which was used in the next step without further purification. MS (m/z): 219.8 [M+H$^+$]

Step B: Synthesis of 6-(6-(2-(4-(*tert*-butoxycarbonyl)piperazin-1-yl)ethylcarbamoyl)pyridin-3-yl)quinoline-4-carboxylic acid (**21**). A mixture of 6-(6-(methoxycarbonyl)pyridin-3-yl)quinoline-4-carboxylic acid (**20**, 122.3 mg, 0.397 mmol) and *tert*-butyl 4-(2-aminoethyl)piperazine-1-carboxylate (207.4 mg, 1.190 mmol) was stirred at 40 °C over

night. After complete conversion, the crude product was purified by flash column chromatography on silica gel (elution with DCM/ MeOH) to give the title compound. MS (m/z): 504.1 [M-H$^+$]

Step C: Synthesis of *tert-butyl* 4-(2-(5-(4-(2-methoxy-2-oxoethylcarbamoyl)quinolin-6-yl)picolinamido)ethyl)piperazine-1-carboxylate (**22**). 6-(6-(2-(4-(*tert*-Butoxycarbonyl)piperazin-1-yl)ethylcarbamoyl)pyridin-3-yl)quinoline-4-carboxylic acid (**21**, 136.0 mg, 0.269 mmol), glycine methyl ester hydrochloride (47.3 mg, 0.377 mmol), HATU (153.4 mg, 0.404 mmol), and DIPEA (183.0 µL, 1.076 mmol) were reacted according to the synthesis of *tert-butyl* 3-(4-(2-methoxy-2-oxoethylcarbamoyl)quinolin-7-yl)benzoate (**11**) to yield the title compound after flash column chromatography (elution with DCM/ MeOH). MS (m/z): 577.7 [M+H$^+$]

Step D: Synthesis of 2-(6-(6-(2-(4-(*tert*-butoxycarbonyl)piperazin-1-yl)ethylcarbamoyl)pyridin-3-yl)quinoline-4-carboxamido)acetic acid (**23**). 4-(2-(5-(4-(2-Methoxy-2-oxoethylcarbamoyl)quinolin-6-yl)picolinamido)ethyl)piperazine-1-carboxylate (**22**, 96.0 mg, 0.166 mmol) was reacted with lithiumhydroxyde monohydrate (24.5 mg, 0.583 mmol) according to the Synthesis of 2-(7-(3-(*tert*-butoxycarbonyl)phenyl)quinoline-4-carboxamido)acetic acid (**12**) to yield the title compound. The product was used without further purification. MS (m/z): 563.5 [M+H$^+$]

Step E: Synthesis of (*S*)-*tert*-butyl 4-(2-(5-(4-(2-(2-(benzo[*d*]oxazole-2-carbonyl)pyrrolidin-1-yl)-2-oxoethylcarbamoyl)quinolin-6-yl)picolinamido)ethyl)piperazine-1-carboxylate (**6**). 2-(6-(6-(2-(4-(*tert*-Butoxycarbonyl)piperazin-1-yl)ethylcarbamoyl)pyridin-3 -yl)quinoline-4-carboxamido)acetic acid (**23**, 30.9 mg, 0.055 mmol), (*S*)-benzo[*d*]oxazol-2-yl(pyrrolidin-2-yl)methanone 2,2,2-trifluoroacetate (**25**, 22.2 mg, 0.071 mmol), HATU (31.4 mg, 0.082 mmol), and DIPEA (18.7 µL, 0.110 mmol) were reacted in dry DMF (550 µL) according to the synthesis of *tert*-butyl 3-(4-(2-methoxy-2-oxoethylcarbamoyl)quinolin-7-yl)benzoate (**11**) to afford the title compound after reverse phase HPLC. MS (m/z): 761.5 [M+H$^+$]

Step F: Synthesis of (*S*)-*tert*-butyl 2-(benzo[*d*]oxazole-2-carbonyl)pyrrolidine-1-carboxylate (**24**) (2*S*)-*tert*-butyl 2-(benzo[*d*]oxazol-2-yl(hydroxy)methyl)pyrrolidine-1-carboxylate (**17**, 185.0 mg, 0.581 mmol) was reacted with DMP (492.9 mg, 1.162 mmol) in DCM (5.8 mL) according to the synthesis of (*S*)-*tert*-butyl 3-(4-(2-(2-(benzo[*d*]oxazole-2-carbonyl)pyrrolidin-1-yl)-2-oxoethylcarbamoyl)quinolin-7-yl)benzoate (**14**) to yield the title compound after flash column chromatography (elution with EA/ heptane). MS (m/z): 316.8 [M+H$^+$]

Step G: Synthesis of (*S*)-benzo[*d*]oxazol-2-yl(pyrrolidin-2-yl)methanone 2,2,2-trifluoroacetate (**25**) (*S*)-*tert*-Butyl 2-(benzo[*d*]oxazole-2-carbonyl)pyrrolidine-1-carboxylate (**24**, 108.0 mg, 0.341 mmol) was reacted with TFA (526.0 µL, 6.828 mmol) in dry DCM (1.7 mL) according to the synthesis of (2*S*)-2-(benzo[*d*]oxazol-2-yl(hydroxy)methyl)pyrrolidinium 2,2,2-trifluoroacetate to yield the title compound. MS (m/z): 216.6 [M+H$^+$]

Preparation of Boc-protected precursor. Intermediate (**7**), for the synthesis of DOTA-compound 3BP-4810

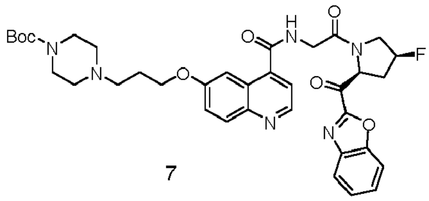

7

The synthesis of the title compound is depicted in the following reaction scheme.

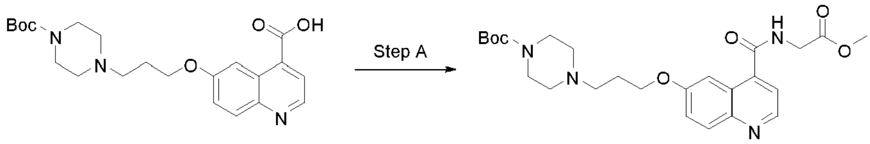


26 27

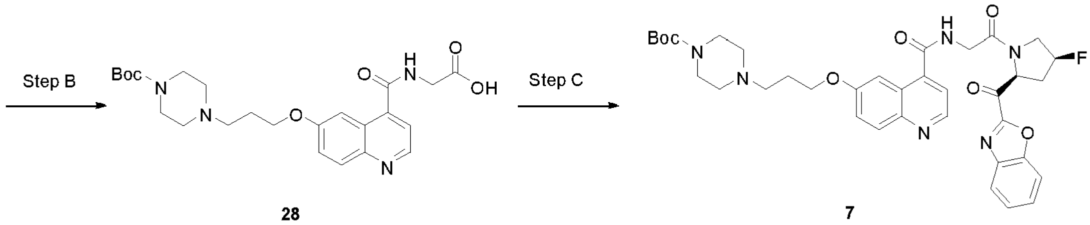


28 7

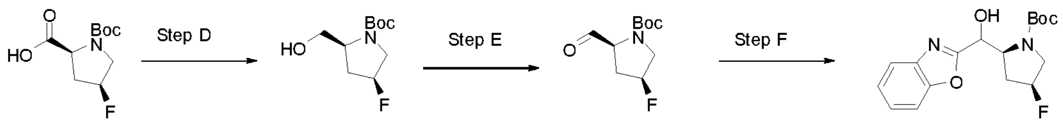


29 30 31 32

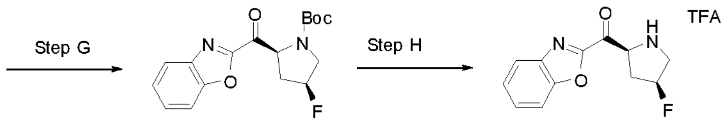


33 34

Step A: Synthesis of *tert*-butyl 4-(3-(4-(2-methoxy-2-oxoethylcarbamoyl)quinolin-6-yloxy)propyl)piperazine-1-carboxylate (**27**). 6-(3 -(4-(*tert*-Butoxycarbonyl)piperazin-1-yl)propoxy)quinoline-4-carboxylic acid (**26**, 160 mg, 0.385 mmol), glycine methyl ester hydrochloride (67.7 mg, 0.539 mmol), HATU (219.6 mg, 0.578 mmol), and DIPEA (196.5 μL, 1.155 mmol) were reacted according to the synthesis of *tert*-butyl 3-(4-(2-methoxy-2-oxoethylcarbarnoyl)quinolin-7-yl)benzoate (11) to yield the title compound after flash column chromatography (elution with DCM/ MeOH). MS (m/z): 487.3 [M+H$^+$]

Step B: Synthesis of 2-(6-(3-(4-(*tert*-butoxycarbonyl)piperazin-1-yl)propoxy)quinoline-4-carboxamido)acetic acid (**28**). *tert*-Butyl 4-(3-(4-(2-methoxy-2-oxoethylcarbarnoyl)quinolin-6-yloxy)propyl)piperazine-1-carboxylate (**27**, 202.0 mg, 0.415 mmol) was reacted with lithiumhydroxide monohydrate (34.9 mg, 0.830 mmol) according to the synthesis of 2-(7-(3-(*tert*-butoxycarbonyl)phenyl)quinoline-4-carboxamido)acetic acid (**12**) to yield the title compound. The product was used without further purification. MS (m/z): 473.3 [M+H$^+$]

Step C: Synthesis of *tert*-butyl 4-(3-(4-(2-((2S,4S)-2-(benzo[*d*]oxazole-2-carbonyl)-4-fluoropyrrolidin-1-yl)-2-oxoethylcarbamoyl)quinolin-6-yloxy)propyl)piperazine-1-carboxylate (**7**). 2-(6-(3-(4-(*tert*-Butoxycarbonyl)piperazin-1-yl)propoxy)quinoline-4-carboxamido)acetic acid (**28**, 100 mg, 0.212 mmol), benzo[*d*]oxazol-2-yl((2*S*,4*S*)-4-fluoropyrrolidin-2-yl)methanone 2,2,2-trifluoroacetate (**34**, 69.4 mg, 0.296 mmol), HATU (112.7 mg, 0.296 mmol), and DIPEA (72.0 μL, 0.423 mmol) were reacted according to the synthesis of (S)-*tert*-butyl 4-(2-(5-(4-(2-(2-(benzo[*d*]oxazole-2-carbonyl)pyrrolidin-1-yl)-2-oxoethylcarbamoyl)quinolin-6-yl)picolinamido)ethyl)piperazine-1-carboxylate (**6**) and yielded the title compound after reverse phase HPLC. MS (m/z): 689.2 [M+H$^+$]

Step D: Synthesis of (2*S*,4*S*)-*tert*-butyl 4-fluoro-2-(hydroxymethyl)pyrrolidine-1-carboxylate (**30**). Lithium aluminium hydride (195.2 mg, 5.140 mmol) was added at 0 °C to a solution of (2*S*,4*S*)-1-(*tert*-butoxycarbonyl)-4-fluoropyrrolidine-2-carboxylic acid (1000.0 mg, 4.290 mmol) in dry THF (4.3 mL) and the mixture was allowed to reach rt. After 30 min, Sodium sulfate decahydrate was added to quench the reaction and stirring was continued for 30 min. The mixture was filtrated and the filtrate was carefully evaporated *in vacuo* (p > 120 mbar). MS (m/z): 219.8 [M+H$^+$]

Step E: Synthesis of (2*S*,4*S*)-*tert*-butyl 4-fluoro-2-formylpyrrolidine-1-carboxylate (**31**). DMP (1021.0 mg, 2.408 mmol) was added to a solution of (2*S*,4*S*)-*tert*-butyl 4-fluoro-2-(hydroxymethyl)pyrrolidine-1-carboxylate (**30**, 480.0 mg, 2.189 mmol) in DCM (20 mL). The mixture was stirred for 3 h at rt. After complete conversion, the volatiles were removed in vacuo and the crude product was subject to flash column chromatography on silica gel (elution with DCM → EA). MS (m/z): 216.0 [M-H$^+$]

Step F: Synthesis of (2*S*,4*S*)-*tert*-butyl 2-((*R*)-benzo[*d*]oxazol-2-yl(hydroxy)methyl)-4-fluoropyrrolidine-1-carboxylate (**32**). (2*S*,4*S*)-*tert*-Butyl 4-fluoro-2-formylpyrrolidine-1-carboxylate (**31**, 480.0 mg, 2.210 mmol), benzo[d]oxazole (315.8 mg, 2.651 mmol), and isopropylmagnesium chloride (2 M in diethyl ether, 1590.9 μL, 3.182 mmol) were reacted according to the synthesis of (2*S*)-*tert*-butyl 2-(benzo[*d*]oxazol-2-yl(hydroxy)methyl)pyrrolidine-1-carboxylate (**17**) to yield the title compound after flash column chromatography on silica gel (elution with heptane/ EA). MS (m/z): 337.0 [M+H$^+$]

Step G: Synthesis of (2*S*,4*S*)-*tert*-butyl 2-(benzo[*d*]oxazole-2-carbonyl)-4-fluoropyrrolidine-1-carboxylate (**33**). (2*S*,4*S*)-*tert*-Butyl 2-((*R*)-benzo[d]oxazol-2-yl(hydroxy)methyl)-4-fluoropyrrolidine-1-carboxylate (**32**, 180 mg, 0.535 mmol) was reacted with DMP (249.7 mg, 0.589 mmol) according to the synthesis of (*S*)-*tert*-butyl 2-(benzo[*d*]oxazole-2-carbonyl)pyrrolidine-1-carboxylate (**24**) to afford the title compound after flash column chromatography (elution with heptane/ EA). MS (m/z): 334.8 [M+H$^+$]

Step H: Synthesis of benzo[*d*]oxazol-2-yl((2*S*,4*S*)-4-fluoropyrrolidin-2-yl)methanone 2,2,2-trifluoroacetate (**34**). (2*S*,4*S*)-*tert*-Butyl 2-(benzo[*d*]oxazole-2-carbonyl)-4-fluoropyrrolidine-1-carboxylate (**33**, 140 mg, 0.419 mmol) was reacted with TFA (645.2 μL, 8.375 mmol) according to the synthesis of (2S)-2-(benzo[*d*]oxazol-2-yl(hydroxy)methyl)pyrrolidinium 2,2,2-trifluoroacetate (**18**). MS (m/z): 234.9 [M+H$^+$]

The Boc protected precursor, Intermediate (**5**), for the preparation of 3BP-4808 was prepared following the procedure described for intermediate (**7**), where in step C building-block (**25**) was used instead of (**34**).

Compound 3BP-3412 was prepared in analogy to these syntheses by using the corresponding building block with two fluorine-substituents in the 4-position of the pyrrolidine.

Preparation of Boc-protected precursor. Intermediate (**8**), for the synthesis of 3BP-4811

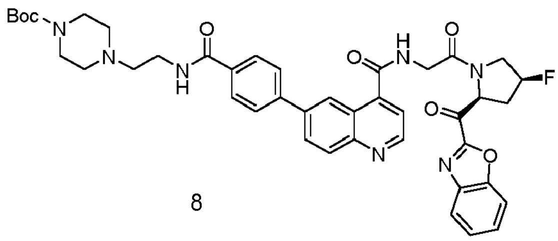

8

The synthesis of the title compound is depicted in the following reaction scheme.

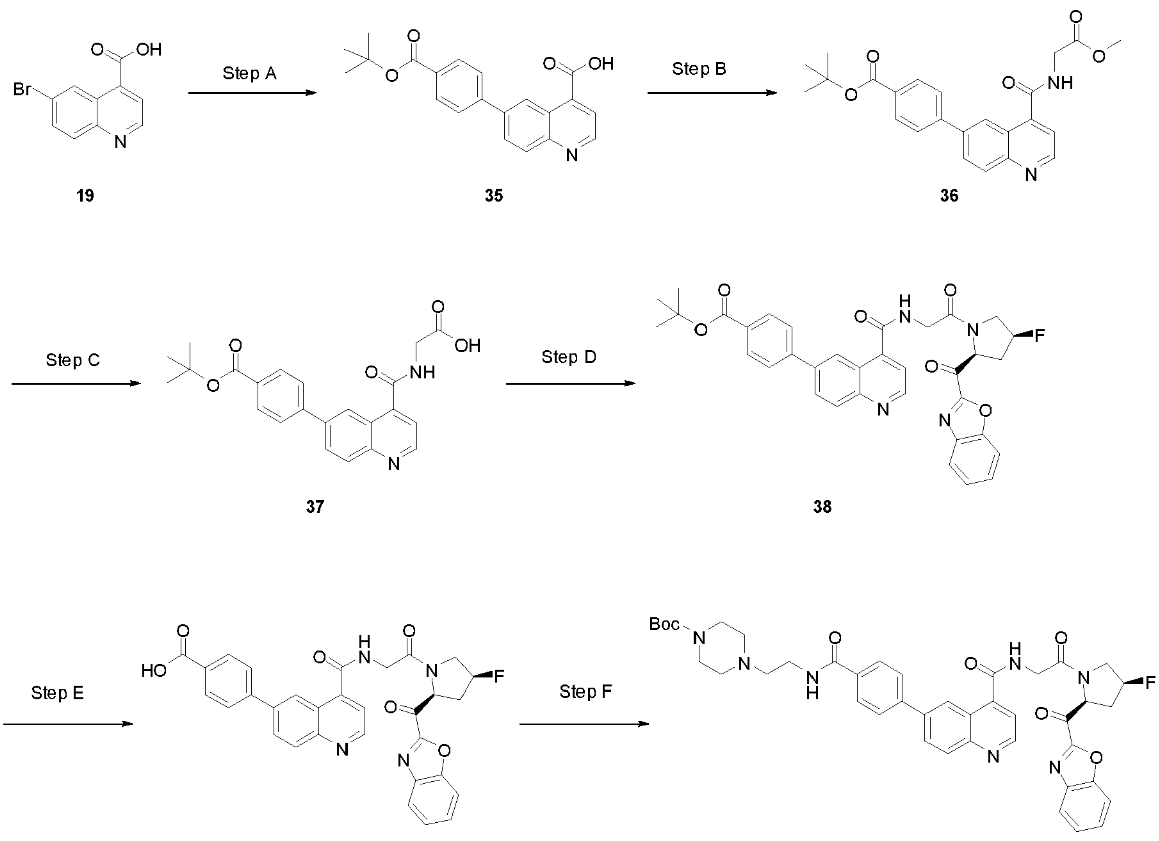


19 35 36

37 38

39 8

Step A: Synthesis of 6-(4-(*tert*-butoxycarbonyl)phenyl)quinoline-4-carboxylic acid (35). 6-Bromoquinoline-4-carboxylic acid (**19,** 800.0 mg, 3.174 mmol), 4-(*tert*-butoxycarbonyl)phenylboronic acid (1057.1 mg, 4.761 mmol), bis(triphenylphosphine)palladium(II) dichloride (603.4 mg, 1.587 mmol), and aqueous 2 M $NaHCO_3$ solution (9.5 mL, 19.043 mmol) were reacted according to the synthesis of 7-(3-(*tert*-butoxycarbonyl)phenyl)quinoline-4-carboxylic acid (10) to afford the title compound. It was used in the next step without further purification. MS (m/z): 350.3 [$M+H^+$]

Step B: Synthesis of *tert*-butyl 4-(4-(2-methoxy-2-oxoethylcarbamoyl)quinolin-6-yl)benzoate (**36**). 6-(4-(*tert*-Butoxycarbonyl)phenyl)quinoline-4-carboxylic acid (**35**, 554.4 mg, 1.587 mmol), glycine methyl ester hydrochloride (278.9 mg, 2.222 mmol), HATU (905.0 mg, 2.380 mmol), and DIPEA (1079.4 μL, 6.347 mmol) were reacted according to the synthesis of *tert*-butyl 3-(4-(2-methoxy-2-oxoethylcarbamoyl)quinolin-7-yl)benzoate (**11**) to yield the title compound after flash column chromatography (elution with DCM/ MeOH). MS (m/z): 421.5 [$M+H^+$]

Step C: Synthesis of 2-(6-(4-(*tert*-butoxycarbonyl)phenyl)quinoline-4-carboxamido)acetic acid (**37**). *tert*-Butyl 4-(4-(2-methoxy-2-oxoethylcarbamoyl)quinolin-6-yl)benzoate (**36**, 1334.5 mg, 3.174 mmol) was reacted with lithiumhydroxide monohydrate (266.4 mg, 6.348 mmol) according to the synthesis of 2-(7-(3-(*tert*-butoxycarbonyl)phenyl)quinoline-4-carboxamido)acetic acid (**12**) to yield the title compound. The product was used without further purification. MS (m/z): 407.0 [$M+H^+$]

Step D: Synthesis of *tert*-butyl 4-(4-(2-((2*S*,4*S*)-2-(benzo[*d*]oxazole-2-carbonyl)-4-fluoropyrrolidin-1-yl)-2-oxoethylcarbamoyl)quinolin-6-yl)benzoate (**38**). 2-(6-(4-(*tert*-Butoxycarbonyl)phenyl)-quinoline-4-carboxamido)acetic acid (**37**, 128.9 mg, 0.317 mmol), benzo[*d*]oxazol-2-yl((2*S*,4*S*)-4-fluoropyrrolidin-2-yl)methanone 2,2,2-trifluoroacetate (**34**, 89.1 mg, 0.381 mmol), HATU (180.9 mg, 0.476 mmol), and DIPEA (107.9 μL, 0.634 mmol) were reacted according to the synthesis of (*S*)-*tert*-butyl 4-(2-(5-(4-(2-(2-(benzo[*d*]oxazole-2-carbonyl)pyrrolidin-1-yl)-2-oxoethylcarbamoyl)quinolin-6-yl)picolinamido)ethyl)piperazine-1-carboxylate (**6**) and yielded the title compound after flash chromatography on silica gel (elution with DCM/ MeOH). MS (m/z): 623.1 [$M+H^+$]

Step E: Synthesis of 4-(4-(2-((2S,4S)-2-(benzo[*d*]oxazole-2-carbonyl)-4-fluoropyrrolidin-1-yl)-2-oxoethylcarbamoyl)quinolin-6-yl)benzoic acid (**39**). *tert*-Butyl 4-(4-(2-((2*S*,4*S*)-2-(benzo[*d*]oxazole-2-carbonyl)-4-fluoropyrrolidin-1-yl)-2-oxoethylcarbamoyl)quinolin-6-yl)benzoate (**38**, 184.0 mg, 0.296 mmol) was reacted with TFA (455.3 μL,

5.910 mmol) according to the synthesis of (S)-3-(4-(2-(2-(benzo[*d*]oxazole-2-carbonyl)pyrrolidin-1-yl)-2-oxoethylcarbamoyl)quinolin-7-yl)benzoic acid (**15**) to afford the title compound. It was used in the next step without further purification. MS (m/z): 567.2 [M+H$^+$]

Step F: Synthesis of *tert*-butyl 4-(2-(4-(4-(2-((2*S*,4*S*)-2-(benzo[*d*]oxazole-2-carbonyl)-4-fluoropyrrolidin-1-yl)-2-oxoethylcarbamoyl)quinolin-6-yl)benzamido)ethyl)piperazine-1-carboxylate (**8**). 4-(4-(2-((2S,4S)-2-(Benzo[*d*]oxazole-2-carbonyl)-4-fluoropyrrolidin-1-yl)-2-oxoethylcarbamoyl)quinolin-6-yl)benzoic acid (**39**, 167.4 mg, 0.295 mmol), *tert*-butyl 4-(2-aminoethyl)piperazine-1-carboxylate (115.2 mg, 0.502 mmol), HATU (168.5 mg, 0.443 mmol), and DIPEA (100.5 μL, 0.591 mmol) were reacted in DMF (2.95 mL) according to the synthesis of (*S*)-*tert*-butyl 4-(2-(3-(4-(2-(2-(benzo[*d*]oxazole-2-carbonyl)pyrrolidin-1-yl)-2-oxoethylcarbamoyl)quinolin-7-yl)benzamido)ethyl)piperazine-1-carboxylate (**4**) to afford the title compound after reverse phase HPLC. MS (m/z): 778.5 [M+H$^+$]

**Final conversion of eight Boc-protected precursors (Intermediates 1-8) to the compounds of the invention**

General procedure for the conversion of Boc-protected compounds (**1-8**) to DOTA-functionalized compounds: 1) Boc-deprotection and 2) DOTA-coupling:

1) The respective compound (**1-8**) is dissolved in a mixture of 20% TFA and 80% DCM and stirred for 20 min. After complete conversion, the mixture is concentrated to dryness and used without further purification.

2) The crude material from 1) was dissolved in dry DMSO (typically as a 0.05-0.1 M solution) and adjusted to pH 5-7 with DIPEA. DOTA-NHS ester (1.5 eq) is added as solid it was stepwise neutralized to pH 7 by addition of small amounts of DIPEA. The solution was stirred at RT for 2 h. After complete conversion, the DMSO solution was subject to HPLC purification to afford the respective title compound.

The compounds prepared, starting from Intermediates 1-8 and following the procedure described above, are shown in Table 4:

Table 4: Compounds prepared from precursor Intermediates 1-8

| Intermediate | Prepared compound | Amount [mg] | MS (m/z): [M+H$^+$] |
|---|---|---|---|
| **1** | 3BP-4663 | 12.5 | 1046.5 |
| **2** | 3BP-4664 | 12.0 | 1046.5 |
| **3** | 3BP-4665 | 19.7 | 1046.5 |
| **4** | 3BP-4694 | 11.0 | 1046.5 |
| **5** | 3BP-4808 | 5.0 | 957.5 |
| **6** | 3BP-4809 | 10.5 | 975.4 |
| **7** | 3BP-4810 | 14.1 | 1064.5 |
| **8** | 3BP-4811 | 5.9 | 1047.5 |

More detailed results from the mass-spectrometry analysis (calculated vs. found) for title compounds including compounds of the present invention are given in Table 7, which appears after Example 8.

**Example 2c: Preparation of Compound 3BP-3376**

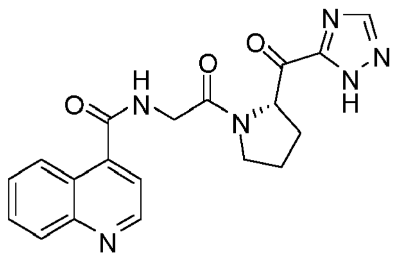

(*S*)-*N*-(2-(2-(1*H*-1,2,4-triazole-5-carbonyl)pyrrolidin-1-yl)-2-oxoethyl)quinoline-4-carboxamide

The synthesis of the title compound is depicted in the following reaction scheme.

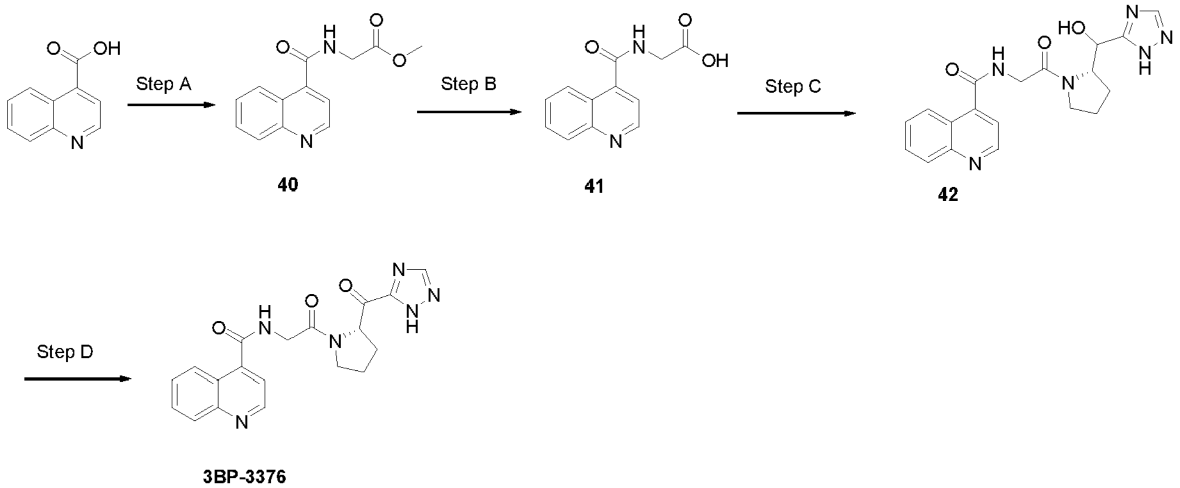


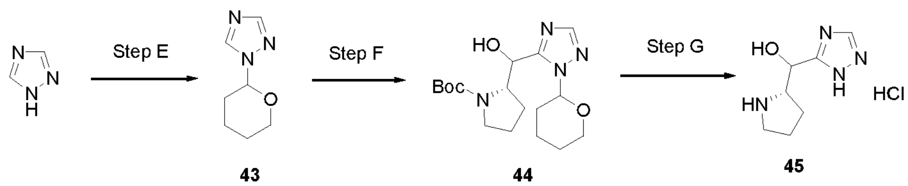


Step A: Synthesis of methyl 2-(quinoline-4-carboxamido)acetate (**40**)

A solution of quinoline-4-carboxylic acid (3.29 g, 18.99 mmol) and methyl 2-aminoacetate hydrochloride (2.50 g, 19.98 mmol) in dry DMF (25 mL) were cooled to 0 °C. At this temperature, HATU (8.36 g, 21.98 mmol) and DIPEA (13.59 mL, 79.94 mmol) were added and the resulting solution was stirred at RT over night. After complete conversion of the starting material, the mixture was evaporated to dryness. The remaining residue was re-dissolved in DCM and washed with water. The organic phase was dried ($Na_2SO_4$) and concentrated *in vacuo.* The crude product was purified via flash chromatography on silica gel (elution with DCM/ MeOH) to give the title compound. MS (m/z): 245.2 [$M+H^+$]

Step B: Synthesis of 2-(quinoline-4-carboxamido)acetic acid (**41**)

Methyl 2-(quinoline-4-carboxamido)acetate (**40**, 4.63 g, 18.96 mmol) was dissolved in methanol (9.8 mL) and water (9.8 mL). 1 M aqueous NaOH solution (28.44 mL, 28.44 mmol) was added and the mixture was stirred at RT over night. After complete consumption of the starting material, the mixture was concentrated *in vacuo.* The remaining residue was dissolved in a minimum amount of water. The aqueous solution was washed with DCM twice. Subsequently, the organic phase was acidified using conc. HCl. The product was collected by filtration and dried *in vacuo.* MS (m/z): 230.8 [$M+H^+$]

Step C: Synthesis of *N*-(2-((2*S*)-2-(hydroxy(1*H*-1,2,4-triazol-5-yl)methyl)pyrrolidin-1-yl)-2-oxoethyl)quinoline-4-carboxamide (**42**)

A solution of 2-(quinoline-4-carboxamido)acetic acid (**41**, 82.1 mg, 0.36 mmol) and (S)-pyrrolidin-2-yl(1*H*-1,2,4-triazol-5-yl)methanol (**45**, 50.0 mg, 0.30 mmol) in dry DMF (3 mL) was cooled to 0 °C. HATU (146.9 mg, 0.39 mmol) and DIPEA (202.2 μL, 1.19 mmol) were added at this temperature and the solution was stirred at RT over night. After complete consumption of the starting material, the mixture was evaporated to dryness and the crude product was purified by flash chromatography on silica gel (elution with DCM/ MeOH) to give the title compound. MS (m/z) 381.1 $[M+H^+]$

Step D: Synthesis of (*S*)-*N*-(2-(2-(1*H*-1,2,4-triazole-5-carbonyl)pyrrolidin-1-yl)-2-oxoethyl)quinoline-4-carboxamide (3BP-3376)

Dess-Martin periodinane (196.7 mg, 0.46 mmol) was added to a solution of *N*-(2-((2*S*)-2-(hydroxy(1*H*-1,2,4-triazol-5-yl)methyl)pyrrolidin-1-yl)-2-oxoethyl)quinoline-4-carboxamide (**42**, 88.2 mg, 0.23 mmol) in DCM (820 μL) and the mixture was stirred over night at RT. After complete consumption of the starting material, a solution of $NaHCO_3/Na_2S_2O_3$ (10 %/ 10 %; 850 μL) was added and the phases were separated. The organic phase was washed with aqueous $Na_2S_2O_3$ solution (25 %, 850 μL) and brine (850 μL), dried ($Na_2SO_4$), filtered and evaporated to dryness. The crude was subject to HPLC purification. MS (m/z) 429.3 [M+H+]

Step E: Synthesis of 1-(tetrahydro-2*H*-pyran-2-yl)-1*H*-1,2,4-triazole (**43**)

3,4-Dihydro-2*H*-pyran (4.57 mL, 50 mmol) and 4-methylbenzene-1-sulfonic acid (480 mg, 2.5 mmol) were added to a solution of 1*H*-1,2,4-triazole (1.73 g, 25 mmol) in dry THF (12 mL) under an atmosphere of nitrogen. The reaction mixture was stirred for 4 h at 70 °C. After complete consumption of starting material, the mixture was diluted with EA (6 mL) and washed with aqueous $NaHCO_3$ solution (1M, 10 mL). The organic phase was dried ($Na_2SO_4$), filtrated and evaporated to dryness. The crude product was purified by flash chromatography on silica gel (elution with heptane/ EA) to give the title compound. MS (m/z) 153.9 $[M+H^+]$

Step F: Synthesis of (2*S*)-*tert*-butyl 2-(hydroxy(1-(tetrahydro-2*H*-pyran-2-yl)-1*H*-1,2,4-triazol-5-yl)methyl)pyrrolidine-1-carboxylate (**44**)

A Schlenck flask was evacuated and ventilated with argon. 1-(tetrahydro-2*H*-pyran-2-yl)-1*H*-1,2,4-triazole (**43**, 490.4 mg, 3.2 mmol) and dry THF (7.5 mL) were added and cooled to -78 °C. At this temperature, BuLi (2.5 M in hexanes, 1280 μL, 3.2 mmol) was added and stirring at -78 °C was continued for 1 h. Then, a solution of *tert*-butyl (2*S*)-2-formylpyrrolidine-1-carboxylate (500 mg, 2.7 mmol) in dry THF (2.5 mL) was added and stirring at -78 °C was continued for 3 h. The reaction was quenched by addition of sat. aqueous $NH_4Cl$ solution (2 mL) and stirring for 1 h. The mixture is extracted with EA (3 x 3 mL). The combined organic layers were washed with brine, dried ($Na_2SO_4$), and evaporated to dryness. The crude product was purified by flash chromatography on silica gel (elution with heptane/ EA) to give the title compound. MS (m/z): 353.6 $[M+H^+]$

Step G: Synthesis of (*S*)-pyrrolidin-2-yl(1*H*-1,2,4-triazol-5-yl)methanol hydrochloride (**45**)

A solution of (2S)-*tert*-butyl 2-(hydroxy(1-(tetrahydro-2*H*-pyran-2-yl)-1*H*-1,2,4-triazol-5-yl)methyl)pyrrolidine-1-carboxylate (**44**, 799.3 mg, 2.27 mmol) in a mixture of 3 M HCl in methanol (3 mL) and methanol (3 mL) was stirred at room temperature. After complete consumption of starting material (4 h, TLC), the mixture was evaporated and used without further purification. MS (m/z) 168.7 $[M+H^+]$

**Example 2d: Preparation of Compound 3BP-3707**

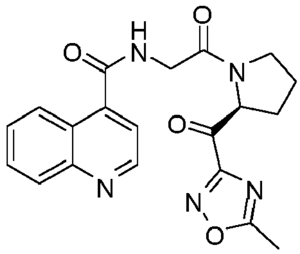

(*S*)*N*-(2-(2-(5-methyl-1,2,4-oxadiazole-3-carbonyl)pyrrolidin-1-yl)-2-oxoethyl)quinoline-4-carboxamide

The synthesis of the title compound is depicted in the following reaction scheme.

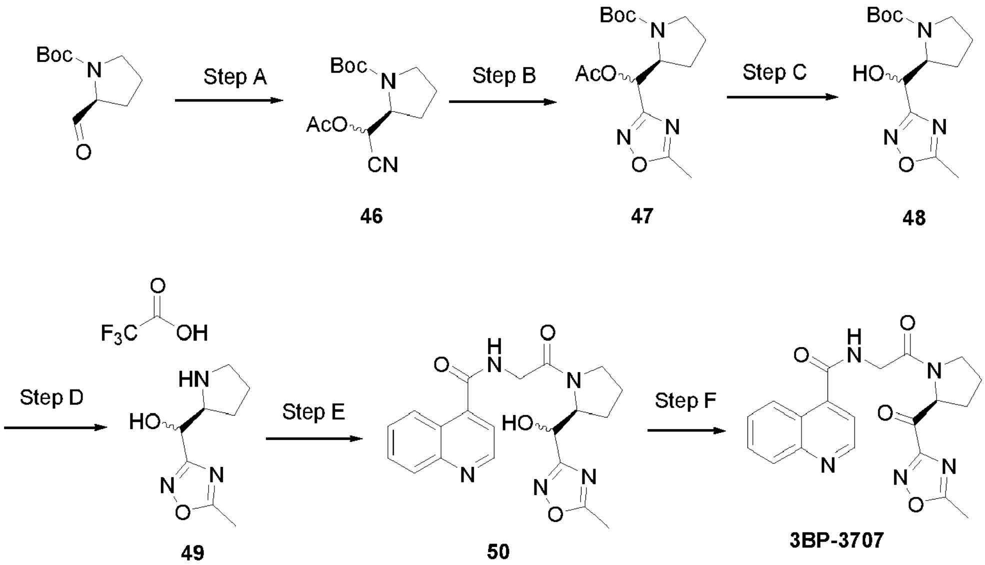


46 47 48

49 50 3BP-3707

Step A: Synthesis of (*S*)-*tert*-butyl 2-(acetoxy(cyano)methyl)pyrrolidine-1-carboxylate (**46**)

Acetic anhydride (379.5 μL, 4.02 mmol) and sodium cyanide (59.0 mg, 1.21 mmol) were added to a solution of (*S*)-*tert*-butyl 2-formylpyrrolidine-1-carboxylate (200.0 mg, 1.00 mmol) in DME (5 mL). The mixture was stirred at RT over night. After complete consumption of starting material, it was extracted with EA (2 x 25 mL). The combined organic phases were washed with water (2 x 10 mL), dried ($Na_2SO_4$) and evaporated to dryness. The crude product was used without further purification. MS (m/z): 286.1 [$M+NH_4^+$]

Step B: Synthesis of (*S*)-*tert*-butyl 2-(acetoxy(5-methyl-1,2,4-oxadiazol-3-yl)methyl)pyrrolidine-1-carboxylate (**47**)

(*S*)-*tert*-Butyl 2-(acetoxy(cyano)methyl)pyrrolidine-1-carboxylate (**46**, 269.3 mg, 1.00 mmol) was dissolved in EtOH/ $H_2O$ = 5 :1 (2.1 mL). Hydroxylamine hydrochloride (97.6 mg, 1.41 mmol) and sodium acetate (205.8 mg, 2.51 mmol) were added and the mixture was stirred at 40 °C for 4.5 h. Subsequently, the mixture was evaporated to dryness. The remaining residue was re-dissolved in EA (2.5 mL) and washed with brine (2 x 5 mL). The organic phase was dried ($Na_2SO_4$) and evaporated to dryness. The resulting intermediate was used without further purification.

The intermediate was dissolved in toluene (0.7 mL) and acetic anhydride (286.5 μL, 3.01 mmol) was added. the mixture was stirred at 110 °C over night. After complete conversion, the mixture was evaporated to dryness and the crude product was subject to flash chromatography on silica gel (eluent: DCM/ MeOH) to give the title compound. MS (m/z): 225.9 [$M-Boc+H^+$]

Step C: Synthesis of (*S*)-*tert*-butyl 2-(hydroxy(5-methyl-1,2,4-oxadiazol-3-yl)methyl)pyrrolidine-1-carboxylate (**48**)

A solution of (*S*)-*tert*-butyl 2-(acetoxy(5-methyl-1,2,4-oxadiazol-3-yl)methyl)pyrrolidine-1-carboxylate (**47**, 218.3 mg, 0.67 mmol) in a mixture of methanolic ammonia/ MeOH = 1: 1 (2 mL) was stirred at RT over night. After complete conversion the solution was concentrated in vacuo. The crude product was used without further purification. MS (m/z): 283.9 [$M+H^+$]

Step D: Synthesis of (*S*)-(5-methyl-1,2,4-oxadiazol-3-yl)(pyrrolidin-2-yl)methanol 2,2,2-trifluoroacetate (**49**)

2,2,2-Trifluoroacetic acid (1.03 mL, 13.42 mmol) was added to a solution of (*S*)-*tert*-butyl 2-(hydroxy(5-methyl-1,2,4-oxadiazol-3-yl)methyl)pyrrolidine-1-carboxylate (**48**, 190.1 mg, 0.67 mmol) in DCM (1.34 mL) and stirred at RT over night. After complete conversion the solution was conenctrated *in vacuo* to yield the title compound. MS (m/z): 184.0 [$M+H^+$]

Step E: Synthesis of (*S*)-*N*-(2-(2-(hydroxy(5-methyl-1,2,4-oxadiazol-3-yl)methyl)pyrrolidin-1-yl)-2-oxoethyl)quinoline-4-carboxamide (**50**)

(*S*)-(5-Methyl-1,2,4-oxadiazol-3-yl)(pyrrolidin-2-yl)methanol 2,2,2-trifluoroacetate (**49**, 100 mg. 0.42 mmol) was reacted with 2-(quinoline-4-carboxamido)acetic acid (**41**, 121.63 mg, 0.35 mmol) according to the synthesis of *N*-(2-((2*S*)-2-(hydroxy(1*H*-1,2,4-triazol-5-yl)methyl)pyrrolidin-1-yl)-2-oxoethyl)quinoline-4-carboxamide (**42**) to give the title compound which was used without further purification. MS (m/z): 396.2 [$M+H^+$]

Step F: Synthesis of (*S*)-*N*-(2-(2-(5-methyl-1,2,4-oxadiazole-3-carbonyl)pyrrolidin-1-yl)-2-oxoethyl)quinoline-4-carboxamide (3BP-3707)

(*S*)-*N*-(2-(2-(hydroxy(5-methyl-1,2,4-oxadiazol-3-yl)methyl)pyrrolidin-1-yl)-2-oxoethyl)quinoline-4-carboxamide (**50**, 137.6 mg, 0.35 mmol) was reacted with Dess-Martin-Periodinane (295.2 mg, 0.70 mmol) according to the synthesis of (*S*)-*N*-(2-(2-(1*H*-1,2,4-triazole-5-carbonyl)pyrrolidin-1-yl)-2-oxoethyl)quinoline-4-carboxamide (3BP-3376). The crude product was purified by HPLC. MS (m/z): 394.3 [$M+H^+$]

**Example 2e: Preparation of Compound 3BP-3295**

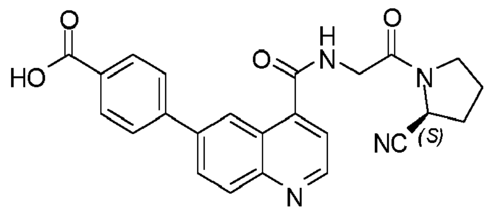

((*S*)-4-(4-(2-(2-cyanopyrrolidin-1-yl)-2-oxoethylcarbamoyl)quinolin-6-yl)benzoic acid

The synthesis of the title compound is depicted in the following reaction scheme.

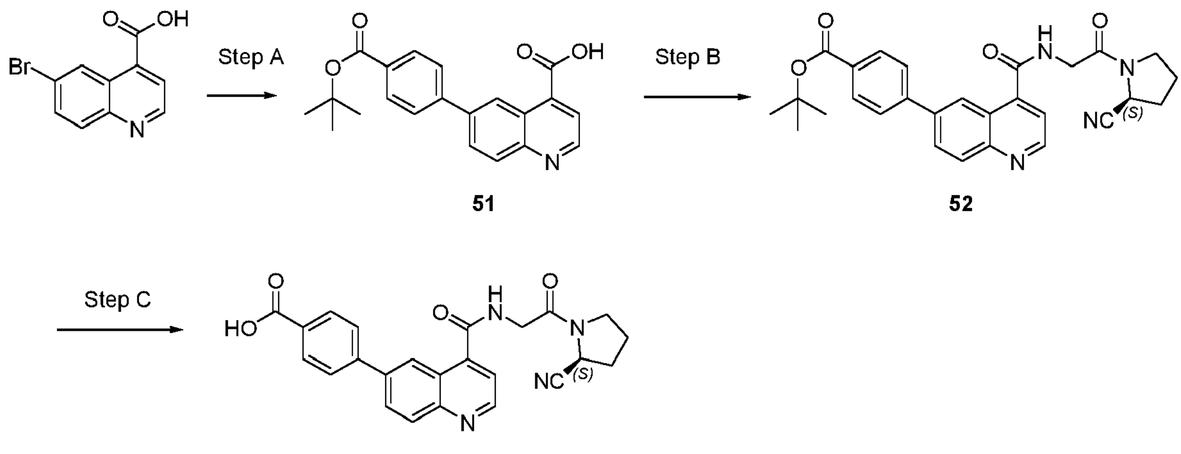


51

52

3BP-3295

Step A: Synthesis of 6-(4-(*tert*-butoxycarbonyl)phenyl)quinoline-4-carboxylic acid (**51**)

6-Bromoquinoline-4-carboxylic acid (50.0 mg, 0.20 mmol), 4-(*tert*-butoxycarbonyl)phenylboronic acid (66.1 mg,

0.30 mmol) and bis(triphenylphosphine)palladium(II) dichloride (3.8 mg, 0.01 mmol) were placed in a flask and evacuated. Subsequently, the flask was ventilated with Nitrogen. The solids were dissolved in DME (2 mL) and 2 M aqueous $NaHCO_3$ solution (595.1 µL, 1.19 mmol) was added and the resulting mixture was stirred over night at 80 °C. After full consumption of starting material, the mixture was cooled to RT and evaporated to dryness. The crude product was used without further purification. MS (m/z): 350.3 [$M+H^+$].

Step B: Synthesis of (*S*)-*tert*-butyl 4-(4-(2-(2-cyanopyrrolidin-1-yl)-2-oxoethylcarbamoyl)quinolin-6-yl)benzoate (**52**)

A solution of 6-(4-(*tert*-butoxycarbonyl)phenyl)quinoline-4-carboxylic acid (**51**, 69.3 mg, 0.20 mmol), (S)-2-(2-cyanopyrrolidin-1-yl)-2-oxoethanaminium 4-methylbenzenesulfonate [J. Med. Chem. 2017, 60, 8385.] (118.4 mg, 0.24 mmol), HATU (117.6 mg, 0.31 mmol), and DIPEA (202.4 µL, 1.19 mmol) in dry DMF (1.25 mL) were stirred at RT over night. After complete conversion of starting material, the mixture was concentrated *in vacuo*. The crude product was used without further purification. MS (m/z): 485.4 [$M+H^+$].

Step C: Synthesis of ((*S*)-4-(4-(2-(2-cyanopyrrolidin-1-yl)-2-oxoethylcarbamoyl)quinolin-6-yl)benzoic acid (3BP-3295)

Water (475 µL) was added to a solution of (*S*)-*tert*-butyl 4-(4-(2-(2-cyanopyrrolidin-1-yl)-2-oxoethylcarbamoyl)quinolin-6-yl)benzoate **52** in $Et_2O$ (1450 µL). The mixture was cooled to 0 °C and conc. HCl (475 µL) was added over a period of 10 min and stirring at 0 °C was continued for 1 h. Subsequently, the mixture was evaporated to dryness (bath temperature 25 °C). The residue was suspended in $Et_2O$, sonicated and evaporated. Purification of the crude product by reverse phase HPLC afforded the title compound. MS (m/z): 429.1 [$M+H^+$].

**Example 2f: Preparation of Compound 3BP-4084**

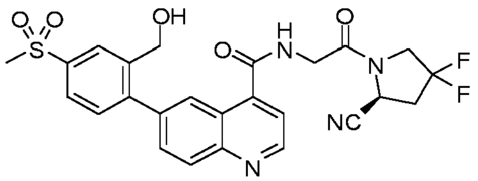

(*S*)-*N*-(2-(2-cyano-4,4-difluoropyrrolidin-1-yl)-2-oxoethyl)-6-(2-(hydroxymethyl)-4-(methylsulfonyl)phenyl)quinoline-4-carboxamide

The synthesis of the title compound is depicted in the following reaction scheme.

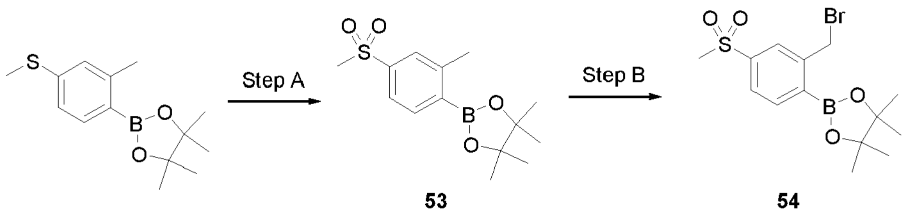


53 54

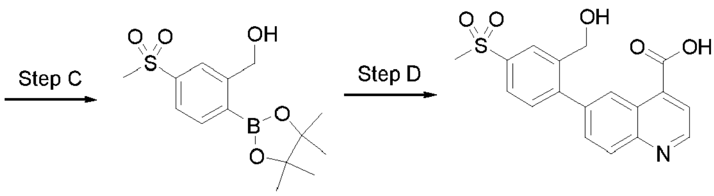


55 56

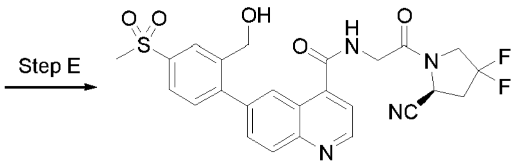


3BP-4084

Step A: Synthesis of 4,4,5,5-tetramethyl-2-(2-methyl-4-(methylsulfonyl)phenyl)-1,3,2-dioxaborolane (53)

To a solution of 4,4,5,5-tetramethyl-2-(2-methyl-4-(methylthio)phenyl)-1,3,2-dioxaborolane (200.0 mg, 0.76 mmol) in methanol (22.5 mL) was added a solution of $NaOI_4$ (300.0 mg, 1.40 mmol) in water (9 mL). The mixture was stirred over night at RT. After complete conversion, the mixture was filtrated. $KMnO_4$ was added to the filtrate and it was stirred for 10 min. Subsequently, the mixture was filtrated again. The methanol was removed in vacuo and the aqueous solution was extracted with EA. The combined organic phases were dried ($Na_2SO_4$) and concentrated *in vacuo*. The crude product was purified by flash chromatography on silica (eluent: Heptane/ EA) to afford the title compound. MS (m/z): 296.7 $[M+H^+]$

Step B: Synthesis of 2-(2-(bromomethyl)-4-(methylsulfonyl)phenyl)-4,4,5,5-tetramethyl-1,3,2-dioxaborolane (**54**)

4,4,5,5-Tetramethyl-2-(2-methyl-4-(methylsulfonyl)phenyl)-1,3,2-dioxaborolane (**53**, 58.0 mg, 0.20 mmol)**53**, NBS (36.6 mg, 0.21 mmol) and AIBN (0.5 mg, 3 μmol) were dissolved in $CCl_4$ and refluxed over night. After cooling to RT the mixture was filtrated and evaporated to dryness. The crude product was purified by flash chromatography on silica (eluent: Heptane/ EA). MS (m/z): 418.7 $[M+HCOO^-]$

Step C: Synthesis of (5-(methylsulfonyl)-2-(4,4,5,5-tetramethyl-l,3,2-dioxaborolan-2-yl)phenyl)methanol (**55**)

2-(2-(Bromomethyl)-4-(methylsulfonyl)phenyl)-4,4,5,5-tetramethyl-1,3,2-dioxaborolane (**54**, 62.0 mg, 0.17 mmol) was suspended in 1 M aqueous NaOH (248 μL) and THF (248 μL) was added until a clear solution resulted. The solution was stirred over night at RT. After complete conversion of the starting material, pH was adjusted to 6 by addition of 1 M aqueous HCl and it was concentrated *in vacuo* to afford the title compound. MS (m/z): 310.9 $[M-H^+]$

Step D: Synthesis of 6-(2-(hydroxymethyl)-4-(methylsulfonyl)phenyl)quinoline-4-carboxylic acid (**56**)

(5-(Methylsulfonyl)-2-(4,4,5,5-tetramethyl-1,3,2-dioxaborolan-2-yl)phenyl)methanol (**55**, 51.6 mg, 0.17 mmol) was reacted with 6-bromo-4-carboxylic acid (45.8 mg, 0.18 mmol) and bis(triphenylphosphine)palladium(II) dichloride (31.4 mg, 0.08 mmol) according to the synthesis of 6-(4-(*tert*-butoxycarbonyl)phenyl)quinoline-4-carboxylic acid (**51**). The crude product was purified by flash chromatography on silica (eluent: DCM/ MeOH/ AcOH). MS (m/z): 357.9 $[M+H^+]$

Step E: Synthesis of (*S*)-*N*-(2-(2-cyano-4,4-difluoropyrrolidin-1-yl)-2-oxoethyl)-6-(2-(hydroxymethyl)-4-(methylsulfonyl)phenyl)quinoline-4-carboxamide (3BP-4084)

6-(2-(Hydroxymethyl)-4-(methylsulfonyl)phenyl)quinoline-4-carboxylic acid (28.8 mg, 0.08 mmol) **56** was reacted with (*S*)-1-(2-aminoacetyl)-4,4-difluoropyrrolidine-2-carbonitrile 2,2,2-trifluoroacetate (31.8 mg, 0.11 mmol) according to the synthesis of (S)-*tert*-butyl 4-(4-(2-(2-cyanopyrrolidin-1-yl)-2-oxoethylcarbamoyl)quinolin-6-yl)benzoate (**52**). The crude product was purified by HPLC to afford the title compound. MS (m/z): 529.1 [M+H$^+$]

**Example 2g: Preparation of Compound 3BP-4152**

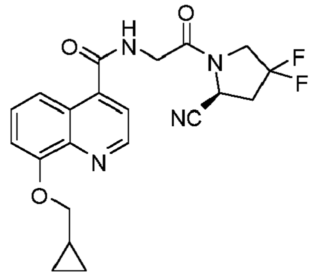

(*S*)-*N*-(2-(2-cyano-4,4-difluoropyrrolidin-l-y1)-2-oxoethy1)-8-(cyclopropylmethoxy)quinoline-4-carboxamide

The synthesis of the title compound is depicted in the following reaction scheme.

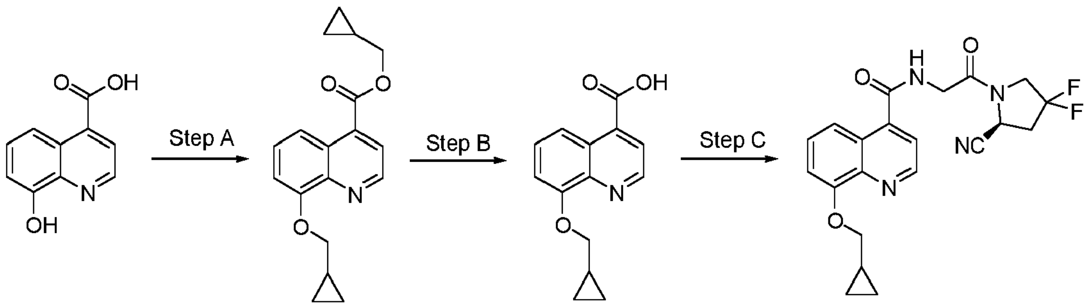


**57** **58** **3BP-4152**

Step A: Synthesis of cyclopropylmethyl 8-(cyclopropylmethoxy)quinoline-4-carboxylate (57)

Caesium carbonate (387.5 mg, 1.19 mmol) and TBAI (7.3 mg, 0.02 mmol) were added to a solution of 8-hydroxy-quinoline-4-carboxylic acid (75.0 mg, 0.40 mmol) in acetonitrile (10 mL). Subsequently bromomethylcyclopropane (267.6 μL, 1.98 mmol) was added and the mixture was stirred over night at RT. After complete conversion of starting material, the mixture was filtrated. The filtrated was concentrated *in vacuo* to afford the title compound. MS (m/z): 297.8 [M+H$^+$]

Step B: Synthesis of 8-(cyclopropylmethoxy)quinoline-4-carboxylic acid (**58**)

To a solution of cyclopropylmethyl 8-(cyclopropylmethoxy)quinoline-4-carboxylate (**57**, 117.9 mg, 0.40 mmol) in THF (4.0 mL) a solution of lithium hydroxide monohydrate (17.5 mg, 0.42 mmol) in water (0.2 mL) was added and the solution was stirred at RT for 2 h. After complete conversion of the starting material, the solution was concentrated *in vacuo* to afford the title compound. MS (m/z): 243.8 [M+H$^+$]

Step C: Synthesis of (*S*)-*N*-(2-(2-cyano-4,4-difluoropyrrolidin-1-yl)-2-oxoethyl)-8-(cyclopropylmethoxy)quinoline-4-carboxamide (3BP-4152)

8-(Cyclopropylmethoxy)quinoline-4-carboxylic acid (**58**, 25.0 mg, 0.10 mmol) was reacted with (*S*)-1-(2-aminoacetyl)-4,4-difluoropyrrolidine-2-carbonitrile 2,2,2-trifluoroacetate (34.3 mg, 0.11 mmol) according to the synthesis of

(*S*)-*tert*-butyl 4-(4-(2-(2-cyanopyrrolidin-1-yl)-2-oxoethylcarbamoyl)quinolin-6-yl)benzoate (**52**). The crude product was purified by HPLC to afford the title compound. MS (m/z): 415.0 [M+H$^+$]

**Example 2h: Preparation of Compound 3BP-4025**

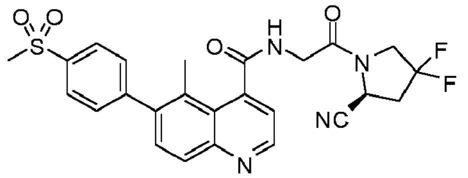

(*S*)-*N*-(2-(2-cyano-4,4-difluoropyrrolidin-1-yl)-2-oxoethyl)-5-methyl-6-(4-(methylsulfonyl)phenyl)quinoline-4-carboxamide

The synthesis of the title compound is depicted in the following reaction scheme.

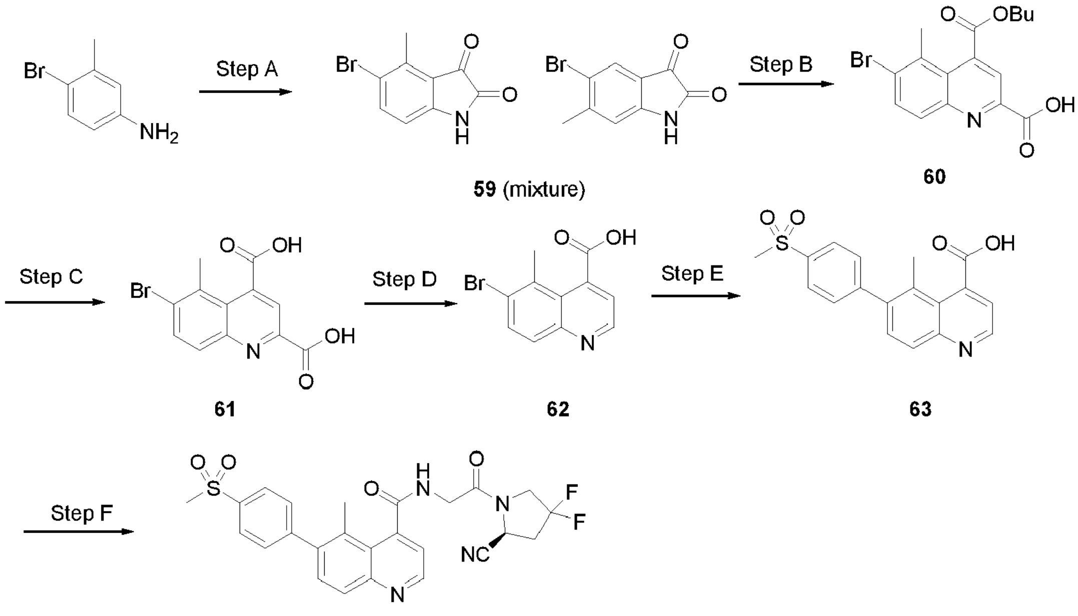


**59** (mixture)

**60**

**61**

**62**

**63**

**3BP-4025**

Step A: Synthesis of 5-bromo-4-methylindoline-2,3-dione and 5-bromo-6-methylindoline-2,3-dione and 5-bromo-6-methylindoline-2,3-dione (**59**)

A mixture of 4-bromo-3-methylaniline (10.00 g, 53.75 mmol), 2,2,2-Trichloroethane-1,1-diol (9.42 g, 46.97 mmol), hydroxylamine hydrochloride (5.83 g, 83.85 mmol), conc. HCl (6.90 mL,83.31 mmol) and $Na_2SO_4$ (61.60 g, 433.68 mmol) in water (206 mL) was stirred at 80 °C over night. After complete consumption of 4-bromo-3-methylaniline and cooling to RT, the precipitated crude product was filtrated off. The crude product was carefully dissolved in a mixture of conc. H2SO4 (27.4 mL) and water (9.6 mL) and the temperature was maintained at 50 - 60 °C. After complete addition, the solution was stirred at 80 °C for 1 h. After complete consumption of the starting material, the mixture was filtrated and re-dissolved in 2 M NaOH (137 mL) and stirred for 1.5 h at RT. The undissolved solids were filtered off. The filtrate was acidified with conc. HCl to pH 1 and extracted with EA to give the title compounds. MS (m/z): 239.9 [M+H$^+$]

Step B: Synthesis of 6-bromo-4-(butoxycarbonyl)-5-methylquinoline-2-carboxylic acid (**60**)

A solution of 5-bromo-4-methylindoline-2,3-dione and 5-bromo-6-methylindoline-2,3-dione (**59**, 56.0 mg, 2.33 mmol) and pyruvic acid (194.4 μL, 2.80 mmol) in 20% aqueous NaOH (5 mL) was refluxed over night. After complete consumption of the starting material and cooling to RT, the mixture was filtrated. The filter cake was washed with *n*-BuOH. Subsequently, the filtrate was acidified to pH 2 with 2 M HCl. The aqueous solution was extracted with *n*-BuOH. The

kombined organic phases were concentrated *in vacuo* and the crude product was purified by HPLC to afford 6-bromo-4-(butoxycarbonyl)-7-methylquinoline-2-carboxylic acid and the title compound. MS (m/z): 365.7 [M+H$^+$]

Step C: Synthesis of 6-bromo-5-methylquinoline-2,4-dicarboxylic acid (**61**)

To a solution of 6-bromo-4-(butoxycarbonyl)-5-methylquinoline-2-carboxylic acid (**60**, 26.2 mg, 72 μmol) in MeOH (2 mL) was added at 0 °C lithium hydroxide monohydrate (6.0 mg, 143 μmol). The solution was stirred at RT over night. Subsequently, the solution was carefully neutralized using Amberlyst IR120 (H$^+$-Form). The resin was filtered of and carefully washed with MeOH. The filtrate was evaporated to yield the title compound. MS (m/z): 309.7 M+H$^+$]

Step D: Synthesis of 6-bromo-5-methylquinoline-4-carboxylic acid (**62**)

A solution of 6-bromo-5-methylquinoline-2,4-dicarboxylic acid (**61**, 22.9 mg, 74 μmol) in water (2 mL) was heated using the microwave to 200 °C for 5 min.. After complete conversion of the starting material, the solution was lyophilized to afford the title compound. MS (m/z): 265.6 [M+H$^+$]

Step E: Synthesis of 5-methyl-6-(4-(methylsulfonyl)phenyl)quinoline-4-carboxylic acid (63)

6-Bromo-5-methylquinoline-4-carboxylic acid (**62**, 30.0 mg, .011 mmol) was reacted with 4-(methylsulfonyl)phenylboronic acid (45.8 mg, 0.18 mmol) and bis(triphenylphosphine)palladium(II) dichloride (31.4 mg, 0.08 mmol) according to the synthesis of 6-(4-(*tert*-butoxycarbonyl)phenyl)quinoline-4-carboxylic acid (**51**). The crude product was purified by flash chromatography on silica (eluent: DCM/ MeOH/ AcOH). MS (m/z): 357.9 [M+H+]

Step F: Synthesis of (*S*)-*N*-(2-(2-cyano-4,4-difluoropyrrolidin-1-yl)-2-oxoethyl)-5-methy1-6-(4-(methylsulfonyl)phenyl)quinoline-4-carboxamide (3BP-4025)

5-methyl-6-(4-(methylsulfonyl)phenyl)quinoline-4-carboxylic acid (**63**, 16.8 mg, 49 μmol) was reacted with (*S*)-1-(2-aminoacetyl)-4,4-difluoropyrrolidine-2-carbonitrile 2,2,2-trifluoroacetate (16.4 mg, 54 μmol) according to the synthesis of (*S*)-*tert*-butyl 4-(4-(2-(2-cyanopyrrolidin-1-yl)-2-oxoethylcarbamoyl)quinolin-6-yl)benzoate (**52**). The crude product was purified by HPLC to afford the title compound. MS (m/z): 513.0 [M+H$^+$]

**Example 2i: Preparation of Compound 3BP-4197**

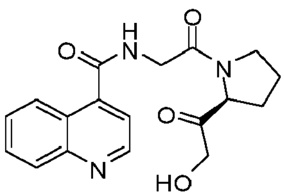

(*S*)-*N*-(2-(2-(2-hydroxyacetyl)pyrrolidin-1-yl)-2-oxoethyl)quinoline-4-carboxamide

The synthesis of the title compound is depicted in the following reaction scheme.

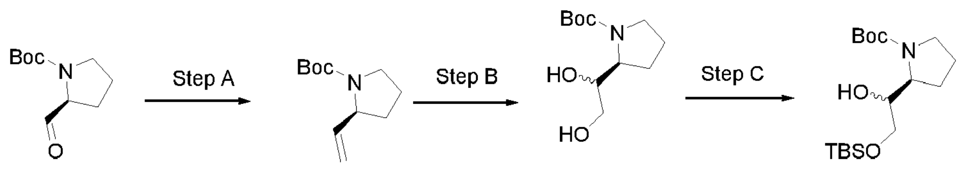


64 65 66

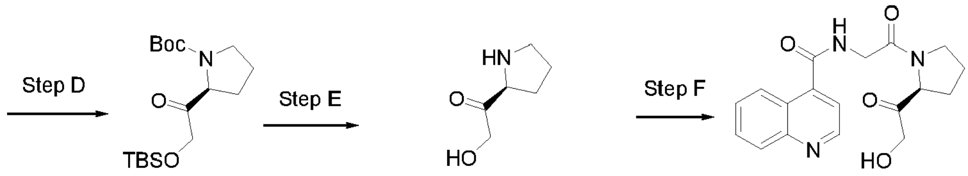


67 68 3BP-4197

Step A: Synthesis of (*S*)-*tert*-butyl 2-vinylpyrrolidine-1-carboxylate (**64**)

KO$^t$Bu (2.0 g, 18.82 mmol) was added to a solution of methyltriphenylphosphonium iodide (7.6 g, 18.82 mmol) in dry THF (33.5 mL) under an atmosphere of nitrogen and the mixture was stirred for 15 min at RT. Subsequently, a solution of (S)-tert-butyl 2-formylpyrrolidine-1-carboxylate (2.5 g, 12.5 mmol) in dry THF (22.2 mL) was added and the reaction mixture was stirred over night at RT. After complete conversion of starting material, water (33.5 mL) was added and the mixture was extracted with diethyl ether. The combined organic phases were dried ($Na_2SO_4$) and concentrated *in vacuo.* The crude product was purified by flash chromatography on silica (eluent: heptane/ EA) to afford the title compound.

Step B: Synthesis of (*S*)-*tert*-butyl 2-(1,2-dihydroxyethyl)pyrrolidine-1-carboxylate (**65**)

Potassium osmate dihydrate and 4-methylmorpholine *N*-oxide were added to a solution of (*S*)-*tert*-butyl 2-vinylpyrrolidine-1-carboxylate (**64**) in a mixture of acetone/ water (10 mL/mmol). The mixture was stirred over night at RT. Then, 10 % aqueous $Na_2S_2O_3$ was added and stirring at RT was continued for 1 h. The organic layer was separated and the auqeous solution was extracted with EA. The combined organic phases were washed with brine, dried ($Na_2SO_4$) and concentrated *in vacuo.* The crude product was purified by flash chromatography (eluent: Heptane/ EA) to afford the title compound. MS (m/z): 231.9 [M+H$^+$]

Step C: Synthesis of (*S*)-*tert*-butyl 2-(2-(*tert*-butyldimethylsilyloxy)-1-hydroxyethyl)pyrrolidine-1-carboxylate (**66**)

A solution of (*S*)-*tert*-butyl 2-(1,2-dihydroxyethyl)pyrrolidine-1-carboxylate (**65**, 2.6 g, 11.18 mmol) and imidazole (1.1 g, 16.77 mmol) in dry DCM (3.5 mL/ mmol (*S*)-*tert*-butyl 2-(1,2-dihydroxyethyl)pyrrolidine-1-carboxylate (**65**)) was cooled to 0 °C. At this temperature, a solution of TBSCl (1.9 mg, 12.29 mmol) in dry DCM (1.5 mL/ mmol TBSCl) was slowly added. The mixture was stirred over night and allowed to reach RT. After complete conversion, the mixture was washed with sat. aqueous $NH_4Cl$ solution and water. The organic phase was dried ($Na_2SO_4$) and concentrated *in vacuo.* The crude product was purified by flash chromatography on silica (eluent: Heptane/ EA) to afford the title compound. MS (m/z): 346.0 [M+H$^+$]

Step D: Synthesis of (*S*)-*tert*-butyl 2-(2-(*tert*-butyldimethylsilyloxy)acetyl)pyrrolidine-1-carboxylate (**67**)

A solution of oxalyl chloride (35.3 μL, 0.42 mmol) in dry DCM (972 μL) was cooled to -78 °C. At this temperature DMSO (59.2 μL, 0.83 mmol) was added. After stirring for 30 min at -78 °C a solution of (S)-*tert*-butyl 2-(2-(*tert*-butyldimethylsilyloxy)-1-hydroxyethyl)pyrrolidine-1-carboxylate (**66**, 120.0 mg, 0.35 mmol) in dry DCM (324 μL) was slowly added. After stirring for 50 min, $NEt_3$ (293.3 μL, 2.08 mmol) was added and stirring was continued over night. During this time the mixture was allowed to reach RT. After complete conversion, the reaction was quenched with water and extracted with DCM. The combined organic phases were washed with brine, dried ($Na_2SO_4$) and evaporated to dryness. The crude product was purified by flash chromatography on silica (eluent: Heptane/ EA). to afford the title compound. MS (m/z): 344.0 [M+H$^+$]

Step E: Synthesis of (*S*)-2-hydroxy-1-(pyrrolidin-2-yl)ethanone (**68**)

(*S*)-*tert*-Butyl 2-(2-(*tert*-butyldimethylsilyloxy)acetyl)pyrrolidine-1-carboxylate (**67**, 35.4 mg, 0.10 mmol) was dissolved in 1 M HCl in methanol (300.0 μL) and stirred at RT for 1 h. After complete conversion the mixture was evaporated to dryness. The crude product was used without further purification.

Step F: Synthesis of (*S*)-*N*-(2-(2-(2-hydroxyacetyl)pyrrolidin-1-yl)-2-oxoethyl)quinoline-4-carboxamide (3BP-4197)

(*S*)-2-Hydroxy-1-(pyrrolidin-2-yl)ethanone (**68**, 13.3 mg, 0.10 mmol) and 2-(quinoline-4-carboxamido)acetic acid (**41**, 25.6 mg, 0.10 mmol) were dissolved in DMF (1 mL)and cooled to 0 °C. At this temperature, HATU (58.8 mg, 0.16 mmol) was added and the pH was adjusted to 6 using DIPEA. The mixture was stirred for 2 h and allowed to reach RT. After complete conversion, the mixture was evaporated to dryness and purified by HPLC to afford the title compound. MS (m/z): 342.1 [M+H$^+$]

**Example 2j: Preparation of Compound 3BP-3581**

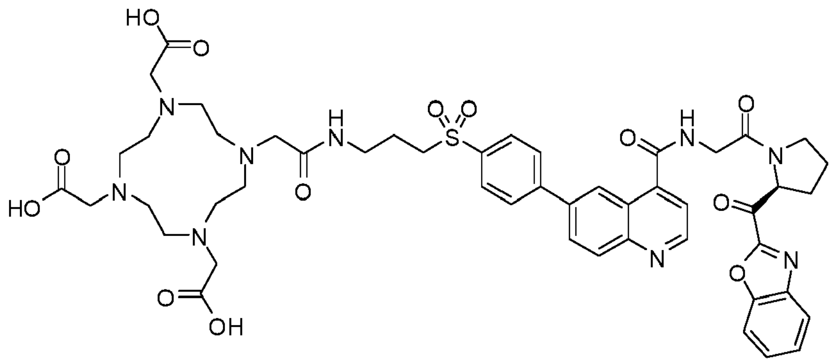

(*S*)-2,2',2"-(10-(2-(3-(4-(4-(2-(2-(benzo[*d*]oxazole-2-carbonyl)pyrrolidin-1-yl)-2-oxoethylcarbamoyl)quinolin-6-yl)phenylsulfonyl)propylamino)-2-oxoethyl)-1,4, 7,10-tetraazacyclododecane-1,4,7-triyl)triacetic acid

The synthesis of the title compound is depicted in the following reaction scheme.

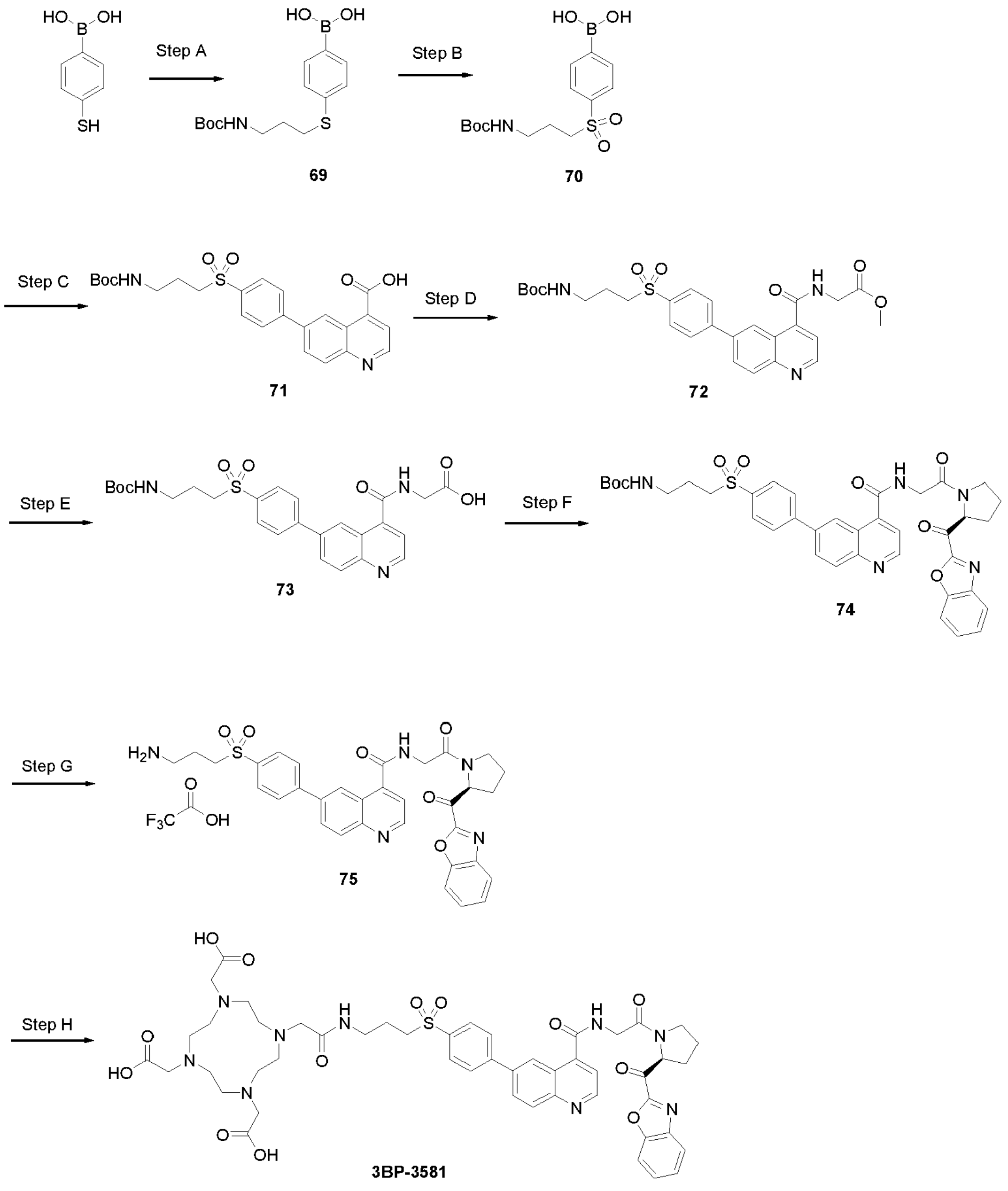


69 70 71 72 73 74 75 3BP-3581

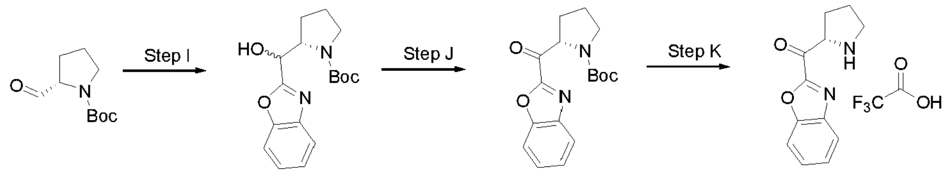


76 77 78

Step A: Synthesis of 4-(3-(tert-butoxycarbonylamino)propylthio)phenylboronic acid (**69**)

To a mixture of 4-mercaptophenylboronic acid (500.0 mg, 3.25 mmol), cesium carbonate (2.12 g, 6.49 mmol), and sodium iodide (486.7 mg, 3.25 mmol) in acetonitrile (12 mL) was slowly added a solution of *tert*-butyl 3-bromopropylcarbamate (773.2 mg. 3.25 mmol) in acetonitrile (1.2 mL). The mixture was stirred over night a roomtemperature. After complete conversion, the mixture was filtrated and the filtrate was evaporated to give the title compound. MS (m/z): 310.0 [M-$H^+$]

Step B: Synthesis of 4-(3-(*tert*-butoxycarbonylamino)propylsulfonyl)phenylboronic acid (**70**)

A solution of 4-(3-(*tert*-butoxycarbonylamino)propylthio)phenylboronic acid (**69**, 1.01 g, 3.25 mmol) in methanol (43.7 mL) was added to a solution of sodium periodate (2.08 g, 9.74 mmol) in water (17 mL). The solution was stirred at RT over night. After complete conversion, the mixture was filtrated. Sodium permanganate (0.31 g, 1.95 mmol) was added and stirring at RT was continued for 2 h. After complete conversion, the methanol is evaporated and the remaining aqueous phase was extracted with EA. The combined organic phases were dried ($Na_2SO_4$) and evaporated to dryeness. The crude product was purified by flash chromatography on silica gel (eluent: DCM/ MeOH)to yield the title compound. MS (m/z): 243.3 [M-Boc+$H^+$]

Step C: Synthesis of 6-(4-(3-(*tert*-butoxycarbonylamino)propylsulfonyl)phenyl)quinoline-4-carboxylic acid (**71**)

4-(3-(*tert*-Butoxycarbonylamino)propylsulfonyl)phenylboronic acid (**70**, 310.4 mg, 0.91 mmol) was reacted with 6-bromoquinoline-4-carboxylic acid (190.0 mg, 0.75 mmol) using bis(triphenylphosphine)palladium(II) dichloride (28.7 mg, 0.08 mmol) according to the synthesis of 6-(4-(*tert*-butoxycarbonyl)phenyl)quinoline-4-carboxylic acid 5**1**. The crude product was used without further purification. MS (m/z): 470.9 [M+$H^+$]

Step D: Synthesis of methyl 2-(6-(4-(3-(*tert*-butoxycarbonylamino)propylsulfonyl)phenyl)quinoline-4-carboxamido)acetate (**72**)

6-(4-(3-(*tert*-Butoxycarbonylamino)propylsulfonyl)phenyl)quinoline-4-carboxylic acid (**71**, 282.1 mg, 0.60 mmol) was reacted with methyl 2-aminoacetate hydrochloride (150.0 mg, 1.20 mmol) according to the synthesis of 2-(quinoline-4-carboxamido)acetate (**40**) to yield the title compound. MS (m/z): 542.4 [M+$H^+$]

Step E: Synthesis of 2-(6-(4-(3-(*tert*-butoxycarbonylamino)propylsulfonyl)phenyl)quinoline-4-carboxamido)acetic acid (**73**)

A solution of methyl 2-(6-(4-(3-(*tert*-butoxycarbonylamino)propylsulfonyl)phenyl)quinoline-4-carboxamido)acetate (**72**, 146.3 mg, 0.27 mmol) in 1,4-dioxane (8.4 mL) was cooled to 0 °C and a solution of lithiumhydroxyde monohydrate (22.7 mg, 0.54 mmol) was added. The mixture was stirred for 4 h at room temperature. Subsequently, the 1,4-dioxane was evaporated and the remaining aqueous solution was carefully acidified (pH 1-2) with 2 M conc. HCl at 0 °C and directly extracted with DCM (3 x 5 mL). The combined organic phases were dried ($Na_2SO_4$) and concentrated *in vacuo* to afford the title compound. MS (m/z): 428.1 [M-Boc+$H^+$]

Step F: Synthesis of (*S*)-*tert*-butyl 3-(4-(4-(2-(2-(benzo[d]oxazole-2-carbonyl)pyrrolidin-1-yl)-2-oxoethylcarbamoyl)quinolin-6-yl)phenylsulfonyl)propylcarbamate (**74**)

A solution of (*S*)-benzo[*d*]oxazol-2-yl(pyrrolidin-2-yl)methanone (77, 55.4 mg, 0.26 mmol) and 2-(6-(4-(3-(*tert*-butoxycarbonylamino)propylsulfonyl)phenyl)quinoline-4-carboxamido)acetic acid (**73**, 123.0 mg, 0.23 mmol) in dry DMF (1.2 mL) and cooled to 0 °C. At this temperature, HATU (115.2 mg. 0.30 mmol) and 2,4,6-trimethylpyridine (90.1 μL, 0.70 mmol) were added and the mixture was stirred at RT over night. After complete conversion, the mixture was evaporated to dryness, re-dissolved in DCM and washed with water. The organic phase was dried ($Na_2SO_4$) and concentrated in vacuo. The crude product was purified by HPLC to afford the title product. MS (m/z): 726.5 [M+$H^+$]

Step G: Synthesis of (*S*)-6-(4-(3-aminopropylsulfonyl)phenyl)-*N*-(2-(2-(benzo[*d*]oxazole-2-carbonyl)pyrrolidin-1-yl)-2-oxoethyl)quinoline-4-carboxamide 2,2,2-trifluoroacetate (**75**)

TFA (50.3 μL, 0.44 mmol) was added to a solution of (*S*)-*tert*-butyl 3-(4-(4-(2-(2-(benzo[*d*]oxazole-2-carbonyl)pyrrolidin-1-yl)-2-oxoethylcarbamoyl)quinolin-6-yl)phenylsulfonyl)propylcarbamate (**74**, 16.0 mg, 0.02 mmol) in DCM (260 μL) and the solution was stirred at RT for 1 h. Subsequently, the volatiles were removed in vacuo to afford the title compound. MS (m/z): 626.1 [M+$H^+$]

Step H: Synthesis of (*S*)-2,2',2"-(10-(2-(3-(4-(4-(2-(2-(benzo[*d*]oxazole-2-carbonyl)pyrrolidin-1-yl)-2-oxoethylcarbamoyl)quinolin-6-yl)phenylsulfonyl)propylamino)-2-oxoethyl)-1,4,7,10-tetraazacyclododecane-1,4,7-triyl)triacetic acid (3BP-3581)

A solution of (*S*)-6-(4-(3-aminopropylsulfonyl)phenyl)-*N*-(2-(2-(benzo[*d*]oxazole-2-carbonyl)pyrrolidin-1-yl)-2-oxoethyl)quinoline-4-carboxamide 2,2,2-trifluoroacetate (**75**, 13.8 mg, 18.7 μmol) DOTA-NHS ester (25.7 mg, 31.8 μmol)

in DMSO (0.7 mL) was neutralized to pH 7 with DIPEA. The solution was stirred at RT for 2 h. After complete conversion, the DMSo solution was subject to HPLC purification to afford the title compound. MS (m/z): 1012.3 [M+H$^+$]

Step I: Synthesis of (*S*)-*tert*-butyl 2-(benzo[*d*]oxazol-2-yl(hydroxy)methyl)pyrrolidine-1-carboxylate (**76**)

A Schlenck flask was evacuated and ventilated with argon. benzoxazole (762.5 mg, 6.40 mmol) and dry THF (13.3 mL) were added and cooled to -20 °C. At this temperature, isopropylmagnesium chloride (2.0 M in diethyl ether, 3.84 mL, 7.68 mmol) was added and stirring at -20 °C was continued for 1 h. Then, a solution of *tert*-butyl (2*S*)-2-formylpyrrolidine-1-carboxylate (1000.0 mg, 5.34 mmol) in dry THF (5.0 mL) was added at -20 °C. The mixture was stirred over night and allowed to reach RT. After complete conversion, the reaction was quenched by addition of sat. aqueous $NH_4Cl$ solution (9 mL) and stirring for 1 h. The mixture is extracted with EA (3 x 9 mL). The combined organic layers were washed with brine, dried ($Na_2SO_4$), and evaporated to dryness. The crude product was purified by flash chromatography on silica gel (elution with heptane/ EA) to give the title compound. MS (m/z): 353.6 [M+H$^+$]

Step J: Synthesis of (*S*)-*tert*-butyl 2-(benzo[*d*]oxazole-2-carbonyl)pyrrolidine-1-carboxylate (**77**)

Dess-Martin periodinane (199.8 mg, 0.47 mmol) was added to a solution of (*S*)-*tert*-butyl 2-(benzo[*d*]oxazol-2-yl(hydroxy)methyl)pyrrolidine-1-carboxylate (**76**, 100.0 mg, 0.31 mmol) in $CHCl_3$ (300 μL) and the mixture was stirred for 4 h at RT. After complete consumption of the starting material, a solution of $NaHCO_3$/ $Na_2S_2O_3$ (10 %/ 10 %; 3 mL) was added and the phases were separated. The organic phase was washed with aqueous $Na_2S_2O_3$ solution (25 %, 3 mL) and brine (3 mL), dried ($Na_2SO_4$), filtered and evaporated to dryness to afford the title compound. MS (m/z): 317.1 [M+H$^+$]

Step K: Synthesis of (*S*)-benzo[*d*]oxazol-2-yl(pyrrolidin-2-yl)methanone 2,2,2-trifluoroacetate (**78**)

TFA (486.5 μL, 6.32 mmol) was added dropwise to a solution of (*S*)-*tert*-butyl 2-(benzo[*d*]oxazole-2-carbonyl)pyrrolidine-1-carboxylate (**77**, 100.0 mg, 0.32 mmol) in DCM (630 μL). The reaction mixture was stirred at RT for 1 h. After complete conversion, the solution was concentrated *in vacuo* to give the title compound. MS (m/z): 216.9 [M+H$^+$]

**Example 2k: Preparation of Compound 3BP-3622**

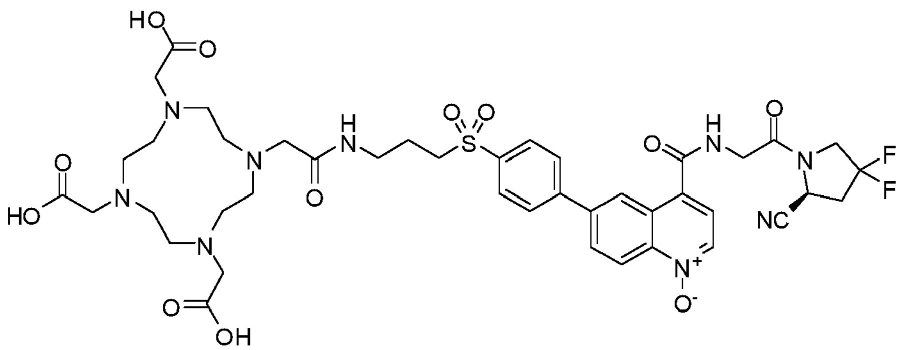

(*S*)-4-(2-(2-cyano-4,4-difluoropyrrolidin-1-yl)-2-oxoethylcarbamoyl)-6-(4-(3-(2-(4,7,10-tris(carboxymethyl)-1,4,7,10-tetraazacyclododecan-1-yl)acetamido)propylsulfonyl)phenyl)quinoline 1-oxide

The synthesis of the title compound is depicted in the following reaction scheme.

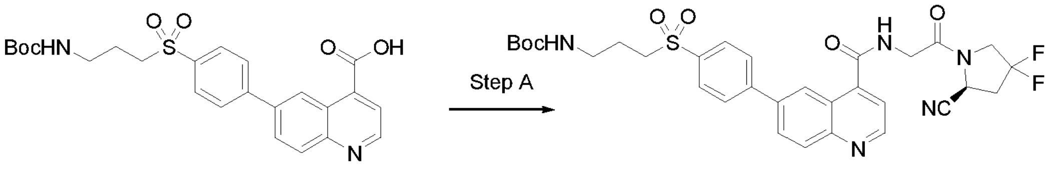


**71** **79**

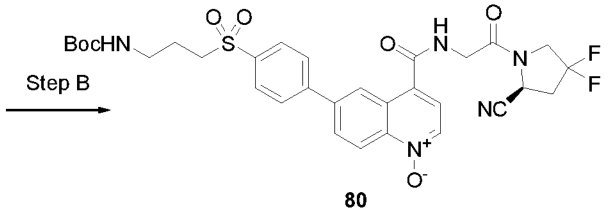


**80**

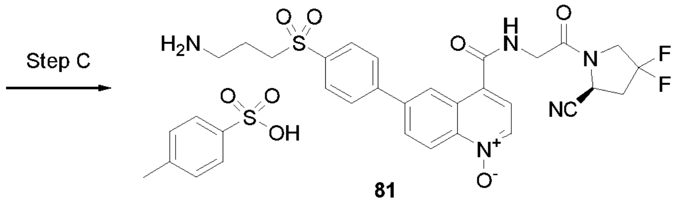


**81**

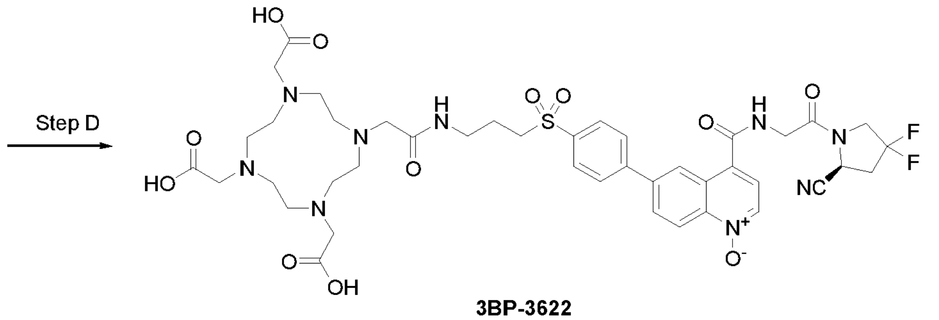


**3BP-3622**

Step A: Synthesis of (*S*)-*tert*-butyl 3-(4-(4-(2-(2-cyano-4,4-difluoropyrrolidin-1-yl)-2-oxoethylcarbamoyl)quinolin-6-yl)phenylsulfonyl)propylcarbamate (**79**)

6-(4-(3-(*tert*-Butoxycarbonylamino)propylsulfonyl)phenyl)quinoline-4-carboxylic acid (**71**, 391.4 mg, 0.44 mmol) was reacted with (*S*)-1-(2-aminoacetyl)-4,4-difluoropyrrolidine-2-carbonitrile 2,2,2-trifluoroacetate (110.0 mg, 0.36 mmol) according to the synthesis of (*S*)-*tert*-butyl 4-(4-(2-(2-cyanopyrrolidin-1-yl)-2-oxoethylcarbamoyl)quinolin-6-yl)benzoate (**52**). The crude product was purified by flash chromatography on silica gel (eluent: DCM/ MeOH) to afford the title compound. MS (m/z): 640.0 [M-H$^+$]

Step B: Synthesis of (*S*)-6-(4-(3-(*tert*-butoxycarbonylamino)propylsu]fonyl)phenyl)-4-(2-(2-cyano-4,4-difluoropyrrolidin-1-yl)-2-oxoethylcarbamoyl)quinoline 1-oxide (**80**)

*m*CPBA (20.7 mg, 0.12 mmol) was added in several portions to a solution of (*S*)-*tert*-butyl 3-(4-(4-(2-(2-cyano-4,4-difluoropyrrolidin-1-yl)-2-oxoethylcarbamoyl)quinolin-6-yl)phenylsulfonyl)propylcarbamate (**79**, 70.0 mg, 0.11 mmol) in DCM (1.1 mL) and the mixture was stirred over night at RT. The mixture was filtrated and the residue was washed with DCM (1mL). The filtrate was dried ($Na_2SO_4$) and concentrated in vacuo. the crude product was purified by flash chromatography on silica gel (eluent: DCM/ MeOH) to yield the title compound. MS (m/z): 658.2 [M+H$^+$]

Step C: Synthesis of (*S*)-6-(4-(3-aminopropylsulfonyl)phenyl)-4-(2-(2-cyano-4,4-difluoropyrrolidin-1-yl)-2-oxoethylcarbamoyl)quinoline 1-oxide 4-methylbenzenesulfonate (**81**)

A solution of (*S*)-6-(4-(3-(*tert*-butoxycarbonylamino)propylsu]fonyl)phenyl)-4-(2-(2-cyano-4,4-difluoropyrrolidin-1-yl)-2-oxoethylcarbamoyl)quinoline 1-oxide (**80**, 80.5 mg, 0.12 mmol) and 4-methylbenzenesulfonic acid monohydrate (32.6 mg, 0.17 mmol) in acetonitrile was stirred at RT over night. After complete conversion, the volatiles were evaporated to give the title compound. MS (m/z): 558.0 [M+H+]

Step D: Synthesis of (*S*)-4-(2-(2-cyano-4,4-difluoropyrrolidin-1-yl)-2-oxoethylcarbamoyl)-6-(4-(3-(2-(4,7,10-tris(carboxymethyl)-1,4,7,10-tetraazacyclododecan-1-yl)acetamido)propylsulfonyl)phenyl)quinoline 1-oxide (3BP-3622)

(*S*)-6-(4-(3-Aminopropylsulfonyl)phenyl)-4-(2-(2-cyano-4,4-difluoropyrrolidin-1-yl)-2-oxoethylcarbamoyl)quinoline 1-oxide 4-methylbenzenesulfonate (**81**, 33.8 mg, 0.05 mmol) was reacted with DOTA-NHS ester (83.1 mg, 0.09 mmol) according to the synthesis of (*S*)-2,2',2"-(10-(2-(3-(4-(4-(2-(2-(benzo[*d*]oxazole-2-carbonyl)pyrrolidin-1-yl)-2-oxoethylcarbamoyl)quinolin-6-yl)phenylsulfonyl)propylamino)-2-oxoethyl)-1,4,7,10-tetraazacyclododecane-1,4,7-triyl)triacetic acid (3BP-3581) to afford the title compound. MS (m/z): 982.6 [M+K+]

3BP-3467 was prepared following a similar method to that described for 3BP-3622, without performing the oxidation step B.

**Example 2l: Preparation of Compound 3BP-3951**

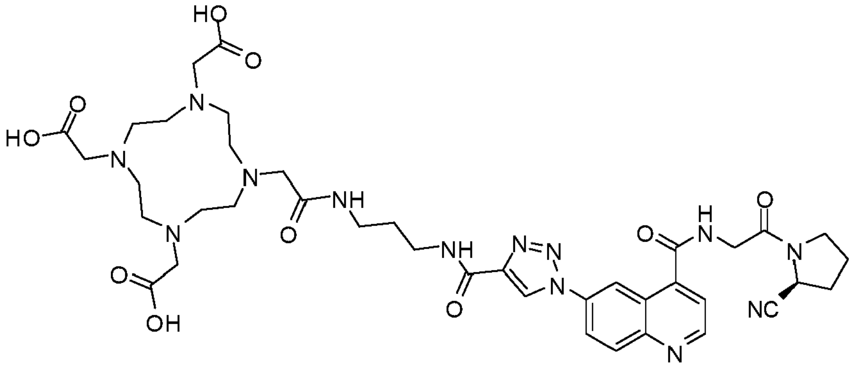

(*S*)-2,2',2"-(10-(2-(3-(1-(4-(2-(2-cyanopyrrolidin-1-y1)-2-oxoethylcarbamoyl)quinolin-6-yl)-1H-1,2,3-triazole-4-carboxamido)propylamino)-2-oxoethyl)-1,4,7,10-tetraazacyclododecane-1,4,7-triyl)triacetic acid

The synthesis of the title compound is depicted in the following reaction scheme.

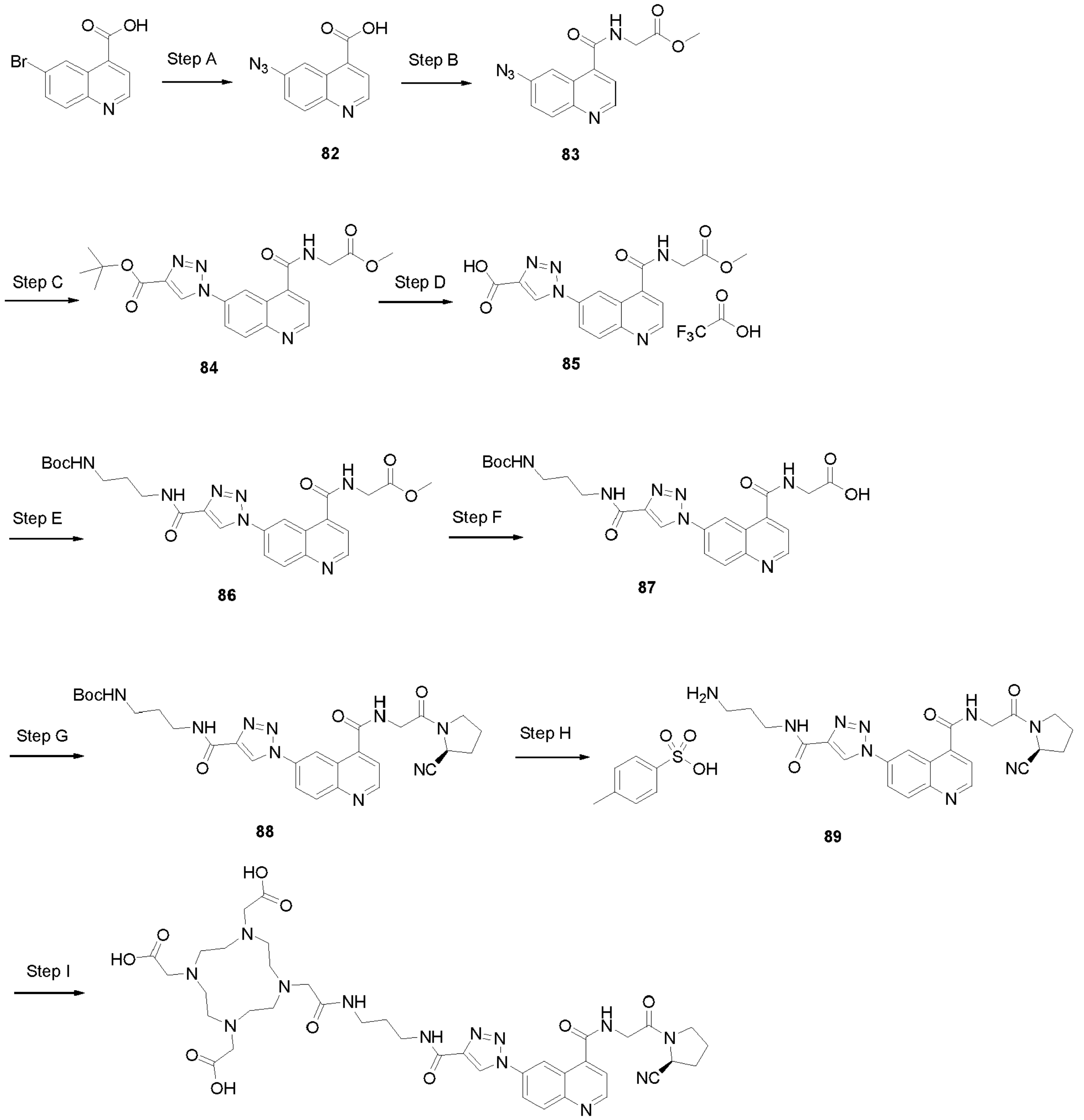


82 83 84 85 86 87 88 89

3BP-3951

Step A: Synthesis of 6-azidoquinoline-4-carboxylic acid **(82)**

Sodium azide (515.8 mg, 7.94 mmol) and trans-*N*,*N'*-dimethylcyclohexane-1,2-diamine (1.25 mL, 7.94 mmol) were added to a solution of 6-bromoquinoline-4-carboxylic acid (1000.0 mg, 3.97 mmol) in a mixture of ethanol/ water = 7 : 1 (52.8 mL). Subsequently, sodium ascorbate (1 M in Ethanol, 396.7 μL, 0.4 mmol) and CuI (1 M in Ethanol, 396.7 μL, 0.4 mmol) were added and stirring was continued at 80 °C for 15 min. After complete consumption of starting material, the mixture was diluted with water and extracted with EA. The combined organic phases were dried ($Na_2SO_4$) and concentrated *in vacuo* to yield the title compound. MS (m/z): 214.8 [M+H$^+$]

Step B: Synthesis of methyl 2-(6-azidoquinoline-4-carboxamido)acetate (83)

6-Azidoquinoline-4-carboxylic acid (82, 832.5 mg, 3.89 mmol) was reacted with methyl 2-aminoacetate hydrochloride (610.0 mg, 4.86 mmol) according to the synthesis of 2-(quinoline-4-carboxamido)acetate **(40)** to yield the title compound. MS (m/z): 285.9 [M+H$^+$]

Step C: Synthesis of *tert*-butyl 1-(4-(2-methoxy-2-oxoethylcarbamoyl)quinolin-6-yl)-1*H*-1,2,3-triazole-4-carboxylate **(84)**

Copper(II) sulfate pentahydrate (48.6 mg, 0.19 mmol) and sodium ascorbate (77.0 mg, 0.39 mmol) were added to a solution of *tert-butyl* propiolate (640.6 μL, 4.67 mmol) and methyl 2-(6-azidoquinoline-4-carboxamido)acetate **(83,** 1109.4 mg, 3.89 mmol) in a mixture of water and $^t$BuOH = 1 : 1 (20 mL). The mixture was stirred under an atmosphere of nitrogen. After complete consumption of the starting material, the mixture was evaporated to dryness and purified by flash chromatography on silica gel (eluent: DCM/ MeOH) to afford the title compound. MS (m/z): 412.0 [M+H$^+$]

Step D: Synthesis of 1-(4-(2-methoxy-2-oxoethylcarbamoyl)quinolin-6-yl)-1*H*-1,2,3-triazole-4-carboxylic acid 2,2,2-trifluoroacetate **(85)**

*tert*-Butyl 1-(4-(2-methoxy-2-oxoethylcarbamoyl)quinolin-6-yl)-1*H*-1,2,3-triazole-4-carboxylate **(84,** 490.0 mg, 1.19 mmol) was dissolved in dry DCM (5 mL) and TFA (1.84 mL, 23.82 mmol) was added dropwise. The mixture was stirred at RT for 4 h. After complete conversion of the starting material, the mixture was concentrated *in vacuo* to yield the title compound. MS (m/z): 355.9 [M+H$^+$]

Step E: Synthesis of methyl 2-(6-(4-(3-(*tert*-butoxycarbonylamino)propylcarbamoyl)-1*H*-1,2,3-triazol-1-yl)quinoline-4-carboxamido)acetate **(86)**

A solution of 1-(4-(2-methoxy-2-oxoethylcarbamoyl)quinolin-6-yl)-1*H*-1,2,3-triazole-4-carboxylic acid 2,2,2-trifluoroacetate **(85,** 560.0 mg, 1.19 mmol) and *tert*-butyl 3-aminopropylcarbamate (312.4 μL, 1.79 mmol) werde dissolved in DMF (7.95 mL) and cooled to 0 °C. Subsequently, HATU (680.5 mg, 1.79 mmol) and DIPEA (811.6 μL, 4.77 mmol) were added at this temperature. The mixture was stirred over night at RT. After complete consumption, the mixture was diluted with DCM and washed with water. The organic phase was dried ($Na_2SO_4$) and evaporated to dryness. The crude product was purified by flash chromatography on silica (eluent: DCM/ MeOH) to afford the title compound. MS (m/z): 512.1 [M+H$^+$]

Step F: Synthesis of 2-(6-(4-(3-(*tert*-butoxycarbonylamino)propylcarbamoyl)-1H-1,2,3-triazol-1-yl)quinoline-4-carboxamido)acetic acid **(87)**

3-(*tert*-Butoxycarbonylamino)propyl 1-(4-(2-methoxy-2-oxoethylcarbamoyl)quinolin-6-yl)-1*H*-1,2,3-triazole-4-carboxylate **(86,** 457.5 mg, 0.89 mmol) was reacted with lithiumhydroxyde monohydrate (75.1 mg, 1.79 mmol) according to the synthesis of 2-(6-(4-(3-(*tert*-butoxycarbonylamino)propylsulfonyl)phenyl)quinoline-4-carboxamido)acetic acid (73) to afford the title compound. MS (m/z): 498.2 [M+H$^+$]

Step G: Synthesis of (*S*)-3-(*tert*-butoxycarbonylamino)propyl 1-(4-(2-(2-cyanopyrrolidin-1-yl)-2-oxoethylcarbamoyl)quinolin-6-yl)-1*H*-1,2,3-triazole-4-carboxylate **(88)**

2-(6-(4-(3-(*tert*-Butoxycarbonylamino)propylcarbarnoyl)-1*H*-1,2,3-triazol-1-yl)quinoline-4-carboxamido)acetic acid **(87,** 50.0 mg, 0.10 mmol) was reacted with (2*S*)-pyrrolidine-2-carbonitrile hydrochloride (20.0 mg, 0.15 mmol) according to the synthesis of *N*-(2-((2*S*)-2-(hydroxy(1*H*-1,2,4-triazol-5-yl)methyl)pyrrolidin-1-yl)-2-oxoethyl)quinoline-4-carboxamide **(42)** to afford the title compound. MS (m/z): 576.2 [M+H$^+$]

Step H: Synthesis of (*S*)-3-aminopropyl 1-(4-(2-(2-cyanopyrrolidin-1-yl)-2-oxoethylcarbamoyl)quinolin-6-yl)-1*H*-1,2,3-triazole-4-carboxylate 4-methylbenzenesulfonate **(89)**

(*S*)-3-(*tert*-Butoxycarbonylamino)propyl 1-(4-(2-(2-cyanopyrrolidin-1-yl)-2-oxoethylcarbamoyl)quinolin-6-yl)-1*H*-1,2,3-triazole-4-carboxylate (**88**, 48.6 mg, 0.08 mmol) was reacted with 4-methylbenzenesulfonic acid monohydrate (22.5 mg, 0.12 mmol) according the synthesis of (*S*)-6-(4-(3-aminopropylsulfonyl)phenyl)-4-(2-(2-cyano-4,4-difluoropyrrolidin-1-yl)-2-oxoethylcarbamoyl)quinoline 1-oxide 4-methylbenzenesulfonate **(81)** to give the title compound. MS (m/z): 476.1 [M+H$^+$]

Step K: Synthesis of (*S*)-2,2',2"-(10-(2-(3-(1-(4-(2-(2-cyanopyrrolidin-1-yl)-2-oxoethylcarbamoyl)quinolin-6-yl)-1*H*-1,2,3-triazole-4-carboxamido)propylamino)-2-oxoethyl)-1,4,7,10-tetraazacyclododecane-1,4,7-triyl)triacetic acid (3BP-3951)

(S)-3 -Aminopropyl 1-(4-(2-(2-cyanopyrrolidin-1-yl)-2-oxoethylcarbamoyl)quinolin-6-yl)-1*H*-1,2,3-triazole-4-carboxylate 4-methylbenzenesulfonate **(89,** 55.1 mg, 0.09 mmol) was reacted with DOTA-NHS ester (116.0 mg, 0.14 mmol) according to the synthesis of (*S*)-2,2',2"-(10-(2-(3-(4-(4-(2-(2-(benzo[*d*]oxazole-2-carbonyl)pyrrolidin-1-yl)-2-oxoethylcarbamoyl)quinolin-6-yl)phenylsulfonyl)propylamino)-2-oxoethyl)-1,4,7,10-tetraazacyclododecane-1,4,7-triyl)triacetic acid (3BP-3581) to afford the title compound. MS (m/z): 1012.2 [M+H$^+$]

**Example 2m: Preparation of Compound 3BP-4076**

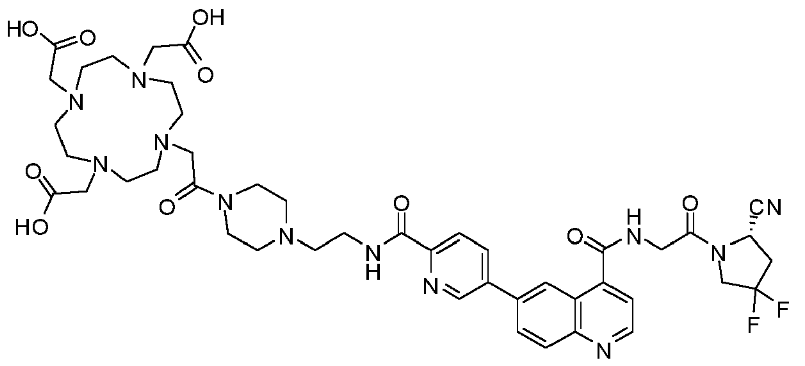

(*S*)-2,2',2"-(10-(2-(4-(2-(5-(4-(2-(2-cyano-4,4-difluoropyrrolidin-1-yl)-2-oxoethylcarbamoyl)quinolin-6-yl)picolinamido)ethyl)piperazin-1-yl)-2-oxoethyl)-1,4,7,10-tetraazacyclododecane-1,4,7-triyl)triacetic acid 3BP-4076

The synthesis of the title compound is depicted in the following reaction scheme.

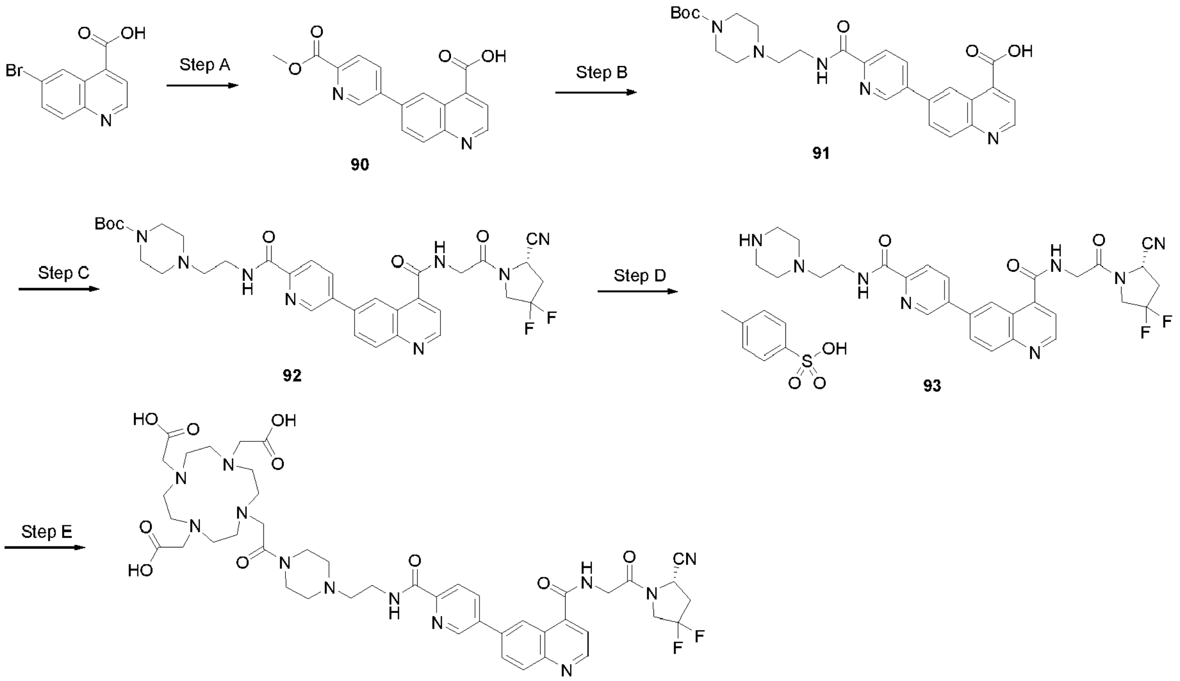

90
91
92
93
3BP-4076

Step A: Synthesis of 6-(6-(methoxycarbonyl)pyridin-3-yl)quinoline-4-carboxylic acid **(90)**

6-Bromoquinoline-4-carboxylic acid (100 mg, 0.40 mmol) and methyl 5-(4,4,5,5-tetramethyl-1,3,2-dioxaborolan-2-yl)picolinate (313.1 mg, 1.19 mmol) were reacted using bis(triphenylphosphine)palladium(II) dichloride (150.8 mg, 0.40 mmol) according to the synthesis of 6-(4-(*tert*-butoxycarbonyl)phenyl)quinoline-4-carboxylic acid **(51).** The crude product was used without further purification. MS (m/z): 309.0 [M+H$^+$]

Step B: Synthesis of 6-(6-(2-(4-(*tert*-butoxycarbonyl)piperazin-1-yl)ethylcarbamoyl)pyridin-3-yl)quinoline-4-carboxylic acid **(91)**

6-(6-(Methoxycarbonyl)pyridin-3-yl)quinoline-4-carboxylic acid (20.0 mg, 0.065 mmol) (90), *tert*-butyl 4-(2-aminoethyl)piperazine-1-carboxylate (113.0 mg, 0.65 mmol) and 1,2-dimethoxyethane (150 μL) were stirred at 40 °C over night. After complete conversion the mixture was evaporated to dryness and purified by flash chromatography on silica gel (eluent: DCM/ MeOH --> DCM/ MeOH/ acetic acid). MS (m/z): 506.5 M+H$^+$]

Step C: Synthesis of (*S*)-*tert*-butyl 4-(2-(5-(4-(2-(2-cyano-4,4-difluoropyrrolidin-1-yl)-2-oxoethylcarbamoyl)quinolin-6-yl)picolinamido)ethyl)piperazine-1-carboxylate **(92)**

6-(6-(2-(4-(*tert*-butoxycarbonyl)piperazin-1-yl)ethylcarbamoyl)pyridin-3-yl)quinoline-4-carboxylic acid (91, 38.8 mg, 0.073 mmol) was reacted with (*S*)-2-(2-cyanopyrrolidin-l-yl)-2-oxoethanaminium 4-methylbenzenesulfonate [J. Med. Chem. 2017, 60, 8385.] (26.5 mg, 0.087 mmol), HATU (36.0 mg, 0.095 mmol), and DIPEA (49.5 μL, 0.291 mmol) according to the synthesis of (*S*)-*tert*-butyl 4-(4-(2-(2-cyanopyrrolidin-1-yl)-2-oxoethylcarbamoyl)quinolin-6-yl)benzoate **(52).** The crude product was purified by flash chromatography on silica gel (eluent: DCM/ MeOH) to afford the title compound. MS (m/z): 677.7 [M+H$^+$].

Step D: Synthesis of (*S*)-N-(2-(2-cyano-4,4-difluoropyrrolidin-1-yl)-2-oxoethyl)-6-(6-(2-(piperazin-1-yl)ethylcarbamoyl)pyridin-3-yl)quinoline-4-carboxamide 4-methylbenzenesulfonate **(93)**

(*S*)-*tert*-Butyl 4-(2-(5-(4-(2-(2-cyano-4,4-difluoropyrrolidin-1-yl)-2-oxoethylcarbamoyl)quinolin-6-yl)picolinamido)ethyl)piperazine-1-carboxylate (23.2 mg, 0.034 mmol) **92** was reacted with p-toluenesulfonic acid monohydrate (13.0 mg, 0.069 mmol) according to the synthesis of (S)-6-(4-(3-aminopropylsulfonyl)phenyl)-4-(2-(2-cyano-4,4-difluoropyrrolidin-1-yl)-2-oxoethylcarbamoyl)quinoline 1-oxide 4-methylbenzenesulfonate (**81**). The crude product was used without further purification. MS (m/z): 577.2 [M+H+]

Step E: Synthesis of (*S*)-2,2',2"-(10-(2-(4-(2-(5-(4-(2-(2-cyano-4,4-difluoropyrrolidin-1-yl)-2-oxoethylcarbamoyl)quinolin-6-yl)picolinamido)ethyl)piperazin-1-yl)-2-oxoethyl)-1,4,7,10-tetraazacyclododecane-1,4,7-triyl)triacetic acid (3BP-4076)

(*S*)-*N*-(2-(2-cyano-4,4-difluoropyrrolidin-1-yl)-2-oxoethyl)-6-(6-(2-(piperazin-1-yl)ethylcarbamoyl)pyridin-3-yl)quinoline-4-carboxamide 4-methylbenzenesulfonate **(93,** 19.8 mg, 0.034 mmol) was reacted with DOTA-NHS ester (47.1 mg, 0.058 mmol) according to the synthesis of (*S*)-2,2',2"-(10-(2-(3-(4-(4-(2-(2-(benzo[*d*]oxazole-2-carbonyl)pyrrolidin-1-yl)-2-oxoethylcarbamoyl)quinolin-6-yl)phenylsulfonyl)propylamino)-2-oxoethyl)-1,4,7,10-tetraazacyclododecane-1,4,7-triyl)triacetic acid (3BP-3581). The crude product was purified by HPLC. MS (m/z): 1001.6 [M+K$^+$]

The synthesis of 3BP-3772 followed similar procedures as described for 3BP-4076, utilizing another Boc-protected diamine compound in step B.

**Example 2n: Preparation of Compound 3BP-3954**

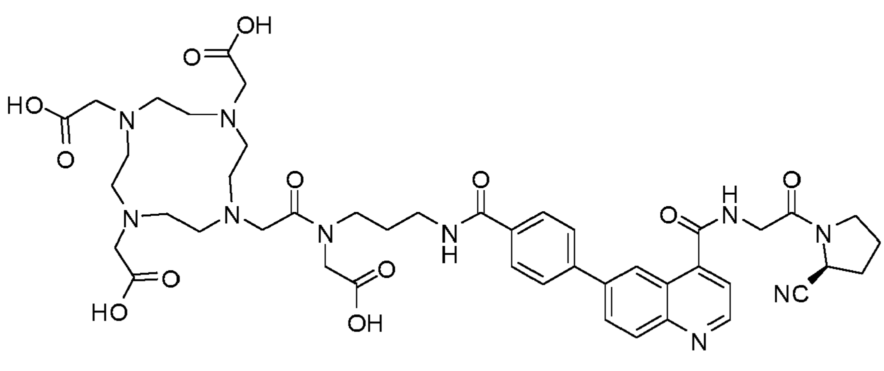

(*S*)-2,2',2"-(10-(2-((carboxymethyl)(3-(4-(4-(2-(2-cyanopyrrolidin-1-yl)-2-oxoethylcarbamoyl)quinolin-6-yl)benzamido)propyl)amino)-2-oxoethyl)-1,4,7,10-tetraazacyclododecane-1,4,7-triyl)triacetic acid

The synthesis of the title compound is depicted in the following reaction scheme.

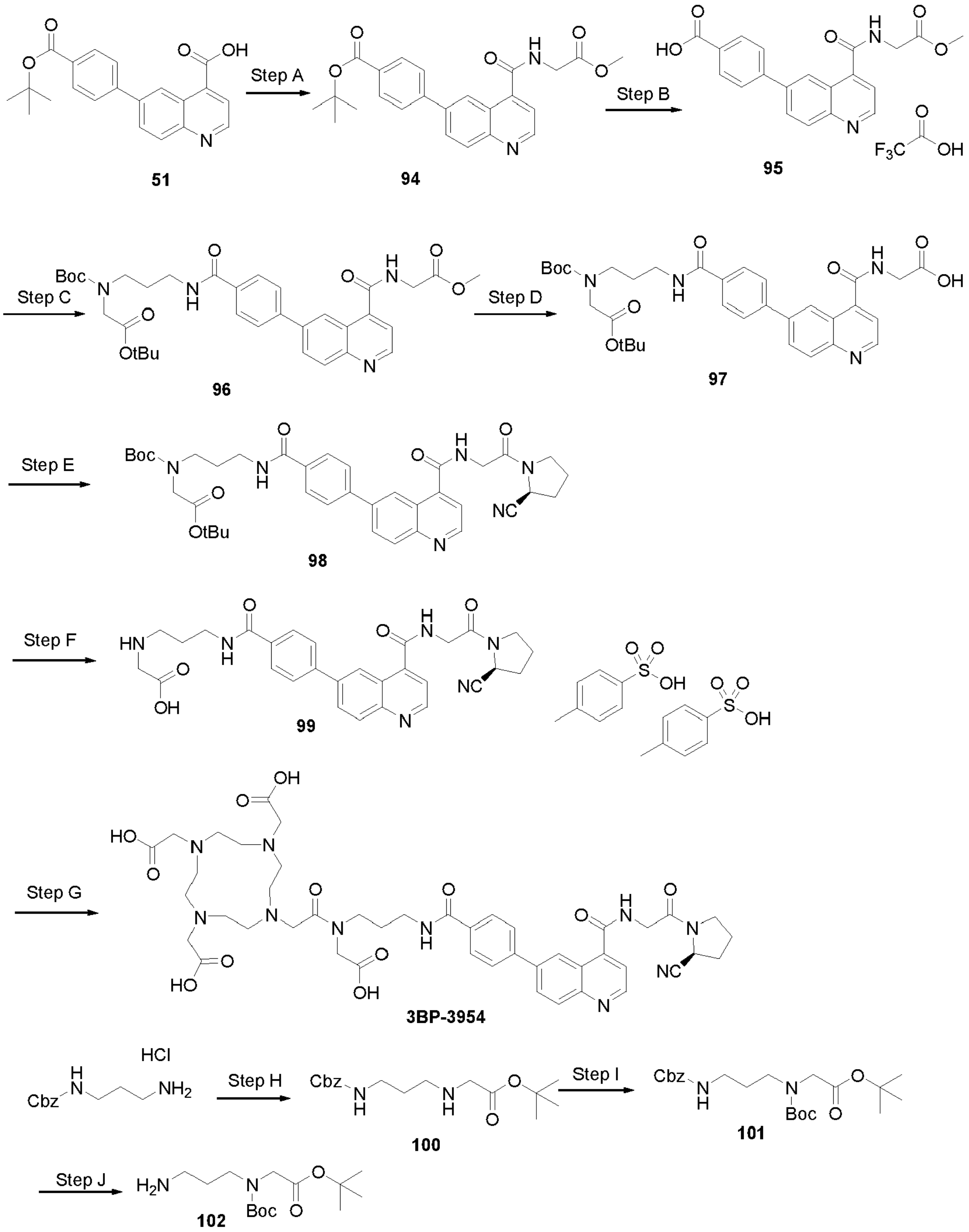


51 94 95 96 97 98 99 3BP-3954 100 101 102

Step A: Synthesis of *tert*-butyl 4-(4-(2-methoxy-2-oxoethylcarbamoyl)quinolin-6-yl)benzoate **(94)**

6-(4-(*tert*-Butoxycarbonyl)phenyl)quinoline-4-carboxylic acid **(51,** 1987.3 mg, 6.07 mmol) was reacted with methyl 2-aminoacetate hydrochloride (635.0 mg, 5.06 mmol) according to the synthesis of 2-(quinoline-4-carboxamido)acetate **(40)** to yield the title compound. MS (m/z): 421.3 [M+H$^+$]

Step B: Synthesis of 4-(4-(2-methoxy-2-oxoethylcarbamoyl)quinolin-6-yl)benzoic acid trifluoroacetate **(95)**

TFA (7.80 mL, 101.2 mmol) was slowly added to a solution of *tert-butyl* 4-(4-(2-methoxy-2-oxoethylcarbamoyl)quinolin-6-yl)benzoate **(94,2.55** g, 6.07 mmol) in dry DCM (26.4 mL) and the solution was stirred at RT for 3 h. After complete conversion, the volatiles were removed *in vacuo* to afford the title compound. MS (m/z): 355.2 [M+H$^+$]

Step C: Synthesis of *tert*-butyl 2-(*tert*-butoxycarbonyl(3-(4-(4-(2-methoxy-2-oxoethylcarbamoyl)quinolin-6-yl)benzamido)propyl)amino)acetate (**96**)

4-(4-(2-methoxy-2-oxoethylcarbamoyl)quinolin-6-yl)benzoic acid trifluoroacetate (340.3 mg, 0.93 mmol) (**95**) was reacted with tert-butyl 2-((3-aminopropyl)(*tert*-butoxycarbonyl)amino)acetate (**102**, 215.5 mg, 0.75 mmol) according to the synthesis of 2-(quinoline-4-carboxamido)acetate (**40**) to yield the title compound. MS (m/z): 289.3 [M+H$^+$]

Step D: Synthesis of 2-(6-(4-(3-((2-*tert*-butoxy-2-oxoethyl)(*tert*-butoxycarbonyl)amino)propylcarbamoyl)-phenyl)quinoline-4-carboxamido)acetic acid **(97)**

*tert*-Butyl 2-(*t*ert-butoxycarbonyl(3-(4-(4-(2-methoxy-2-oxoethylcarbamoyl)quinolin-6-yl)benzamido)propyl)amino)acetate (96, 421.8 mg, 0.67 mmol) was reacted with lithiumhydroxyde monohydrate (55.8 mg, 1.33 mmol) according to the synthesis of 2-(6-(4-(3-(*tert*-butoxycarbonylamino)propylsulfonyl)phenyl)quinoline-4-carboxamido)acetic acid **(73)** to afford the title compound. MS (m/z): 621.3 [M+H$^+$]

Step E: Synthesis of (*S*)-*tert*-butyl 2-(*tert*-butoxycarbonyl(3-(4-(4-(2-(2-cyanopyrrolidin-1-yl)-2-oxoethylcarbamoyl)quinolin-6-yl)benzamido)propyl)amino)acetate (**98**)

2-(6-(4-(3-((2-*tert*-Butoxy-2-oxoethyl)(*tert*-butoxycarbonyl)amino)propylcarbamoyl)phenyl)quinoline-4-carboxamido)acetic acid **(97,** 412.5 mg, 0.67 mmol) was reacted with (*S*)-pyrrolidine-2-carbonitrile hydrochloride (132.2 mg, 1.00 mmol) according to the synthesis of 2-(quinoline-4-carboxamido)acetate **(40)** to yield the title compound. MS (m/z): 699.6 [M+H$^+$]

Step F: Synthesis of (*S*)-2-(3-(4-(4-(2-(2-cyanopyrrolidin-1-yl)-2-oxoethylcarbamoyl)quinolin-6-yl)benzamido)propylamino)acetic acid bis(4-methylbenzenesulfonate) (**99**)

(*S*)-*tert*-Butyl 2-(tert-butoxycarbonyl(3-(4-(4-(2-(2-cyanopyrrolidin-1-yl)-2-oxoethylcarbamoyl)quinolin-6-yl)benzamido)propyl)amino)acetate (**98**, 36.6 mg, 0.052 mmol) was reacted with *p*-toluenesulfonic acid monohydrate (13.9 mg, 0.073 mmol) according to the synthesis of (S)-6-(4-(3-aminopropylsulfonyl)phenyl)-4-(2-(2-cyano-4,4-difluoropyrrolidin-1-yl)-2-oxoethylcarbamoyl)quinoline 1-oxide 4-methylbenzenesulfonate (**81**). The crude product was used without further purification. MS (m/z): 543.2 [M+H$^+$]

Step G: Synthesis of (*S*)-2,2',2"-(10-(2-((carboxymethyl)(3-(4-(4-(2-(2-cyanopyrrolidin-1-yl)-2-oxoethylcarbamoyl)quinolin-6-yl)benzamido)propyl)amino)-2-oxoethyl)-1,4,7,10-tetraazacyclododecane-1,4,7-triyl)triacetic acid (3BP-3954)

(*S*)-2-(3-(4-(4-(2-(2-Cyanopyrrolidin-1-yl)-2-oxoethylcarbamoyl)quinolin-6-yl)benzamido)propylamino)acetic acid bis(4-methylbenzenesulfonate) **(99,** 51.4 mg, 0.058 mmol) was reacted with DOTA-NHS ester (47.1 mg, 0.058 mmol) according to the synthesis of (*S*)-2,2',2"-(10-(2-(3-(4-(4-(2-(2-(benzo[*d*]oxazole-2-carbonyl)pyrrolidin-1-yl)-2-oxoethylcarbamoyl)quinolin-6-yl)phenylsulfonyl)propylamino)-2-oxoethyl)-1,4,7,10-tetraazacyclododecane-1,4,7-triyl)triacetic acid (3BP-3581). The crude product was purified by HPLC. MS (m/z): 929.4 [M+H$^+$]

Step H: Synthesis of *tert-butyl* 2-(3-(benzyloxycarbonylamino)propylamino)acetate **(100)**

Bromoacetic acid *tert-butyl* ester (603.4 μL, 4.09 mmol) was added slowly to a solution of triethylamine (556.4 mL, 4.09 mmol), sodium iodide (678.3 mg, 4.09 mmol), and benzyl 3-aminopropylcarbamate (1000.0 mg, 4.09 mmol) in dry DCM (5 mL). The mixture was stirred at RT over night. After complete conversion of the starting material, water (5 mL) was added. the aqueous phase was extracted with DCM (5 mL). The combined organic phases were washed with brine (10 mL), dried ($Na_2SO_4$) and evaporated to dryness. The crude product was purified by flash chromatography on silica (eluent: DCM/ MeOH) to afford the title compound. MS (m/z): 323.3 [M+H$^+$]

Step I: Synthesis of *tert*-butyl 2-((3-(benzyloxycarbonylamino)propyl)*tert*-butoxycarbonyl)amino)acetate (101)

Sodium carbonate (223.4 mg, 106.0 mmol) was added to a solution of *tert-butyl* 2-(3-(benzyloxycarbonylamino)propylamino)acetate **(100,** 453.1 mg, 1.41 mmol) in a mixture of dioxane and water (1 : 1, 14.3 mL). The mixture was cooled to 0 °C and $BoC_2O$ (306.7 mg, 1.41 mmol) was added. The mixture was stirred over night and allowed to reach RT. After complete conversion of the starting material, water was added until a clear solution resulted and it was extracted with EA. The combined organic layers were dried ($Na_2SO_4$) and concentrated *in vacuo* to afford the title compound. MS (m/z): 423.1 [M+H$^+$]

Step J: Synthesis of *tert*-butyl 2-((3-aminopropyl)(*tert*-butoxycarbonyl)amino)acetate (**102**)

Palladium on activated charcoal (10 wt % loading, 40 mg) was added to a solution of *tert-butyl* 2-((3-(benzyloxycarbonylamino)propyl)(*tert*-butoxycarbonyl)amino)acetate (**101**, 315.5 mg, 0.75 mmol) in methanol (3.2 mL). The mixture was three times evacuated and ventilated with nitrogen. After evacuating and ventilating the mixture with hydrogen it was stirred under an atmosphere of hydrogen over night. After complete conversion, the mixture was filtered over a pad of Celite®. The filter cake was carefully rinsed with methanol. Subsequently, the filtrate was concentrated *in vacuo* to afford the title compound. MS (m/z): 289.3 [M+H$^+$]

The synthesis of 3BP-3785 followed similar procedures as described for 3BP-3954, but differed because the corresponding Boc-protected diamine introduced in step C was Boc-protected diaminopropane.

The synthesis of 3BP-3621 followed similar procedures as described for 3BP-3785, but used double fluorinated cyano-substituted pyrrolidine in step E.

The synthesis of 3BP-3631 followed similar procedures as described for 3BP-3621, where piperazine-derived Boc-protected diamine was used in Step C.

The synthesis of 3BP-4201 followed similar procedures as described for 3BP-3785, differing in that the pyrollidine fragment which was introduced in step E was boronate rather than cyano-substituted, and the Boc-cleavage step was performed with HCl with concomitant boronic acid deprotection.

**Example 3: Preparation of DOTA-transition metal complexes of compounds of the disclosure**

General procedure for the preparation of compounds comprising DOTA-transition metal-complexes from corresponding compounds comprising uncomplexed DOTA A 0.1 mM solution of the compound comprised by uncomplexed DOTA in

- 0.4 M sodium acetate, pH= 5 (Buffer A) (in case of Cu(II), Zn(II), In(III), Lu(III) or Ga(III) complexes) or
- 0.1 M ammonium acetate, pH = 8 (Buffer B) (in case of Eu(III) complexes) was diluted with a solution 0.1 mM solution of the corresponding metal salt in water whereby the molar ratio of compound to metal was adjusted to 1 : 3. The solution was stirred
- at 50 °C for 20 minutes (also referred to herein as Condition A) (in case of In(III), Lu(III), Ga(III), Zn(II) or Cu(II) complexes) or
- at room temperature overnight (also referred to herein as Condition B) (in case of Eu(III) complexes).

The solution was then applied to

- HPLC purification (also referred to herein as Purification A) or
- solid phase extraction (also referred to herein as Purification B).

In case of solid phase extraction 250 mg Varian Bondesil-ENV was placed in a 15 ml polystyrene syringe, prewashed with methanol (1 x 5 ml) and water (2 x 5 ml). Then the reaction solution was applied to the column. Thereafter elution was performed with water (2 x 5 ml - to remove excess salt), 5 ml of 50% ACN in water as first fraction and each of the next fractions were eluted with 5 ml of 50% ACN in water containing 0.1% TFA.

In either case (HPLC purification or solid phase extraction) fractions containing the pure product were pooled and freeze dried.

**Example 4: FACS Binding Assay**

In order to determine binding of compounds according to the present invention to FAP-expressing cells, a competitive FACS binding assay was established.

FAP-expressing human WI-38 fibroblasts (ECACC 90020107) were cultured in EMEM (Eagle's Minimum Essential Medium) including 15% fetal bovine serum (FBS), 2mM L-Glutamine and 1% Non-essential amino acids. Cells were detached with Accutase (Biolegend, #BLD-423201) and washed in FACS buffer (PBS including 1% FBS, Sigma-Aldrich, Cat# D8537). Cells were diluted in FACS buffer to a final concentration of 100.000 cells per ml and 200 μl of the cell suspension are transferred to a u-shaped non-binding 96-well plate (Greiner). Cells were washed in ice-cold FACS buffer and incubated with 3 nM of a Cy5-labeled derivative of a known FAP inhibitor (3BP-2935, structure see below) in the presence of increasing concentrations of test compounds at 4°C for 1 hour.

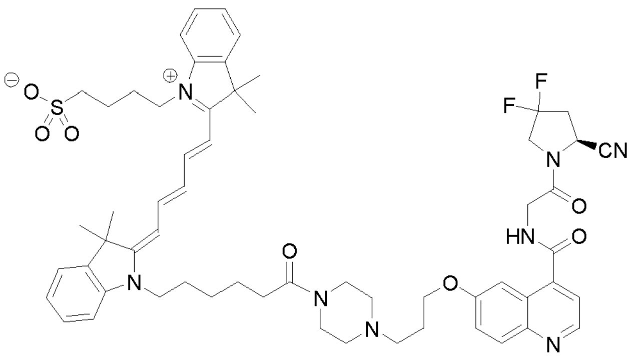

3BP-2935

Cell were washed twice with FACS buffer (see above) and resuspended in 200 µl FACS buffer. Cells were analyzed in an Attune NxT flow cytometer (Thermo Fisher Scientific). Median fluorescence intensities (Cy5 channel) was calculated by Attune NxT software and plotted against test compound concentrations. Four parameter logistic (4PL) curve fitting and pIC50 calculations were performed using ActivityBase software. The results of this assay for select compounds are shown in Table 7.

**Example 5: FAP Protease Activity Assay (human)**

In order to determine the inhibitory activity a FRET-based FAP protease activity assay was established.

Recombinant human FAP (R&D systems, # 3715-SE) was diluted in assay buffer (50 mM Tris, 1 M NaCl, 1 mg/mL BSA, pH 7.5) to a concentration of 3.6 nM. 25 µl of the FAP solution was mixed with 25 µl of a 3-fold serial dilution of the test compounds and incubated for 5 min in a white 96-well ProxiPlate (Perkin Elmer). As specific FAP substrate the FRET-peptide HiLyteFluor™ 488 - VS(D-)P SQG K(QXL® 520) - NH2 was used (Bainbridge, et al., Sci Rep, 2017, 7: 12524). 25 µL of a 30 µM substrate solution, diluted in assay buffer, was added. All solutions were equilibrated at 37°C prior to use. Substrate cleavage and increase in fluorescence (excitation at 485 nm and emission at 538 nm) was measured in a kinetic mode for 5 minutes at 37°C in a SPECTRAmax M5 plate reader (Molecular Devices). RFU/sec was calculated by SoftMax Pro software and plotted against test compound concentration. Four parameter logistic (4PL) curve fitting and pIC50 calculations were performed using ActivityBase software (IDBS). The results of this assay for select compounds are shown in Table 7.

**Example 6: Surface Plasmon Resonance Assay**

Surface plasmon resonance studies were performed using a Biacore™ T200 SPR system (Cytiva, formerly GE Healthcare). Briefly, polarized light is directed towards a gold-labeled sensor surface, and minimum intensity reflected light is detected. The angle of reflected light changes as molecules bind and dissociate. The gold-labeled sensor surface is loaded with FAP antibodies bearing FAP target proteins, whereby antibody binding does not occur at the substrate-binding site of FAP. Test compounds are contacted with the loaded surface, and a real-time interaction profile with the FAP ligand is recorded in a sensorgram. In real-time, the association and dissociation of a binding interaction is measured, enabling calculation of association and dissociation rate constants and the corresponding affinity constants. Importantly, a background response is generated due to the difference in the refractive indices of the running and sample buffers, as well as unspecific binding of the test compounds to the flow cell surface. This background is measured and subtracted by running the sample on a control flow cell coated with the same density of capture antibody in the absence of immobilized FAP. Furthermore, baseline drift correction of the binding data is performed, which is caused by slow dissociation of the captured FAP from the immobilized antibody. This drift is measured by injecting running buffer through a flow cell with the antibody and FAP immobilized to the sensor surface.

Biacore™ CM5 sensor chips were used. Human anti-FAP antibody (MAB3715, R&D systems) was diluted in 10 mM acetate buffer, pH 4.5, to a final concentration of 50 µg/mL. A 150 µL aliquot was transferred into plastic vials and placed into the sample rack of the Biacore™ T200 instrument. Amine Coupling Kit Reagent solutions were transferred into plastic vials and placed into the sample rack: 90 µL of 0.4 M 1-ethyl-3-(3-dimethylaminopropyl)carbodiimide (EDC), and 90 µL of 0.1 M N-hydroxysuccinimide (NHS). A 130 µL aliquot of 1 M ethanolamine-HCl, pH 8.5, was transferred into plastic vials and placed into the sample rack. The Biacore™ liquid system was set-up as follows: Separate bottles containing distilled water (1 L), Running Buffer (500 mL), as well as an empty bottle for waste were placed onto the buffer tray. A preinstalled program for immobilization was used, with an immobilization level of 7000 RU. Immobilization was performed at 25°C. The immobilization procedure of anti-FAP antibodies was performed, as described in the Table 5.

**Table 5: Immobilization protocol for anti-FAP antibodies used on the CM5 sensor chip.**

| Step | Injected solution | Contact time | Flow rate |
|---|---|---|---|
| Surface conditioning | 50 mM NaOH | 300 s | 10 μL/min |
| Surface activation | EDC/NHS | 420 s | 10 μL/min |
| Washing | Ethanolamine | 90 s | 10 μL/min |
| Ligand binding | Human/mouse antibodies diluted in acetate buffer | 420 s | 10 μL/min |
| Washing | Running Buffer | 40 s | 10 μL/min |
| Deactivation of reactive, non-ligand bound surface | 1 M ethanolamine | 420 s | 10 μL/min |
| Washing | Running Buffer | 30 s | 10 μL/min |

Human recombinant FAP was diluted in Running Buffer to a final concentration of 20 μg/mL. A 100 μL aliquot of human FAP-Working-Solution was transferred into plastic vials and placed into a sample rack. A 0.5 mM Compound-Stock-Solution was prepared by dissolving each compound in DMSO. For each test compound, Compound-Stock-Solutions were diluted in Running Buffer (HBST) at 500 nM and further diluted with HBST-DMSO Buffer (0.1% DMSO). SPR binding analyses for binary complexes were performed in SCK mode at 25°C. Table 6 describes the protocol for capturing and assessment of the binding kinetics. Following three SCK measurements, a baseline drift was assessed by injecting running buffer through a flow cell, with the antibody and FAP immobilized to the sensor surface.

**Table 6: Protocol for assessing the binding kinetics.**

| Step | Injected solution | Contact time | Flow rate |
|---|---|---|---|
| Startup cycle as a triple run: Washing & surface regeneration | HBST-DMSO Buffer<br>10 mM glycine, pH 2 | 60 s<br>5s | 30 μL/min |
| Binding target protein FAP (capturing) | 20 μg/mL rhFAP or<br>4 μg/mL rmFAP | 600 s | 5 μL/min |
| Washing (removal of unbound FAP) | HBST-DMSO-Buffer | 2700 s | 30 μL/min |
| 1. Binding kinetics of test compound | Dilution no. 5 (0.19 nM) | 120 s | 30 μL/min |
| 2. Binding kinetics of test compound | Dilution no. 4 (0.78 nM) | 120 s | 30 μL/min |
| 3. Binding kinetics of test compound | Dilution no. 3 (3.125 nM) | 120 s | 30 μL/min |
| 4. Binding kinetics of test compound | Dilution no. 2 (12.5 nM) | 120 s | 30 μL/min |
| 5. Binding kinetics of test compound | Dilution no. 1 (50 nM) | 120 s | 30 μL/min |
| Dissociation cycle | HBST-DMSO Buffer | 1800 s | 30 μL/min |
| Regeneration | 10 mM glycine, pH 2 | 7s | 30 μL/min |

For each test compound, SPR raw data in the form of resonance units (RU) were plotted as sensorgrams using the Biacore™ T200 control software. The signal from the blank sensorgram was subtracted from that of the test compound sensorgram (blank corrected). The blank corrected sensorgram was corrected for baseline drift by subtracting the sensorgram of a SCK run without the test compound (running buffer only). The association rate ($k_{on}$), dissociation rate ($k_{off}$),

dissociation constant ($K_D$), and $t_{1/2}$ were calculated from Blank-normalized SPR data using the 1:1 Langmuir binding model from the Biacore™ T200 evaluation software. Raw data and fit results were imported as text files in IDBS. The $pK_D$ value (negative decadic logarithm of dissociation constant) was calculated in the IDBS excel template.

The results of this assay for select compounds are shown in Table 7.

**Example 7: FAP Protease Activity Assay (mouse)**

In order to determine the inhibitory activity a FRET-based FAP protease activity assay was established.

Recombinant mouse FAP (R&D systems, # 8647-SE) was diluted in assay buffer (50 mM Tris, 1 M NaCl, 1 mg/mL BSA, pH 7.5) to a concentration of 3.6 nM. 25 μl of the FAP solution was mixed with 25 μl of a 3-fold serial dilution of the test compounds and incubated for 5 min in a white 96-well ProxiPlate (Perkin Elmer). As specific FAP substrate the FRET-peptide HiLyteFluor™ 488 - VS(D-)P SQG K(QXL® 520) - NH2 was used (Bainbridge, et al., Sci Rep, 2017, 7: 12524). 25 μL of a 30 μM substrate solution, diluted in assay buffer, was added. All solutions were equilibrated at 37°C prior to use. Substrate cleavage and increase in fluorescence (excitation at 485 nm and emission at 538 nm) was measured in a kinetic mode for 5 minutes at 37°C in a SPECTRAmax M5 plate reader. RFU/sec was calculated by SoftMax Pro software and plotted against test compound concentration. Four parameter logistic (4PL) curve fitting and pIC50 calculations were performed using ActivityBase software. The results of this assay for select compounds are shown in Table 7.

**Example 8: PREP and DPP4 Protease Activity Assay**

In order to test selectivity of FAP binding compounds toward both PREP and DPP4, protease activity assays were performed analogous to the FAP activity assay described above (Example 5) with following exceptions.

PREP activity was measured with recombinant human PREP (R&D systems, #4308-SE). The PREP activity assay was performed using the FACS buffer (PBS including 1% FBS, Sigma-Aldrich, Cat# D8537). As substrate 50 μM Z-GP-AMC (Bachem, # 4002518) was used. The DPP4 activity assay was performed in DPP assay buffer (25 mM Tris, pH 8.0). Recombinant human DPP4 was purchased from R&D systems (#9168-SE). 20 μM of GP-AMC (Santa Cruz Biotechnology, #115035-46-6) was used as substrate.

Fluorescence of AMC (excitation at 380 nm and emission at 460 nm) after cleavage was measured in a kinetic mode for 5 minutes at 37°C in a SPECTRAmax M5 plate reader. RFU/sec was calculated by SoftMax Pro software and plotted against test compound concentration. Four parameter logistic (4PL) curve fitting and pIC50 calculations were performed using ActivityBase software. The results of this assay for select compounds are shown in the following Table 7.

Table 7. Compound ID, structure, Exact Mass ([M+H] calculated), Exact Mass ([M+H] found), pIC50 (FACS), pIC50 (Activity), pKD (Biacore), HL (Biacore) pIC50 (mFAP), pIC50 (DPP4) and pIC50(PREP)

**Compound ID/Structure**

3BP-2762

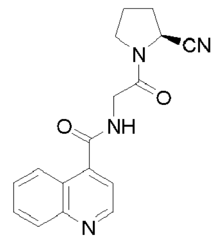

| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
|---|---|---|---|---|---|---|---|---|
| 309.135 | 309.135 | 7.8 | 8.1 | 8.5 | 1.6 | 8.2 | <6 | 6.2 |

**Compound ID/Structure**

3BP-2929

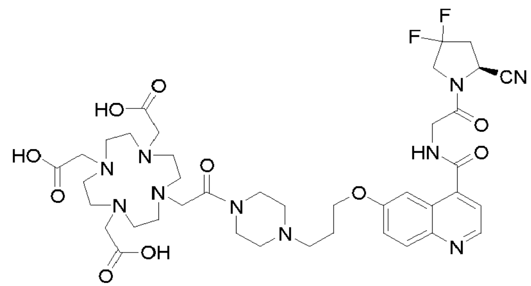

| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
|---|---|---|---|---|---|---|---|---|
| 873.400 | 873.410 | 8.9 | 9 | >11 | >500 | 8.8 | <6 | <6 |

(continued)

| Compound ID/Structure | | | | | | | | |
|---|---|---|---|---|---|---|---|---|
| 3BP-3126 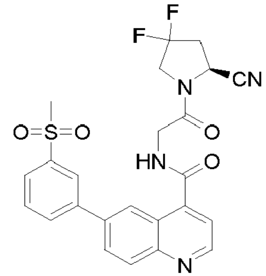 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 499.118 | 499.126 | 8.8 | 9.2 | 10.1 | 29 | 9 | 6.1 | <6 |
| **Compound ID/Structure** | | | | | | | | |
| 3BP-3127 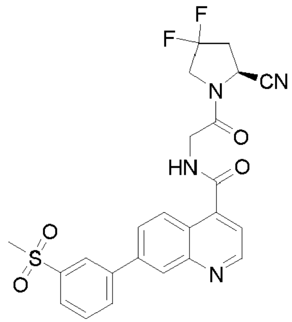 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 499.118 | 499.128 | 8.7 | 9.1 | 10.1 | 29.5 | 8.9 | <6 | <6 |

(continued)

| Compound ID/Structure | | | | | | | | |
|---|---|---|---|---|---|---|---|---|
| 3BP-3128 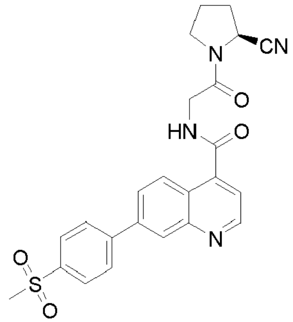 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 463.13 | 463.146 | 8.2 | 8.8 | 9.2 | 4 | n.d. | <6 | 6.1 |
| **Compound ID/Structure** | | | | | | | | |
| 3BP-3132 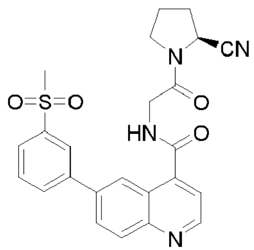 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 463.137 | 463.146 | 8.4 | 9 | 9.4 | 2.5 | n.d. | <6 | <6 |

(continued)

| Compound ID/Structure | | | | | | | | |
|---|---|---|---|---|---|---|---|---|
| 3BP-3133 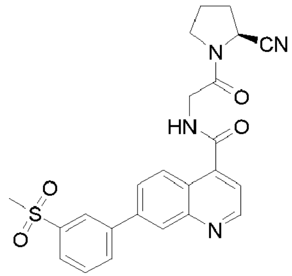 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 463.137 | 463.145 | 8.2 | 8.7 | 9.3 | 5.7 | n.d. | <6 | <6 |
| **Compound ID/Structure** | | | | | | | | |
| 3BP-3134 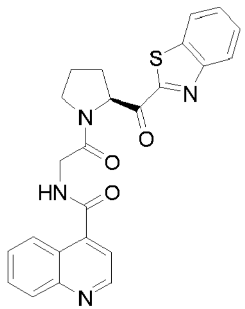 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 445.127 | 445.132 | 7.2 | 7.4 | 8.4 | 0.3 | n.d. | <6 | 7.8 |

(continued)

| Compound ID/Structure | | | | | | | | |
|---|---|---|---|---|---|---|---|---|
| 3BP-3205 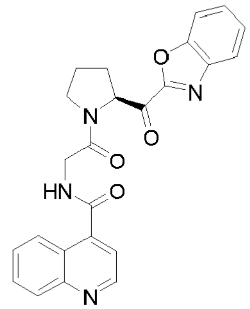 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 429.149 | 429.159 | 8.2 | 8.8 | 9.3 | 3.7 | 8.7 | <6 | 9 |
| **Compound ID/Structure** | | | | | | | | |
| 3BP-3206 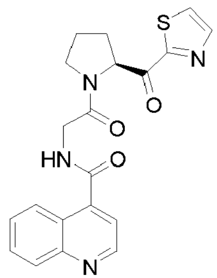 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 395.111 | 395.119 | 7 | 7.3 | 7.9 | 0.3 | n.d. | <6 | 7.6 |

(continued)

| Compound ID/Structure | | | | | | | | |
|---|---|---|---|---|---|---|---|---|
| 3BP-3207 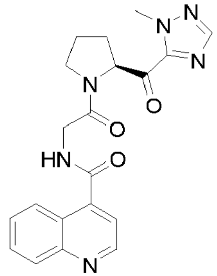 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 393.161 | 393.169 | <6 | 6.5 | n.d. | n.d. | n.d. | n.d. | n.d. |
| **Compound ID/Structure** | | | | | | | | |
| 3BP-3208 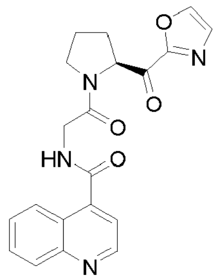 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 379.134 | 379.141 | 7.9 | 8.2 | 8.6 | 0.6 | n.d. | <6 | 9.2 |

(continued)

| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
|---|---|---|---|---|---|---|---|---|
| **Compound ID/Structure** | | | | | | | | |
| 3BP-3232 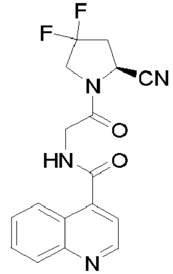 | | | | | | | | |
| 345.109 | 345.117 | 8.4 | 8.8 | 9.5 | 10.4 | 8.6 | <6 | 6.4 |
| **Compound ID/Structure** | | | | | | | | |
| 3BP-3233 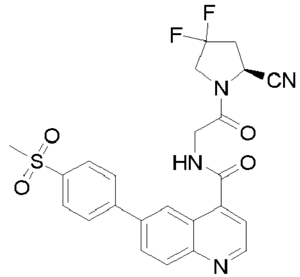 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 499.118 | 499.128 | 9 | 9.2 | 10.6 | 125 | 9.1 | <6 | <6 |

(continued)

| Compound ID/Structure | | | | | | | | |
|---|---|---|---|---|---|---|---|---|
| 3BP-3234 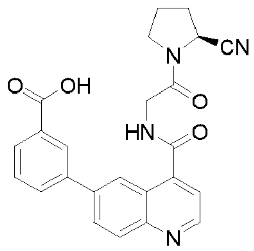 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 499.128 | 429.158 | 8.5 | 8.8 | n.d. | n.d. | n.d. | <6 | <6 |
| **Compound ID/Structure** | | | | | | | | |
| 3BP-3235 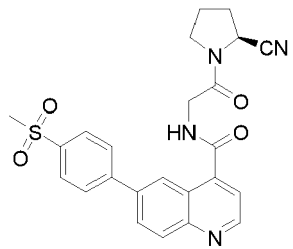 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 463.137 | 463.144 | 8.4 | 9.2 | 9.8 | 12 | n.d. | <6 | <6 |
| **Compound ID/Structure** | | | | | | | | |
| 3BP-3236 | | | | | | | | |

(continued)

| Compound ID/Structure | | | | | | | | |
|---|---|---|---|---|---|---|---|---|
| 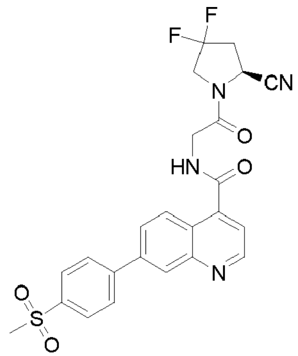 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 499.118 | 499.129 | 8.5 | 9.1 | 10 | 37 | n.d. | <6 | <6 |
| Compound ID/Structure | | | | | | | | |
| 3BP-3293 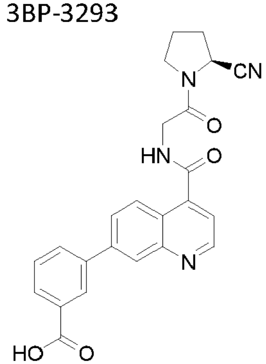 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 429.149 | 429.157 | 8.6 | 8.8 | n.d. | n.d. | n.d. | <6 | <6 |

(continued)

| Compound ID/Structure | | | | | | | | |
|---|---|---|---|---|---|---|---|---|
| 3BP-3294 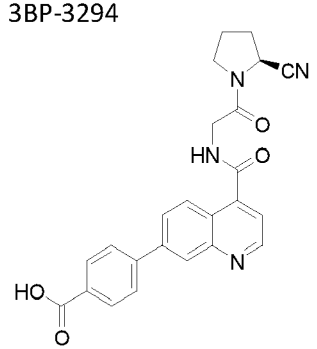 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 429.149 | 429.157 | 8.4 | 8.7 | n.d. | n.d. | n.d. | <6 | <6 |
| **Compound ID/Structure** | | | | | | | | |
| 3BP-3295 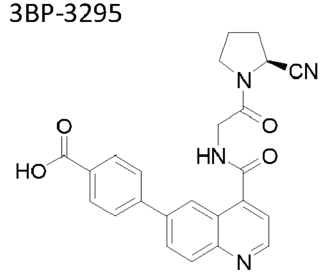 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 429.149 | 429.157 | 8.6 | 8.8 | 9.7 | 11 | n.d. | <6 | <6 |

(continued)

| Compound ID/Structure | | | | | | | | |
|---|---|---|---|---|---|---|---|---|
| 3BP-3376 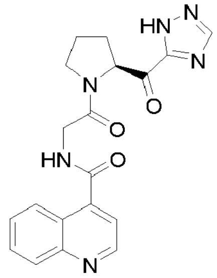 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 379.145 | 379.152 | 6.7 | 7.2 | n.d. | n.d. | 7.1 | n.d. | n.d. |
| **Compound ID/Structure** | | | | | | | | |
| 3BP-3412 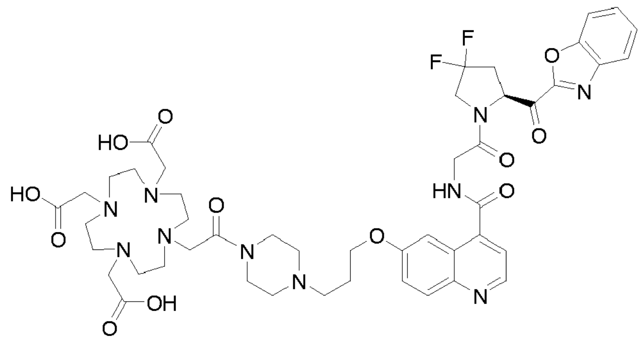 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 993.421 | 993.429 | 7.9 | 8.6 | 8.6 | 57 | 8.3 | <6 | 6.7 |

(continued)

| Compound ID/Structure | | | | | | | | |
|---|---|---|---|---|---|---|---|---|
| 3BP-3467<br>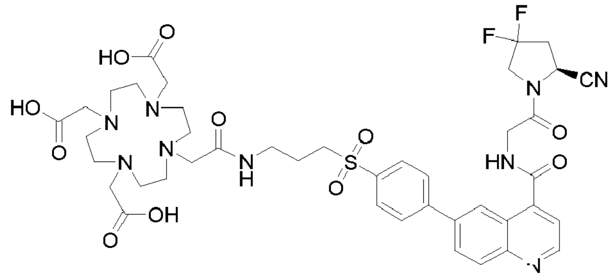 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 928.341 | 928.351 | 9.2 | 9.1 | >11 | >500 | 9 | 6.7 | <6 |
| **Compound ID/Structure** | | | | | | | | |
| 3BP-3468<br>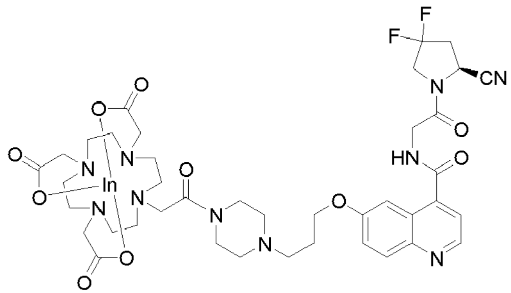 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 492.137 [M]/2 | 492.148 [M]/2 | 8.7 | 9.2 | 10.5 | 69 | 9 | <6 | <6 |

(continued)

| Compound ID/Structure | | | | | | | | |
|---|---|---|---|---|---|---|---|---|
| 3BP-3469 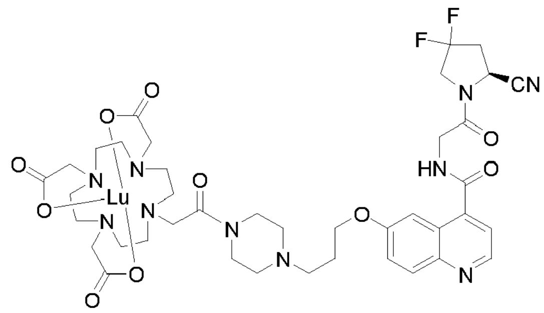 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 1045.318 | 1045.323 | 8.7 | 9.3 | 10.2 | 35.5 | 9 | <6 | n.d. |
| **Compound ID/Structure** | | | | | | | | |
| 3BP-3515 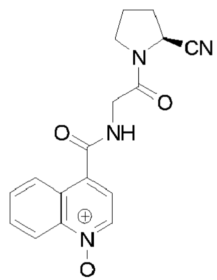 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 325.123 | 325.131 | 7.1 | 7.6 | n.d. | n.d. | 7.5 | <6 | 6.3 |

(continued)

| Compound ID/Structure | | | | | | | | |
|---|---|---|---|---|---|---|---|---|
| 3BP-3577 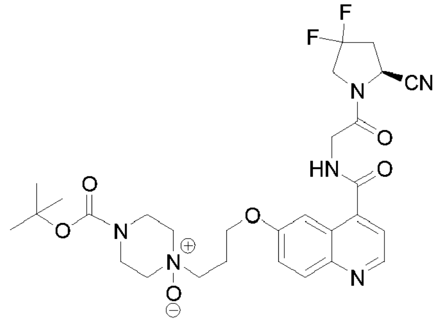 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 603.267 | 603.277 | 8.6 | 9 | n.d. | n.d. | n.d. | n.d. | n.d. |
| **Compound ID/Structure** | | | | | | | | |
| 3BP-3578 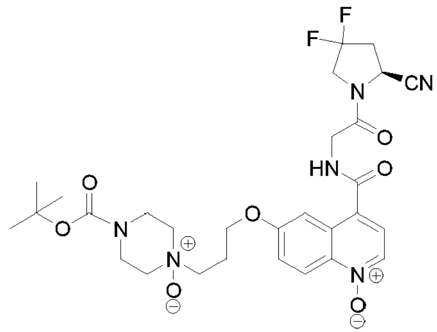 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 619.262 | 619.269 | 8 | 8.6 | n.d. | n.d. | n.d. | n.d. | n.d. |

(continued)

| Compound ID/Structure | | | | | | | | |
|---|---|---|---|---|---|---|---|---|
| 3BP-3579 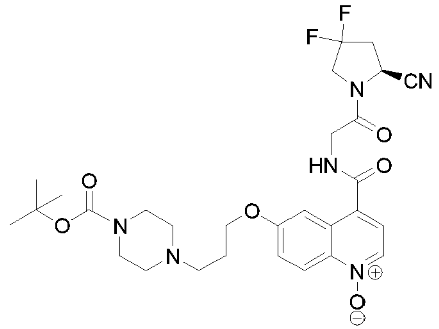 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 603.267 | 603.284 | 8 | 8.6 | n.d. | n.d. | n.d. | n.d. | n.d. |
| **Compound ID/Structure** | | | | | | | | |
| 3BP-3580 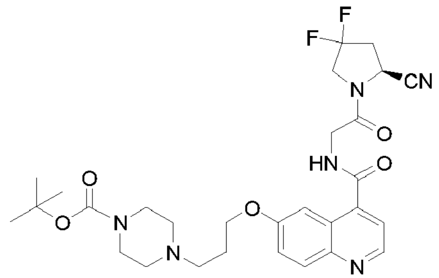 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 587.273 | 587.280 | 8.4 | 8.8 | n.d. | n.d. | n.d. | n.d. | n.d. |

(continued)

| Compound ID/Structure | | | | | | | | |
|---|---|---|---|---|---|---|---|---|
| 3BP-3581 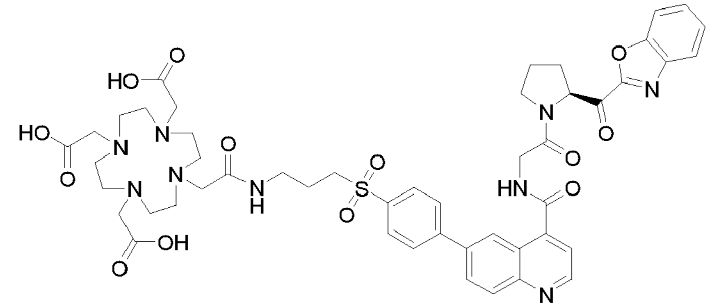 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 1012.381 | 1012.388 | 8.8 | 8.9 | >11 | >500 | 8.6 | 6.4 | 8.2 |
| Compound ID/Structure | | | | | | | | |
| 3BP-3582 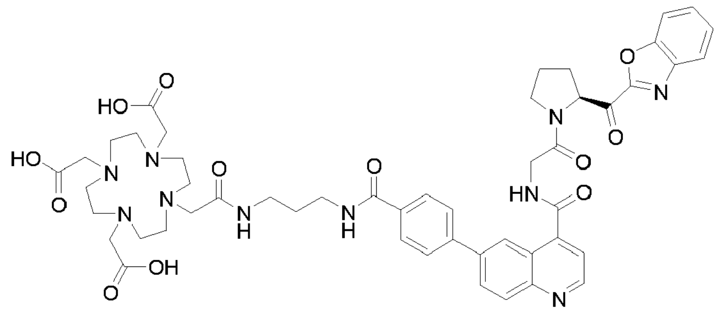 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 991.425 | 991.432 | 8.6 | 8.8 | 10.3 | 259.3 | 8.7 | <6 | 8.2 |

(continued)

| Compound ID/Structure | | | | | | | | |
|---|---|---|---|---|---|---|---|---|
| 3BP-3611 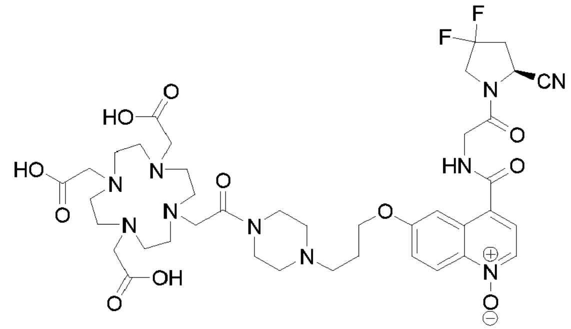 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 889.395 | 889.403 | 8.5 | 9 | 9.8 | 19 | 8.8 | <6 | n.d. |
| Compound ID/Structure | | | | | | | | |
| 3BP-3621 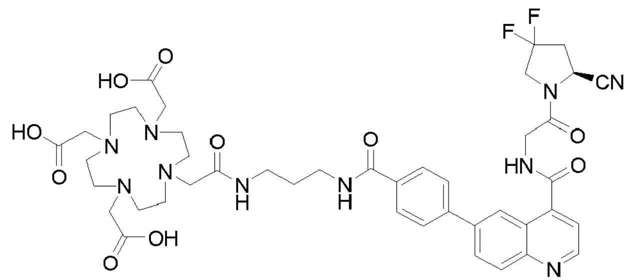 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 907.385 | 907.398 | 8.9 | 9.2 | >11 | 110 | 9.2 | <6 | n.d. |

(continued)

| Compound ID/Structure | | | | | | | | |
|---|---|---|---|---|---|---|---|---|
| 3BP-3622 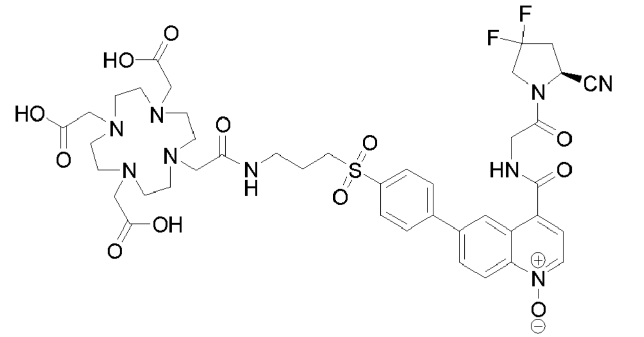 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 944.336 | 944.343 | 8.8 | 9.2 | >11 | 110 | 9.1 | 7 | n.d. |
| **Compound ID/Structure** | | | | | | | | |
| 3BP-3631 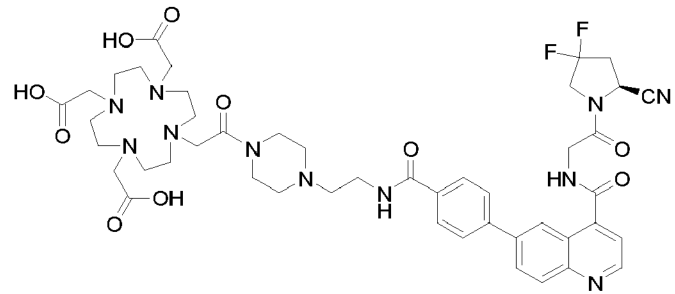 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 962.427 | 962.434 | 8.7 | 9.1 | >11 | >500 | 8.9 | <6 | <6 |

(continued)

| Compound ID/Structure | | | | | | | | |
|---|---|---|---|---|---|---|---|---|
| 3BP-3632 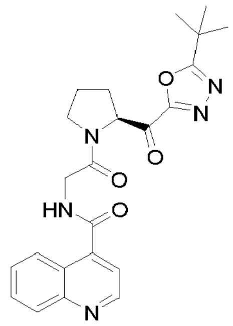 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 436.192 | 436.199 | 7.8 | 8.4 | 8.6 | 0.4 | 8.4 | <6 | 8.9 |
| **Compound ID/Structure** | | | | | | | | |
| 3BP-3663 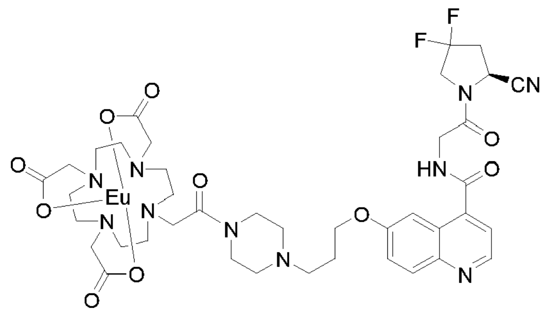 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 1023.298 | 1023.301 | 8.6 | 9.3 | n.d. | n.d. | 9.1 | n.d. | n.d. |

(continued)

| Compound ID/Structure | | | | | | | | |
|---|---|---|---|---|---|---|---|---|
| 3BP-3707 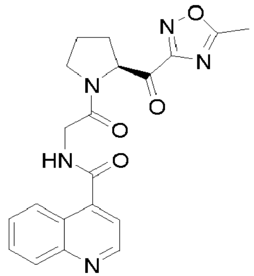 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 394.145 | 394.153 | 6.6 | 7.1 | n.d. | n.d. | 6.9 | n.d. | 8.9 |
| **Compound ID/Structure** | | | | | | | | |
| 3BP-3772 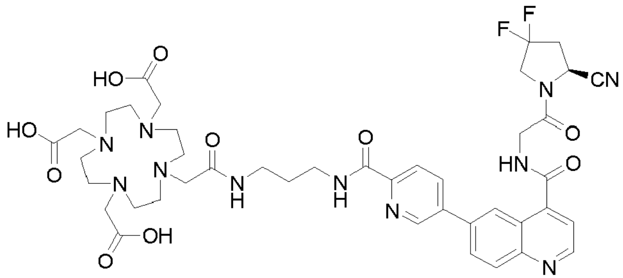 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 908.380 | 908.387 | 8.8 | 9.1 | >11 | >500 | 8.8 | <6 | <6 |

(continued)

| Compound ID/Structure | | | | | | | | |
|---|---|---|---|---|---|---|---|---|
| 3BP-3773 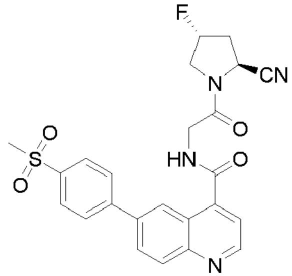 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 481.128 | 481.136 | 7.3 | 8.5 | n.d. | n.d. | 8 | <6 | n.d. |
| Compound ID/Structure | | | | | | | | |
| 3BP-3774 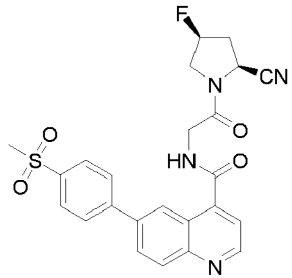 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 481.128 | 481.136 | 8.8 | 9.2 | n.d. | n.d. | 9.2 | <6 | n.d. |

(continued)

| Compound ID/Structure | | | | | | | | |
|---|---|---|---|---|---|---|---|---|
| 3BP-3785 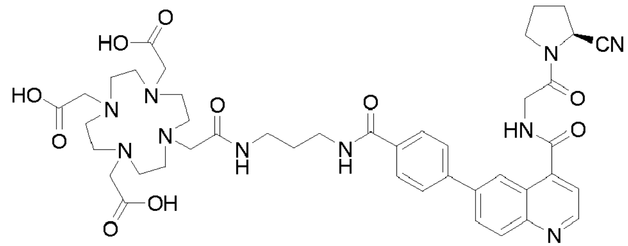 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 871.403 | 871.409 | 8.7 | 9 | 10.2 | 160 | 9 | <6 | n.d. |
| Compound ID/Structure | | | | | | | | |
| 3BP-3828 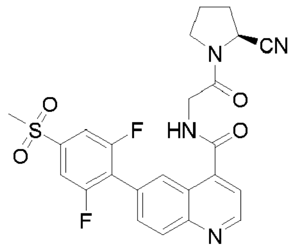 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 499.118 | 499.125 | 8.5 | 9.3 | n.d. | n.d. | 9 | <6 | n.d. |
| Compound ID/Structure | | | | | | | | |
| 3BP-3829 | | | | | | | | |

(continued)

| Compound ID/Structure | | | | | | | | |
|---|---|---|---|---|---|---|---|---|
| 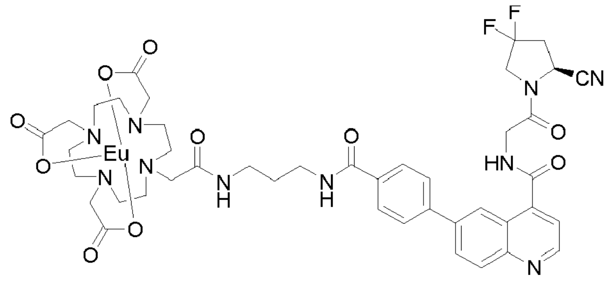 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 1057.282 | 1057.293 | 8.9 | 9.4 | >11 | >500 | 8.9 | <6 | n.d. |
| Compound ID/Structure | | | | | | | | |
| 3BP-3830 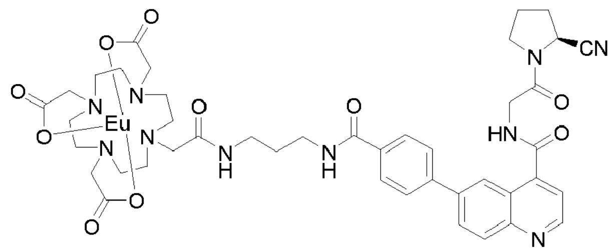 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 1021.301 | 1021.312 | 8.5 | 9.3 | 10 | 64 | 8.7 | <6 | n.d. |

(continued)

| Compound ID/Structure | | | | | | | | |
|---|---|---|---|---|---|---|---|---|
| 3BP-3951 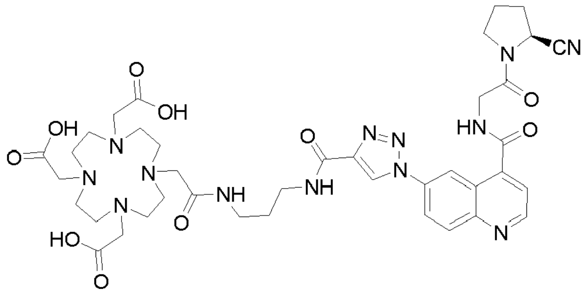 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 862.389 | 862.402 | 8.4 | 9.1 | 10 | 95 | 9.1 | <6 | <6 |
| **Compound ID/Structure** | | | | | | | | |
| 3BP-3952 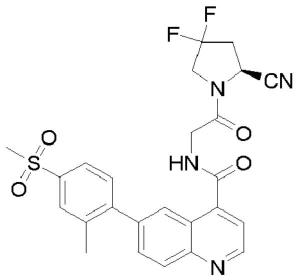 | | | | | | | | |
| **Exact Mass (calc)** | **Exact Mass (found)** | **pIC50 (FACS)** | **pIC50 (Activity)** | **pKD (Biacore)** | **HL [min] (Biacore)** | pIC50 (mouseFAP) | **pIC50 (DPP4)** | **pIC50 (PREP)** |
| 513.134 | 513.146 | 8.7 | 9.3 | n.d. | n.d. | 8.8 | 6.1 | n.d. |

(continued)

| Compound ID/Structure | | | | | | | | |
|---|---|---|---|---|---|---|---|---|
| 3BP-3953 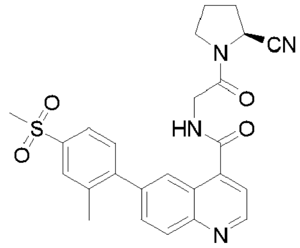 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 477.153 | 477.165 | 7.9 | 9.2 | n.d. | n.d. | 8.9 | <6 | n.d. |
| **Compound ID/Structure** | | | | | | | | |
| 3BP-3954 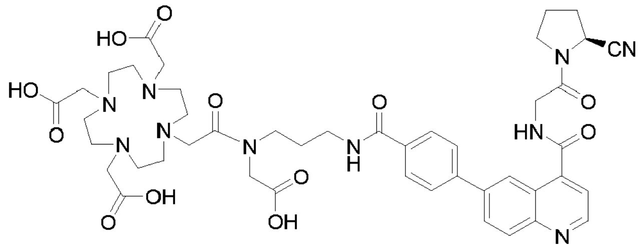 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | |
| 929.409 | 929.417 | 8.4 | 8.9 | >11 | >500 | 8.9 | <6 | |

(continued)

| Compound ID/Structure | | | | | | | | |
|---|---|---|---|---|---|---|---|---|
| 3BP-4025 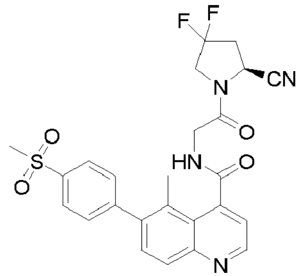 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 513.134 | 513.148 | 8.7 | 9.3 | n.d. | n.d. | 8.7 | n.d. | n.d. |
| Compound ID/Structure | | | | | | | | |
| 3BP-4026 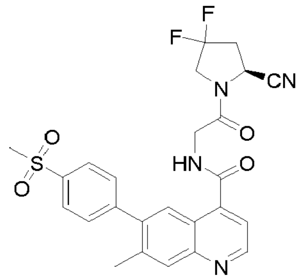 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 513.134 | 513.148 | 8.9 | 9.3 | n.d. | n.d. | 8.8 | n.d. | n.d. |

(continued)

| Compound ID/Structure | | | | | | | | |
|---|---|---|---|---|---|---|---|---|
| 3BP-4076 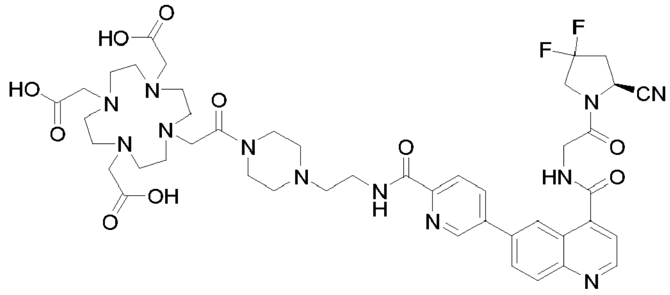 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 963.422 | 963.428 | 8.9 | 9.2 | >11 | >500 | n.d. | n.d. | n.d. |
| **Compound ID/Structure** | | | | | | | | |
| 3BP-4081 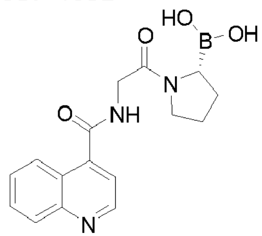 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 328.140 | 328.152 | 8.5 | 8.9 | n.d. | n.d. | n.d. | n.d. | n.d. |

(continued)

| Compound ID/Structure | | | | | | | | |
|---|---|---|---|---|---|---|---|---|
| 3BP-4084 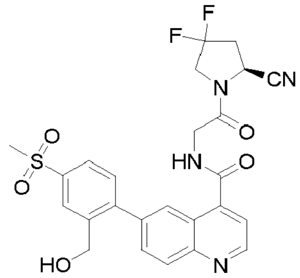 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 529.129 | 529.142 | 8.7 | 9.3 | >11 | >500 | n.d. | n.d. | n.d. |

| Compound ID/Structure | | | | | | | | |
|---|---|---|---|---|---|---|---|---|
| 3BP-4085 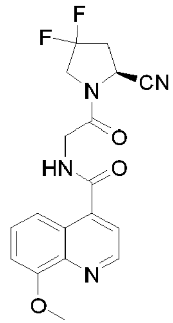 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 375.120 | 375.132 | 7.7 | 8.2 | n.d. | n.d. | n.d. | n.d. | n.d. |

(continued)

| Compound ID/Structure | | | | | | | | |
|---|---|---|---|---|---|---|---|---|
| 3BP-4150 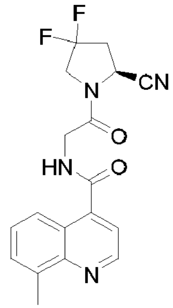 | | | | | | | | |
| **Exact Mass (calc)** | **Exact Mass (found)** | **pIC50 (FACS)** | **pIC50 (Activity)** | **pKD (Biacore)** | HL **[min] (Biacore)** | **pIC50 (mouseFAP)** | **pIC50 (DPP4)** | **pIC50 (PREP)** |
| 359.125 | 359.133 | 7.6 | 7.9 | n.d. | n.d. | n.d. | n.d. | n.d. |
| **Compound ID/Structure** | | | | | | | | |
| 3BP-4151 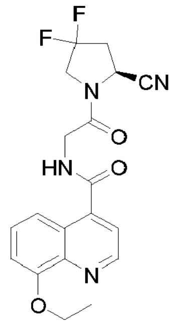 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 389.136 | 389.144 | 7.7 | 8.5 | n.d. | n.d. | n.d. | n.d. | n.d. |

(continued)

| Compound ID/Structure | | | | | | | | |
|---|---|---|---|---|---|---|---|---|
| 3BP-4152 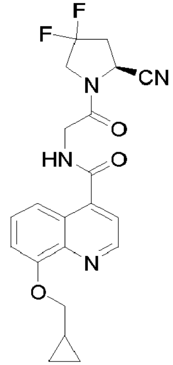 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 415.151 | 415.160 | 7.8 | 8.5 | n.d. | n.d. | n.d. | n.d. | n.d. |
| Compound ID/Structure | | | | | | | | |
| 3BP-4197 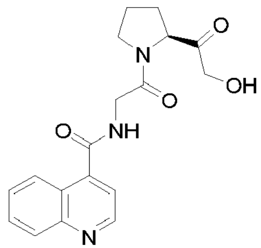 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 342.139 | 342.150 | 8 | 8.5 | 9 | 3.1 | 8.2 | n.d. | n.d. |
| Compound ID/Structure | | | | | | | | |
| 3BP-4199 | | | | | | | | |

(continued)

| Compound ID/Structure | | | | | | | | |
|---|---|---|---|---|---|---|---|---|
| 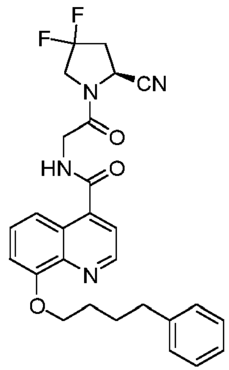 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 493.198 | 493.209 | 7.7 | 8.2 | n.d. | n.d. | n.d. | n.d. | n.d. |
| **Compound ID/Structure** | | | | | | | | |
| 3BP-4200 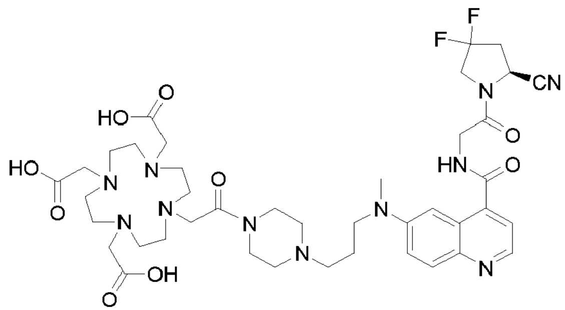 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 886.432 | 886.454 | 9 | 8.9 | >11 | >500 | 8.6 | n.d. | n.d. |

(continued)

| Compound ID/Structure | | | | | | | | |
|---|---|---|---|---|---|---|---|---|
| 3BP-4201 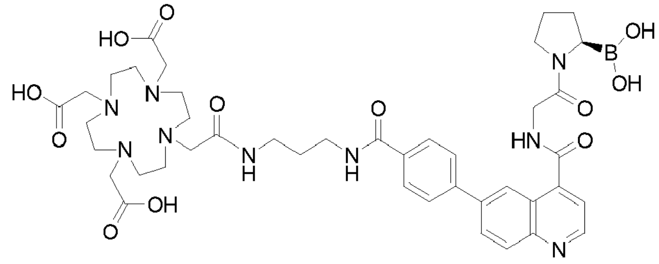 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 872.404 [M+H-$H_2O$] | 872.427 [M+H-$H_2O$] | 8.9 | 8.8 | >11 | >500 | 8.5 | <6 | 8.2 |
| **Compound ID/Structure** | | | | | | | | |
| 3BP-4663 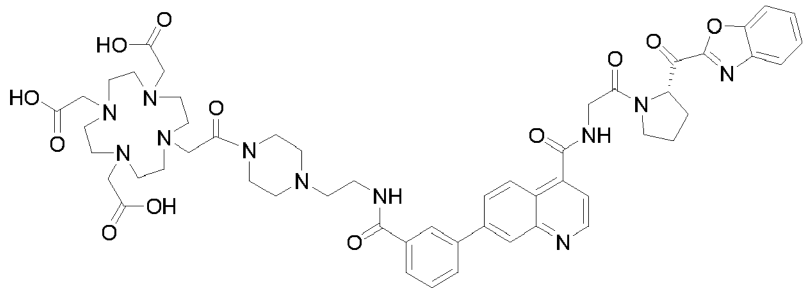 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 1046.467 | 1046.490 | 8.4 | 8.7 | >11 | >500 | 8.9 | <6 | 8.4 |

(continued)

| Compound ID/Structure | | | | | | | | |
|---|---|---|---|---|---|---|---|---|
| 3BP-4664 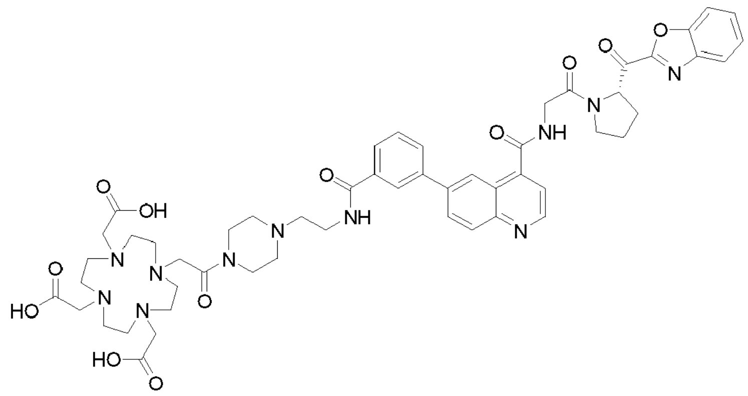 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 1046.467 | 1046.503 | 8.5 | 8.6 | 10.1 | 289.5 | 8.6 | <6 | 7.8 |
| Compound ID/Structure | | | | | | | | |
| 3BP-4665 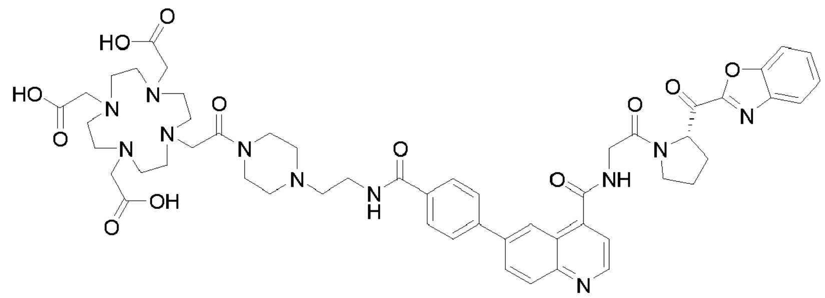 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 1046.467 | 1046.495 | 7.9 | 8.4 | 9.1 | 10.5 | 8.1 | <6 | 8.2 |

(continued)

| Compound ID/Structure | | | | | | | | |
|---|---|---|---|---|---|---|---|---|
| 3BP-4694 | | | | | | | | |
| 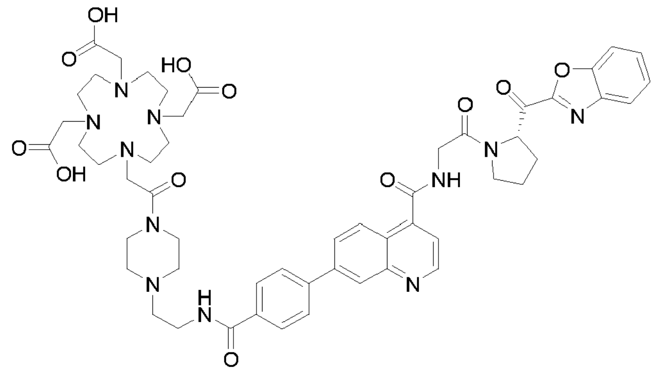 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 1046.467 | 1046.488 | 8.4 | 8.7 | >11 | >500 | 8.8 | <6 | 8.7 |
| **Compound ID/Structure** | | | | | | | | |
| 3BP-4808 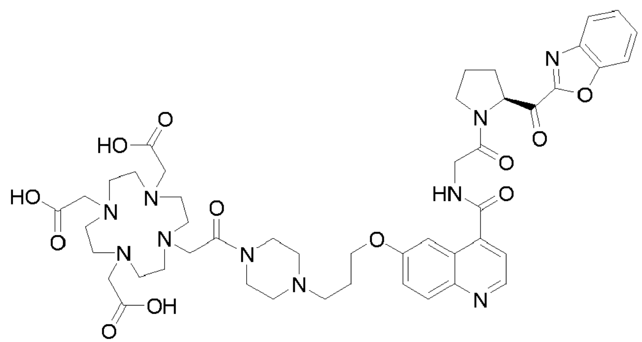 | | | | | | | | |
| **Exact Mass** (calc) | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 957.440 | 957.496 | 8.6 | 8.9 | >11 | >500 | 8.5 | <6 | 8.6 |

(continued)

| Compound ID/Structure | | | | | | | | |
|---|---|---|---|---|---|---|---|---|
| 3BP-4809 | | | | | | | | |
| 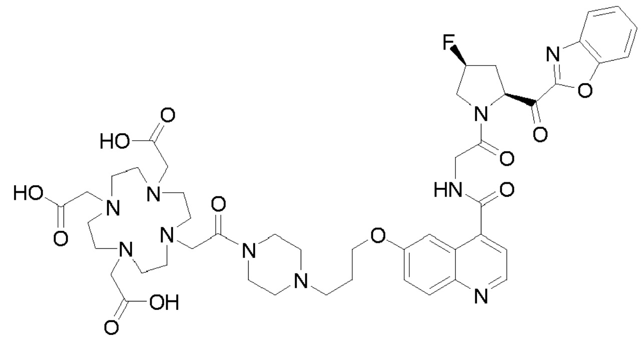 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 975.431 | 975.433 | 8.6 | 9 | >11 | >500 | 8.5 | <6 | 7.8 |
| **Compound ID/Structure** | | | | | | | | |
| 3BP-4810 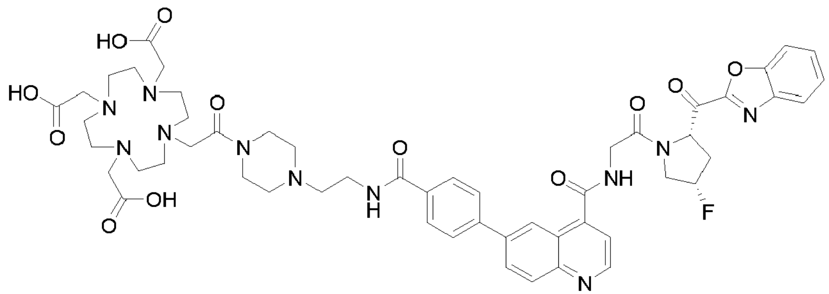 | | | | | | | | |
| Exact Mass (calc) | Exact Mass (found) | pIC50 (FACS) | pIC50 (Activity) | pKD (Biacore) | HL [min] (Biacore) | pIC50 (mouseFAP) | pIC50 (DPP4) | pIC50 (PREP) |
| 1064.457 | 1064.514 | 7.6 | 8.5 | 9 | 4.8 | 7.9 | <6 | 7.3 |

(continued)

| Compound ID/Structure | | | | | | | | |
|---|---|---|---|---|---|---|---|---|
| 3BP-4811 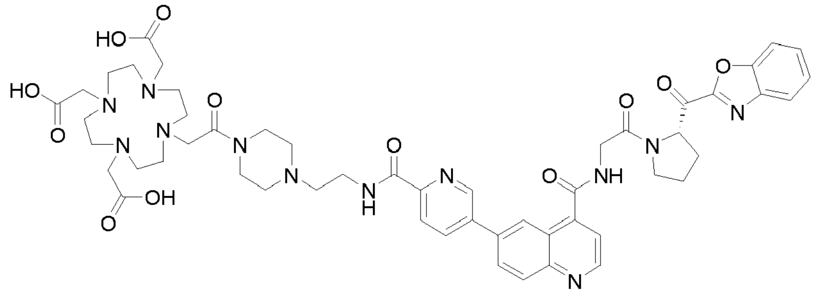 | | | | | | | | |
| **Exact Mass (calc)** | **Exact Mass (found)** | **pIC50 (FACS)** | **pIC50 (Activity)** | **pKD (Biacore)** | **HL [min] (Biacore)** | **pIC50 (mouseFAP)** | **pIC50 (DPP4)** | **pIC50 (PREP)** |
| 1047.462 | 1047.521 | 7.6 | 8.4 | 8.7 | 4.1 | 7.8 | <6 | 7.9 |

**Example 9: Plasma stability assay**

In order to determine the stability of selected compounds of the invention in human and mouse plasma, a plasma stability assay was carried out. Such plasma stability assay measures degradation of compounds of the present invention in blood plasma. This is an important characteristic of a compound as compounds, with the exception of pro-drugs, which rapidly degrade in plasma, generally show poor *in vivo* efficacy. The results show that those compounds are highly stable in human and mouse plasma. The stability is sufficient for the diagnostic, therapeutic and theragnostic use of these compounds according to the present invention.

The plasma stability samples were prepared by spiking 50 µl plasma aliquots (all sodium citrate 3.8%) with 1 µl of a 0.5 mM compound stock solution in DMSO. After vortexing, the samples were incubated in a Thermomixer at 37°C for 4 or 6 hours. After incubation the samples were stored on ice until further treatment. All samples were prepared in duplicates.

A suitable internal standard was added to each sample (1 µl of a 0.5 mM stock solution in DMSO). Protein precipitation was performed using two different methods depending on the compound conditions as indicated in Table 8.

A) 250 µl of acetonitrile containing 1% trifluoroacetic acid was added. After incubation at room temperature for 30 min the precipitate was separated by centrifugation and 150 µl of the supernatant was diluted with 150 µl of 1% aqueous formic acid.

B) 150 µl of a zinc sulphate precipitation agent containing 78% 0.1 M zinc sulphate and 22% acetonitrile was added. After incubation at room temperature for 30 min the precipitate was separated by centrifugation. To 100 µl of the supernatant 10 µl of 1% formic acid was added followed by further incubation at 60°C for 10 min to complete the formation of the zinc chelate, if the compound contains a free DOTA moiety.

The determination of the analyte in the clean sample solutions was performed on an Agilent 1290 UHPLC system coupled to an Agilent 6530 Q-TOF mass spectrometer. The chromatographic separation was carried out on a Phenomenex BioZen XB-C18 HPLC column (50 x 2 mm, 1.7 µm particle size) with gradient elution using a mixture of 0.1% formic acid in water as eluent A and acetonitrile as eluent B (2% B to 41% in 7 min, 800 µl/min, 40°C). Mass spectrometric detection was performed in positive ion ESI mode by scanning the mass range from m/z 50 to 3000 with a sampling rate of 2 / sec.

From the mass spectrometric raw data the ion currents for the double or triple charged monoisotopic signal was extracted for both, the compound and the internal standard.

Quantitation was performed by external matrix calibration with internal standard using the integrated analyte signals.

Additionally, recovery was determined by spiking a pure plasma sample that only contained the internal standard after treatment with a certain amount of the compound.

Carry-over was evaluated by analysis of a blank sample (20% acetonitrile) after the highest calibration sample.

The results of this assay performed on select compounds according to the present invention are given in the following Table 8. The result is stated as "% intact compound remaining after 4 h or 6 h" and means that from the amount of material at the start of the experiment the stated percentage is detected as unchanged material at the end of the experiment by LC-MS quantification. Since all compounds are more than 50% intact after at least 4 h they are considered as stable enough for diagnostic and therapeutic applications.

Table 8: Results of the plasma stability assay

| | **Protein precipitation method** | **% intact compound remaining after incubation** | |
|---|---|---|---|
| **Compound** | | **Human plasma** | **Mouse plasma** |
| 3BP-3126 | A | n.d. | 78% (4 h) |
| 3BP-3127 | A | n.d. | > 95% (4 h) |
| 3BP-3128 | A | n.d. | > 95% (4 h) |
| 3BP-3205 | A | n.d. | > 95% (4 h) |
| 3BP-3208 | B | n.d. | 92% (4 h) |
| 3BP-4663 | A | 89% (6 h) | 92% (6 h) |

(continued)

| | Protein precipitation method | % intact compound remaining after incubation | |
|---|---|---|---|
| Compound | | Human plasma | Mouse plasma |
| 3BP-4694 | B | n.d. | 90% (6 h) |

**Example 10: Stability in the presence of tumor cells**

The colorectal cancer cell line HT-29 (ECACC Cat. No. 91072201) was purchased from ECACC. Cells were cultured in McCoys's 5A modified medium (Biochrom, #F1015) including 10% fetal bovine serum (FBS), 2 mM L-glutamine, 100 U/ml penicillin and 100 μg/mL streptomycin (growth medium). Cells were detached with Accutase (Biolegend, #BLD-423201) and resuspended in growth medium at a concentration of 500.000 cells per mL. 1 mL of the cell suspension were seeded in 24-well plates and incubated overnight at 37°C and 5% $CO_2$. The next day, cells were washed twice and growth medium was exchanged against DMEM without phenol red (Biochrom, #F0475) including 2 mM L-glutamine. Test compound was diluted in DMEM without phenol red (Biochrom, #F0475) including 2 mM L-glutamine. 400 μL of a 10 μM solution of the test compound was incubated for 24 hours at 37°C and 5% $CO_2$ in the presence of cells as well as in control wells (without cells) of the same 24-well plate.

For IC50 determination, supernatants including test compounds were diluted 1:3.3 in assay buffer (50 mM Tris, 1 M NaCl, 1 mg/mL BSA, pH 7.5) and further sequentially diluted 1:5. 25 μl of each dilution containing test compound were mixed with 25 μl of a 3.6 nM recombinant human FAP solution (R&D systems, # 3715-SE) diluted in assay buffer and incubated for 5 min in a white 96-well ProxiPlate (Perkin Elmer). As specific FAP substrate the FRET-peptide HiLyteFluor™ 488 - VS(D-)P SQG K(QXL® 520) - NH2 was used (Bainbridge, et al., Sci Rep, 2017, 7: 12524). 25 μL of a 30 μM substrate solution, diluted in assay buffer, was added. All solutions were equilibrated at 37°C prior to use. Substrate cleavage and increase in fluorescence (excitation at 485 nm and emission at 538 nm) was measured in a kinetic mode for 5 minutes at 37°C in a SPECTRAmax M5 plate reader. RFU/sec was calculated by SoftMax Pro software and plotted against test compound concentration. Four parameter logistic (4PL) curve fitting and IC50/pIC50 calculations were performed using GraphPad Prism 9.1 software. Residual activity was calculated as follows:

$$\text{Residual activity (\%)} = (10^{-\text{pIC50 (w/o cells)}}/10^{-\text{pIC50 (with HT29 cells)}})*100\ \%$$

The results of this assay for select compounds are given in Table 9.

Table 9:

| ID | pIC50 (incubation in cell medium w/o HT-29) | pIC50 (incubation in the presence of HT-29 cells) | Residual activity after incubation with HT-29 cells for 24 hours (%) |
|---|---|---|---|
| 3BP-2762 | 8.4 | 8.2 | 63 |
| 3BP-2929 | 8.7 | 7.9 | 16 |
| 3BP-3205 | 8.7 | <6 | <1 |
| 3BP-3232 | 8.9 | 8.0 | 13 |
| 3BP-3412 | 8.6 | 8.6 | 100 |
| 3BP-3467 | 9.3 | 8.4 | 13 |
| 3BP-3469 | 9.2 | 8.3 | 13 |
| 3BP-3515 | 7.8 | 7.7 | 79 |
| 3BP-3581 | 8.7 | 8.7 | 100 |
| 3BP-4200 | 8.7 | 7.9 | 16 |
| 3BP-4663 | 8.6 | 8.6 | 100 |
| 3BP-4664 | 8.6 | 8.6 | 100 |
| 3BP-4665 | 8.4 | 8.4 | 100 |
| 3BP-4694 | 8.5 | 8.5 | 100 |
| 3BP-4808 | 8.6 | 8.6 | 100 |
| 3BP-4809 | 8.6 | 8.6 | 100 |
| 3BP-4810 | 8.2 | 8.2 | 100 |
| 3BP-4811 | 8.1 | 8.1 | 100 |

**Example 11: $^{111}$In-labeling of selected compounds**

In order to serve as a diagnostically, therapeutically, or theragnostically active agent, a compound needs to be labeled with a radioactive isotope. The labeling procedure needs to be appropriate to ensure a high radiochemical yield and purity of the radiolabeled compound of the invention. This example shows that the compounds of the present invention are appropriate for radio labeling and can be labeled in high radiochemical yield and purity.

**Compounds: 3BP-3467, 3BP-3581, 3BP-3621, 3BP-3631, and 3BP-4076**

20 - 40 MBq of $^{111}InCl_3$ (in 0.02 M HCl; Curium, Germany) were mixed with 1 nmol of the respective compound (250 μM stock solution in ultrapure water) per 30 MBq and buffer (1 M sodium acetate pH 5) at a final buffer concentration of 0.2 M. The mixture was heated to 95 °C for 20 min. After cooling down, DTPA (Heyl, Germany) and TWEEN-20 were added at a final concentration of 0.1 mg/mL and 0.1%, respectively.

Radiochemical incorporation yield was between 91 - 96 % and the radiochemical purity was between 60 - 86 % at end of synthesis (cf. Table 10).

Table 10: Radiochemical incorporation yield (RCY) and radiochemical purity (RCP) of compounds $^{111}$In-3BP-3467, -3581, -3621, -3631, and -4076 at end of synthesis (EOS).

| Compound | RCY [%] | RCP [%] | $R_t$ [min] | HPLC method |
|---|---|---|---|---|
| $^{111}$In-3BP-3467 | > 91 | > 86 | 8.0 | A |
| $^{111}$In-3BP-3581 | > 96 | > 80 | 13.7 | A |
| $^{111}$In-3BP-3621 | > 94 | > 60 | 7.5 | A |
| $^{111}$In-3BP-3631 | > 93 | >71 | 7.8 | A |
| $^{111}$In-3BP-4076 | > 93 | > 77 | 6.4 | A |

**Compound: 3BP-3622**

Around 100 MBq of $^{111}InCl_3$ (in 0.02 M HCl; Curium, Germany) was mixed with 1 nmol of the respective compound (250 μM stock solution in ultrapure water) per 30 MBq and buffer (1 M sodium acetate pH 5) at a final buffer concentration of 0.2 M. The mixture was heated to 80 °C for 20 min. After cooling down, DTPA (Heyl, Germany) and TWEEN-20 were added at a final concentration of 0.08 mg/mL and 0.1%, respectively.

Radiochemical incorporation yield was > 96 % and the radiochemical purity was > 86 % ($R_t$ = 4.9 min, HPLC method B) at end of synthesis.

**Compounds: 3BP-3772, 3BP-3785**

90 - 100 MBq of $^{111}InCl_3$ (in 0.02 M HCl; Curium, Germany) were mixed with 1 nmol of the respective compound (250 μM stock solution in ultrapure water) per 30 MBq and buffer (1 M sodium acetate pH 5) at a final buffer concentration of 0.1 M. The mixture was heated to 80 °C for 25 min. After cooling down, DTPA (Heyl, Germany) and TWEEN-20 were added at a final concentration of 0.1 mg/mL and 0.1%, respectively.

Radiochemical incorporation yield was between 87 - 94 % and the radiochemical purity was between 48 - 71 % at end of synthesis (cf, Table 11).

Table 11: Radiochemical incorporation yield (RCY) and radiochemical purity (RCP) of compounds $^{111}$In-3BP-3772, and -3785 at end of synthesis (EOS).

| Compound | RCY [%] | RCP [%] | $R_t$ [min] | HPLC method |
|---|---|---|---|---|
| $^{111}$In-3BP-3772 | > 87 | >48 | 4.3 | B |
| $^{111}$In-3BP-3785 | > 94 | >71 | 6.3 | A |

**Compounds: 3BP-4808, 3BP-4809, 3BP-4810, and 3BP-4811**

Around 102 MBq of $^{111}InCl_3$ (in 0.02 M HCl; Curium, Germany) were mixed with 1 nmol of the respective compound (250 μM stock solution in ultrapure water) per 30 MBq and buffer (1 M sodium acetate pH 5) at a final buffer concentration of 0.1 M. The mixture was heated to 80 °C for 25 min. After cooling down, ascorbic acid (Woerwag Pharma, Germany), DTPA (Heyl, Germany) and TWEEN-20 were added at a final concentration of 25 mg/mL, 0.1 mg/mL, and 0.1%, respectively.

Radiochemical incorporation yield was between 89 - 95 % and the radiochemical purity was between 87 - 93 % at end of synthesis (cf. Table 12).

Table 12: Radiochemical incorporation yield (RCY) and radiochemical purity (RCP) of compounds $^{111}$In-3BP-4808 - 4811 at end of synthesis (EOS).

| Compound | RCY [%] | RCP [%] | $R_t$ [min] | HPLC method |
|---|---|---|---|---|
| $^{111}$In-3BP-4808 | > 95 | > 92 | 5.0 | B |
| $^{111}$In-3BP-4809 | > 94 | > 93 | 4.8 | B |
| $^{111}$In-3BP-4810 | > 93 | > 91 | 5.7 | B |
| $^{111}$In-3BP-4811 | > 89 | > 87 | 5.7 | B |

**Compounds: 3BP-4663, 3BP-4664, 3BP-4665, and 3BP-4694**

30 - 35 MBq of $^{111}InCl_3$ (in 0.02 M HCl; Curium, Germany) were mixed with 1 nmol of the respective compound (250 μM stock solution in ultrapure water) per 30 MBq and buffer (1 M sodium acetate pH 5) at a final buffer concentration of 0.1 M. The mixture was heated to 80 °C for 25 min. After cooling down, ascorbic acid (Woerwag Pharma, Germany), DTPA (Heyl, Germany) and TWEEN-20 were added at a final concentration of 25 mg/mL, 0.1 mg/mL, and 0.1%, respectively. Afterwards, the mixture was diluted with sodium hydrogen phosphate buffer (0.1 M, pH 8.5) (1:1). In some instances, purification of the reaction mixture by solid phase extraction was performed. The reaction mixture was applied to a pre-conditioned Oasis HLB 1 cc Vac cartridge, 10 mg sorbent, 30 μm (Waters, USA) and washed with 300 μL water. The $^{111}$In-labeled compound was then eluted in 30 μL ethanol fractions, which were diluted to an ethanol content of appr. 10% with 10 mg/mL ascorbic acid in PBS.

Radiochemical incorporation yield was between 80 - 90 % and the radiochemical purity was between 80 - 89 % at end of synthesis (cf. Table 13).

Table 13: Radiochemical incorporation yield (RCY) and radiochemical purity (RCP) of compounds $^{111}$In-3BP-4808 - 4811 at end of synthesis (EOS).

| Compound | RCY [%] | RCP [%] | $R_t$ [min] | HPLC method |
|---|---|---|---|---|
| $^{111}$In-3BP-4663 | > 89 | > 89 | 6.4 | B |
| $^{111}$In-3BP-4664 | > 80 | > 80 | 6.2 | B |
| $^{111}$In-3BP-4665 | > 90 | > 88 | 5.7 | B |
| $^{111}$In-3BP-4694 | > 88 | > 83 | 6.6 | B |

**Quality control:**

Radiochemical purity was analyzed by HPLC. 5 μl of diluted labeling solution was analyzed with a Poroshell SB-C18 2.7 μm, 2.1 x 50 mm (Agilent). Eluent A: $H_2O$, 0.1 % TFA eluent B: MeCN, and a gradient from either 10% B to 18% B within 15 min (method A) or from 5% B to 70% B within 15 min (method B), flow rate 0.5 mL/min; detector: NaI (Raytest), DAD 230 nm. The peak eluting with the dead volume represents free radionuclide, the peak eluting with the compoundspecific retention time as determined with an unlabeled sample represents radiolabeled compound.

**Example 12: LigandTracer® assay**

Affinity studies were performed with $^{111}$In-labeled compounds using the LigandTracer® (Ridgeview Instruments AB, Sweden). HEK293 cells (human embryonic kidney cells) were engineered to express the human FAP. HEK-FAP cells were cultured in DMEM (Sigma-Aldrich, #D6545) including 10% fetal calf serum (FCS), 2 mM L-glutamine, 100 U/ml penicillin, 100 μg/mL streptomycin, 200 μg/ml Zeocin and 100 μg/ml Hygromycin B. Cells were detached with Accutase (Biolegend, #BLD-423201) and counted using a particle counter (CASY Model TT; Schärfe Systems). Cell concentrations were adjusted to 3 x $10^5$ $mL^{-1}$, and 3.000 μL of the suspension was dispensed into a poly-lysine-coated, clear cell dish (10cm).

Approximately 24 hours after re-seeding, the medium was replaced by assay medium (Ham's medium with 20 mM HEPES) containing 1 % FCS. The dish was placed in the LigandTracer® device and a baseline measurement was obtained using the LigandTracer® Control software (version 2.4). To determine the association rate ($k_a$) the HEK-FAP cells were incubated consecutively with two or three different concentrations of the $^{111}$In-labeled compound. To determine

the dissociation rate ($k_d$) the cells were washed and subsequently incubated with fresh assay medium for several hours. Obtained association and dissociation rates were used to calculate the dissociation constant ($K_D$) via the LigandTracer® TracerDrawer software (version 1.92; fit: ligand depletion).

Table 14 Results of the LigandTracer® assay

| Compound | ka (1/M*s) | HL (min) | $pK_D$ | n |
|---|---|---|---|---|
| 3BP-2929 | 2.5 ± 1.5 E+05 | 276 ± 142 | 9.6 ± 0.3 | 4 |
| 3BP-3581 | 2.0 ± 1.8 E+05 | 345 ± 218 | 10.1 ± 0.1 | 2 |
| 3BP-4200 | 1.6 ± 0.1 E+05 | 1798 ± 24 | 10.4 ± 0.0 | 2 |
| 3BP-4663 | 1.5 ± 0.7 E+05 | 1477 ± 2013 | 9.8 ± 0.7 | 4 |
| 3BP-4664 | 1.6 ± 0.6 E+05 | 1030 ± 1439 | 9.3 ± 0.7 | 3 |
| 3BP-4665 | 2.2 ± 1.0 E+05 | 22 ± 16 | 8.8 ± 0.2 | 3 |
| 3BP-4694 | 5.7 ± 0.7 E+04 | 3946 ± 1189 | 10.3 ± 0.1 | 2 |
| 3BP-4808 | 9.4 ± 1.9 E+04 | 20 ± 5 | 8.2 ± 0.0 | 2 |
| 3BP-4809 | 1.0 ± 0.2 E+05 | 18 ± 1 | 8.2 ± 0.1 | 3 |
| 3BP-4810 | 8.8 ± 2.1 E+04 | 38 ± 4 | 8.5 ± 0.1 | 2 |
| 3BP-4811 | 4.8 ± 3.7 E+05 | 27 ± 12 | 8.3 ± 0.2 | 2 |

**Example 13: Imaging and biodistribution studies**

Radioactively labeled compounds can be detected by imaging methods such as SPECT and PET. Furthermore, the data acquired by such techniques can be confirmed by direct measurement of radioactivity contained in the individual organs prepared from an animal injected with a radioactively labeled compound of the disclosure. Thus, the biodistribution (the measurement of radioactivity in individual organs) of a radioactively labeled compound can be determined and analyzed. This example shows that the compounds of the present disclosure show a biodistribution appropriate for diagnostic imaging and therapeutic treatment of tumors.

All animal experiments were conducted in compliance with the German animal protection laws. Female Swiss nude mice (6-8 weeks old, Charles River Laboratories, France) were inoculated with $5x10^6$ HEK-FAP cells in the right shoulder. For selected compounds an additional model was used, here female SCID beige mice (8-weeks old, Charles River, Germany) were inoculated with $5x10^6$ HEK-FAP cells in the right and $5x10^6$ CHO-FAP cells in the left shoulder. When tumors reached an appropriate size, the mice received ~30 MBq $^{111}$In-labelled compounds of the disclosure (diluted to 100 µL with PBS) administered intravenously via the tail vein. Images were obtained on a NanoSPECT/CT system (Mediso Medical Imaging Systems, Budapest, Hungary) using exemplarily the following acquisition and reconstruction parameters (Table 15).

Table 15: Acquisition and reconstruction parameters of NanoSPECT/CT imaging

| Acquistion parameters SPECT | |
|---|---|
| System | NanoSPECT/CT ™ |
| Scan range | whole body, 3-bed holder (mouse hotel) |
| Time per projection | 60s |
| Aperture model, pinhole diameter | Aperture #2, 1,5 mm |
| **Reconstruction parameters** | |
| Method | HiSPECT (Scivis), iterative reconstruction |
| Smoothing | 35% |
| Iterations | 9 |
| Voxel size | 0.15 mm x 0.15 mm x 0.15 mm |

(continued)

| Acquisition parameters CT | |
|---|---|
| System | NanoSPECT/CT ™ |
| Scan range | whole body, 3-bed holder (mouse hotel) |
| Scan duration | 7 minutes |
| Tube voltage | 45 kVp |
| Exposure time | 500 ms |
| Number of projections | 240 |

Imaging data were saved as DICOM files and analysed using VivoQuant™ software (Invicro, Boston, USA). Two to three animals were used per time point. Results are expressed as a percentage of injected dose per gram of tissue (%ID/g).

Figures 1(a) - 1(p) show the percentage of injected dose per gram of tissue uptake (mean %ID/g, error bars indicate standard deviation) in the kidneys, liver, bloodpool and HEK-FAP tumor as determined by SPECT/CT imaging of $^{111}$In-labeled compounds at the indicated time points post injection into Female Swiss nude mice.

Figures 2(a)-2(f) show the percentage of injected dose per gram of tissue uptake (mean %ID/g, error bars indicate standard deviation) in the kidneys, liver, bloodpool, HEK-FAP and/or CHO-FAP tumor as determined by SPECT-imaging of select $^{111}$In-labeled compounds post injection into SCID beige mice.

Figures 3(a)-3(d) show SPECT/CT-images of select $^{111}$In-labeled compounds post injection into Swiss nude mice with HEK-FAP tumors.

Figures 4(a)-4(f) show SPECT/CT-images of select $^{111}$In-labeled compounds post injection into SCID beige mice with HEK-FAP (right shoulder) and CHO-FAP (left shoulder) tumors.

**References**

**Claims**

**1.** A compound of formula (I):

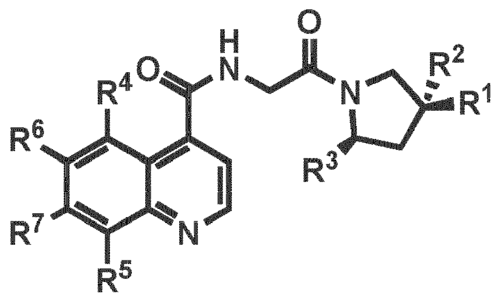

I,

wherein:

$R_1$ is H or F,
$R_2$ is H or F,
$R_3$ is -C(O)-Het1,
wherein -C(O)-Het1 is:

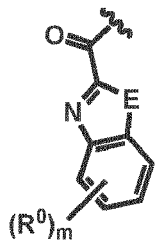,

wherein:

E is O or S;
$R^0$ is independently selected from the group consisting of $(C_1\text{-}C_4)$alkyl, -O-$(C_1\text{-}C_4)$alkyl, - COO-$(C_1\text{-}C_4)$alkyl, F, Cl, Br, I, OH, COOH, and CN; and
m is selected from the group consisting of 0, 1, and 2;

$R^4$ is selected from the group consisting of H, Cl, Br, F, and $(C_1\text{-}C_2)$alkyl;
one of $R^5$, $R^6$, and $R^7$ is $R^8$-L-, and the other two of $R^5$, $R^6$, and $R^7$ are each independently selected from the group consisting of H, $(C_1\text{-}C_4)$alkyl, -O-$(C_1\text{-}C_4)$alkyl, -O-$(C_1\text{-}C_3)$alkylidene-$(C_6)$aryl, F, Cl, Br, and O-$CF_3$; and $R^8$-L- is $R^8$-Lin4-Lin3-Lin2-Lin1-,
wherein:

Lin1 is selected from the group consisting of

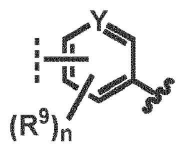, 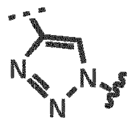,

-O-, -N($CH_3$)-, and ⊢ $(C_1\text{-}C_4)$alkylidene-C(O)NH-, wherein in Lin1:

Y is CH or N;
⊢ indicates attachment to Lin2, and when Lin1 is

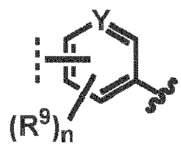,

Lin2 is in *meta*-or *para*-position to —⌇ and

—⌇ indicates attachment to the quinoline;
$R^9$ is selected from the group consisting of halogen, $CO_2H$, $(C_1\text{-}C_6)$alkyl, hydroxy substituted $(C_1\text{-}C_6)$alkyl, and -O-$(C_1\text{-}C_6)$alkyl; and
n is selected from the group consisting of 0, 1, and 2, preferably n is 0 or 1, more preferably n is 0;

Lin2 is selected from the group consisting of -C(O)-NH-, -NH-C(O)-, and -S(O)$_2$- when Lin1 is selected from the group consisting of

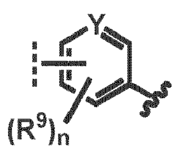, 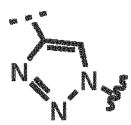,

and 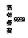 ($C_1$-$C_4$)alkylidene-C(O)NH 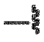, and Lin2 is absent when Lin1 is -O- or - N($CH_3$)-;
Lin3 is ($C_2$-$C_4$)alkylidene;
Lin4 is selected from the group consisting of -$NR^{10}$,

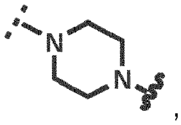,

and

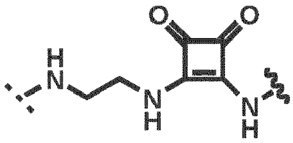,

wherein in Lin4:

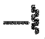 indicates attachment to Lin3,

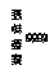 indicates the attachment to $R_8$;
$R^{10}$ is selected from the group consisting of H, $CH_2$-COOH, and ($C_1$-$C_4$)alkyl; and

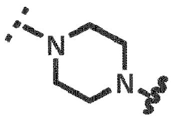

is optionally oxidized to

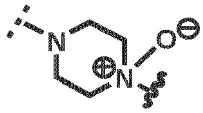;

$R^8$ is a chelator or a cytotoxic agent; and wherein

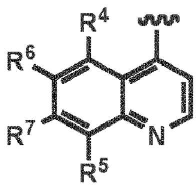

in the formula (I) may optionally be oxidized to its N-oxide

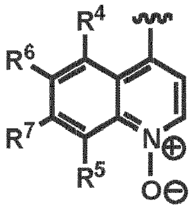.

2. The compound of Claim 1, wherein -C(O)-Het1 is selected from the group consisting of:

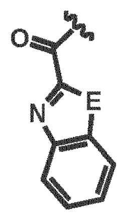, 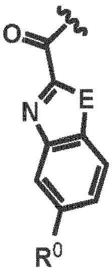, and 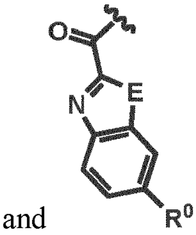,

wherein:

E is O or S; and
$R^0$ is selected from the group consisting of $-CH_3$, $-O-CH_3$, $-COOCH_3$, F, Cl, and Br;

and preferably the compound has a structure of formula (II):

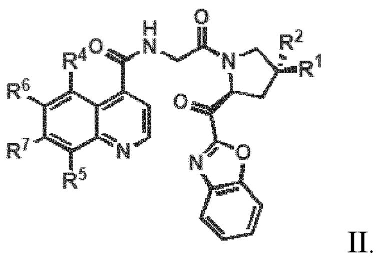
II.

3. The compound of any one of Claims 1, and 2, wherein $R^4$ is H or $-CH_3$.

4. The compound of any one of Claims 1, 2, and 3, wherein $R^6$ is $R^8$-L; preferably $R^5$ and $R^7$ are each independently selected from the group consisting of H and $-CH_3$.

5. The compound of any one of Claims 1, 2, and 3, wherein $R^7$ is $R^8$-L; preferably $R^5$ and $R^6$ are each independently selected from the group consisting of H and $-CH_3$.

6. The compound of any one of Claims 1, 2, 3, 4, and 5 preferably of any one of Claims 2, 3, 4, and 5, wherein Lin1 is

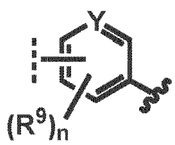 or 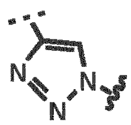.

7. The compound of any one of Claims 1, 2, 3, 4, 5, and 6, wherein Lin1 is

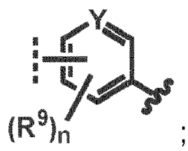;

preferably Y is CH; more preferably Lin1 is

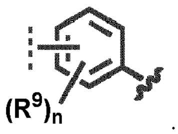

.

8. The compound of any one of Claims 1, 2, 3, 4, 5, and 6, preferably any one of Claims 2, 3, 4, 5, and 6, wherein Lin2-Lin1 is selected from the group consisting of

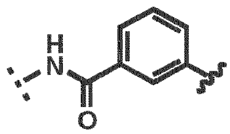

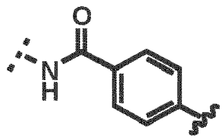 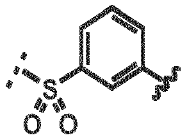 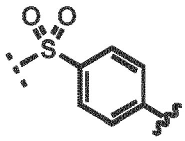, 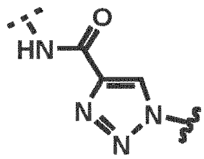, and 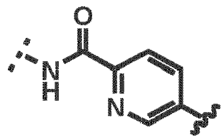,

wherein indicates attachment to the quinoline and indicates attachment to Lin3; preferably Lin2-Lin1 is selected from the group consisting of

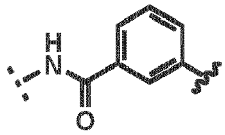, 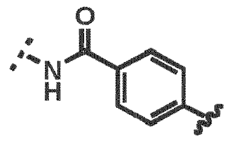,

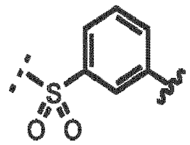, and 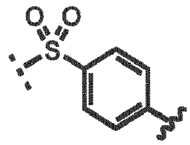,

wherein indicates attachment to the quinoline and indicates attachment to Lin3.

9. The compound of any one of Claims 1, 2, 3, 4, 5, 6, 7, and 8, preferably any one of Claims 2, 3, 4, 5, 6, 7, and 8, wherein Lin3 is -($C_2$-$C_3$)alkylidene.

10. The compound of any one of Claims 1, 2, 3, 4, and 5, wherein $R^8$-L- is selected from the group consisting of:

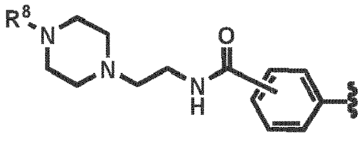, 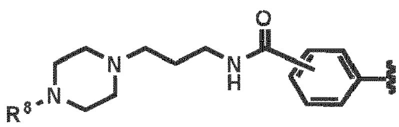, 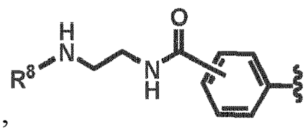,

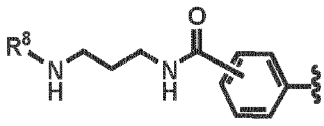, 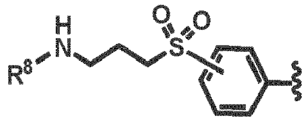,

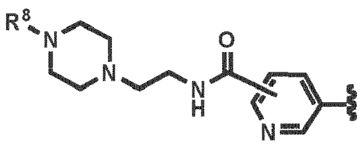, 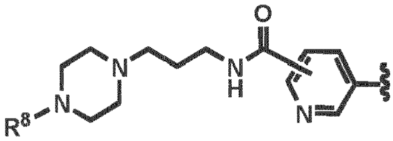, 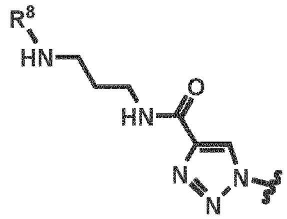,

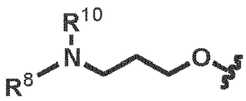, 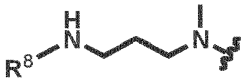, 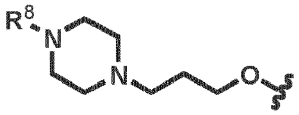, 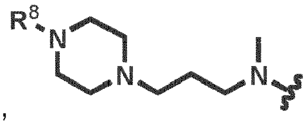,

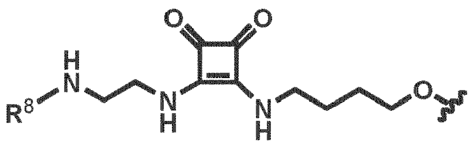, and 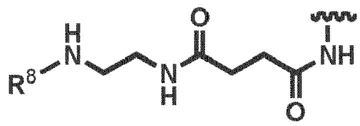;

preferably $R^8$-L- is selected from the group consisting of:

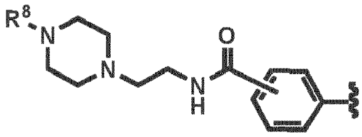, 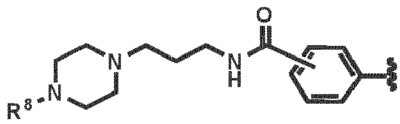, 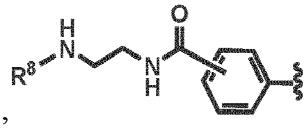,

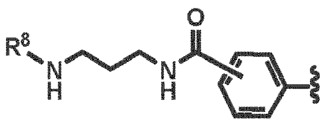, 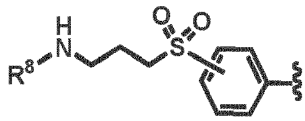, 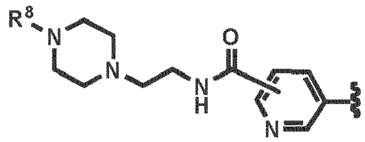,

and

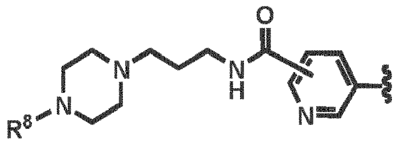, and 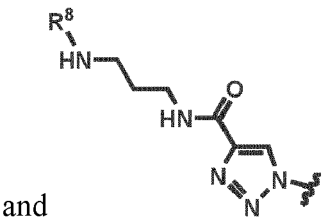;

more preferably $R^8$-L- is selected from the group consisting of:

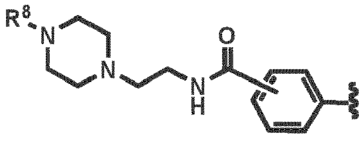, 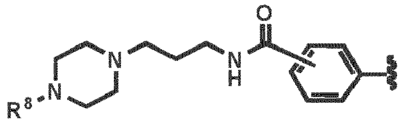, 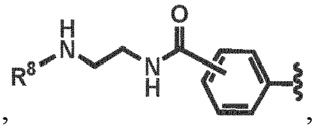,

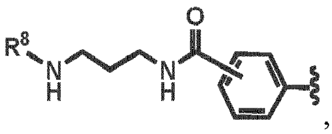, and 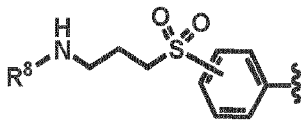.

**11.** The compound of any one of Claims 1, 2, 3, 4, 5, 6, 7, 8, 9, and 10, wherein $R^8$ is a chelator.

**12.** The compound of Claim 11, wherein the chelator is selected from the group consisting of DOTA, DOTAGA, NOPO, PCTA, NOTA, NODAGA, NODA-MPAA, HBED, TETA, CB-TE2A, DTPA, DFO, Macropa, DOTAM, HOPO, TRAP, THP, DATA, NOTP, sarcophagine, FSC, NETA, H4octapa, Pycup, $N_xS_{4-x}$(N4, N2S2, N3S), Hynic, $^{99m}Tc(CO)_3$-Chelators, more preferably DOTA, DOTAGA, NOPO, PCTA, DOTAM, Macropa, NOTA, NODAGA, NODA-MPAA, HBED, CB-TE2A, DFO, THP, N4 and most preferably DOTA, DOTAGA, NOPO, PCTA, DOTAM, Macropa, NOTA, and NODAGA; preferably the chelator is selected from the group consisting of DOTA, NOPO, PCTA and Macropa.

**13.** The compound of any one of Claims 1, 2, 3, 4, 5, 6, 7, 8, 9, and 10, wherein $R^8$ is a cytotoxic agent.

**14.** The compound of Claim 1, wherein the compound is selected from the group consisting of

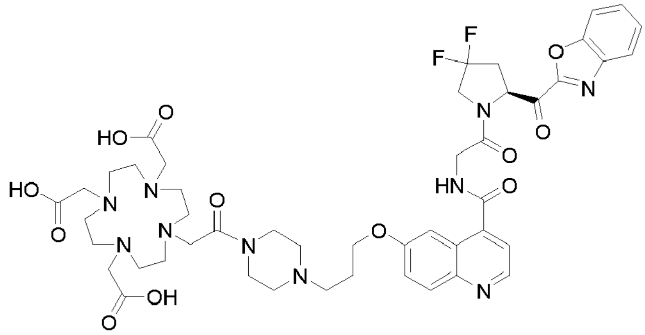

3BP-3412

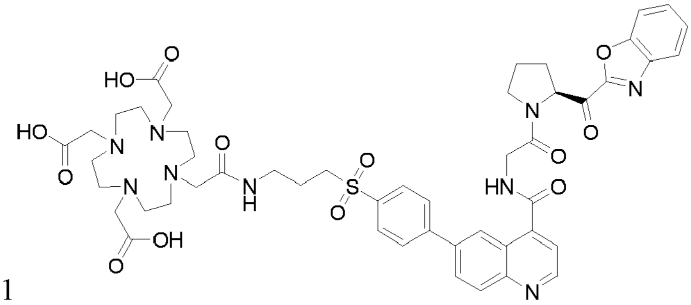

3BP-3581

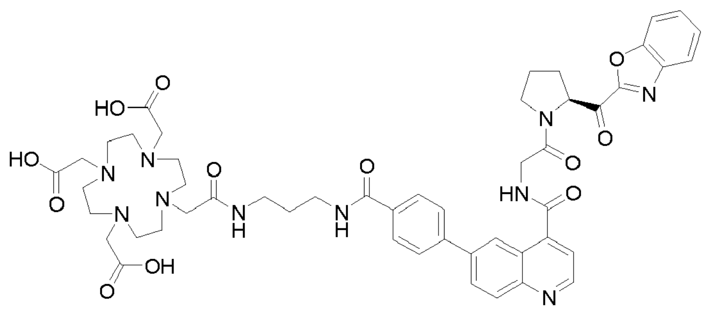

3BP-3582

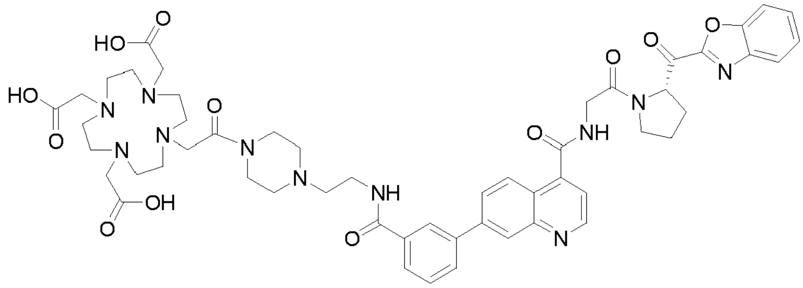

3BP-4663

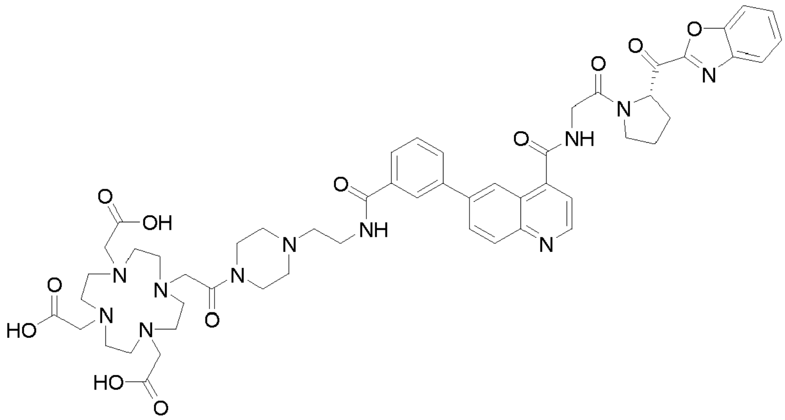

3BP-4664

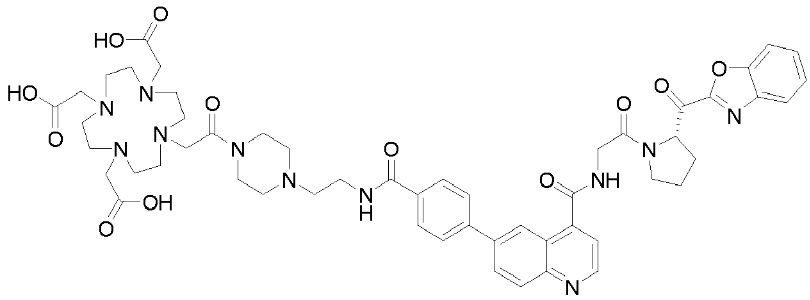

3BP-4665

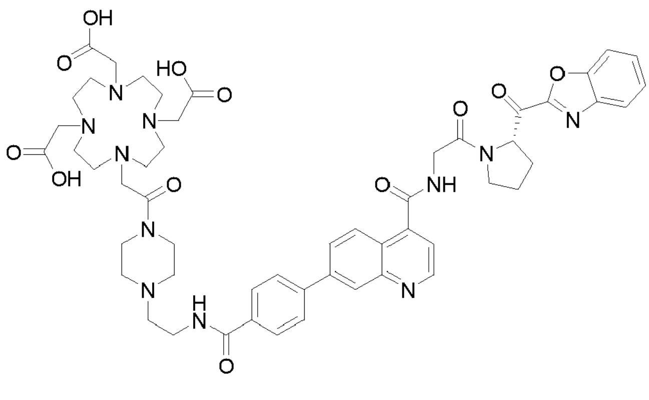

3BP-4694

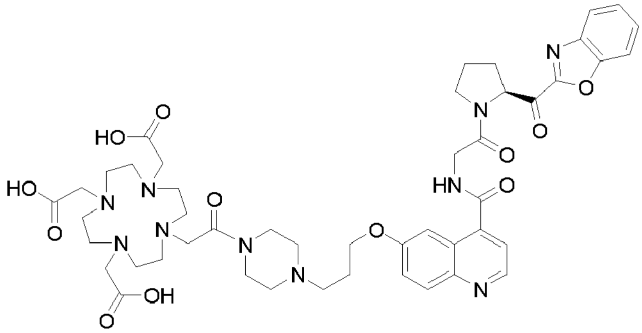

3BP-4808

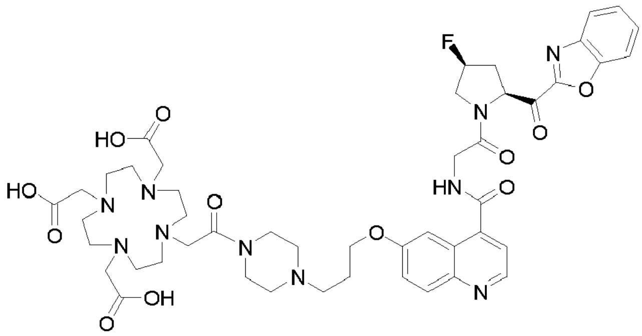

3BP-4809

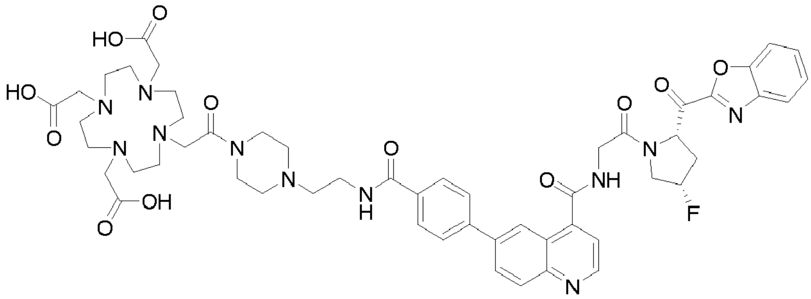

3BP-4810

and

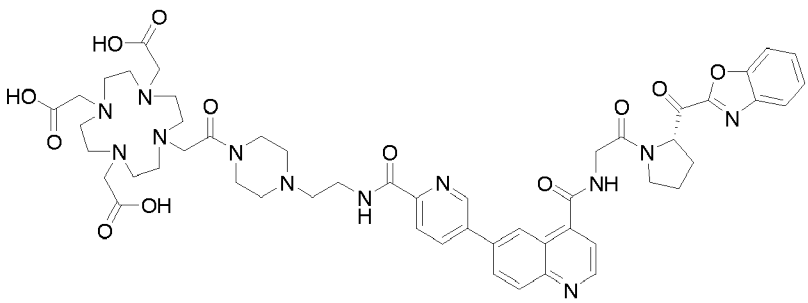

3BP-4811 .

**15.** The compound of any one of Claims 1, 2, 3, 4, 5, 6, 7, 8, 9, 10, 11, 12, 13, and 14, wherein the compound comprises a diagnostically active nuclide and/or a therapeutically active nuclide, preferably the diagnostically active nuclide is a diagnostically active radionuclide and the therapeutically active nuclide is a therapeutically active radionuclide.

**16.** The compound of any one of Claims 1, 2, 3, 4, 5, 6, 7, 8, 9, 10, 11, 12, 13, 14, and 15, for use in a method for diagnosing a disease.

**17.** The compound of any one of Claims 1, 2, 3, 4, 5, 6, 7, 8, 9, 10, 11, 12, 13, 14, and 15, for use in a method for the treatment of a disease.

**18.** A composition, preferably a pharmaceutical composition, wherein the composition comprises the compound according to any one of Claims 1, 2, 3, 4, 5, 6, 7, 8, 9, 10, 11, 12, 13, 14, and 15, and a pharmaceutically acceptable excipient.

Fig. 1(a)

Organ uptake $^{111}$In-3BP-3467

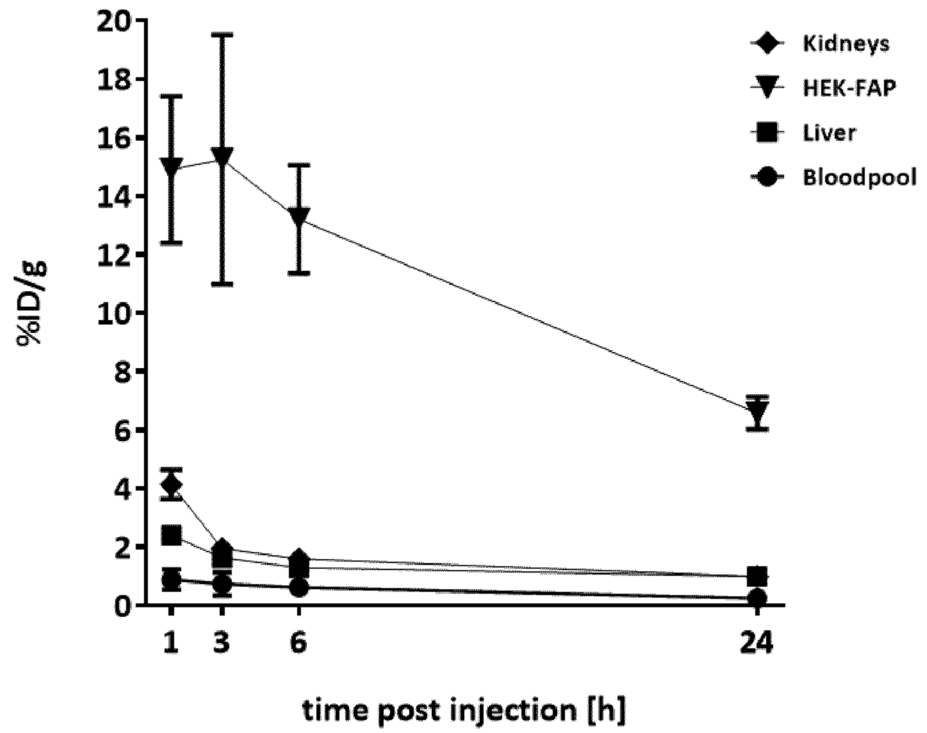

**Fig. 1(b)**

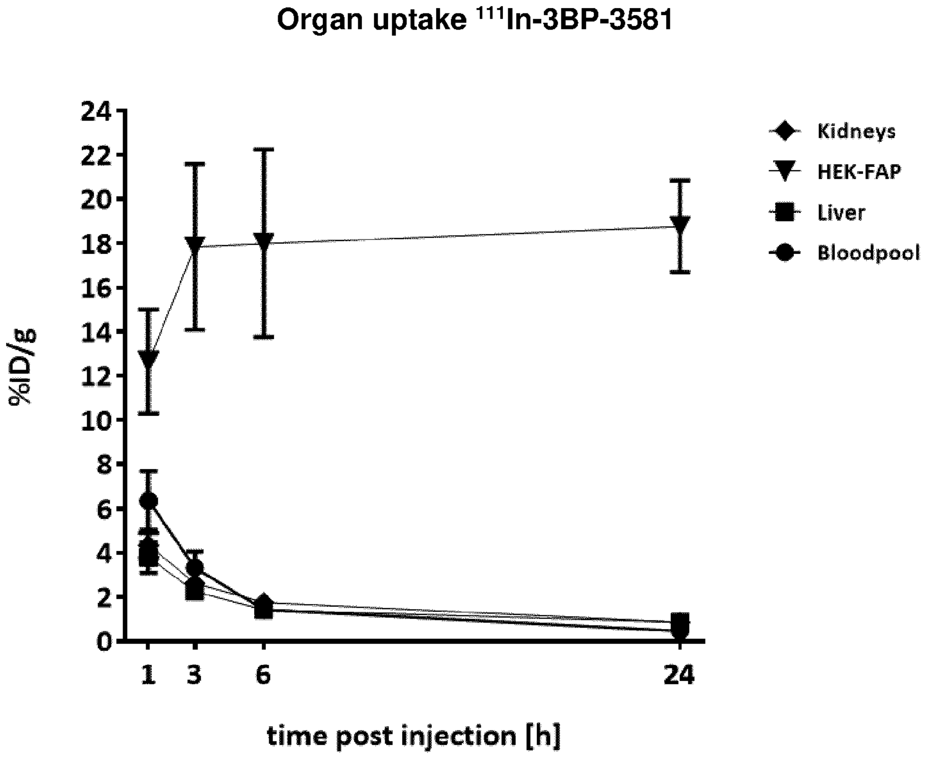
Organ uptake $^{111}$In-3BP-3581
%ID/g
0
2
4
6
8
10
12
14
16
18
20
22
24
1
3
6
24
time post injection [h]
Kidneys
HEK-FAP
Liver
Bloodpool

Fig. 1(c)

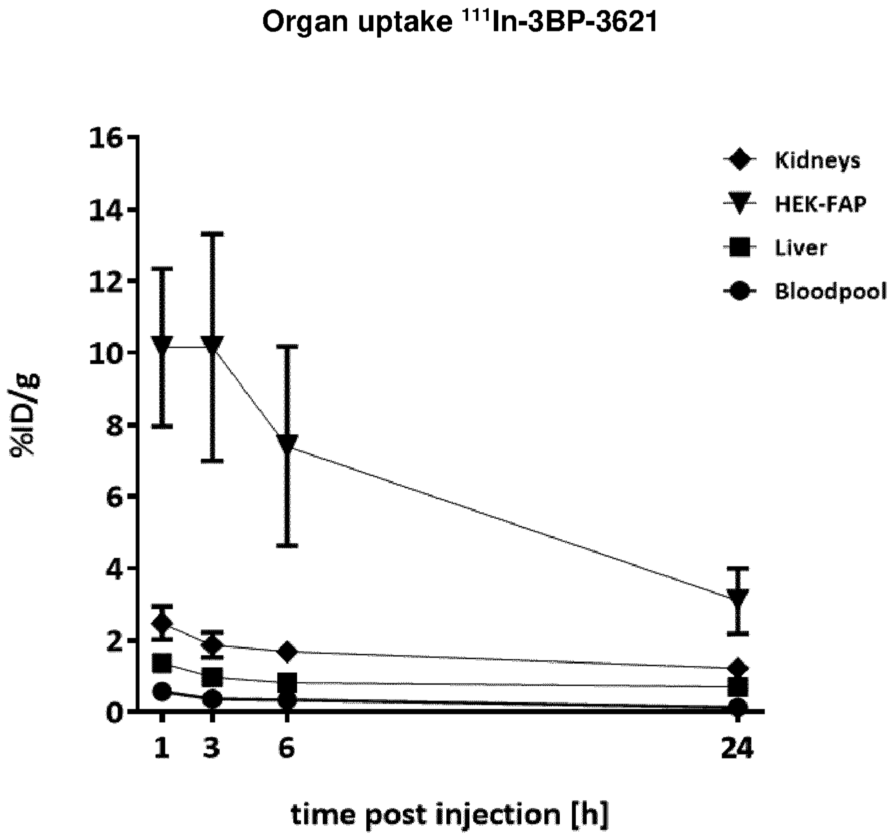
Organ uptake 111In-3BP-3621
16
14
12
10
8
6
4
2
0
%ID/g
1
3
6
24
time post injection [h]
Kidneys
HEK-FAP
Liver
Bloodpool

Fig. 1(d)

Organ uptake $^{111}$In-3BP-3631

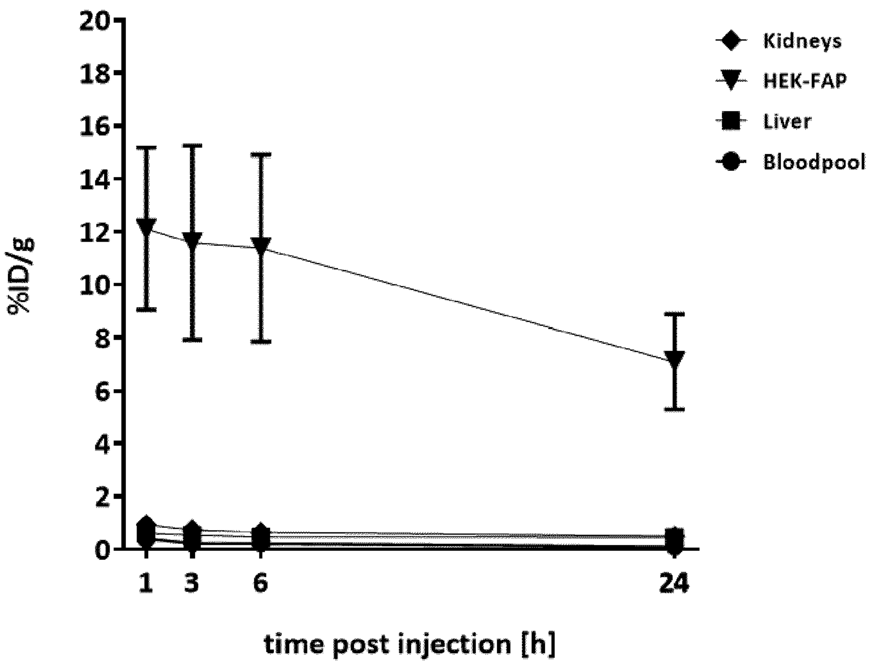

**Fig 1(e)**

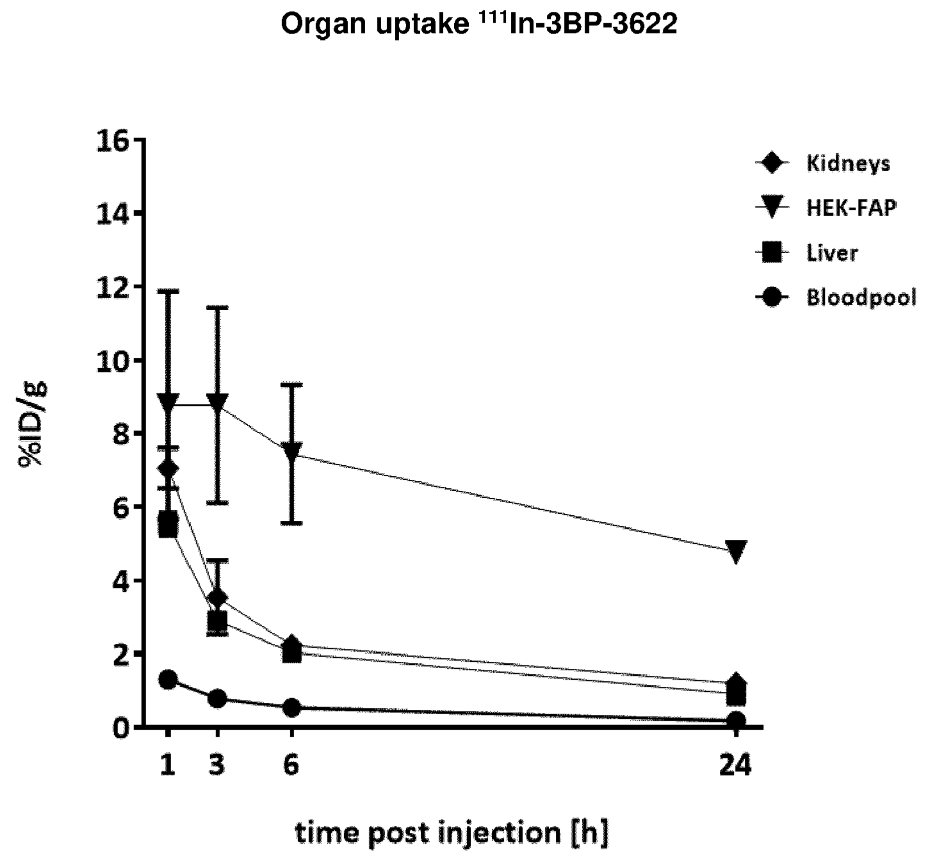
Organ uptake 111In-3BP-3622
16
14
12
10
8
6
4
2
0
%ID/g
1
3
6
24
time post injection [h]
Kidneys
HEK-FAP
Liver
Bloodpool

**Fig 1(f)**

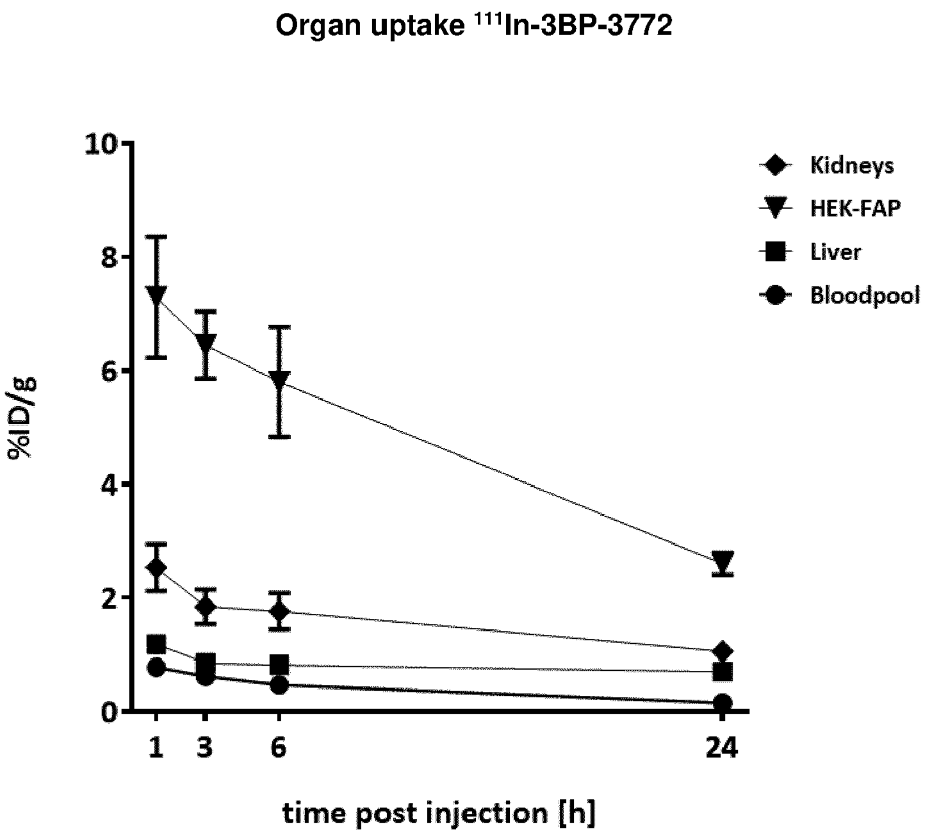
Organ uptake $^{111}$In-3BP-3772
10
8
6
4
2
0
%ID/g
1
3
6
24
time post injection [h]
Kidneys
HEK-FAP
Liver
Bloodpool

**Fig 1(g)**

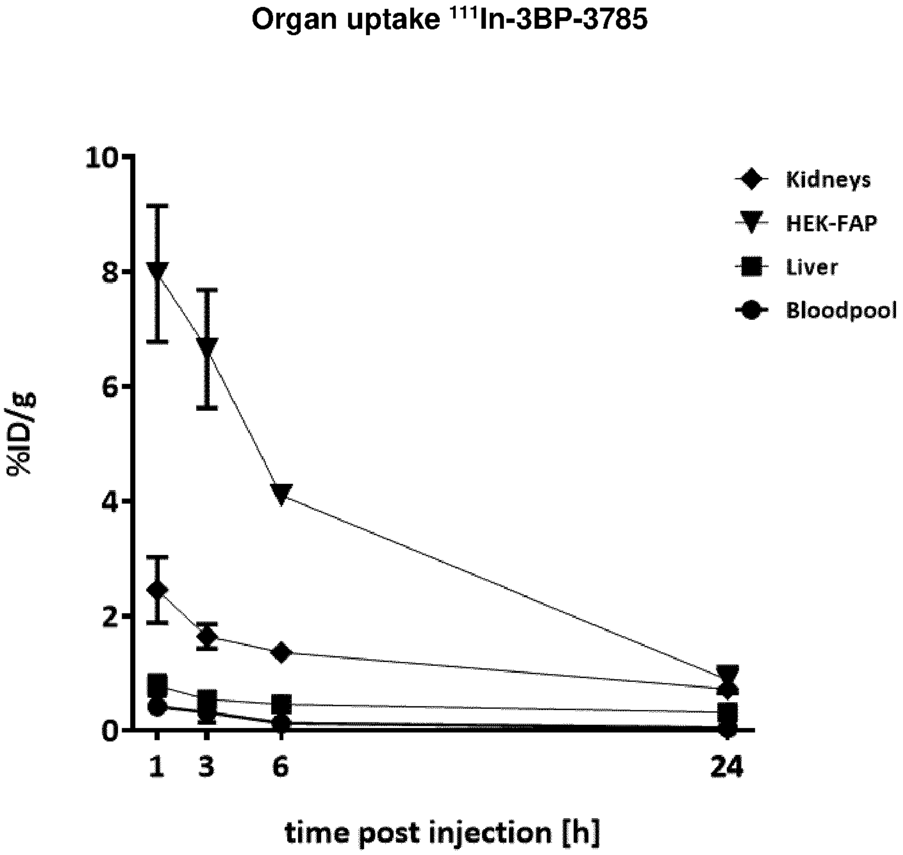
Organ uptake 111In-3BP-3785
10
8
6
4
2
0
%ID/g
1
3
6
24
time post injection [h]
Kidneys
HEK-FAP
Liver
Bloodpool

Fig 1(h)

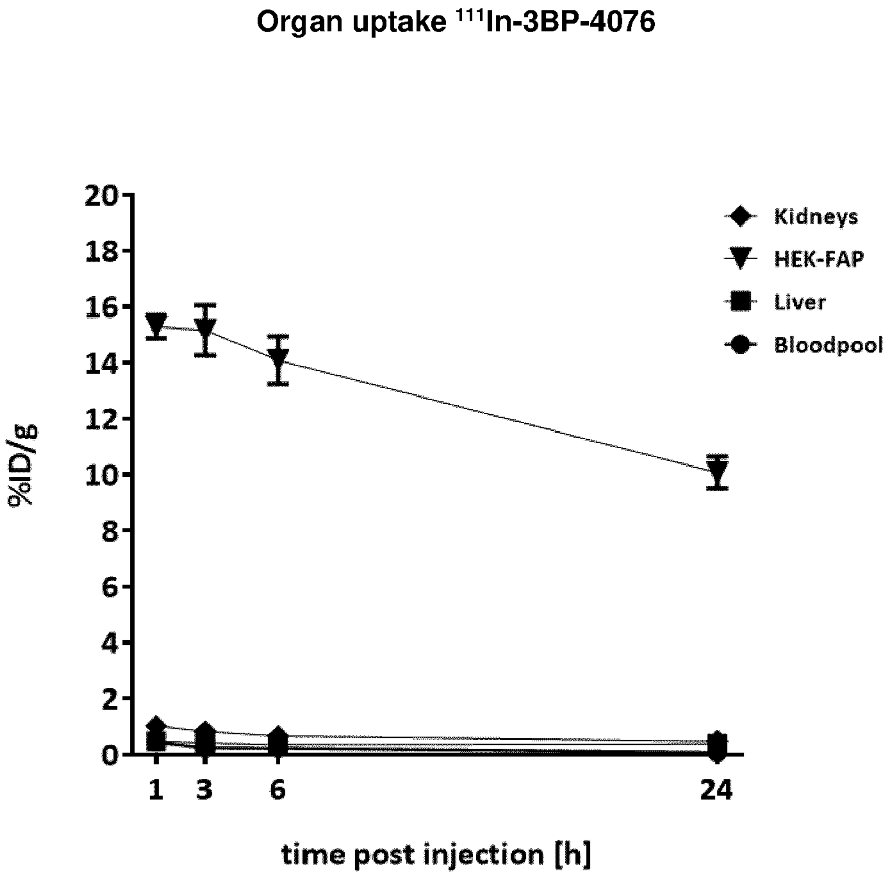
Organ uptake $^{111}$In-3BP-4076
%ID/g
20
18
16
14
12
10
8
6
4
2
0
1
3
6
24
time post injection [h]
Kidneys
HEK-FAP
Liver
Bloodpool

**Fig 1(i)**

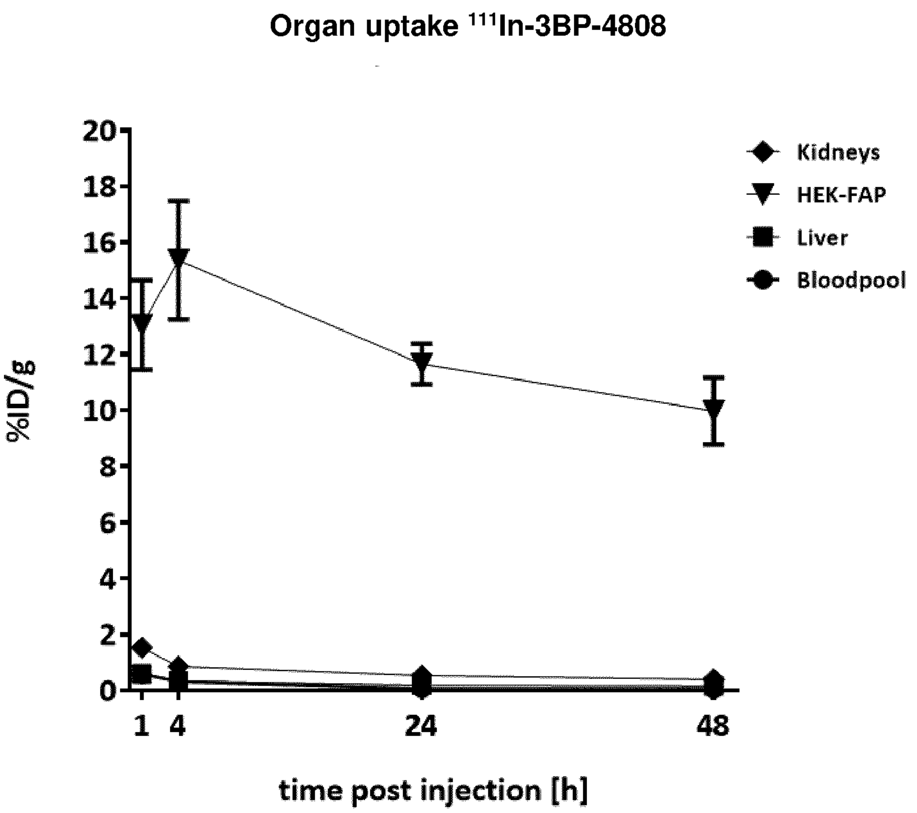
Organ uptake $^{111}$In-3BP-4808
%ID/g
20
18
16
14
12
10
8
6
4
2
0
1
4
24
48
time post injection [h]
Kidneys
HEK-FAP
Liver
Bloodpool

Fig 1(j)

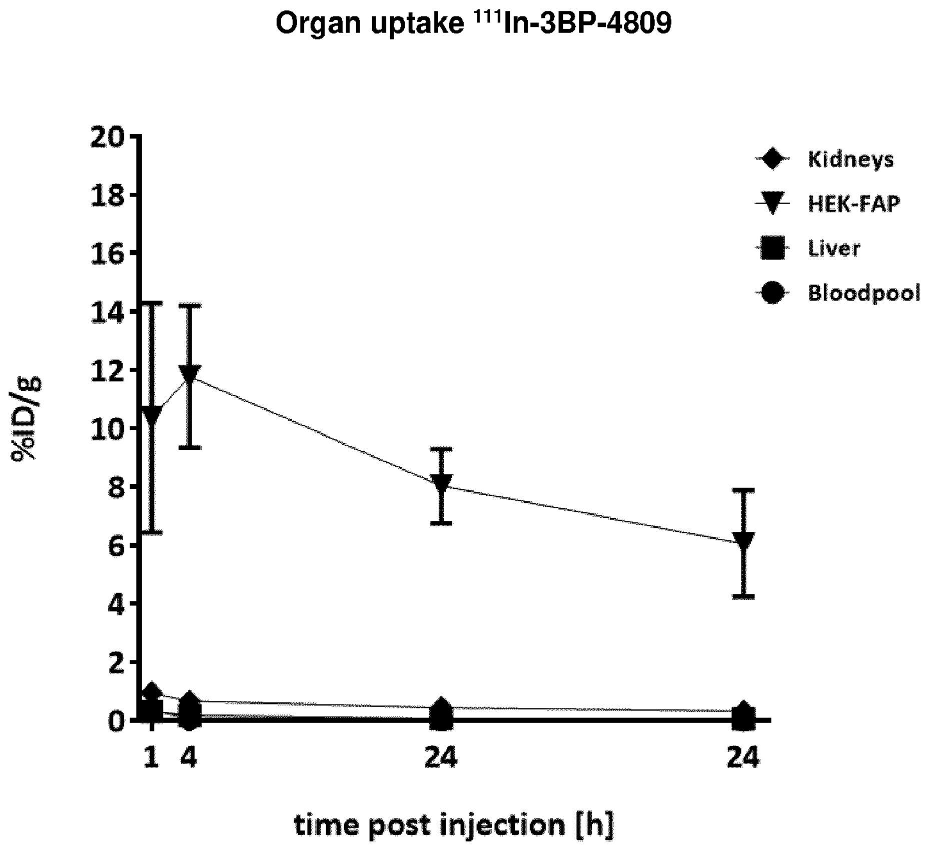
Organ uptake ¹¹¹In-3BP-4809
20
18
16
14
12
10
8
6
4
2
0
%ID/g
1
4
24
24
time post injection [h]
Kidneys
HEK-FAP
Liver
Bloodpool

Fig 1(k)

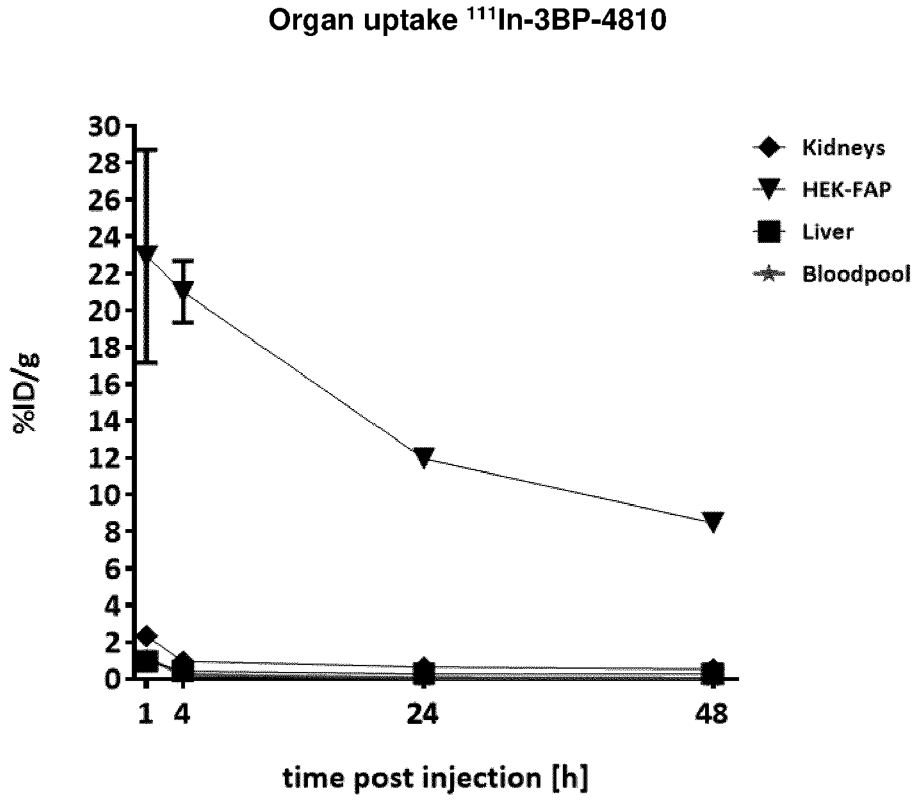
Organ uptake ¹¹¹In-3BP-4810
30
28
26
24
22
20
18
16
14
12
10
8
6
4
2
0
%ID/g
1
4
24
48
time post injection [h]
Kidneys
HEK-FAP
Liver
Bloodpool

Fig. 1(l)

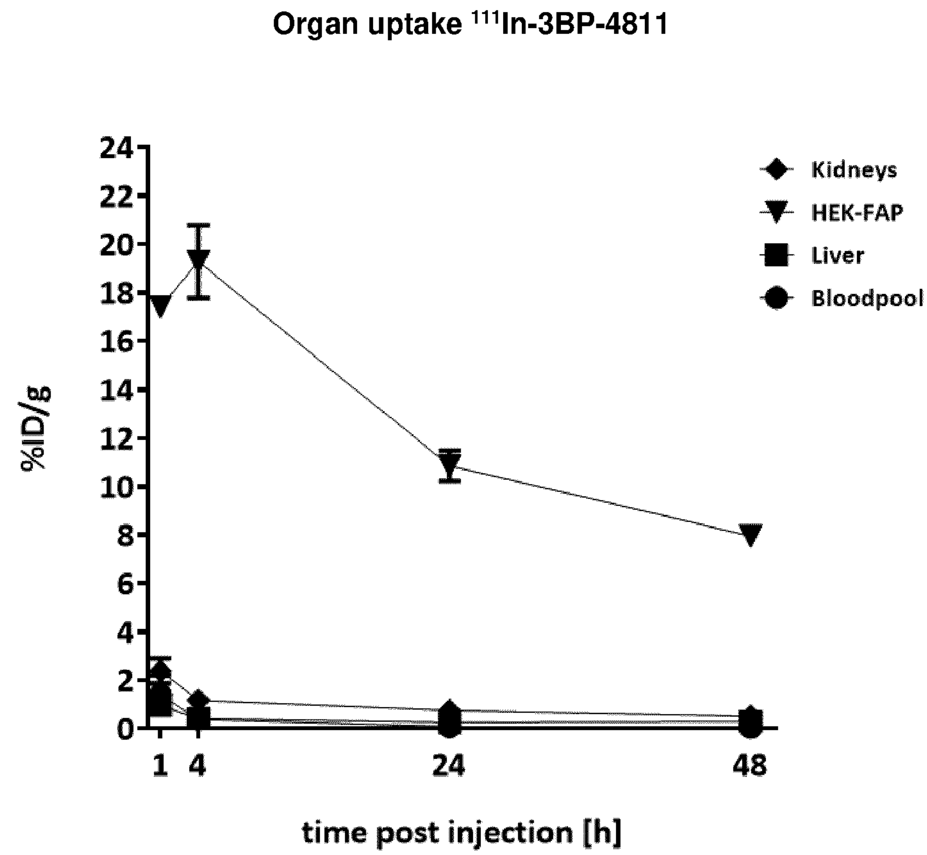
Organ uptake 111In-3BP-4811
%ID/g
24
22
20
18
16
14
12
10
8
6
4
2
0
1
4
24
48
time post injection [h]
Kidneys
HEK-FAP
Liver
Bloodpool

Fig. 1(m)

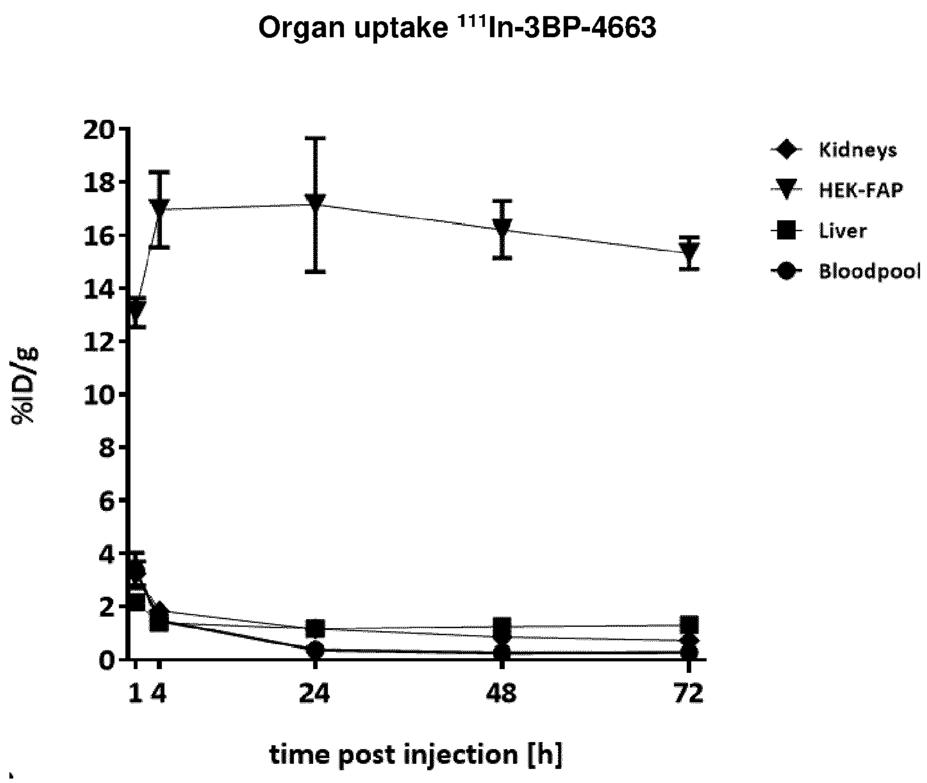
Organ uptake 111In-3BP-4663
%ID/g
20
18
16
14
12
10
8
6
4
2
0
1 4
24
48
72
time post injection [h]
Kidneys
HEK-FAP
Liver
Bloodpool

Fig. 1(n)

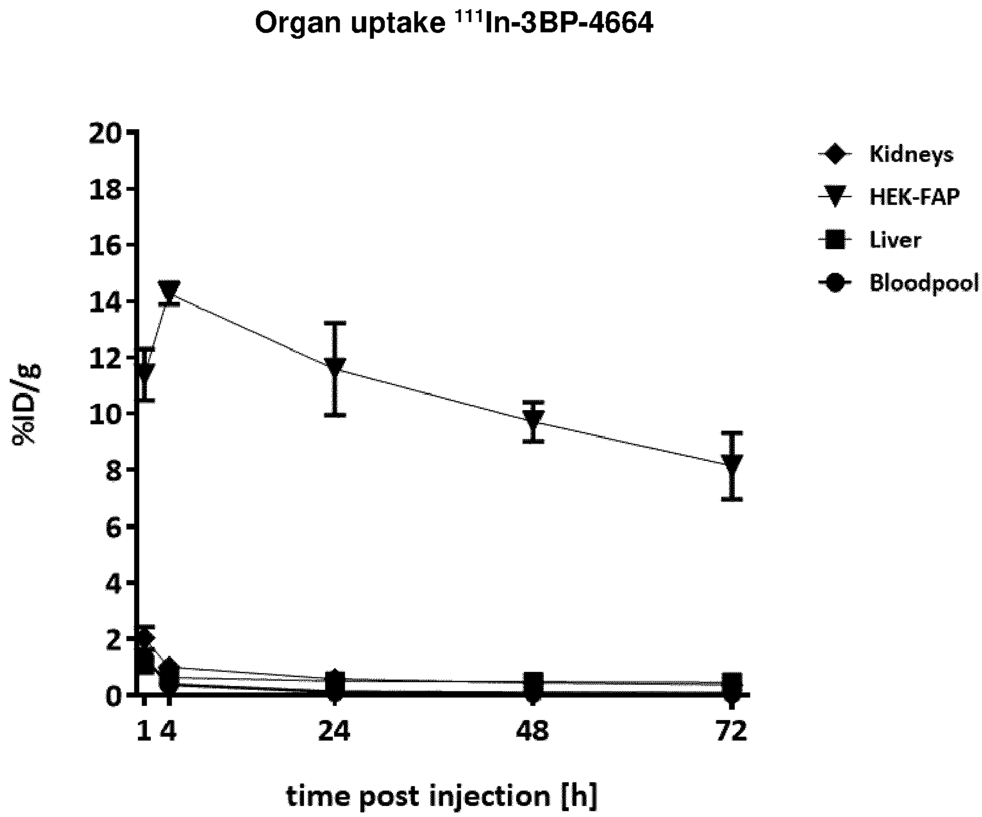
Organ uptake $^{111}$In-3BP-4664
%ID/g
20
18
16
14
12
10
8
6
4
2
0
1 4
24
48
72
time post injection [h]
Kidneys
HEK-FAP
Liver
Bloodpool

Fig. 1(o)

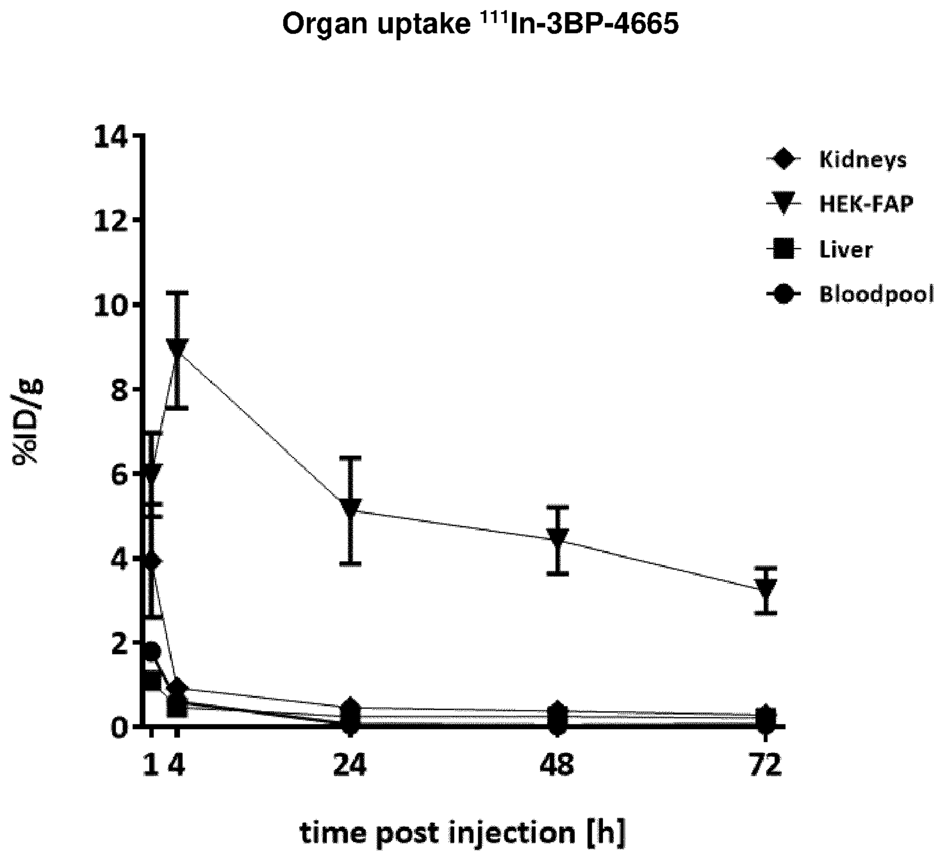
Organ uptake $^{111}$In-3BP-4665
%ID/g
14
12
10
8
6
4
2
0
1 4
24
48
72
time post injection [h]
Kidneys
HEK-FAP
Liver
Bloodpool

**Fig. 1(p)**

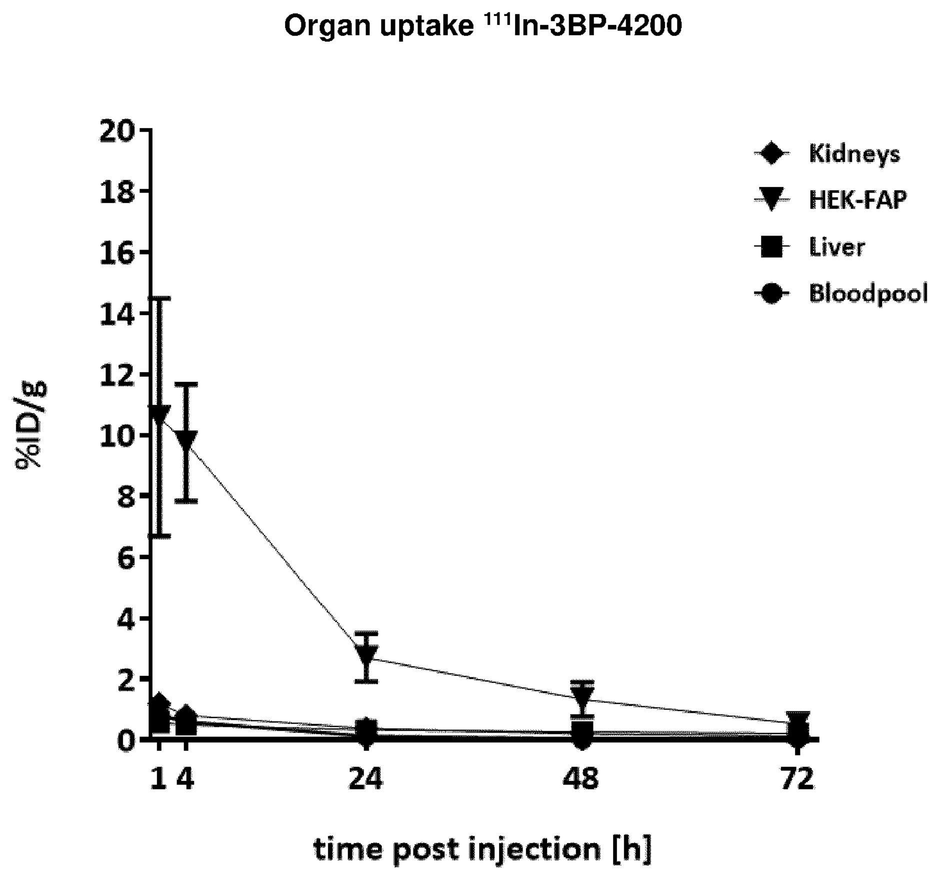
Organ uptake 111In-3BP-4200
%ID/g
20
18
16
14
12
10
8
6
4
2
0
1 4
24
48
72
time post injection [h]
Kidneys
HEK-FAP
Liver
Bloodpool

Fig 2(a)

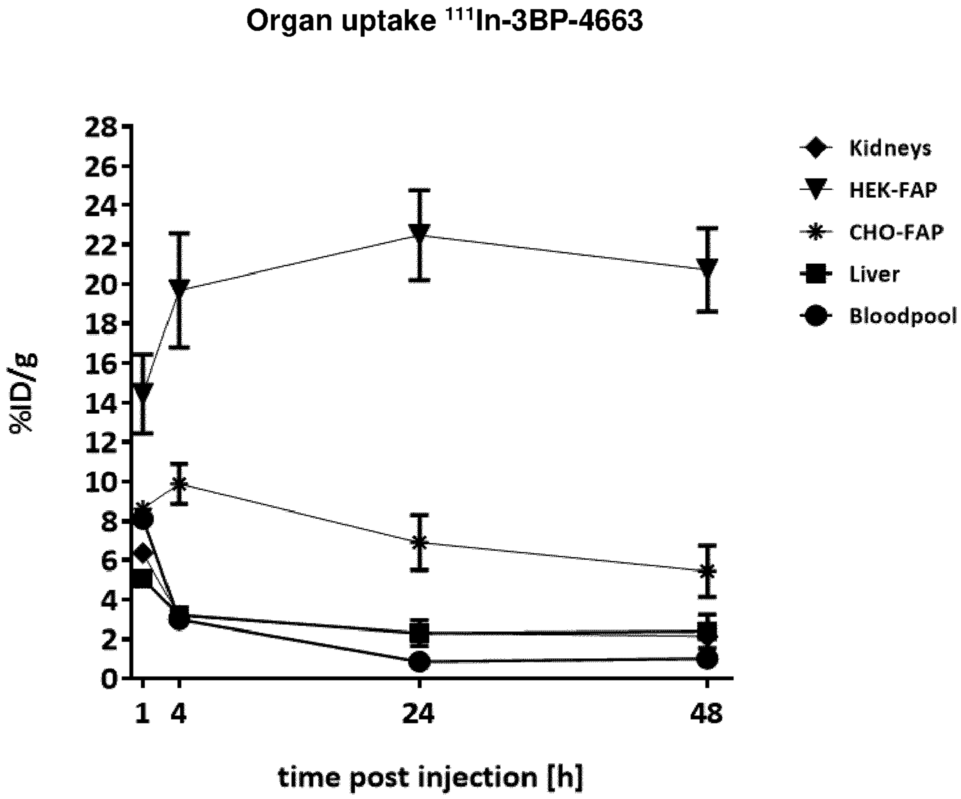
Organ uptake $^{111}$In-3BP-4663
%ID/g
28
26
24
22
20
18
16
14
12
10
8
6
4
2
0
1
4
24
48
time post injection [h]
Kidneys
HEK-FAP
CHO-FAP
Liver
Bloodpool

Fig. 2(b)

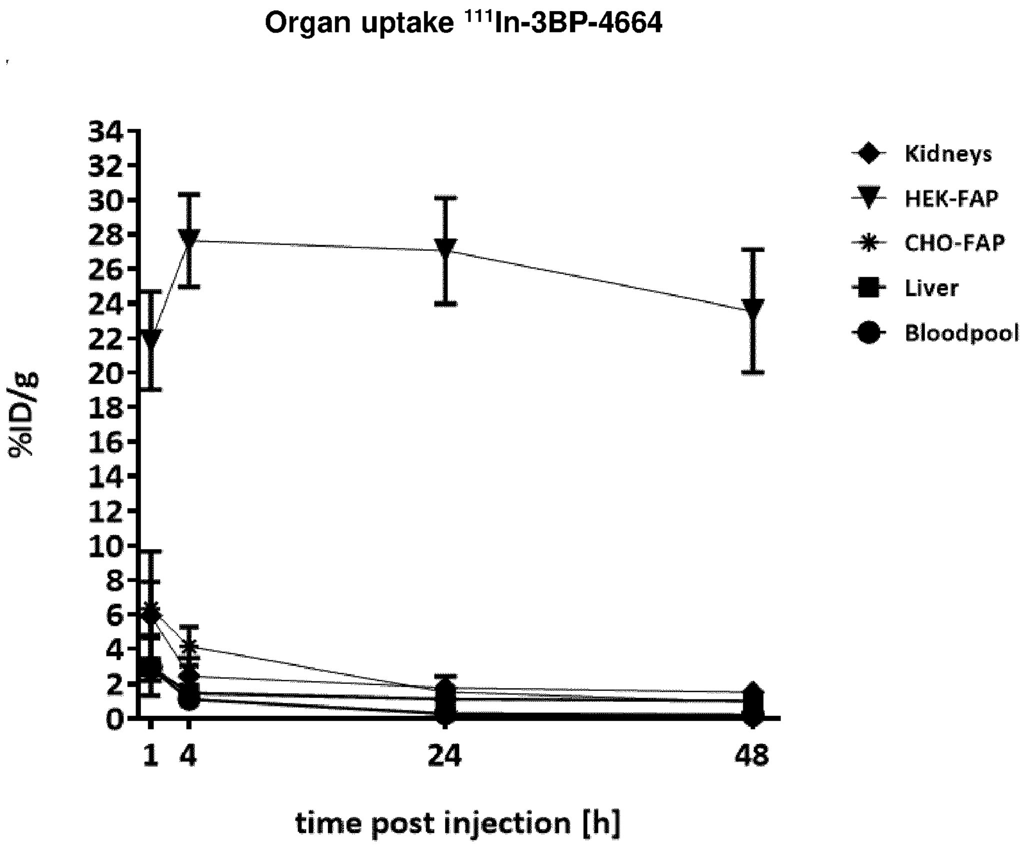
Organ uptake ¹¹¹In-3BP-4664
%ID/g
34
32
30
28
26
24
22
20
18
16
14
12
10
8
6
4
2
0
1
4
24
48
time post injection [h]
Kidneys
HEK-FAP
CHO-FAP
Liver
Bloodpool

Fig. 2(c)

Organ uptake $^{111}$In-3BP-4665

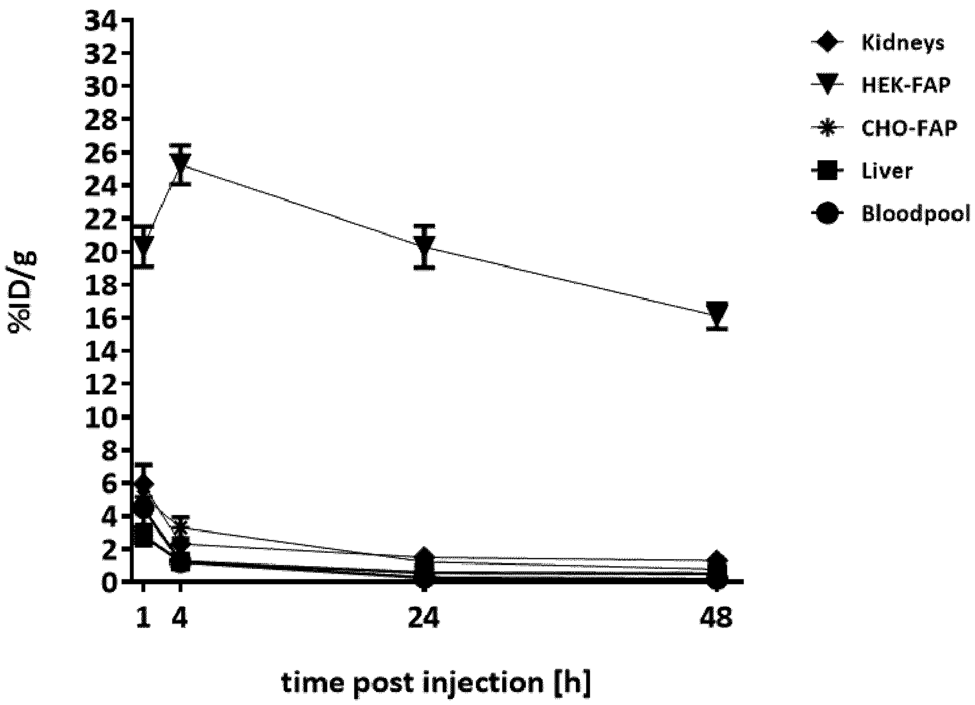

Fig. 2(d)

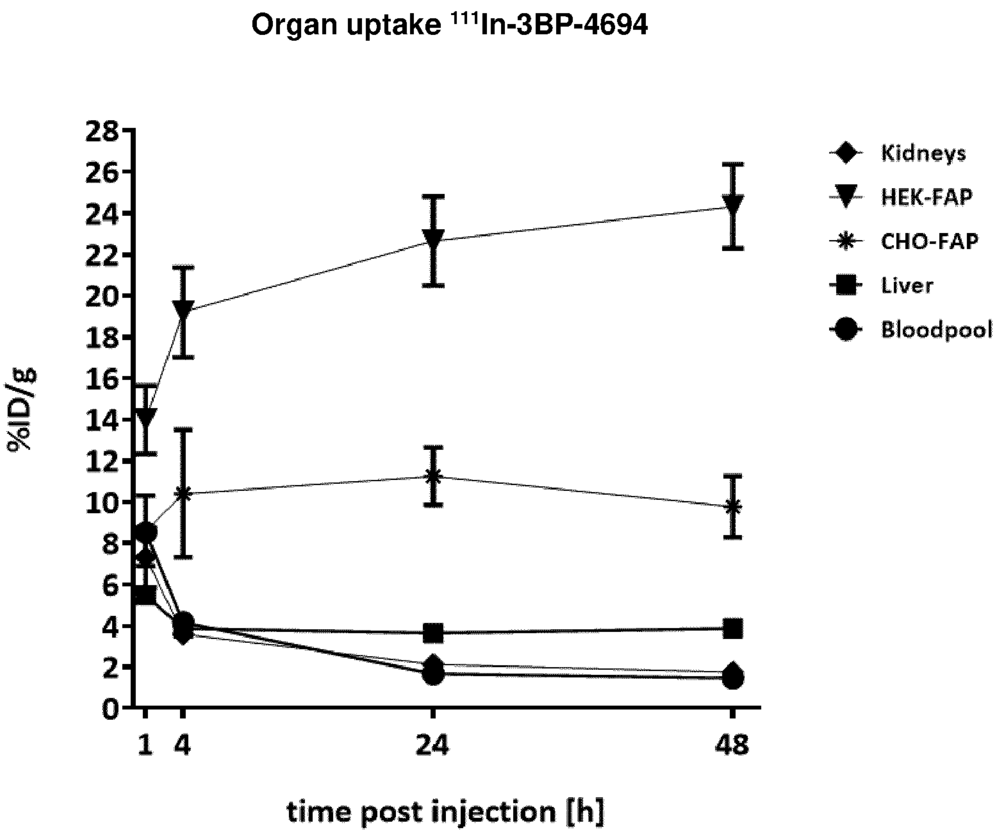
Organ uptake $^{111}$In-3BP-4694
%ID/g
28
26
24
22
20
18
16
14
12
10
8
6
4
2
0
1
4
24
48
time post injection [h]
Kidneys
HEK-FAP
CHO-FAP
Liver
Bloodpool

Fig. 2(e)

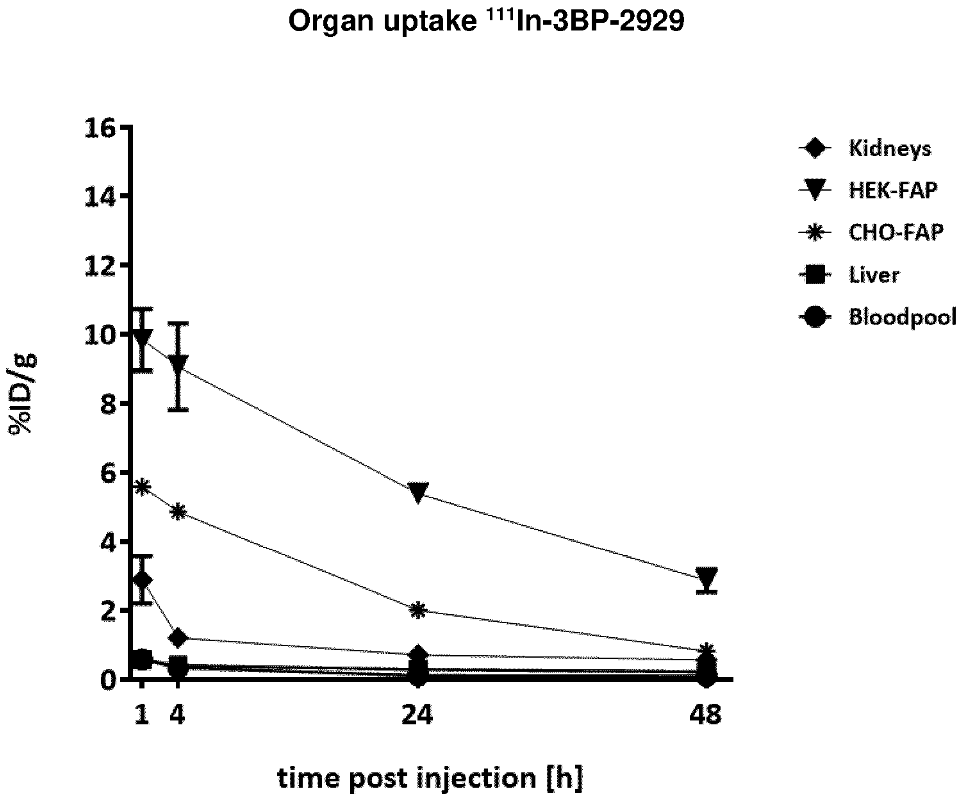
Organ uptake $^{111}$In-3BP-2929
%ID/g
16
14
12
10
8
6
4
2
0
1
4
24
48
time post injection [h]
Kidneys
HEK-FAP
CHO-FAP
Liver
Bloodpool

Fig. 2(f)

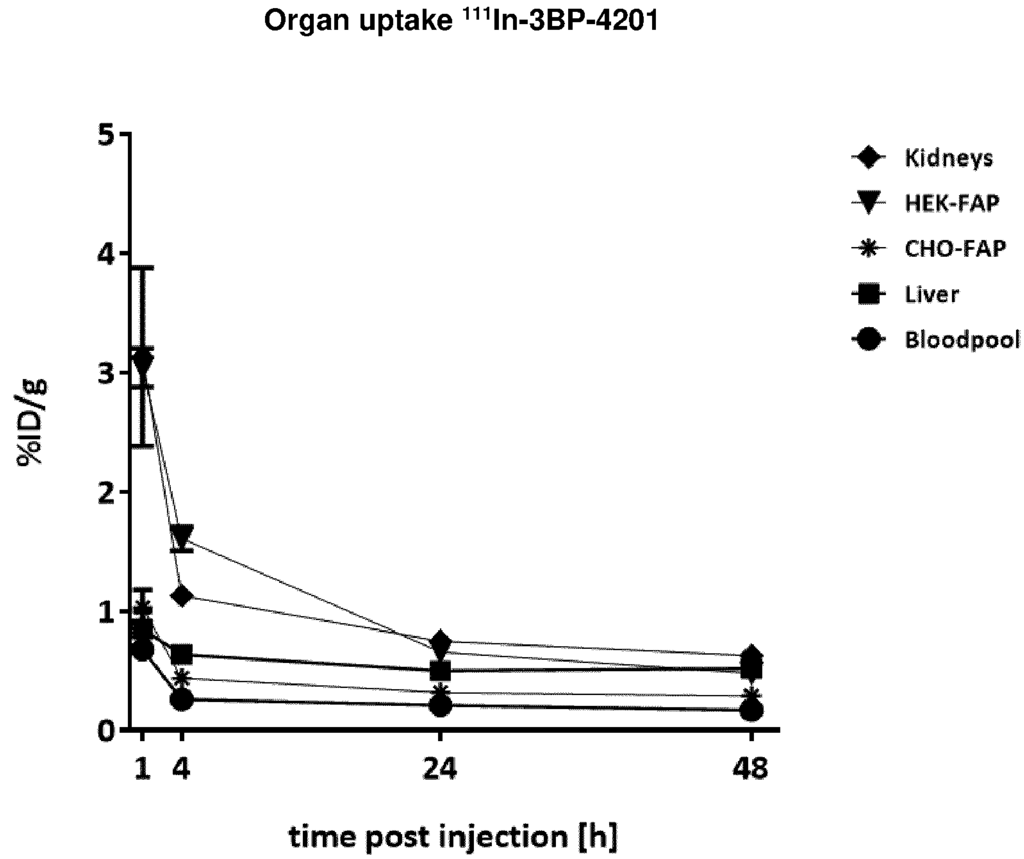
Organ uptake 111In-3BP-4201
5
4
3
2
1
0
%ID/g
1
4
24
48
time post injection [h]
Kidneys
HEK-FAP
CHO-FAP
Liver
Bloodpool

Fig. 3(a)

$^{111}$In-3BP-4809 SPECT/CT Images

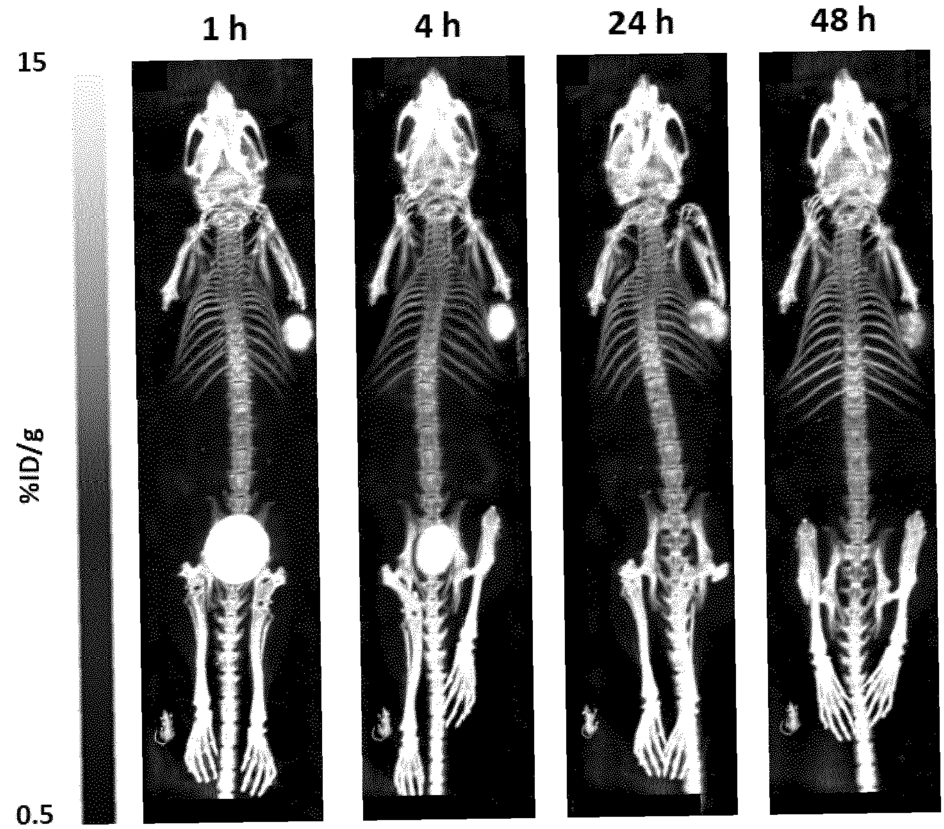

Fig. 3(b)

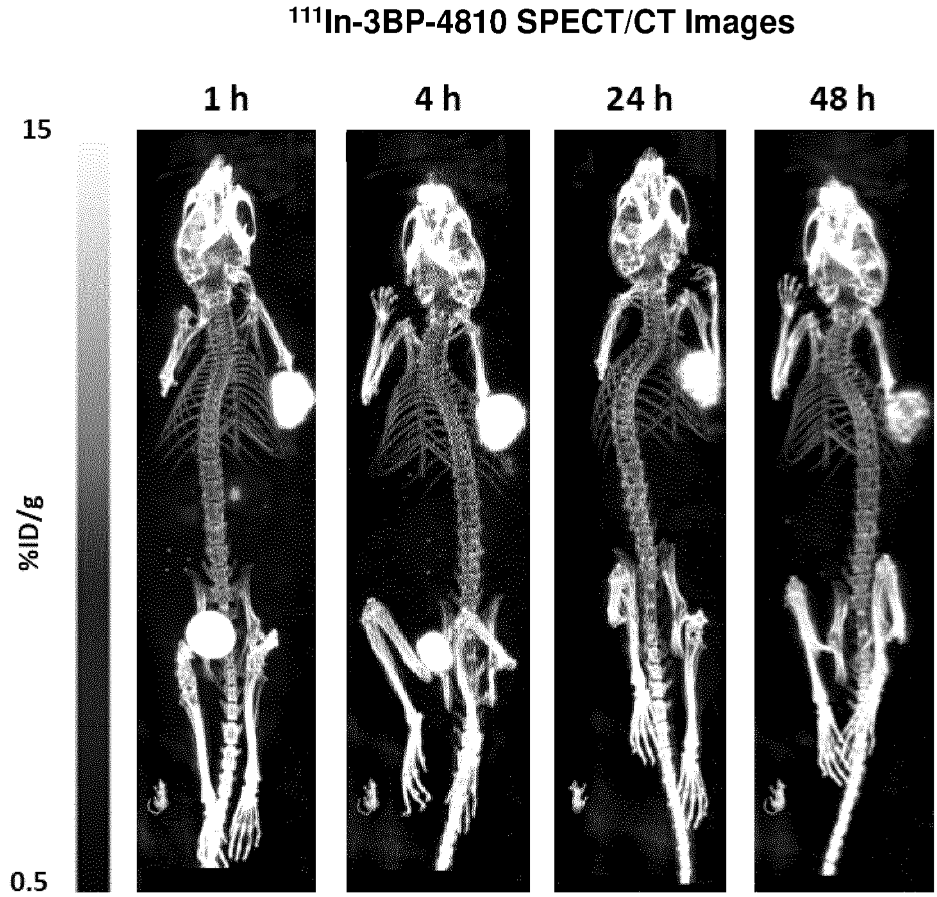

Fig. 3(c)

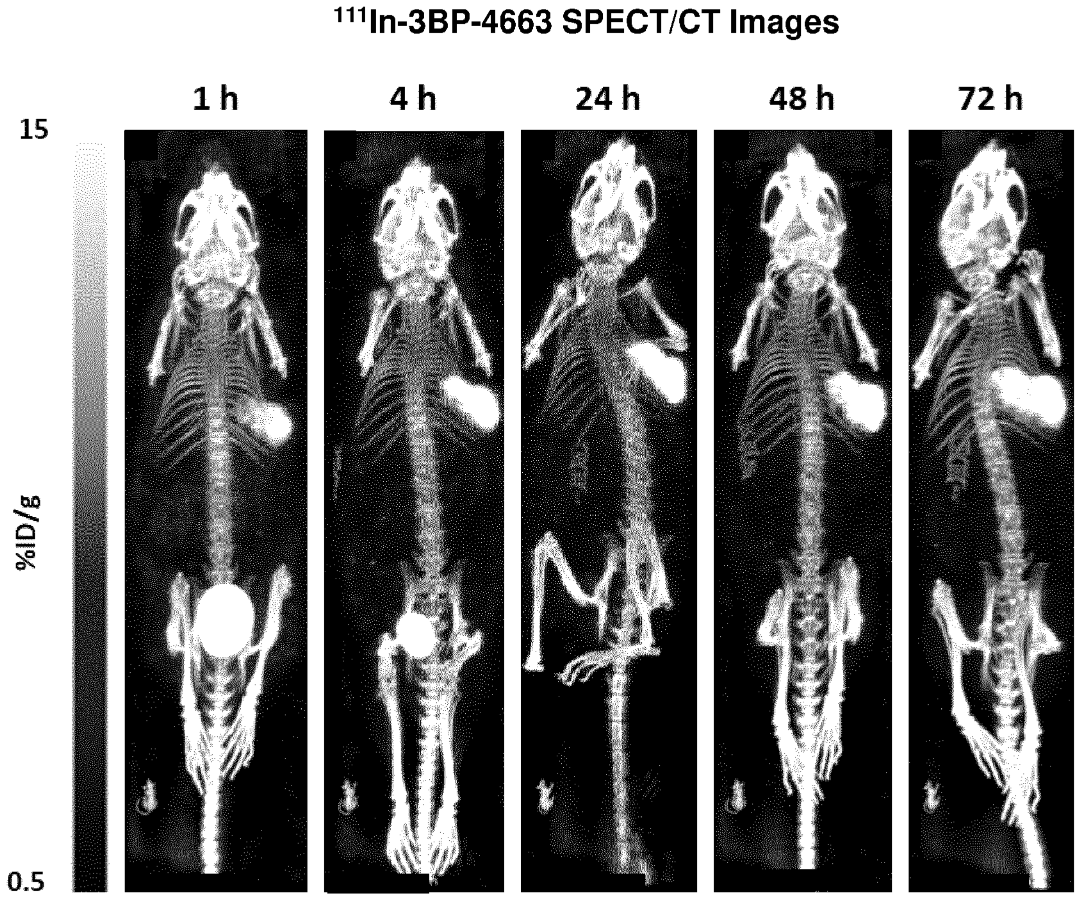

**Figure 3(d)**

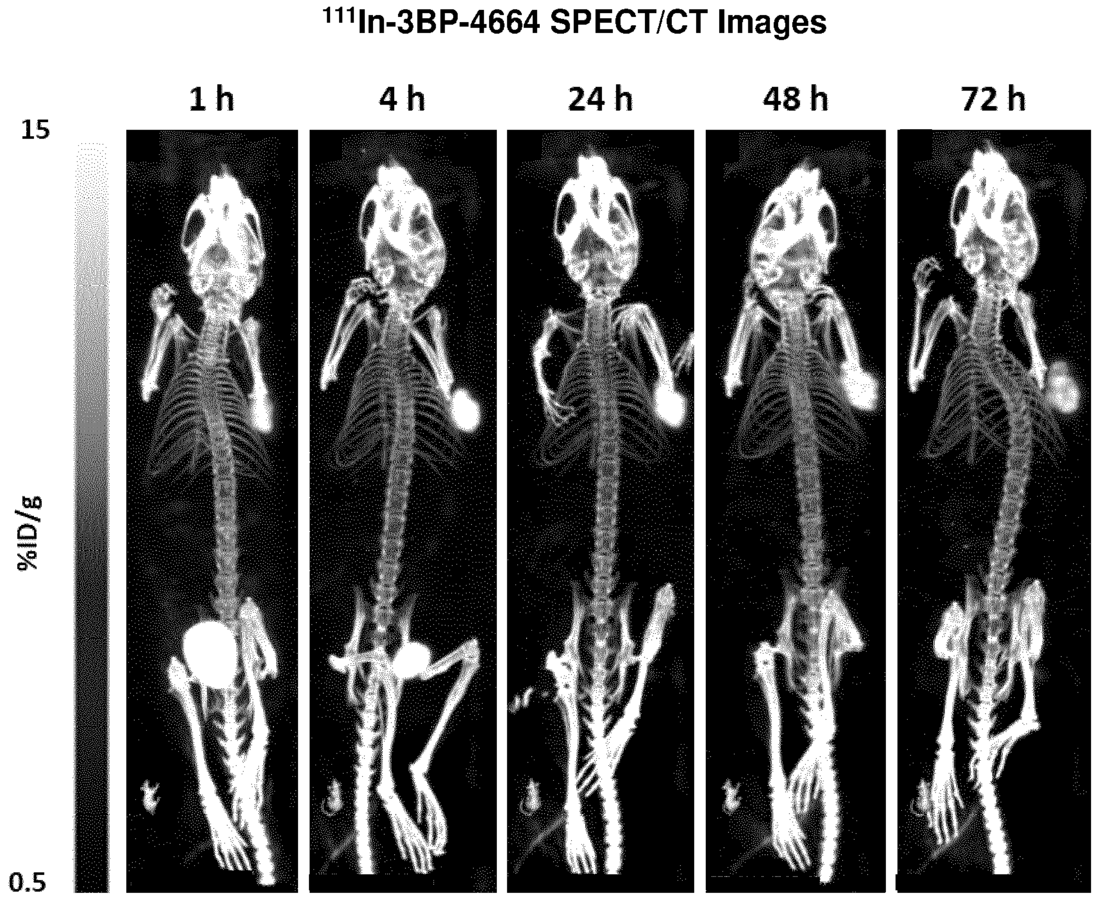

Figure 4(a)

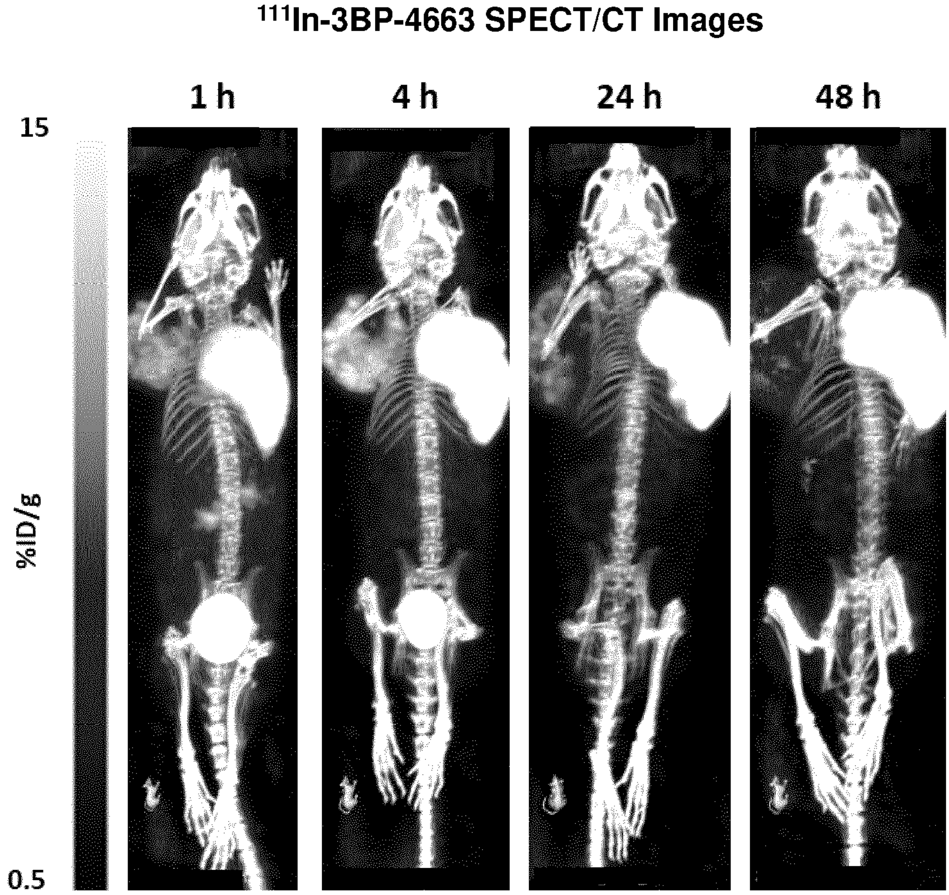

Fig. 4(b)

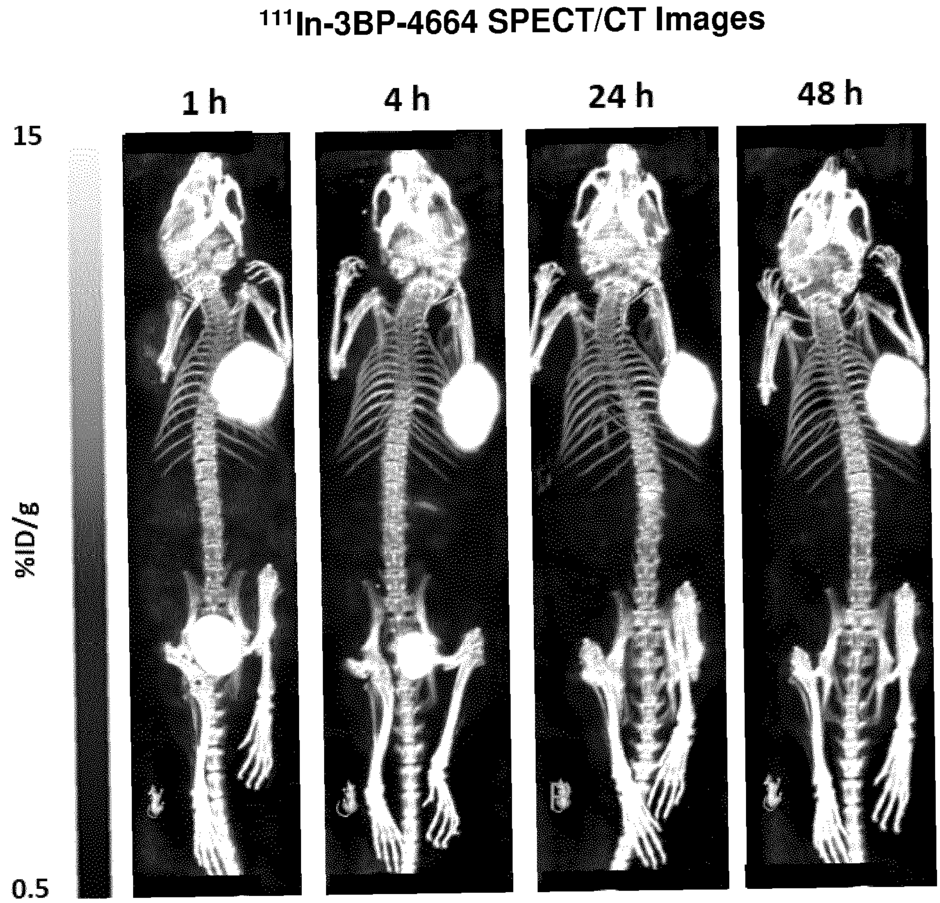

Fig. 4(c)

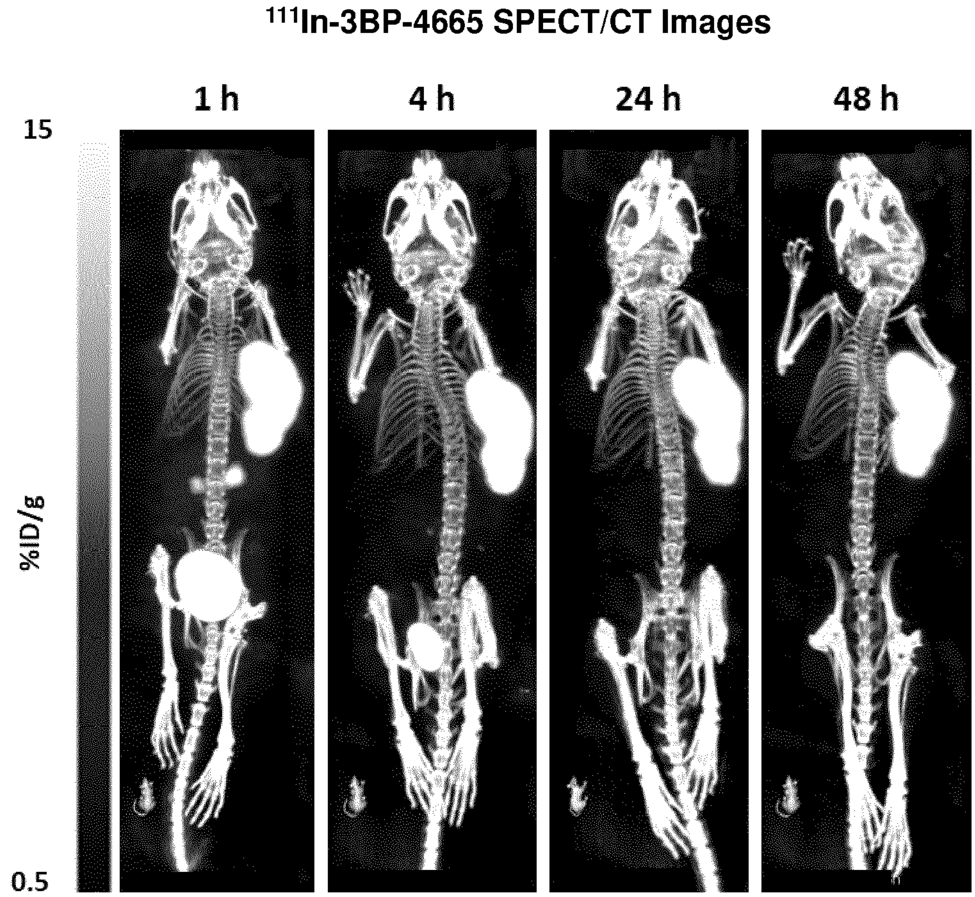

Fig. 4(d)

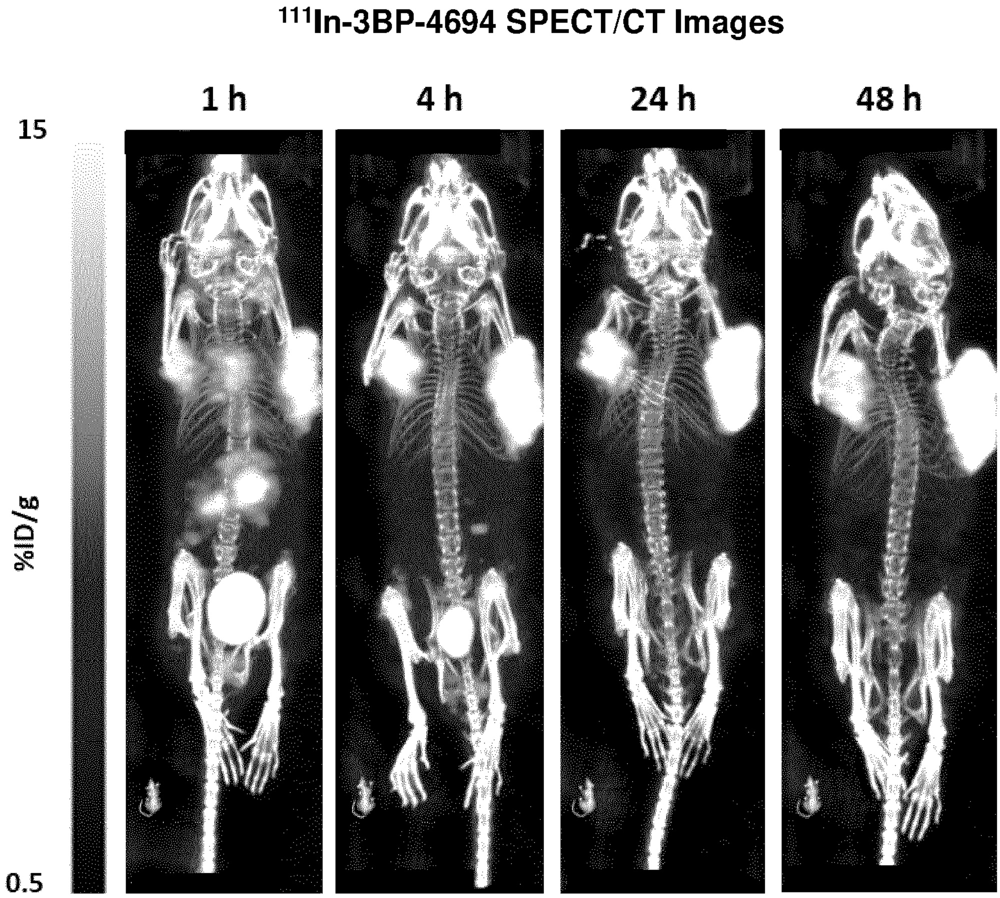

Fig 4(e)

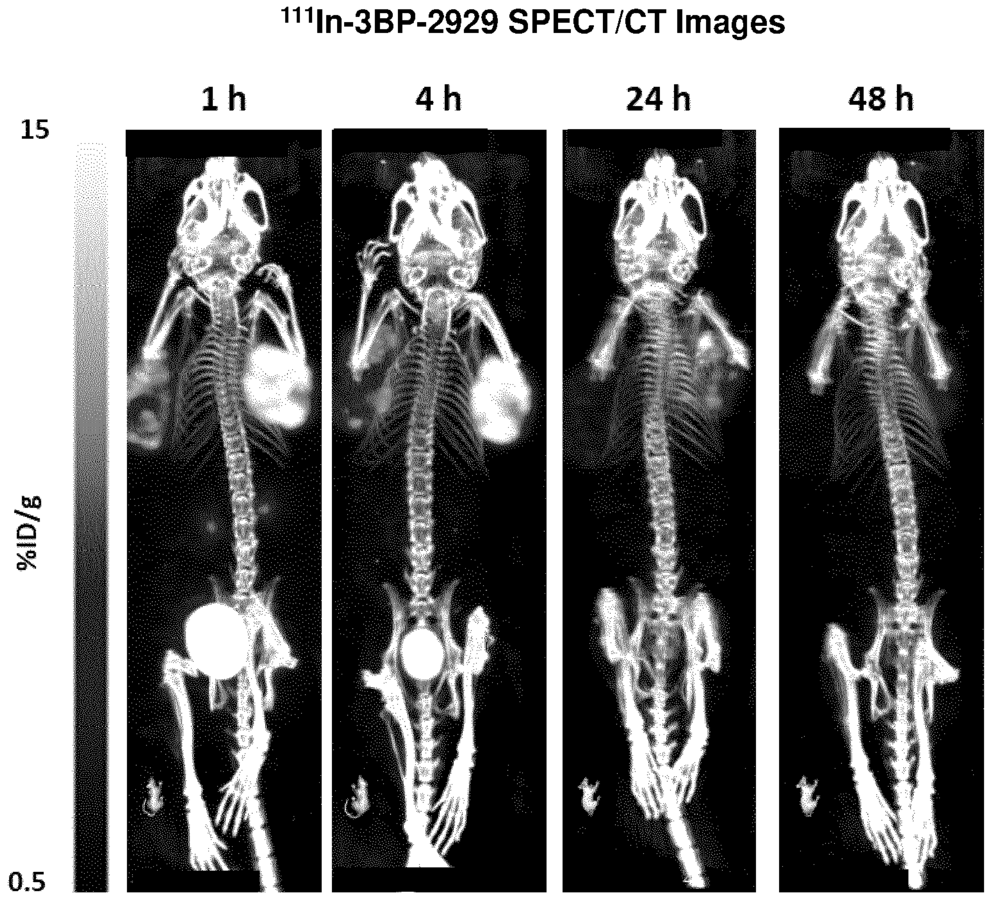

Fig. 4(f)

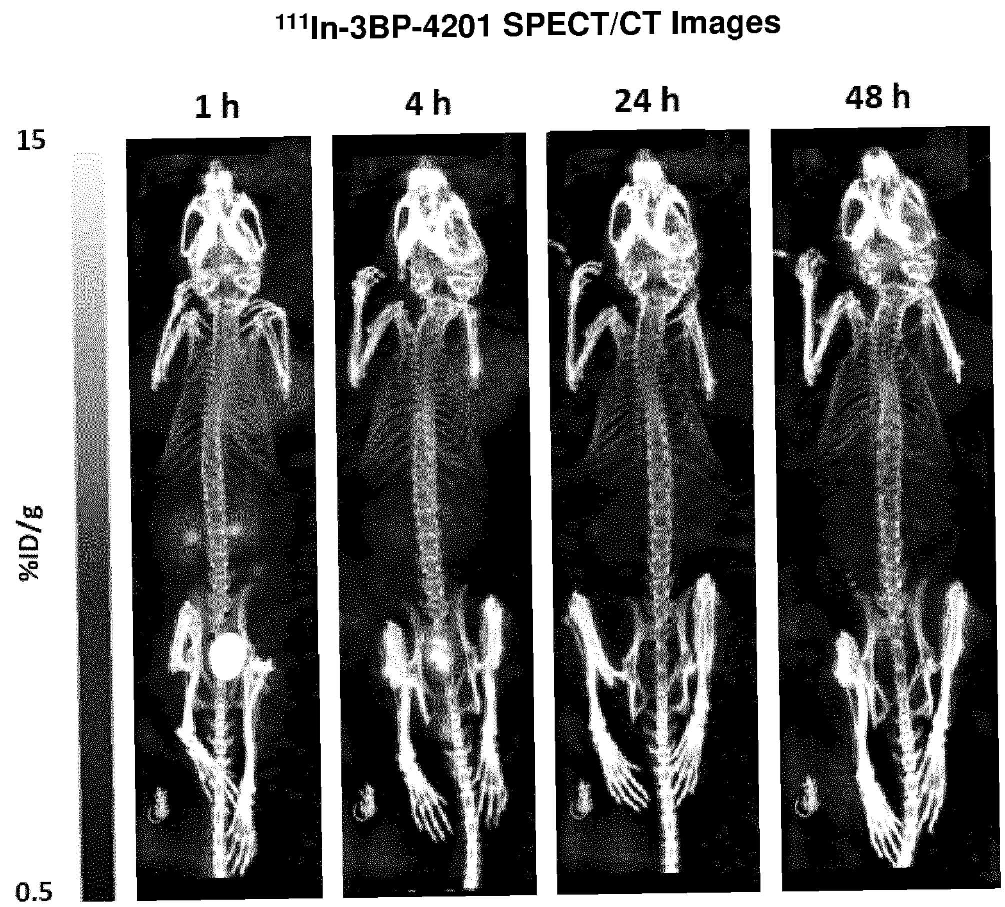

Europäisches Patentamt
European Patent Office
Office européen des brevets

# EUROPEAN SEARCH REPORT

Application Number
EP 21 18 7424

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2020/330624 A1 (YANG XING [US] ET AL) 22 October 2020 (2020-10-22)<br>* claims 1-26 *<br>* examples 1, 2 * | 1-13, 15-18<br>14 | INV.<br>A61K51/04 |
| X<br><br>A | ALTMANN ANNETTE ET AL: "The Latest Developments in Imaging of Fibroblast Activation Protein", THE JOURNAL OF NUCLEAR MEDICINE, vol. 62, no. 2, 19 January 2021 (2021-01-19), pages 160-167, XP055876749, US ISSN: 0161-5505, DOI: 10.2967/jnumed.120.244806<br>* whole document and in particular Figure 2 * | 1-13, 15-18<br><br>14 | |
| X<br><br>A | WO 2020/083853 A1 (UNIV MAINZ JOHANNES GUTENBERG [DE]; HELMHOLTZ ZENTRUM DRESDEN [DE]) 30 April 2020 (2020-04-30)<br>* figures 14, 51 *<br>* claims 1-13 * | 1-13, 15-18<br><br>14 | TECHNICAL FIELDS SEARCHED (IPC)<br>A61K |

1 The present search report has been drawn up for all claims

| Place of search | Date of completion of the search | Examiner |
|---|---|---|
| The Hague | 11 January 2022 | Birikaki, Lemonia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 18 7424

This annex lists the patent family members relating to the patent documents cited in the above-mentioned European search report.
The members are as contained in the European Patent Office EDP file on
The European Patent Office is in no way liable for these particulars which are merely given for the purpose of information.

11-01-2022

| Patent document cited in search report | Publication date | Patent family member(s) | Publication date |
|---|---|---|---|
| US 2020330624 A1 | 22-10-2020 | AU 2018355222 A1 | 30-04-2020 |
| | | BR 112020008011 A2 | 27-10-2020 |
| | | CA 3088138 A1 | 02-05-2019 |
| | | CN 111511408 A | 07-08-2020 |
| | | EA 202090776 A1 | 27-07-2020 |
| | | EP 3700580 A2 | 02-09-2020 |
| | | JP 2021500373 A | 07-01-2021 |
| | | KR 20200063230 A | 04-06-2020 |
| | | US 2020330624 A1 | 22-10-2020 |
| | | WO 2019083990 A2 | 02-05-2019 |
| WO 2020083853 A1 | 30-04-2020 | AU 2019365377 A1 | 29-04-2021 |
| | | BR 112021007659 A2 | 27-07-2021 |
| | | CA 3116272 A1 | 30-04-2020 |
| | | CN 113164630 A | 23-07-2021 |
| | | DE 102018126558 A1 | 30-04-2020 |
| | | EP 3870243 A1 | 01-09-2021 |
| | | KR 20210083289 A | 06-07-2021 |
| | | US 2021369877 A1 | 02-12-2021 |
| | | WO 2020083853 A1 | 30-04-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5367080 A
- US 5364613 A
- US 5021556 A
- US 5075099 A
- US 5886142 A
- US 5720934 A
- US 4885363 A, Tweedle


### Non-patent literature cited in the description


- **E. L. ELIEL ; S. H. WILEN ; L. N. MANDER.** Stereochemistry of Organic Compounds. Wiley-Interscience, 1994
- **BANERJEE et al.** *Dalton Trans,* 2005, vol. 24, 3886
- **PRICE et al.** *Chem Soc Rev,* 2014, vol. 43, 260
- **WADAS et al.** *Chem Rev,* 2010, vol. 110, 2858
- **DOULIAS et al.** *Free Radic BiolMed,* 2003, vol. 35, 719
- **PFISTER et al.** *EJNMMI Res,* 2015, vol. 5, 74
- **CUSNIR et al.** *Int J Mol Sci,* 2017, vol. 18
- **DEMOIN et al.** *Nucl Med Biol,* 2016, vol. 43, 802
- **THIELE et al.** *Angew Chem Int Ed Engl,* 2017, vol. 56, 14712
- **BOROS et al.** *Mol Pharm,* 2014, vol. 11, 617
- **ALLOTT et al.** *Chem Commun (Camb),* 2017, vol. 53, 8529
- **TORNESELLO et al.** *Molecules,* 2017, vol. 22, 1282
- **MCAULEY et al.** *Canadian Journal of Chemistry,* 1989, vol. 67, 1657
- **MA et al.** *Dalton Trans,* 2015, vol. 44, 4884
- **SCHWARTZ et al.** *Bioconjug Chem,* 1991, vol. 2, 333
- **BABICH et al.** *J Nucl Med,* 1993, vol. 34, 1964
- **BABICH et al.** *Nucl Med Biol,* 1995, vol. 22, 25
- **LI et al.** *Nucl Med Biol,* 2001, vol. 28, 145
- **BRECHBIEL et al.** *Bioconjug Chem,* 1991, vol. 2, 187
- **EISENWIENER et al.** *Bioconjug Chem,* 2002, vol. 13, 530
- **NOCK et al.** *J Nucl Med,* 2005, vol. 46, 1727
- **DE KRUIJFF et al.** *Pharmaceuticals,* 2015, vol. 8, 321-336
- **SMITH et al.** *Advances in Applied Mathematics,* 1981, vol. 2, 482
- **NEEDLEMAN et al.** *JMol Biol,* 1970, vol. 48, 443
- **PEARSON et al.** *Proc Natl Acad Sci USA,* 1988, vol. 85, 2444
- **ALTSCHUL et al.** *J Mol Biol,* 1990, vol. 215, 403
- **ALTSCHUL et al.** *Nucleic Acids Res,* 1997, vol. 25, 3389
- **MCGINNIS et al.** *Nucleic Acids Res,* 2004, vol. 32, W20
- **PURE et al.** *Oncogene,* 2018, vol. 37, 4343
- **BUSEK et al.** Front Biosci. 2018, vol. 23, 1933
- **YUAN et al.** *Expert Opin Ther Pat,* 2017, vol. 27, 363
- **PILIE et al.** *Nat Rev Clin Oncol,* 2019, vol. 16, 81
- **ZHANG et al.** *Chin J Cancer,* 2012, vol. 31, 359
- **KHALIL et al.** *Nat Rev Clin Oncol,* 2016, vol. 13, 394
- **HOLLINGSWORTH et al.** *NPJ Vaccines,* 2019, vol. 4, 7
- **WEI et al.** *Cancer Discov,* 2018, vol. 8, 1069
- **GOEL et al.** *Trends Cell Biol,* 2018, vol. 28, 911
- **KELLNER et al.** *Transfus Med Hemother,* 2017, vol. 44, 327
- **YU et al.** *J Cancer Res Clin Oncol,* 2019, vol. 145, 941
- *J. Med. Chem.,* 2017, vol. 60, 8385
- **BAINBRIDGE et al.** *Sci Rep,* 2017, vol. 7, 12524